# EUROPEAN PATENT SPECIFICATION

(11) **EP 3 708 682 B1**
(45) Date of publication and mention of the grant of the patent: **23.11.2022**
(21) Application number: 20161635.6
(22) Date of filing: 02.02.2009
(51) Int. Cl.: C12Q 1/6886, C12Q 1/6883, C12Q 1/6806

(54) **USE OF MICROVESICLES IN DIAGNOSIS AND PROGNOSIS OF MEDICAL DISEASES AND CONDITIONS**
VERWENDUNG VON MIKROVESIKELN BEI DER DIAGNOSE UND PROGNOSE VON MEDIZINISCHEN KRANKHEITEN UND LEIDEN
UTILISATION DE MICROVÉSICULES DANS LE DIAGNOSTIC ET LE PRONOSTIC DE MALADIES ET D'ÉTAT DE SANTÉ

(30) Priority: 01.02.2008 US 2553608 P; 26.09.2008 US 10029308 P
(43) Date of publication of application: 16.09.2020
(62) Divisional of application: 17153084.3
(73) Proprietor: The General Hospital Corporation, Boston, MA 02114 (US)
(72) Inventor: SKOG, Johan Karl Olov, Lincoln, MA 01773 (US); BREAKEFIELD, Xandra, Newton, MA Massachusetts 02459 (US); BROWN, Dennis, Natick, MA Massachusetts 01760 (US); MIRANDA, Kevin, 29018 Malaga (ES); RUSSO, Leileata, Rosebud, Victoria 3939 (AU)
(74) Representative: Kehoe, Laura Ellen

(56) References cited:
- WO-A-2005/121369
- WO-A1-2007/127848
- WO-A1-2009/015357
- WO-A1-2009/036236
- WO-A1-2009/147519
- WO-A2-03/076603
- WO-A2-2006/007529
- CN-A- 101 085 349
- US-A1- 2007 298 118
- VALADI H ET AL: "Exosome-mediated transfer of mRNAs and microRNAs is a novel mechanism of genetic exchange between cells", NATURE CELL BIOLOGY, MACMILLAN MAGAZINES LTD, GB, vol. 9, no. 6, 6 June 2007 (2007-06-06), pages 654-659, XP003016816, ISSN: 1465-7392, DOI: 10.1038/NCB1596
- MONIKA BAJ-KRZYWORZEKA ET AL: "Tumour-derived microvesicles carry several surface determinants and mRNA of tumour cells and transfer some of these determinants to monocytes", CANCER IMMUNOLOGY, IMMUNOTHERAPY, vol. 55, no. 7, 9 November 2005 (2005-11-09), pages 808-818, XP055176484, ISSN: 0340-7004, DOI: 10.1007/s00262-005-0075-9
- SKOG JOHAN ET AL: "Glioblastoma microvesicles transport RNA and proteins that promote tumour growth and provide diagnostic biomarkers.", NATURE CELL BIOLOGY DEC 2008, vol. 10, no. 12, December 2008 (2008-12), pages 1470-1476, XP002526160, ISSN: 1476-4679
- SMALHEISER NEIL R: "Exosomal transfer of proteins and RNAs at synapses in the nervous system", BIOLOGY DIRECT, BIOMED CENTRAL, vol. 2, no. 1, 30 November 2007 (2007-11-30), page 35, XP021039070, ISSN: 1745-6150
- TATISCHEFF I ET AL: "Dictyostelium discoideum cells shed vesicles with associated DNA and vital stain Hoechst 33342", CMLS CELLULAR AND MOLECULAR LIFE SCIENCES, BIRKHAUSER VERLAG, HEIDELBERG, DE, vol. 54, no. 5, 1 May 1998 (1998-05-01), pages 476-487, XP002261692, ISSN: 1420-682X, DOI: 10.1007/S000180050176
- Justine M Carr ET AL: "985;45:5944-5951. Cancer Res Circulating Membrane Vesicles in Leukemic Blood CirculatingMembraneVesicles in LeukemicBlood1", , 1 January 1985 (1985-01-01), pages 5944-5951, XP055114941, Retrieved from the Internet: URL:http://cancerres.aacrjournals.org/cont ent/45/11_Part_2/5944.full.pdf [retrieved on 2014-04-23]
- VALADI H ET AL: "Exosome-mediated transfer of mRNAs and microRNAs is a novel mechanism of genetic exchange between cells", NATURE CELL BIOLOGY, MACMILLAN MAGAZINES LTD, GB, vol. 9, no. 6, 1 June 2007 (2007-06-01), pages 654-659, XP002546815, ISSN: 1465-7392, DOI: 10.1038/NCB1596 [retrieved on 2007-05-07]

## Description

### CROSS REFERENCE TO RELATED APPLICATIONS

This application claims priority to US provisional applications 61/025,536, filed February 1, 2008 and 61/100,293, filed September 26, 2008.

### GOVERNMENTAL SUPPORT

This invention was made with Government support under grants NCI CA86355 and NCI CA69246 awarded by the National Cancer Institute. The Government has certain rights in the invention.

### FIELD OF THE INVENTION

The present disclosure relates to the fields of medical diagnosis, patient monitoring, treatment efficacy evaluation, nucleic acid and protein delivery, and blood transfusion.

### BACKGROUND OF THE INVENTION

Glioblastomas are highly malignant brain tumors with a poor prognosis despite intensive research and clinical efforts (Louis et al., 2007). The invasive nature of this tumor makes complete surgical resection impossible and the median survival time is only about 15 months (Stupp et al., 2005). Glioblastoma cells as well as many other tumor cells have a remarkable ability to mold their stromal environment to their own advantage. Tumor cells directly alter surrounding normal cells to facilitate tumor cell growth, invasion, chemo-resistance, immune-evasion and metastasis (Mazzocca et al., 2005; Muerkoster et al., 2004; Singer et al., 2007). The tumor cells also hijack the normal vasculature and stimulate rapid formation of new blood vessels to supply the tumor with nutrition (Carmeliet and Jain, 2000). Although the immune system can initially suppress tumor growth, it is often progressively blunted by tumor activation of immunosuppressive pathways (Gabrilovich, 2007).

Small microvesicles shed by cells are known as exosomes (Thery et al., 2002). Exosomes are reported as having a diameter of approximately 30-100 nm and are shed from many different cell types under both normal and pathological conditions (Thery et al., 2002). These microvesicles were first described as a mechanism to discard transferrin-receptors from the cell surface of maturing reticulocytes (Pan and Johnstone, 1983). Exosomes are formed through inward budding of endosomal membranes giving rise to intracellular multivesicular bodies (MVB) that later fuse with the plasma membrane, releasing the exosomes to the exterior (Thery et al., 2002). However, there is now evidence for a more direct release of exosomes. Certain cells, such as Jurkat T-cells, are said to shed exosomes directly by outward budding of the plasma membrane (Booth et al., 2006). All membrane vesicles shed by cells are referred to herein collectively as microvesicles.

Microvesicles in *Drosophila melanogaster,* so called argosomes, are said to contain morphogens such as Wingless protein and to move over large distances through the imaginal disc epithelium in developing *Drosophila melanogaster* embryos (Greco et al., 2001). Microvesicles found in semen, known as prostasomes, are stated to have a wide range of functions including the promotion of sperm motility, the stabilization of the acrosome reaction, the facilitation of immunosuppression and the inhibition of angiogenesis (Delves et al., 2007). On the other hand, prostasomes released by malignant prostate cells are said to promote angiogenesis. Microvesicles are said to transfer proteins (Mack et al., 2000) and recent studies state that microvesicles isolated from different cell lines can also contain messenger RNA (mRNA) and microRNA (miRNA) and can transfer mRNA to other cell types (Baj-Krzyworzeka et al., 2006; Valadi et al., 2007).

Microvesicles derived from B-cells and dendritic cells are stated to have potent immuno-stimulatory and antitumor effects *in vivo* and have been used as antitumor vaccines (Chaput et al., 2005). Dendritic cell-derived microvesicles are stated to contain the co-stimulatory proteins necessary for T-cell activation, whereas most tumor cell-derived microvesicles do not (Wieckowski and Whiteside, 2006). Microvesicles isolated from tumor cells may act to suppress the immune response and accelerate tumor growth (Clayton et al., 2007; Liu et al., 2006a). Breast cancer microvesicles may stimulate angiogenesis, and platelet-derived microvesicles may promote tumor progression and metastasis of lung cancer cells (Janowska-Wieczorek et al., 2005; Millimaggi et al., 2007).

Cancers arise through accumulation of genetic alterations that promote unrestricted cell growth. It has been stated that each tumor harbors, on average, around 50-80 mutations that are absent in non-tumor cells (Jones et al., 2008; Parsons et al., 2008; Wood et al., 2007). Current techniques to detect these mutation profiles include the analysis of biopsy samples and the non-invasive analysis of mutant tumor DNA fragments circulating in bodily fluids such as blood (Diehl et al., 2008). The former method is invasive, complicated and possibly harmful to subjects. The latter method inherently lacks sensitivity due to the extremely low copy number of mutant cancer DNA in bodily fluid (Gormally et al., 2007). Therefore, one challenge facing cancer diagnosis is to develop a diagnostic method that can detect tumor cells at different stages non-invasively, yet with high sensitivity and specificity. It has also been stated that gene expression profiles (encoding mRNA or microRNA) can distinguish cancerous and non-cancerous tissue (Jones et al., 2008; Parsons et al., 2008; Schetter et al., 2008). WO2005121369 relates to RNA-containing vesicles and methods thereof. However, current diagnostic techniques to detect gene expression profiles require intrusive biopsy of tissues. Some biopsy procedures cause high risk and are potentially harmful. Moreover, in a biopsy procedure, tissue samples are taken from a limited area and may give false positives or false negatives, especially in tumors which are heterogeneous and/or dispersed within normal tissue. Therefore, a non-intrusive and sensitive diagnostic method for detecting biomarkers would be highly desirable.

### SUMMARY OF THE INVENTION

Present invention is defined by the appended claims. In general, the disclosure relates to a novel method for detecting in a subject the presence or absence of a variety of biomarkers contained in microvesicles, thereby aiding the diagnosis, monitoring and evaluation of diseases, other medical conditions, and treatment efficacy associated with microvesicle biomarkers. The method of the invention is defined in present claim 1.

Disclosed, but not defining the claimed invention, are methods for aiding in the diagnosis or monitoring of a disease or other medical condition in a subject, comprising the steps of: a) isolating a microvesicle fraction from a biological sample from the subject; and b) detecting the presence or absence of a biomarker within the microvesicle fraction, wherein the biomarker is associated with the disease or other medical condition. The methods may further comprise the step or steps of comparing the result of the detection step to a control (e.g., comparing the amount of one or more biomarkers detected in the sample to one or more control levels), wherein the subject is diagnosed as having the disease or other medical condition (e.g., cancer) if there is a measurable difference in the result of the detection step as compared to a control.

Disclosed, but not defining the claimed invention, is a method for aiding in the evaluation of treatment efficacy in a subject, comprising the steps of: a) isolating a microvesicle fraction from a biological sample from the subject; and b) detecting the presence or absence of a biomarker within the microvesicle fraction, wherein the biomarker is associated with the treatment efficacy for a disease or other medical condition. The method may further comprise the step of providing a series of a biological samples over a period of time from the subject. Additionally, the method may further comprise the step or steps of determining any measurable change in the results of the detection step (e.g., the amount of one or more detected biomarkers) in each of the biological samples from the series to thereby evaluate treatment efficacy for the disease or other medical condition.

In certain preferred embodiments, the biological sample from the subject is a sample of bodily fluid. Particularly preferred body fluids are blood and urine. According to the claimed invention, the sample is serum or urine.

In certain preferred embodiments of the foregoing aspects of the invention, the methods further comprise the isolation of a selective microvesicle fraction derived from cells of a specific type (e.g., cancer or tumor cells). Additionally, the selective microvesicle fraction may consist essentially of urinary microvesicles.

In certain embodiments of the foregoing aspects of the invention, the biomarker associated with a disease or other medical condition is i) a species of nucleic acid; ii) a level of expression of one or more nucleic acids; iii) a nucleic acid variant; or iv) a combination of any of the foregoing. Preferred embodiments of such biomarkers include messenger RNA, microRNA, DNA, single stranded DNA, complementary DNA and noncoding DNA.

In certain embodiments of the foregoing aspects of the invention, the disease or other medical condition is a neoplastic disease or condition (e.g., glioblastoma, pancreatic cancer, breast cancer, melanoma and colorectal cancer), a metabolic disease or condition (e.g., diabetes, inflammation, perinatal conditions or a disease or condition associated with iron metabolism), a post transplantation condition, or a fetal condition.

Disclosed, but not part of the claimed invention, is a method for aiding in the diagnosis or monitoring of a disease or other medical condition in a subject, comprising the steps of a) obtaining a biological sample from the subject; and b) determining the concentration of microvesicles within the biological sample.

Disclosed, but not part of the claimed invention, is a method for delivering a nucleic acid or protein to a target cell in an individual comprising the steps of administering microvesicles which contain the nucleic acid or protein, or one or more cells that produce such microvesicles, to the individual such that the microvesicles enter the target cell of the individual. In a preferred embodiment of this aspect of the invention, microvesicles are delivered to brain cells.

Disclosed, but not part of the claimed invention, is a method for performing bodily fluid transfusion (e.g., blood, serum or plasma), comprising the steps of obtaining a fraction of donor body fluid free of all or substantially all microvesicles, or free of all or substantially all microvesicles from a particular cell type (e.g., tumor cells), and introducing the microvesicle-free fraction to a patient. Disclosed, but not part of the claimed invention, is a composition of matter comprising a sample of body fluid (e.g., blood, serum or plasma) free of all or substantially all microvesicles, or free of all or substantially all microvesicles from a particular cell type.

Disclosed, but not part of the claimed invention, is a method for performing bodily fluid transfusion (e.g., blood, serum or plasma), comprising the steps of obtaining a microvesicle-enriched fraction of donor body fluid and introducing the microvesicle-enriched fraction to a patient. In a preferred embodiment, the fraction is enriched with microvesicles derived from a particular cell type. Disclosed, but not part of the claimed invention, is a composition of matter comprising a sample of bodily fluid (e.g., blood, serum or plasma) enriched with microvesicles.

Disclosed, but not part of the claimed invention, is a method for aiding in the identification of new biomarkers associated with a disease or other medical condition, comprising the steps of obtaining a biological sample from a subject; isolating a microvesicle fraction from the sample; and detecting within the microvesicle fraction species of nucleic acid; their respective expression levels or concentrations; nucleic acid variants; or combinations thereof.

Various aspects and embodiments of the invention will now be described in detail. Further, unless otherwise required by context, singular terms shall include pluralities and plural terms shall include the singular.

All patents, patent applications, and publications identified are describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representations as to the contents of these documents are based on the information available to the applicants and do not constitute any admission as to the correctness of the dates or contents of these documents.

### BRIEF DESCRIPTION OF THE DRAWINGS

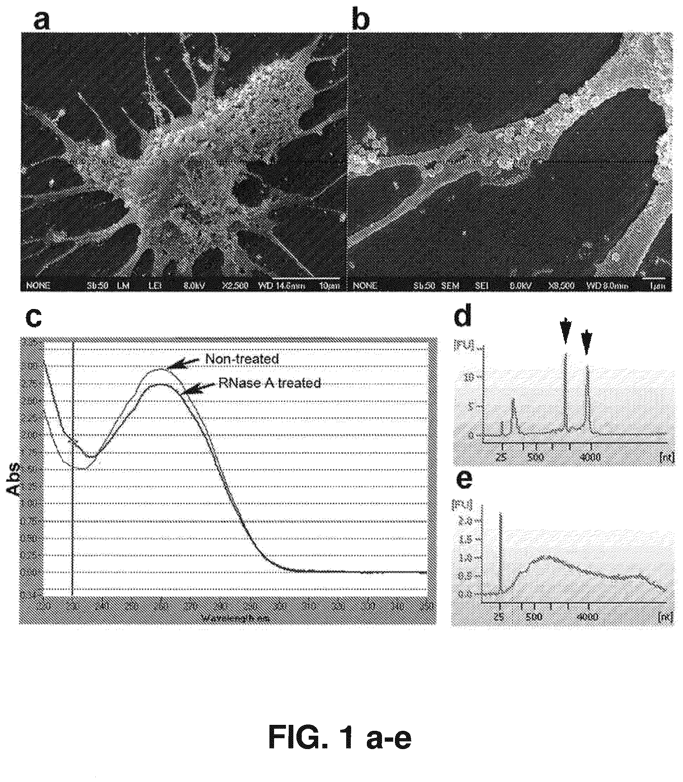

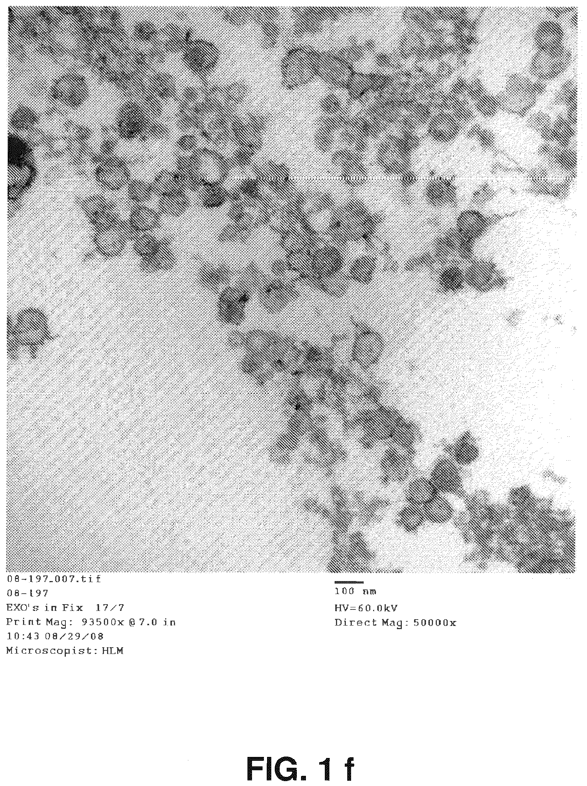

FIGURE 1. Glioblastoma cells produce microvesicles containing RNA.
   (a) Scanning electron microscopy image of a primary glioblastoma cell (bar = 10 µm). (b) Higher magnification showing the microvesicles on the cell surface. The vesicles vary in size with diameters between around 50 nm and around 500 nm (bar = 1 µm). (c) Graph showing the amount of total RNA extracted from microvesicles that were either treated or not treated with RNase A. The amounts are indicated as the absorption (Abs, y-axis) of 260nm wavelength (x-axis). The experiments were repeated 5 times and a representative graph is shown. (d) Bioanalyzer data showing the size distribution of total RNA extracted from primary glioblastoma cells and (e) Bioanalyzer data showing the size distribution of total RNA extracted from microvesicles isolated from primary glioblastoma cells. The 25 nt peak represents an internal standard. The two prominent peaks in (d) (arrows) represent 18S (left arrow) and 28S (right arrow) ribosomal RNA. The ribosomal peaks are absent from RNA extracted from microvesicles (e). (f) Transmission electron microscopy image of microvesicles secreted by primary glioblastoma cells (bar = 100 nm).

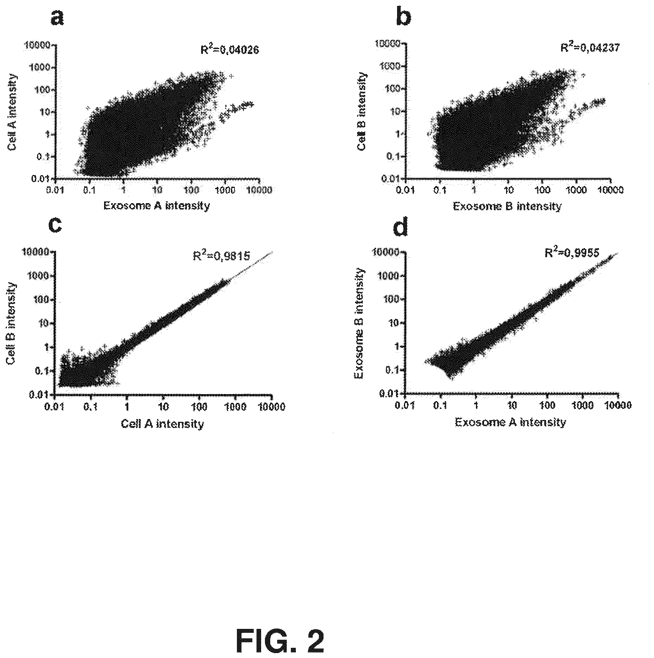

FIGURE 2. Analysis of microvesicle RNA. FIGS. 2 (a) and 2 (b) are scatter plots of mRNA levels in microvesicles and mRNA levels in donor glioblastoma cells from two different experiments. Linear regressions show that mRNA levels in donor cells versus microvesicles were not well correlated. FIGS. 2 (c) and 2 (d) are mRNA levels in two different donor cells or two different microvesicle preparations. In contrast to FIGS. 2 (a) and 2 (b), linear regressions show that mRNA levels between donor cells FIG 2 (c) or between microvesicles FIG 2 (d) were closely correlated.

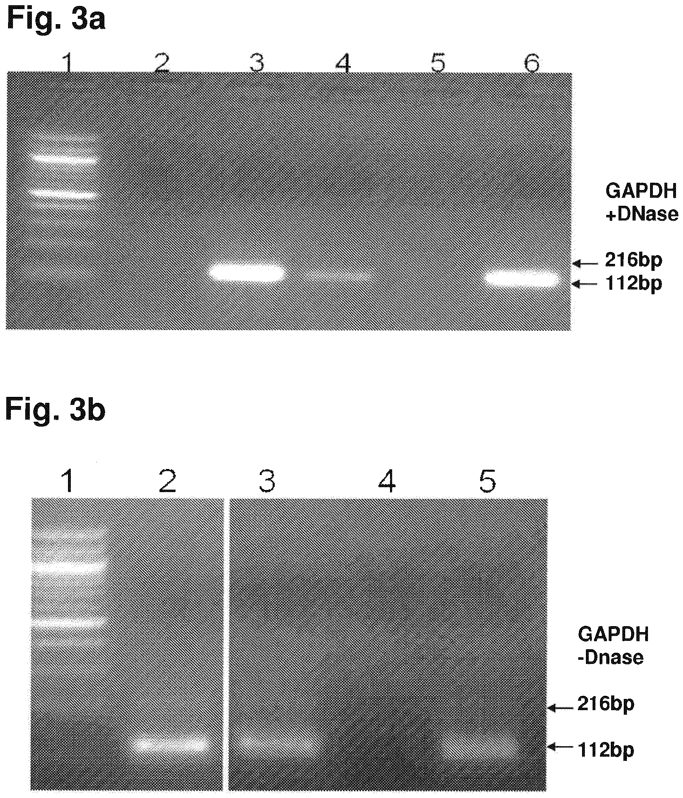

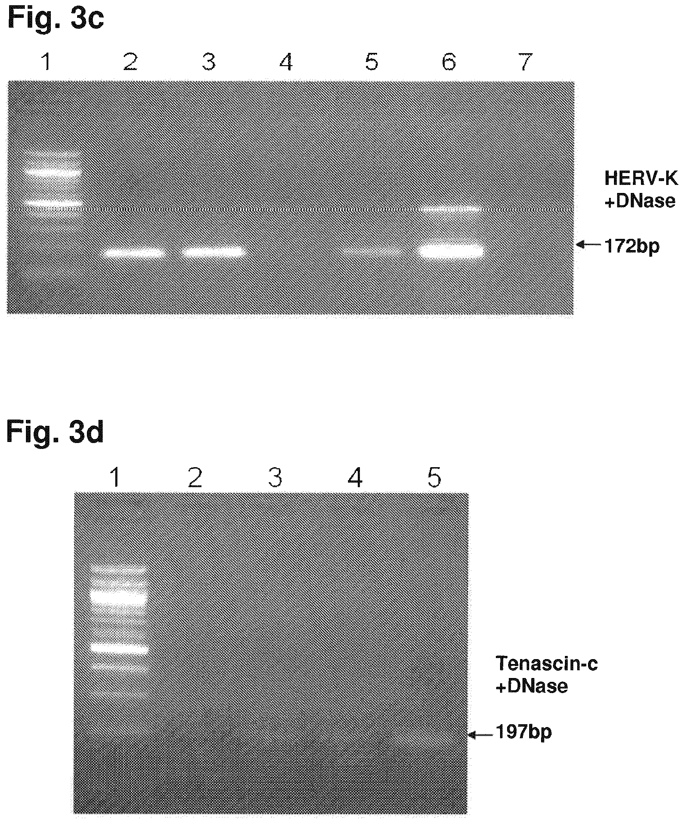

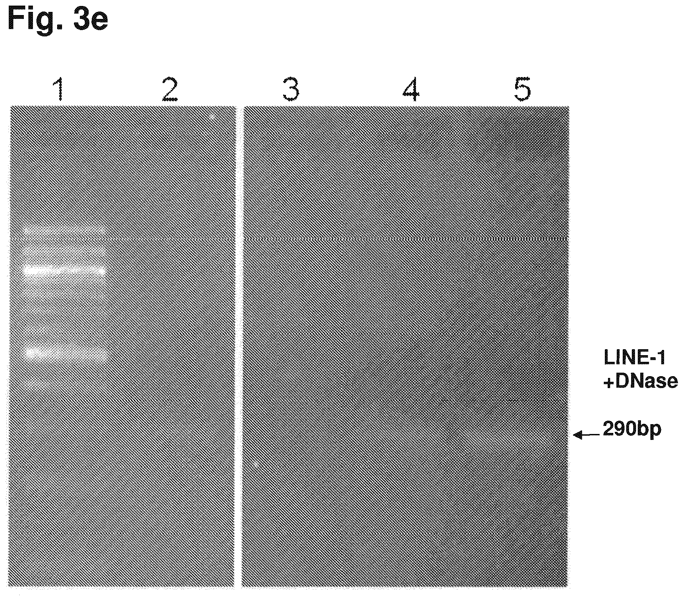

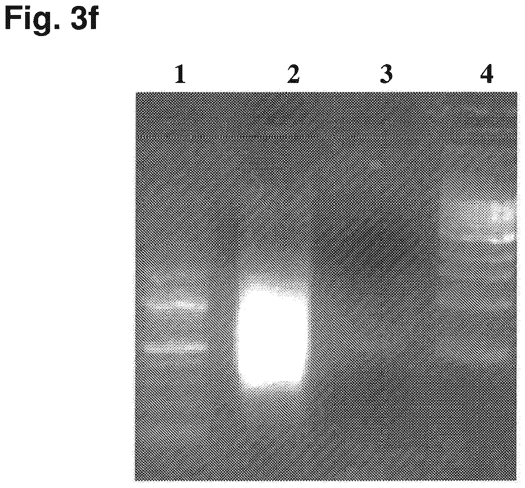

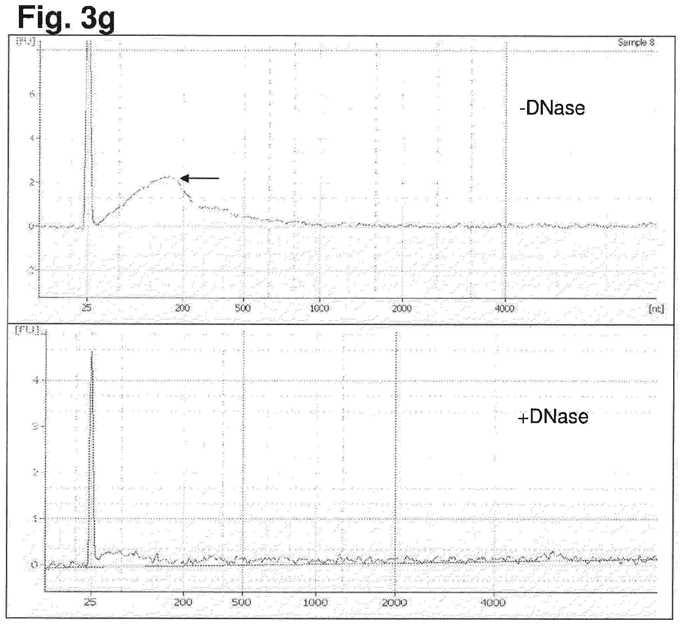

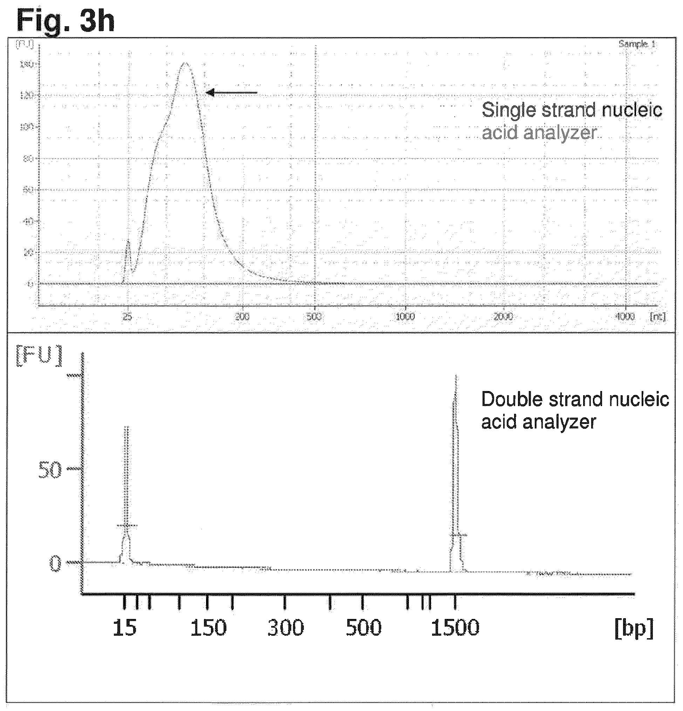

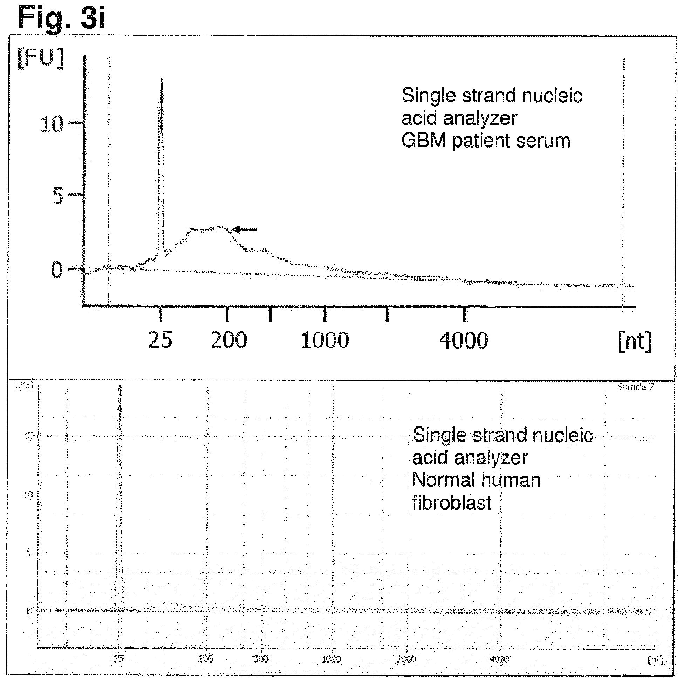

FIGURE 3. Analysis of microvesicle DNA.
   a) GAPDH gene amplification with DNA templates from exosomes treated with DNase prior to nucleic acid extraction. The lanes are identified as follows:
      1. 100bp MW ladder
      2. Negative control
      3. Genomic DNA control from GBM 20/3 cells
      4. DNA from normal serum exosomes (tumor cell-free control)
      5. Exosome DNA from normal human fibroblasts (NHF19)
      6. Exosome DNA from primary medulloblastoma cells (D425)
   b) GAPDH gene amplification with DNA templates from exosomes without prior DNase treatment. The lanes are identified as follows:
      1. 100bp MW ladder
      2. DNA from primary melanoma cell 0105
      3. Exosome DNA from melanoma 0105
      4. Negative Control
      5. cDNA from primary GBM 20/3 (positive control)
   c) Human Endogenous Retrovirus K gene amplification. The lanes are identified as follows:
      1. 100 bp MW ladder
      2. Exosome DNA from medulloblastoma D425 a
      3. Exosome DNA from medulloblasotma D425 b
      4. Exosome DNA from normal human fibroblasts (NHF19)
      5. Exosome DNA from normal human serum
      6. Genomic DNA from GBM 20/3.
      7. Negative Control
   d) Tenascin C gene amplification. The lanes are listed identified follows:
      1. 100bp MW ladder
      2. Exosomes from normal human fibroblasts (NHF19)
      3. Exosomes from serum (tumor cell free individual A)
      4. Exosomes from serum (tumor cell free individual B)
      5. Exosomes from primary medulloblastoma cell D425
   e) Transposable Line 1 element amplification. The lanes are identified as follows:
      1. 100bp MW ladder.
      2. Exosome DNA from normal human serum.
      3. Exosome DNA from normal human fibroblasts
      4. Exosome DNA from medulloblastoma D425 a
      5. Exosome DNA from medulloblastoma D425 b
   f) DNA is present in exosomes from D425 medulloblastoma cell. The lanes are identified as follows:
      1. 100bp marker
      2. D425 no DNase
      3. D425 with DNase
      4. 1kb marker
   g) Single stranded nucleic acid analysis using a RNA pico chip. Upper panel: purified DNA was not treated with DNase; lower panel: purified DNA was treated with DNase. The arrow in the upper panel refers to the detected nucleic acids. The peak at 25 nt is an internal standard.
   h) Analysis of nucleic acids contained in exosomes from primary medulloblastoma D425. Upper panel: single stranded nucleic acids detected by a RNA pico chip. Lower panel: double stranded nucleic acids detected by a DNA 1000 chip. The arrow in the upper panel refers to the detected nucleic acids. The two peaks (15 and 1500 bp) are internal standards.
   i) Analysis of exosome DNA from different origins using a RNA pico chip. Upper panel: DNA was extracted from exosomes from glioblastoma cells. Lower panel: DNA was extracted from exosomes from normal human fibroblasts.
FIGURE 4. Extracellular RNA extraction from serum is more efficient when a serum exosome isolation step is included. a) Exosome RNA from serum. b) Direct whole serum extraction. c) Empty well. Arrows refer to the detected RNA in the samples.
FIGURE 5. Comparison of gene expression levels between microvesicles and cells of origin. 3426 genes were found to be more than 5-fold differentially distributed in the microvesicles as compared to the cells from which they were derived (p-value <0.01).
FIGURE 6. Ontological analysis of microvesicular RNAs. (a) Pie chart displays the biological process ontology of the 500 most abundant mRNA species in the microvesicles. (b) Graph showing the intensity of microvesicle RNAs belonging to ontologies related to tumor growth. The x-axis represents the number of mRNA transcripts present in the ontology. The median intensity levels on the arrays were 182.
FIGURE 7. Clustering diagram of mRNA levels. Microarray data on the mRNA expression profiles in cell lines and exosomes isolated from the culture media of these cell lines were analyzed and clusters of expression profiles were generated. The labels of the RNA species are as follows:
   20/3C-1: Glioblastoma 20/3 Cell RNA, array replicate 1
   20/3C-2: Glioblastoma 20/3 Cell RNA, array replicate 2
   11/5C: Glioblastoma 11/5 Cell RNA
   0105C: Melanoma 0105 Cell RNA
   0664C: Melanoma 0664 Cell RNA
   0664 E-1: Melanoma 0664 exosome RNA, array replicate 1
   0664 E-2: Melanoma 0664 exosome RNA, array replicate 2
   0105E: Melanoma 0105 Exosome RNA
   20/3E: Glioblastoma 20/3 Exosome RNA
   11/5E-1: Glioblastoma 11/5 Exosomes, array replicate 1
   11/5E-2: Glioblastoma 11/5 Exosomes, array replicate 2
   GBM: glioblastoma. The scale refers to the distance between clusters.

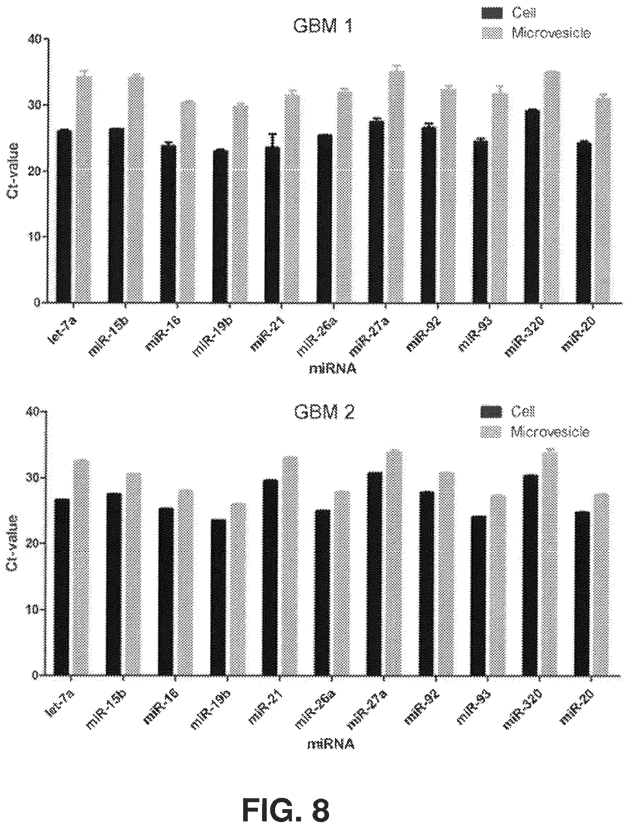

FIGURE 8. Microvesicles from serum contain microRNAs. Levels of mature miRNAs extracted from microvesicles and from glioblastoma cells from two different patients (GBM1 and GBM2) were analysed using quantitative miRNA RT-PCR. The cycle threshold (Ct) value is presented as the mean ± SEM (n = 4).

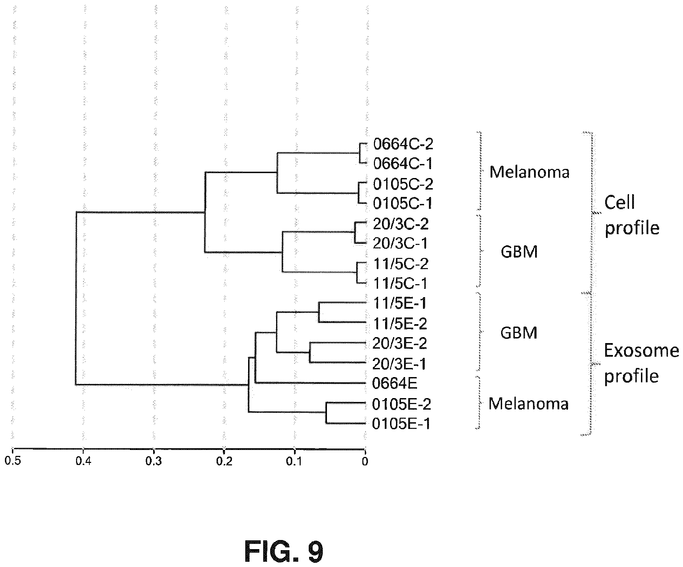

FIGURE 9. Clustering diagram of microRNA levels. Microarray data on the microRNA expression profiles in cell lines and exosomes isolated from the culture media of these cell lines were analyzed and clusters of expression profiles were generated. The labels of the RNA species are as follows:
   0664C-1: Melanoma 0664 Cell RNA, array replicate 1
   0664C-2: Melanoma 0664 Cell RNA, array replicate 2
   0105C-1: Melanoma 0105 Cell RNA, array replicate 1
   0105C-2: Melanoma 0105 Cell RNA, array replicate 2
   20/3C-1: Glioblastoma 20/3 Cell RNA, array replicate 1
   20/3C-2: Glioblastoma 20/3 Cell RNA, array replicate 2
   11/5C-1: Glioblastoma 11/5 Cell RNA, array replicate 1
   11/5C-2: Glioblastoma 11/5 Cell RNA, array replicate 2
   11/5E-1: Glioblastoma 11/5 Exosomes, array replicate 1
   11/5E-2: Glioblastoma 11/5 Exosomes, array replicate 2
   20/3E-1: Glioblastoma 20/3 Exosome RNA, array replicate 1
   20/3E-2: Glioblastoma 20/3 Exosome RNA, array replicate 2
   0664 E: Melanoma 0664 exosome RNA
   0105E-1: Melanoma 0105 Exosome RNA, array replicate 1
   0105E-2: Melanoma 0105 Exosome RNA, array replicate 2
   GBM: Glioblastoma. The scale refers to the distance between clusters.

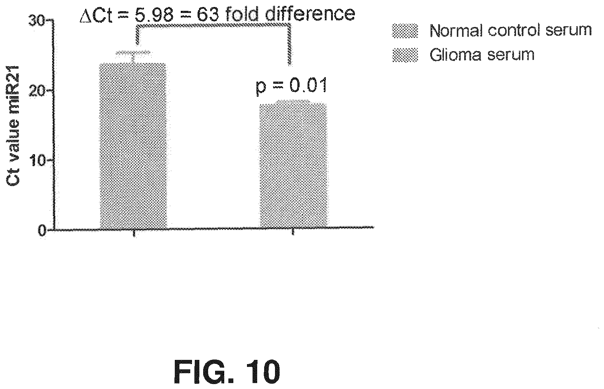

FIGURE 10. The expression level of microRNA-21 in serum microvesicles is associated with glioma. Shown is a bar graph, normal control serum on the left, glioma serum on the right. Quantitative RT-PCR was used to measure the levels of microRNA-21 (miR-21) in exosomes from serum of glioblastoma patients as well as normal patient controls. Glioblastoma serum showed a 5.4 reduction of the Ct-value, corresponding to an approximately 40 (2 ^{Ct})-fold increase of miR21. The miR21 levels were normalized to GAPDH in each sample (n=3).

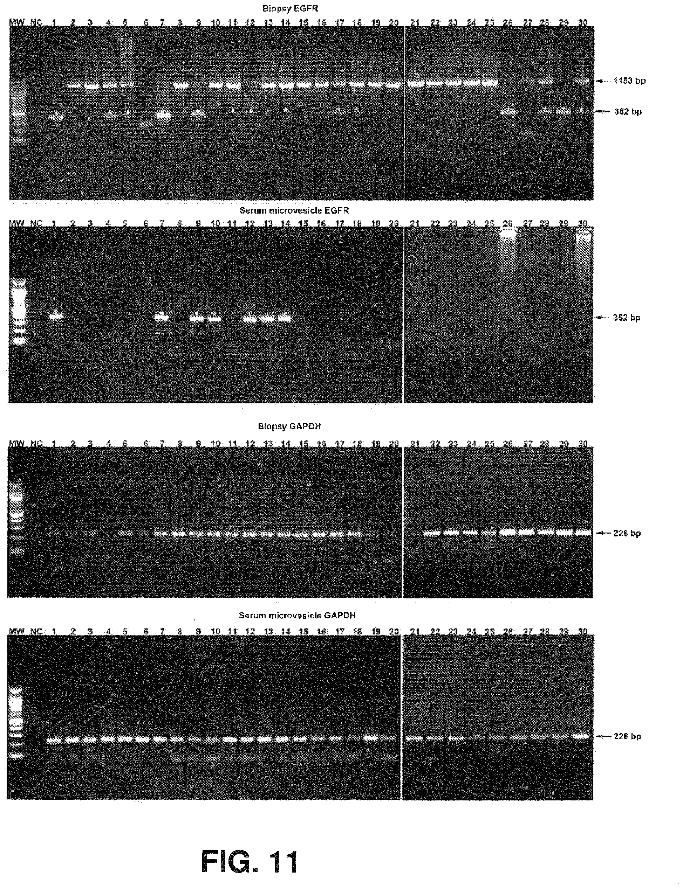

FIGURE 11. Nested RT-PCR was used to detect EGFRvIII mRNA in tumor samples and corresponding serum exosomes. The wild type EGFR PCR product appears as a band at 1153 bp and the EGFRvIII PCR product appears as a band at 352 bp. RT PCR of GAPDH mRNA was included as a positive control (226 bp). Samples considered as positive for EGFRvIII are indicated with an asterisk. Patients 11, 12 and 14 showed only a weak amplification of EGFRvIII in the tumor sample, but it was evident when more samples were loaded.

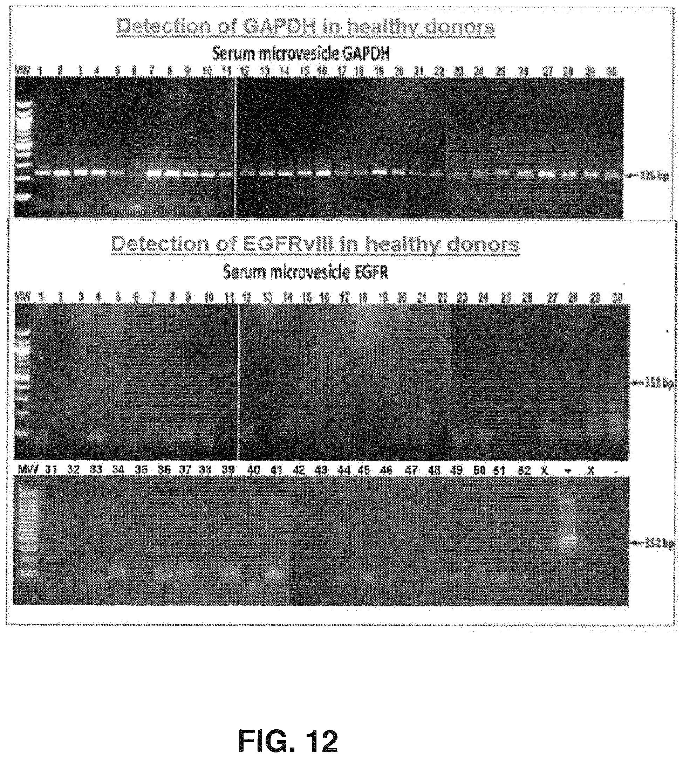

FIGURE 12. Nested RT PCR of EGFRvIII was performed on microvesicles from 52 normal control serums. EGFRvIII (352 bp) was never found in the normal control serums. PCR of GAPDH (226 bp) was included as a control.

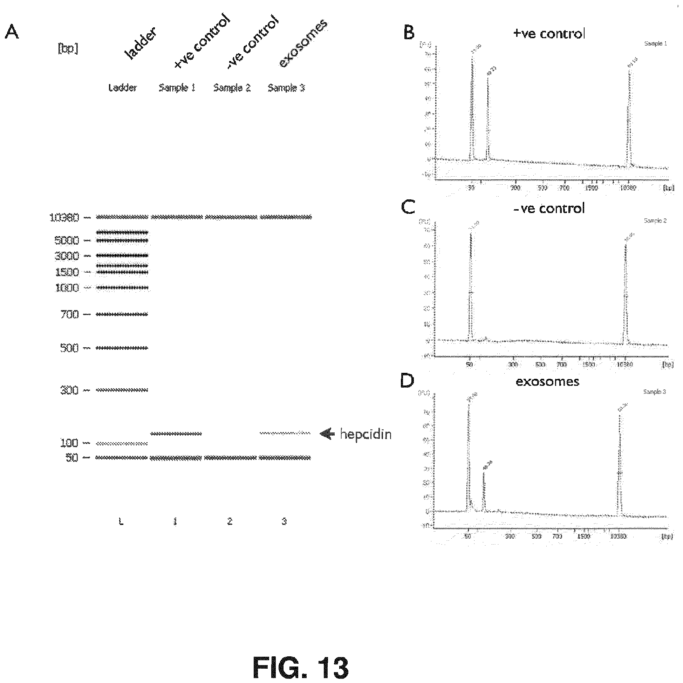

FIGURE 13. Hepcidin mRNA can be detected within exosomes from human serum. A) Pseudo-gel generated by an Agilent Bioanalyzer. B) Raw plot generated by an Agilent Bioanalyser for the positive control (Sample 1). C) Raw plot generated by an Agilent Bioanalyser for the negative control (Sample 2). D) Raw plot generated by an Agilent Bioanalyser for the exosomes (Sample 3).

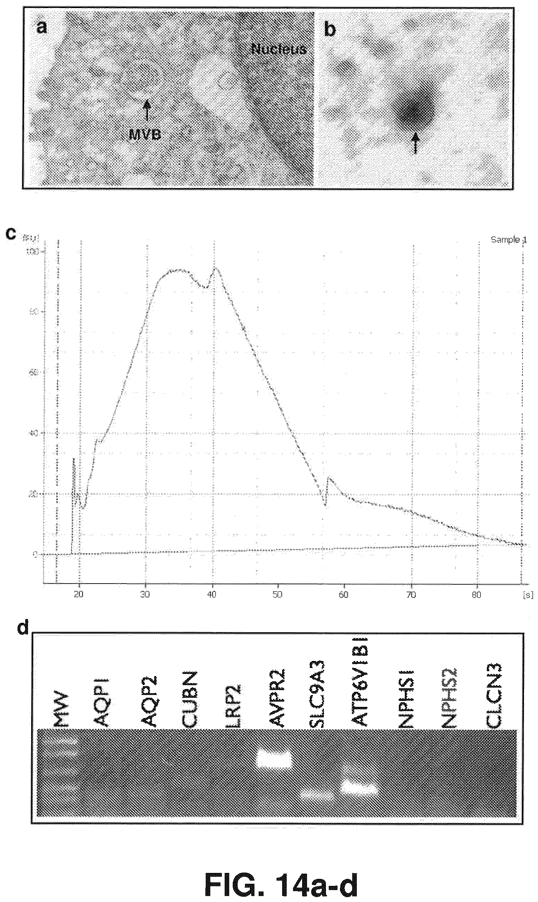

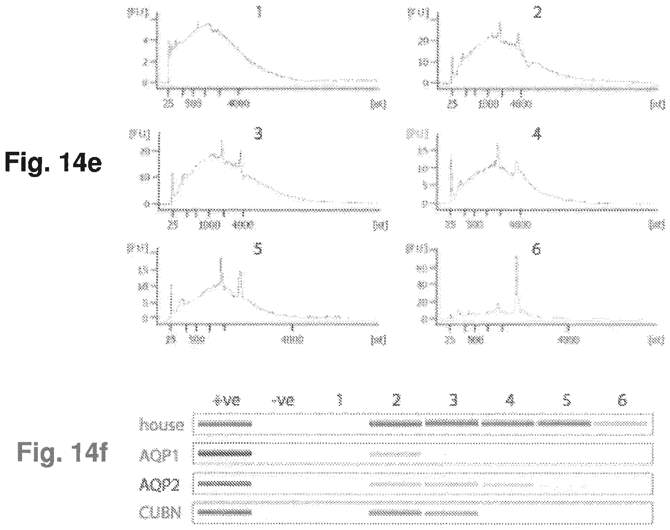

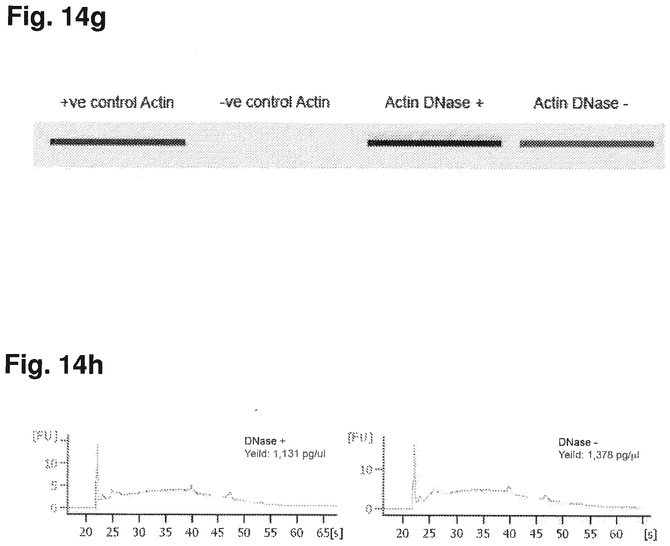

FIGURE 14. Urinary exosome isolation and nucleic acid identification within urinary exosomes. (a) Electron microscopy image of a multivesicular body (MVB) containing many small exosomes in a kidney tubule cell. (b) Electron microscopy image of isolated urinary exosomes. (c) Analysis of RNA transcripts contained within urinary exosomes by an Agilent Bioanalyzer. A broad range of RNA species were identified but both 18S and 28S ribosomal RNAs were absent. (d) Identification of various RNA transcripts in urinary exosomes by PCR. The transcripts thus identified were: Aquaporin 1 (AQP1); Aquaporin 2 (AQP2); Cubulin (CUBN); Megalin (LRP2); Arginine Vasopressin Receptor 2 (AVPR2); Sodium/Hydrogen Exchanger 3 (SLC9A3); V-ATPase B 1 subunit (ATP6V1B 1); Nephrin (NPHS1); Podocin (NPHS2); and Chloride Channel 3 (CLCN3). From top to bottom, the five bands in the molecular weight (MW) lane correspond to 1000, 850, 650, 500, 400, 300 base pair fragments. (e) Bioanalyzer diagrams of exosomal nucleic acids from urine samples. The numbers refer to the numbering of human individuals. (f) Pseudogels depicting PCR products generated with different primer pairs using the nucleic acid extracts described in (e). House refers to actin gene and the actin primers were from Ambion (TX, USA). The +ve control refers to PCRs using human kidney cDNA from Ambion (TX, USA) as templates and the ve control refers to PCRs without nucleic acid templates. (g) Pseudo-gel picture showing positive identification of actin gene cDNA via PCR with and without the DNase treatment of exosomes prior to nucleic acid extraction. (h) Bioanalyzer diagrams showing the amount of nucleic acids isolated from human urinary exosomes.

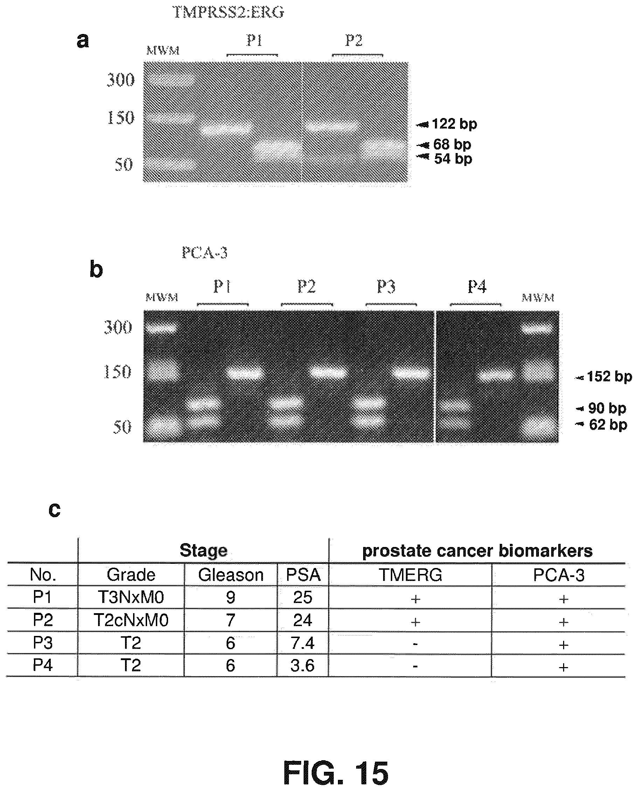

FIGURE 15. Analysis of prostate cancer biomarkers in urinary exosomes. (a) Gel pictures showing PCR products of the *TMPRSS2-ERG* gene and digested fragments of the PCR products. P1 and P2 refer to urine samples from patient 1 and patient 2, respectively. For each sample, the undigested product is in the left lane and the digested product is in the right lane. MWM indicates lanes with MW markers. The sizes of the bands (both undigested and digested) are indicated on the right of the panel. (b) Gel pictures showing PCR products of the *PCA3* gene and digested fragments of the PCR products. P1, P2, P3 and P4 refer to urine samples from patient 1, patient 2, patient 3 and patient 4, respectively. For each sample, the undigested product is in the left lane and the digested product is in the right lane. MWM indicates lanes with MW markers. The sizes of the bands (both undigested and digested) are indicated on the right of the panel. (c) A summary of the information of the patients and the data presented in (a) and (b). TMERG refers to the *TMPRSS2-ERG* fusion gene.

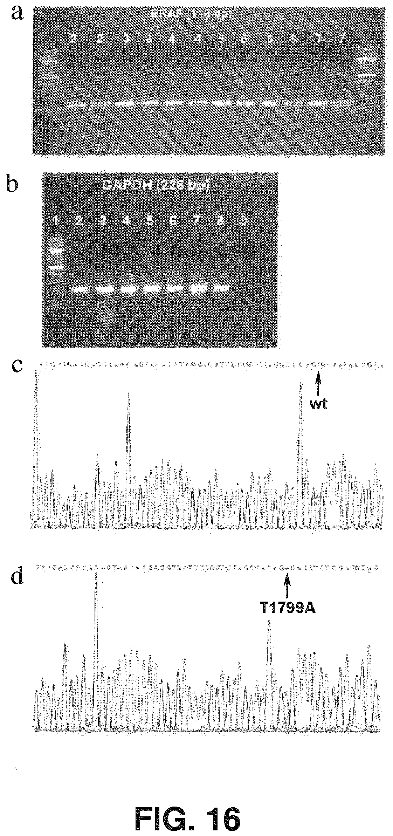

FIGURE 16. BRAF mRNA is contained within microvesicles shed by melanoma cells. (a) An electrophoresis gel picture showing RT-PCR products of BRAF gene amplification. (b) An electrophoresis gel picture showing RT-PCR products of GAPDH gene amplification. The lanes and their corresponding samples are as follows: Lane #1 - 100 bp Molecular Weight marker; Lane #2 - YUMEL-01-06 exo; Lane # 3 - YUMEL-01-06 cell; Lane # 4 YUMEL-06-64 exo; Lane # 5. YUMEL-06-64 cell; Lane # 6. M34 exo; Lane # 7 - M34 cell; Lane # 8 - Fibroblast cell; Lane #9 - Negative control. The reference term exo means that the RNA was extracted from exosomes in the culture media. The reference term cell means that the RNA was extracted from the cultured cells. The numbers following YUMEL refers to the identification of a specific batch of YUMEL cell line. (c) Sequencing results of PCR products from YUMEL-01-06 exo. The results from YUMEL-01-06 cell, YUMEL-06-64 exo and YUMEL-06-64 cell are the same as those from YUMEL-01-06 exo. (d) Sequencing results of PCR products from M34 exo. The results from M34 cell are the same as those from M34 exo.

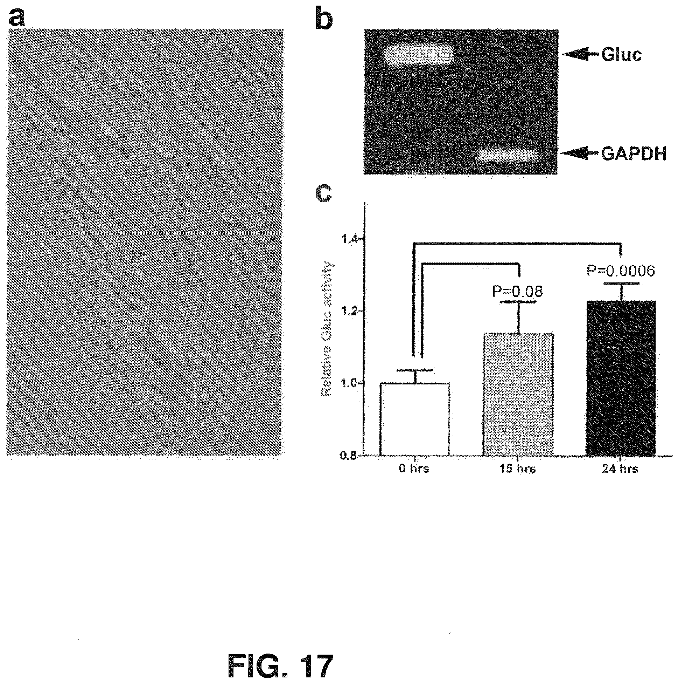

FIGURE 17. Glioblastoma microvesicles can deliver functional RNA to HBMVECs. (a) Purified microvesicles were labelled with membrane dye PKH67 (green) and added to HBMVECs. The microvesicles were internalised into endosome-like structures within an hour. (b) Microvesicles were isolated from glioblastoma cells stably expressing Gluc. RNA extraction and RT-PCR of Gluc and GAPDH mRNAs showed that both were incorporated into microvesicles. (c) Microvesicles were then added to HBMVECs and incubated for 24 hours. The Gluc activity was measured in the medium at 0, 15 and 24 hours after microvesicle addition and normalized to Gluc activity in microvesicles. The results are presented as the mean ± SEM (n = 4).

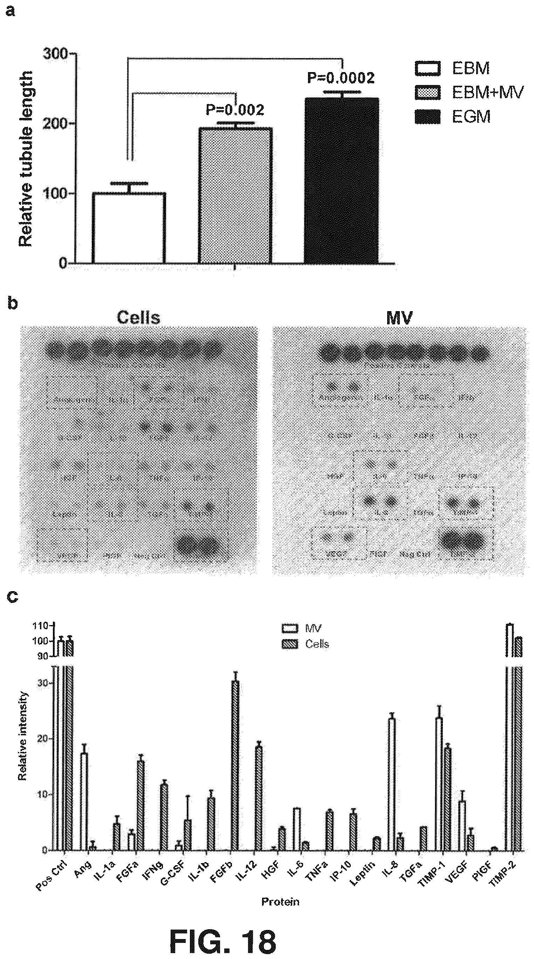

FIGURE 18. Glioblastoma microvesicles stimulate angiogenesis *in vitro* and contain angiogenic proteins. (a) HBMVECs were cultured on Matrigel in basal medium (EBM) alone, or supplemented with GBM microvesicles (EBM+MV) or angiogenic factors (EGM). Tubule formation was measured after 16 hours as average tubule length ± SEM compared to cells grown in EBM (n = 6). (b) Total protein from primary glioblastoma cells and microvesicles (MV) from these cells (1 mg each) were analysed on a human angiogenesis antibody array. (c) The arrays were scanned and the intensities analysed with the Image J software (n = 4).

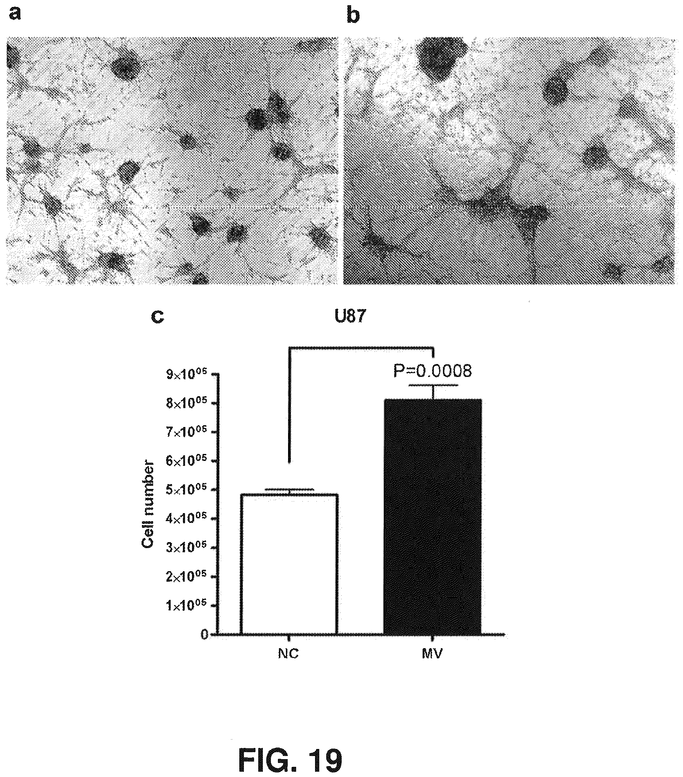

FIGURE 19. Microvesicles isolated from primary glioblastoma cells promote proliferation of the U87 glioblastoma cell line. 100,000 U87 cells were seeded in wells of a 24 well plate and allowed to grow for three days in (a) normal growth medium (DMEM-5% FBS) or (b) normal growth medium supplemented with 125 µg microvesicles. (c) After three days, the non-supplemented cells had expanded to 480,000 cells, whereas the microvesicle-supplemented cells had expanded to 810,000 cells. NC refers to cells grown in normal control medium and MV refers to cells grown in medium supplemented with microvesicles. The result is presented as the mean ± SEM (n=6).

### DETAILED DESCRIPTION OF THE INVENTION

Microvesicles are shed by eukaryotic cells, or budded off of the plasma membrane, to the exterior of the cell. These membrane vesicles are heterogeneous in size with diameters ranging from about 10nm to about 5000 nm. The small microvesicles (approximately 10 to 1000nm, and more often 30 to 200 nm in diameter) that are released by exocytosis of intracellular multivesicular bodies are referred to in the art as exosomes . The methods and compositions described herein are equally applicable to microvesicles of all sizes; preferably 30 to 800 nm; and more preferably 30 to 200 nm.

In some of the literature, the term exosome also refers to protein complexes containing exoribonucleases which are involved in mRNA degradation and the processing of small nucleolar RNAs (snoRNAs), small nuclear RNAs (snRNAs) and ribosomal RNAs (rRNA) (Liu et al., 2006b; van Dijk et al., 2007). Such protein complexes do not have membranes and are not microvesicles or exosomes as those terms are used here in.

### Exosomes As Diagnostic And/Or Prognostic Tools

Certain aspects of the present disclosure are based on the surprising finding that glioblastoma derived microvesicles can be isolated from the serum of glioblastoma patients. This is the first discovery of microvesicles derived from cells in the brain, present in a bodily fluid of a subject. Prior to this discovery it was not known whether glioblastoma cells produced microvesicles or whether such microvesicles could cross the blood brain barrier into the rest of the body. These microvesicles were found to contain mutant mRNA associated with tumor cells. The microvesicles also contained microRNAs (miRNAs) which were found to be abundant in glioblastomas. Glioblastoma-derived microvesicles were also found to potently promote angiogenic features in primary human brain microvascular endothelial cells (HBMVEC) in culture. This angiogenic effect was mediated at least in part through angiogenic proteins present in the microvesicles. The nucleic acids found within these microvesicles, as well as other contents of the microvesicles such as angiogenic proteins, can be used as valuable biomarkers for tumor diagnosis, characterization and prognosis by providing a genetic profile. Contents within these microvesicles can also be used to monitor tumor progression over time by analyzing if other mutations are acquired during tumor progression as well as if the levels of certain mutations are becoming increased or decreased over time or over a course of treatment

Certain aspects of the present disclosure are based on the finding that microvesicles are secreted by tumor cells and circulating in bodily fluids. The number of microvesicles increases as the tumor grows. The concentration of the microvesicles in bodily fluids is proportional to the corresponding tumor load. The bigger the tumor load, the higher the concentration of microvesicles in bodily fluids.

Certain aspects of the present disclosure are based on another surprising finding that most of the extracellular RNAs in bodily fluid of a subject are contained within microvesicles and thus protected from degradation by ribonucleases. As shown in Example 3, more than 90% of extracellular RNA in total serum can be recovered in microvesicles.

Disclosed but not part of the claimed invention are methods for detecting, diagnosing, monitoring, treating or evaluating a disease or other medical condition in a subject by determining the concentration of microvesicles in a biological sample. The determination may be performed using the biological sample without first isolating the microvesicles or by isolating the microvesicles first.

The present invention relates to a method as defined in present claim 1, wherein said method is for for detecting, diagnosing, monitoring, treating or evaluating a disease or other medical condition in a subject comprising the steps of, isolating exosomes from a bodily fluid of a subject, and analyzing one or more nucleic acids contained within the exosomes. The nucleic acids are analyzed qualitatively and/or quantitatively, and the results are compared to results expected or obtained for one or more other subjects who have or do not have the disease or other medical condition. The presence of a difference in microvesicular nucleic acid content of the subject, as compared to that of one or more other individuals, can indicate the presence or absence of, the progression of (e.g., changes of tumor size and tumor malignancy), or the susceptibility to a disease or other medical condition in the subject.

Indeed, the isolation methods and techniques described herein provide the following heretofore unrealized advantages: 1) the opportunity to selectively analyze disease- or tumor-specific nucleic acids, which may be realized by isolating disease- or tumor-specific microvesicles apart from other microvesicles within the fluid sample; 2) significantly higher yield of nucleic acid species with higher sequence integrity as compared to the yield/integrity obtained by extracting nucleic acids directly from the fluid sample; 3) scalability, e.g. to detect nucleic acids expressed at low levels, the sensitivity can be increased by pelleting more microvesicles from a larger volume of serum; 4) purer nucleic acids in that protein and lipids, debris from dead cells, and other potential contaminants and PCR inhibitors are excluded from the microvesicle pellets before the nucleic acid extraction step; and 5) more choices in nucleic acid extraction methods as microvesicle pellets are of much smaller volume than that of the starting serum, making it possible to extract nucleic acids from these microvesicle pellets using small volume column filters.

The microvesicles are preferably isolated from a sample taken of a bodily fluid from a subject. As used herein, a bodily fluid refers to a sample of fluid isolated from anywhere in the body of the subject, preferably a peripheral location, including but not limited to, for example, blood, plasma, serum, urine, sputum, spinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, tumor cyst fluid, amniotic fluid and combinations thereof.

The term subject is intended to include all animals shown to or expected to have microvesicles. In particular embodiments, the subject is a mammal, a human or nonhuman primate, a dog, a cat, a horse, a cow, other farm animals, or a rodent (e.g. mice, rats, guinea pig. etc.). The term subject and individual are used interchangeably herein.

Methods of isolating microvesicles from a biological sample are known in the art. For example, a method of differential centrifugation is described in a paper by Raposo et al. (Raposo et al., 1996) , and similar methods are detailed in the Examples section herein. Methods of anion exchange and/or gel permeation chromatography are described in US Patent Nos. 6,899,863 and 6,812,023. Methods of sucrose density gradients or organelle electrophoresis are described in U.S. Patent No. 7,198,923. A method of magnetic activated cell sorting (MACS) is described in (Taylor and Gercel-Taylor, 2008). A method of nanomembrane ultrafiltration concentrator is described in (Cheruvanky et al., 2007). Preferably, microvesicles can be identified and isolated from bodily fluid of a subject by a newly developed microchip technology that uses a unique microfluidic platform to efficiently and selectively separate tumor derived microvesicles. This technology, as described in a paper by Nagrath et al. (Nagrath et al., 2007), can be adapted to identify and separate microvesicles using similar principles of capture and separation as taught in the paper.

In one embodiment, the microvesicles isolated from a bodily fluid are enriched for those originating from a specific cell type, for example, lung, pancreas, stomach, intestine, bladder, kidney, ovary, testis, skin, colorectal, breast, prostate, brain, esophagus, liver, placenta, fetus cells. Because the microvesicles often carry surface molecules such as antigens from their donor cells, surface molecules may be used to identify, isolate and/or enrich for microvesicles from a specific donor cell type (Al-Nedawi et al., 2008; Taylor and Gercel-Taylor, 2008). In this way, microvesicles originating from distinct cell populations can be analyzed for their nucleic acid content. For example, tumor (malignant and non-malignant) microvesicles carry tumor-associated surface antigens and may be detected, isolated and/or enriched via these specific tumor-associated surface antigens. In one example, the surface antigen is epithelial-cell-adhesion-molecule (EpCAM), which is specific to microvesicles from carcinomas of lung, colorectal, breast, prostate, head and neck, and hepatic origin, but not of hematological cell origin (Balzar et al., 1999; Went et al., 2004). In another example, the surface antigen is CD24, which is a glycoprotein specific to urine microvesicles (Keller et al., 2007). In yet another example, the surface antigen is selected from a group of molecules CD70, carcinoembryonic antigen (CEA), EGFR, EGFRvIII and other variants, Fas ligand, TRAIL, tranferrin receptor, p38.5, p97 and HSP72. Additionally, tumor specific microvesicles may be characterized by the lack of surface markers, such as CD80 and CD86.

The isolation of microvesicles from specific cell types can be accomplished, for example, by using antibodies, aptamers, aptamer analogs or molecularly imprinted polymers specific for a desired surface antigen. In one embodiment, the surface antigen is specific for a cancer type. In another embodiment, the surface antigen is specific for a cell type which is not necessarily cancerous. One example of a method of microvesicle separation based on cell surface antigen is provided in U.S. Patent No. 7,198,923. As described in, e.g., U.S. Patent Nos. 5,840,867 and 5,582,981, WO/2003/050290 and a publication by Johnson et al. (Johnson et al., 2008), aptamers and their analogs specifically bind surface molecules and can be used as a separation tool for retrieving cell type-specific microvesicles. Molecularly imprinted polymers also specifically recognize surface molecules as described in, e.g., US Patent Nos. 6,525,154, 7,332,553 and 7,384,589 and a publication by Bossi et al. (Bossi et al., 2007) and are a tool for retrieving and isolating cell type-specific microvesicles.

It may be beneficial or otherwise desirable to extract the nucleic acid from the exosomes prior to the analysis. Nucleic acid molecules can be isolated from a microvesicle using any number of procedures, which are well-known in the art, the particular isolation procedure chosen being appropriate for the particular biological sample. Examples of methods for extraction are provided in the Examples section herein. In some instances, with some techniques, it may also be possible to analyze the nucleic acid without extraction from the microvesicle.

In one embodiment, the extracted nucleic acids, including DNA and/or RNA, are analyzed directly without an amplification step. Direct analysis may be performed with different methods including, but not limited to, the nanostring technology. NanoString technology enables identification and quantification of individual target molecules in a biological sample by attaching a color coded fluorescent reporter to each target molecule. This approach is similar to the concept of measuring inventory by scanning barcodes. Reporters can be made with hundreds or even thousands of different codes allowing for highly multiplexed analysis. The technology is described in a publication by Geiss et al. (Geiss et al., 2008)

In another embodiment, it may be beneficial or otherwise desirable to amplify the nucleic acid of the microvesicle prior to analyzing it. Methods of nucleic acid amplification are commonly used and generally known in the art, many examples of which are described herein. If desired, the amplification can be performed such that it is quantitative. Quantitative amplification will allow quantitative determination of relative amounts of the various nucleic acids, to generate a profile as described below.

In one embodiment, the extracted nucleic acid is RNA. RNAs are then preferably reverse-transcribed into complementary DNAs before further amplification. Such reverse transcription may be performed alone or in combination with an amplification step. One example of a method combining reverse transcription and amplification steps is reverse transcription polymerase chain reaction (RT-PCR), which may be further modified to be quantitative, e.g., quantitative RT-PCR as described in US Patent No. 5,639,606.

Nucleic acid amplification methods include, without limitation, polymerase chain reaction (PCR) (US Patent No. 5,219,727) and its variants such as in situ polymerase chain reaction (US Patent No. 5,538,871), quantitative polymerase chain reaction (US Patent No. 5,219,727), nested polymerase chain reaction (US Patent No. 5,556,773), self sustained sequence replication and its variants (Guatelli et al., 1990), transcriptional amplification system and its variants (Kwoh et al., 1989), Qb Replicase and its variants (Miele et al., 1983), cold-PCR (Li et al., 2008) or any other nucleic acid amplification methods, followed by the detection of the amplified molecules using techniques well known to those of skill in the art. Especially useful are those detection schemes designed for the detection of nucleic acid molecules if such molecules are present in very low numbers.

The analysis of nucleic acids present in the microvesicles is quantitative and/or qualitative. For quantitative analysis, the amounts (expression levels), either relative or absolute, of specific nucleic acids of interest within the microvesicles are measured with methods known in the art (described below). For qualitative analysis, the species of specific nucleic acids of interest within the microvesicles, whether wild type or variants, are identified with methods known in the art (described below).

Genetic aberrations is used herein to refer to the nucleic acid amounts as well as nucleic acid variants within the microvesicles. Specifically, genetic aberrations include, without limitation, over-expression of a gene (e.g., oncogenes) or a panel of genes, under-expression of a gene (e.g., tumor suppressor genes such as p53 or RB) or a panel of genes, alternative production of splice variants of a gene or a panel of genes, gene copy number variants (CNV) (e.g. DNA double minutes) (Hahn, 1993), nucleic acid modifications (e.g., methylation, acetylation and phosphorylations), single nucleotide polymorphisms (SNPs), chromosomal rearrangements (e.g., inversions, deletions and duplications), and mutations (insertions, deletions, duplications, missense, nonsense, synonymous or any other nucleotide changes) of a gene or a panel of genes, which mutations, in many cases, ultimately affect the activity and function of the gene products, lead to alternative transcriptional splicing variants and/or changes of gene expression level.

The determination of such genetic aberrations can be performed by a variety of techniques known to the skilled practitioner. For example, expression levels of nucleic acids, alternative splicing variants, chromosome rearrangement and gene copy numbers can be determined by microarray analysis (US Patent Nos. 6,913,879, 7,364,848, 7,378,245, 6,893,837 and 6,004,755) and quantitative PCR. Particularly, copy number changes may be detected with the Illumina Infinium II whole genome genotyping assay or Agilent Human Genome CGH Microarray (Steemers et al., 2006). Nucleic acid modifications can be assayed by methods described in, e.g., US Patent No. 7,186,512 and patent publication WO/2003/023065. Particularly, methylation profiles may be determined by Illumina DNA Methylation OMA003 Cancer Panel. SNPs and mutations can be detected by hybridization with allele-specific probes, enzymatic mutation detection, chemical cleavage of mismatched heteroduplex (Cotton et al., 1988), ribonuclease cleavage of mismatched bases (Myers et al., 1985), mass spectrometry (US Patent Nos. 6,994,960, 7,074,563, and 7,198,893), nucleic acid sequencing, single strand conformation polymorphism (SSCP) (Orita et al., 1989), denaturing gradient gel electrophoresis (DGGE)(Fischer and Lerman, 1979a; Fischer and Lerman, 1979b), temperature gradient gel electrophoresis (TGGE) (Fischer and Lerman, 1979a; Fischer and Lerman, 1979b), restriction fragment length polymorphisms (RFLP) (Kan and Dozy, 1978a; Kan and Dozy, 1978b), oligonucleotide ligation assay (OLA), allele-specific PCR (ASPCR) (US Patent No. 5,639,611), ligation chain reaction (LCR) and its variants (Abravaya et al., 1995; Landegren et al., 1988; Nakazawa et al., 1994), flow-cytometric heteroduplex analysis (WO/2006/113590) and combinations/modifications thereof. Notably, gene expression levels may be determined by the serial analysis of gene expression (SAGE) technique (Velculescu et al., 1995). In general, the methods for analyzing genetic aberrations are reported in numerous publications, not limited to those cited herein, and are available to skilled practitioners. The appropriate method of analysis will depend upon the specific goals of the analysis, the condition/history of the patient, and the specific cancer(s), diseases or other medical conditions to be detected, monitored or treated.

A variety of genetic aberrations have been identified to occur and/or contribute to the initial generation or progression of cancer. Examples of genes which are commonly up-regulated (i.e. over-expressed) in cancer are provided in Table 4 (cancers of different types) and Table 6 (pancreatic cancer). Examples of microRNAs which are up-regulated in brain tumor are provided in Table 8. In one embodiment of the disclosure there is an increase in the nucleic acid expression level of a gene listed in Table 4 and/or Table 6 and/or of a microRNA listed in Table 8. Examples of genes which are commonly down-regulated (e.g. under-expressed) in cancer are provided in Table 5 (cancers of different types) and Table 7 (pancreatic cancer). Examples of microRNAs which are down-regulated in brain tumor are provided in Table 9. In one embodiment of the disclosure there is a decrease in the nucleic acid expression level of a gene listed in Table 5 and/or Table 7 and/or a microRNA listed in Table 9. Examples of genes which are commonly under expressed, or over expressed in brain tumors are reviewed in (Furnari et al., 2007). With respect to the development of brain tumors, RB and p53 are often down-regulated to otherwise decrease their tumor suppressive activity. Therefore, in these embodiments, the presence or absence of an increase or decrease in the nucleic acid expression level of a gene(s) and/or a microRNA(s) whose disregulated expression level is specific to a type of cancer can be used to indicate the presence or absence of the type of cancer in the subject.

Likewise, nucleic acid variants, e.g., DNA or RNA modifications, single nucleotide polymorphisms (SNPs) and mutations (e.g., missense, nonsense, insertions, deletions, duplications) may also be analyzed within microvesicles from bodily fluid of a subject, including pregnant females where microvesicles derived from the fetus may be in serum as well as amniotic fluid. Non-limiting examples are provided in Table 3. In yet a further embodiment, the nucleotide variant is in the EGFR gene. In a still further embodiment, the nucleotide variant is the EGFRvIII mutation/variant. The terms EGFR , epidermal growth factor receptor and ErbB1 are used interchangeably in the art, for example as described in a paper by Carpenter (Carpenter, 1987). With respect to the development of brain tumors, RB, PTEN, p16, p21 and p53 are often mutated to otherwise decrease their tumor suppressive activity. Examples of specific mutations in specific forms of brain tumors are discussed in a paper by Furnari et al. (Furnari et al., 2007).

In addition, more genetic aberrations associated with cancers have been identified recently in a few ongoing research projects. For example, the Cancer Genome Atlas (TCGA) program is exploring a spectrum of genomic changes involved in human cancers. The results of this project and other similar research efforts are published (Jones et al., 2008; McLendon et al., 2008; Parsons et al., 2008; Wood et al., 2007). Specifically, these research projects have identified genetic aberrations, such as mutations (e.g., missense, nonsense, insertions, deletions and duplications), gene expression level variations (mRNA or microRNA), copy number variations and nucleic acid modification (e.g. methylation), in human glioblastoma, pancreatic cancer, breast cancer and/or colorectal cancer. The genes most frequently mutated in these cancers are listed in Table 11 and Table 12 (glioblastoma), Table 13 (pancreatic cancer), Table 14 (breast cancer) and Table 15 (colorectal cancer). The genetic aberrations in these genes, and in fact any genes which contain any genetic aberrations in a cancer, are targets that may be selected for use in diagnosing and/or monitoring cancer by the methods described herein.

Detection of one or more nucleotide variants can be accomplished by performing a nucleotide variant screen on the nucleic acids within the microvesicles. Such a screen can be as wide or narrow as determined necessary or desirable by the skilled practitioner. It can be a wide screen (set up to detect all possible nucleotide variants in genes known to be associated with one or more cancers or disease states). Where one specific cancer or disease is suspected or known to exist, the screen can be specific to that cancer or disease. One example is a brain tumor/brain cancer screen (e.g., set up to detect all possible nucleotide variants in genes associated with various clinically distinct subtypes of brain cancer or known drug-resistant or drug-sensitive mutations of that cancer).

In one embodiment, the analysis is of a profile of the amounts (levels) of specific nucleic acids present in the microvesicle, herein referred to as a quantitative nucleic acid profile of the microvesicles. In another embodiment, the analysis is of a profile of the species of specific nucleic acids present in the microvesicles (both wild type as well as variants), herein referred to as a nucleic acid species profile. A term used herein to refer to a combination of these types of profiles is genetic profile which refers to the determination of the presence or absence of nucleotide species, variants and also increases or decreases in nucleic acid levels.

Once generated, these genetic profiles of the microvesicles are compared to those expected in, or otherwise derived from a healthy normal individual. A profile can be a genome wide profile (set up to detect all possible expressed genes or DNA sequences). It can be narrower as well, such as a cancer wide profile (set up to detect all possible genes or nucleic acids derived therefrom, or known to be associated with one or more cancers). Where one specific cancer is suspected or known to exist, the profile can be specific to that cancer (e.g., set up to detect all possible genes or nucleic acids derived therefrom, associated with various clinically distinct subtypes of that cancer or known drug-resistant or sensitive mutations of that cancer).

Which nucleic acids are to be amplified and/or analyzed can be selected by the skilled practitioner. The entire nucleic acid content of the exosomes or only a subset of specific nucleic acids which are likely or suspected of being influenced by the presence of a disease or other medical condition such as cancer, can be amplified and/or analyzed. The identification of a nucleic acid aberration(s) in the analyzed microvesicle nucleic acid can be used to diagnose the subject for the presence of a disease such as cancer, hereditary diseases or viral infection with which that aberration(s) is associated. For instance, analysis for the presence or absence of one or more nucleic acid variants of a gene specific to a cancer (e.g. the EGFRvIII mutation) can indicate the cancer s presence in the individual. Alternatively, or in addition, analysis of nucleic acids for an increase or decrease in nucleic acid levels specific to a cancer can indicate the presence of the cancer in the individual (e.g., a relative increase in EGFR nucleic acid, or a relative decrease in a tumor suppressor gene such as p53).

In one embodiment, mutations of a gene which is associated with a disease such as cancer (e.g. via nucleotide variants, over-expression or under-expression) are detected by analysis of nucleic acids in microvesicles, which nucleic acids are derived from the genome itself in the cell of origin or exogenous genes introduced through viruses. The nucleic acid sequences may be complete or partial, as both are expected to yield useful information in diagnosis and prognosis of a disease. The sequences may be sense or anti-sense to the actual gene or transcribed sequences. The skilled practitioner will be able to devise detection methods for a nucleotide variance from either the sense or anti-sense nucleic acids which may be present in a microvesicle. Many such methods involve the use of probes which are specific for the nucleotide sequences which directly flank, or contain the nucleotide variances. Such probes can be designed by the skilled practitioner given the knowledge of the gene sequences and the location of the nucleic acid variants within the gene. Such probes can be used to isolate, amplify, and/or actually hybridize to detect the nucleic acid variants, as described in the art and herein.

Determining the presence or absence of a particular nucleotide variant or plurality of variants in the nucleic acid within microvesicles from a subject can be performed in a variety of ways. A variety of methods are available for such analysis, including, but not limited to, PCR, hybridization with allele-specific probes, enzymatic mutation detection, chemical cleavage of mismatches, mass spectrometry or DNA sequencing, including minisequencing. In particular embodiments, hybridization with allele specific probes can be conducted in two formats: 1) allele specific oligonucleotides bound to a solid phase (glass, silicon, nylon membranes) and the labeled sample in solution, as in many DNA chip applications, or 2) bound sample (often cloned DNA or PCR amplified DNA) and labeled oligonucleotides in solution (either allele specific or short so as to allow sequencing by hybridization). Diagnostic tests may involve a panel of variances, often on a solid support, which enables the simultaneous determination of more than one variance. In another embodiment, determining the presence of at least one nucleic acid variance in the microvesicle nucleic acid entails a haplotyping test. Methods of determining haplotypes are known to those of skill in the art, as for example, in WO 00/04194.

In one embodiment, the determination of the presence or absence of a nucleic acid variant(s) involves determining the sequence of the variant site or sites (the exact location within the sequence where the nucleic acid variation from the norm occurs) by methods such as polymerase chain reaction (PCR), chain terminating DNA sequencing (US Patent No. 5547859), minisequencing (Fiorentino et al., 2003), oligonucleotide hybridization, pyrosequencing, Illumina genome analyzer, deep sequencing, mass spectrometry or other nucleic acid sequence detection methods. Methods for detecting nucleic acid variants are well known in the art and disclosed in WO 00/04194. In an exemplary method, the diagnostic test comprises amplifying a segment of DNA or RNA (generally after converting the RNA to complementary DNA) spanning one or more known variants in the desired gene sequence. This amplified segment is then sequenced and/or subjected to electrophoresis in order to identify nucleotide variants in the amplified segment.

Disclosed, but not part of the claimed invention is a method of screening for nucleotide variants in the nucleic acid of microvesicles isolated as described herein. This can be achieved, for example, by PCR or, alternatively, in a ligation chain reaction (LCR) (Abravaya et al., 1995; Landegren et al., 1988; Nakazawa et al., 1994). LCR can be particularly useful for detecting point mutations in a gene of interest (Abravaya et al., 1995). The LCR method comprises the steps of designing degenerate primers for amplifying the target sequence, the primers corresponding to one or more conserved regions of the nucleic acid corresponding to the gene of interest, amplifying PCR products with the primers using, as a template, a nucleic acid obtained from a microvesicle, and analyzing the PCR products. Comparison of the PCR products of the microvesicle nucleic acid to a control sample (either having the nucleotide variant or not) indicates variants in the microvesicle nucleic acid. The change can be either an absence or presence of a nucleotide variant in the microvesicle nucleic acid, depending upon the control.

Analysis of amplification products can be performed using any method capable of separating the amplification products according to their size, including automated and manual gel electrophoresis, mass spectrometry, and the like.

Alternatively, the amplification products can be analyzed based on sequence differences, using SSCP, DGGE, TGGE, chemical cleavage, OLA, restriction fragment length polymorphisms as well as hybridization, for example, nucleic acid microarrays.

The methods of nucleic acid isolation, amplification and analysis are routine for one skilled in the art and examples of protocols can be found, for example, in Molecular Cloning: A LaboratoryManual (3-Volume Set) Ed. Joseph Sambrook, David W. Russel, and Joe Sambrook, Cold Spring Harbor Laboratory, 3rd edition (January 15, 2001), ISBN: 0879695773. A particular useful protocol source for methods used in PCR amplification is PCR Basics: From Background to Bench by Springer Verlag; 1st edition (October 15, 2000), ISBN: 0387916008.

Many methods of diagnosis performed on a tumor biopsy sample can be performed with microvesicles since tumor cells, as well as some normal cells are known to shed microvesicles into bodily fluid and the genetic aberrations within these microvesicles reflect those within tumor cells as demonstrated herein. Furthermore, methods of diagnosis using microvesicles have characteristics that are absent in methods of diagnosis performed directly on a tumor biopsy sample. For example, one particular advantage of the analysis of microvesicular nucleic acids, as opposed to other forms of sampling of tumor/cancer nucleic acid, is the availability for analysis of tumor/cancer nucleic acids derived from all foci of a tumor or genetically heterogeneous tumors present in an individual. Biopsy samples are limited in that they provide information only about the specific focus of the tumor from which the biopsy is obtained. Different tumorous/cancerous foci found within the body, or even within a single tumor often have different genetic profiles and are not analyzed in a standard biopsy. However, analysis of the microvesicular nucleic acids from an individual presumably provides a sampling of all foci within an individual. This provides valuable information with respect to recommended treatments, treatment effectiveness, disease prognosis, and analysis of disease recurrence, which cannot be provided by a simple biopsy.

Identification of genetic aberrations associated with specific diseases and/or medical conditions by the methods described herein can also be used for prognosis and treatment decisions of an individual diagnosed with a disease or other medical condition such as cancer. Identification of the genetic basis of a disease and/or medical condition provides useful information guiding the treatment of the disease and/or medical condition. For example, many forms of chemotherapy have been shown to be more effective on cancers with specific genetic abnormalities/aberrations. One example is the effectiveness of EGFR-targeting treatments with medicines, such as the kinase inhibitors gefitinib and erlotinib. Such treatment have been shown to be more effective on cancer cells whose EGFR gene harbors specific nucleotide mutations in the kinase domain of EGFR protein (U.S. Patent publication 20060147959). In other words, the presence of at least one of the identified nucleotide variants in the kinase domain of EGFR nucleic acid message indicates that a patient will likely benefit from treatment with the EGFR-targeting compound gefitinib or erlotinib. Such nucleotide variants can be identified in nucleic acids present in microvesicles by the methods described herein, as it has been demonstrated that EGFR transcripts of tumor origin are isolated from microvesicles in bodily fluid.

Genetic aberrations in other genes have also been found to influence the effectiveness of treatments. As disclosed in the publication by Furnari et al. (Furnari et al., 2007), mutations in a variety of genes affect the effectiveness of specific medicines used in chemotherapy for treating brain tumors. The identification of these genetic aberrations in the nucleic acids within microvesicles will guide the selection of proper treatment plans.

As such, aspects of the present disclosure relate to a method for monitoring disease (e.g. cancer) progression in a subject, and also to a method for monitoring disease recurrence in an individual. These methods comprise the steps of isolating microvesicles from a bodily fluid of an individual, as discussed herein, and analyzing nucleic acid within the microvesicles as discussed herein (e.g. to create a genetic profile of the microvesicles). The presence/absence of a certain genetic aberration/profile is used to indicate the presence/absence of the disease (e.g. cancer) in the subject as discussed herein. The process is performed periodically over time, and the results reviewed, to monitor the progression or regression of the disease, or to determine recurrence of the disease. Put another way, a change in the genetic profile indicates a change in the disease state in the subject. The period of time to elapse between sampling of microvesicles from the subject, for performance of the isolation and analysis of the microvesicle, will depend upon the circumstances of the subject, and is to be determined by the skilled practitioner. Such a method would prove extremely beneficial when analyzing a nucleic acid from a gene that is associated with the therapy undergone by the subject. For example, a gene which is targeted by the therapy can be monitored for the development of mutations which make it resistant to the therapy, upon which time the therapy can be modified accordingly. The monitored gene may also be one which indicates specific responsiveness to a specific therapy.

Aspects of the present disclosure also relate to the fact that a variety of non-cancer diseases and/or medical conditions also have genetic links and/or causes, and such diseases and/or medical conditions can likewise be diagnosed and/or monitored by the methods described herein. Many such diseases are metabolic, infectious or degenerative in nature. One such disease is diabetes (e.g. diabetes insipidus) in which the vasopressin type 2 receptor (V2R) is modified. Another such disease is kidney fibrosis in which the genetic profiles for the genes of collagens, fibronectin and TGF- are changed. Changes in the genetic profile due to substance abuse (e.g. a steroid or drug use), viral and/or bacterial infection, and hereditary disease states can likewise be detected by the methods described herein.

Diseases or other medical conditions for which the inventions described herein are applicable include, but are not limited to, nephropathy, diabetes insipidus, diabetes type I, diabetes II, renal disease glomerulonephritis, bacterial or viral glomerulonephritides, IgA nephropathy, Henoch-Schonlein Purpura, membranoproliferative glomerulonephritis, membranous nephropathy, Sjogren's syndrome, nephrotic syndrome minimal change disease, focal glomerulosclerosis and related disorders, acute renal failure, acute tubulointerstitial nephritis, pyelonephritis, GU tract inflammatory disease, Pre-clampsia, renal graft rejection, leprosy, reflux nephropathy, nephrolithiasis, genetic renal disease, medullary cystic, medullar sponge, polycystic kidney disease, autosomal dominant polycystic kidney disease, autosomal recessive polycystic kidney disease, tuberous sclerosis, von Hippel-Lindau disease, familial thin-glomerular basement membrane disease, collagen III glomerulopathy, fibronectin glomerulopathy, Alport's syndrome, Fabry's disease, Nail-Patella Syndrome, congenital urologic anomalies, monoclonal gammopathies, multiple myeloma, amyloidosis and related disorders, febrile illness, familial Mediterranean fever, HIV infection-AIDS, inflammatory disease, systemic vasculitides, polyarteritis nodosa, Wegener's granulomatosis, polyarteritis, necrotizing and crecentic glomerulonephritis, polymyositis-dermatomyositis, pancreatitis, rheumatoid arthritis, systemic lupus erythematosus, gout, blood disorders, sickle cell disease, thrombotic thrombocytopenia purpura, Fanconi s syndrome, transplantation, acute kidney injury, irritable bowel syndrome, hemolytic-uremic syndrome, acute corticol necrosis, renal thromboembolism, trauma and surgery, extensive injury, burns, abdominal and vascular surgery, induction of anesthesia, side effect of use of drugs or drug abuse, circulatory disease myocardial infarction, cardiac failure, peripheral vascular disease, hypertension, coronary heart disease, non-atherosclerotic cardiovascular disease, atherosclerotic cardiovascular disease, skin disease, soriasis, systemic sclerosis, respiratory disease, COPD, obstructive sleep apnoea, hypoia at high altitude or erdocrine disease, acromegaly, diabetes mellitus, or diabetes insipidus.

Selection of an individual from whom the microvesicles are isolated is performed by the skilled practitioner based upon analysis of one or more of a variety of factors. Such factors for consideration are whether the subject has a family history of a specific disease (e.g. a cancer), has a genetic predisposition for such a disease, has an increased risk for such a disease due to family history, genetic predisposition, other disease or physical symptoms which indicate a predisposition, or environmental reasons. Environmental reasons include lifestyle, exposure to agents which cause or contribute to the disease such as in the air, land, water or diet. In addition, having previously had the disease, being currently diagnosed with the disease prior to therapy or after therapy, being currently treated for the disease (undergoing therapy), being in remission or recovery from the disease, are other reasons to select an individual for performing the methods.

The methods described herein are optionally performed with the additional step of selecting a gene or nucleic acid for analysis, prior to the analysis step. This selection can be based on any predispositions of the subject, or any previous exposures or diagnosis, or therapeutic treatments experienced or concurrently undergone by the subject.

The cancer diagnosed, monitored or otherwise profiled, can be any kind of cancer. This includes, without limitation, epithelial cell cancers such as lung, ovarian, cervical, endometrial, breast, brain, colon and prostate cancers. Also included are gastrointestinal cancer, head and neck cancer, non-small cell lung cancer, cancer of the nervous system, kidney cancer, retina cancer, skin cancer, liver cancer, pancreatic cancer, genital-urinary cancer and bladder cancer, melanoma, and leukemia. In addition, the methods and compositions of the present disclosure are equally applicable to detection, diagnosis and prognosis of non-malignant tumors in an individual (e.g. neurofibromas, meningiomas and schwannomas).

In one embodiment, the cancer is brain cancer. Types of brain tumors and cancer are well known in the art. Glioma is a general name for tumors that arise from the glial (supportive) tissue of the brain. Gliomas are the most common primary brain tumors. Astrocytomas, ependymomas, oligodendrogliomas, and tumors with mixtures of two or more cell types, called mixed gliomas, are the most common gliomas. The following are other common types of brain tumors: Acoustic Neuroma (Neurilemmoma, Schwannoma. Neurinoma), Adenoma, Astracytoma, Low-Grade Astrocytoma, giant cell astrocytomas, Mid- and High-Grade Astrocytoma, Recurrent tumors, Brain Stem Glioma, Chordoma, Choroid Plexus Papilloma, CNS Lymphoma (Primary Malignant Lymphoma), Cysts, Dermoid cysts, Epidermoid cysts, Craniopharyngioma, Ependymoma Anaplastic ependymoma, Gangliocytoma (Ganglioneuroma), Ganglioglioma, Glioblastoma Multiforme (GBM), Malignant Astracytoma, Glioma, Hemangioblastoma, Inoperable Brain Tumors, Lymphoma, Medulloblastoma (MDL), Meningioma, Metastatic Brain Tumors, Mixed Glioma, Neurofibromatosis, Oligodendroglioma. Optic Nerve Glioma, Pineal Region Tumors, Pituitary Adenoma, PNET (Primitive Neuroectodermal Tumor), Spinal Tumors, Subependymoma, and Tuberous Sclerosis (Bourneville s Disease).

In addition to identifying previously known nucleic acid aberrations (as associated with diseases), the methods of the present invention can be used to identify previously unidentified nucleic acid sequences/modifications (e.g. post transcriptional modifications) whose aberrations are associated with a certain disease and/or medical condition. This is accomplished, for example, by analysis of the nucleic acid within microvesicles from a bodily fluid of one or more subjects with a given disease/medical condition (e.g. a clinical type or subtype of cancer) and comparison to the nucleic acid within microvesicles of one or more subjects without the given disease/medical condition, to identify differences in their nucleic acid content. The differences may be any genetic aberrations including, without limitation, expression level of the nucleic acid, alternative splice variants, gene copy number variants (CNV), modifications of the nucleic acid, single nucleotide polymorphisms (SNPs), and mutations (insertions, deletions or single nucleotide changes) of the nucleic acid. Once a difference in a genetic parameter of a particular nucleic acid is identified for a certain disease, further studies involving a clinically and statistically significant number of subjects may be carried out to establish the correlation between the genetic aberration of the particular nucleic acid and the disease. The analysis of genetic aberrations can be done by one or more methods described herein, as determined appropriate by the skilled practitioner.

### Exosomes As Delivery Vehicles

Aspects of the present disclosure also relate to the actual microvesicles described herein. Disclosed, but not part of the claimed invention is an isolated microvesicle as described herein, isolated from an individual. In one embodiment, the microvesicle is produced by a cell within the brain of the individual (e.g. a tumor or non-tumor cell). In another embodiment, the microvesicle is isolated from a bodily fluid of an individual, as described herein. Methods of isolation are described herein.

Disclosed, but not part of the claimed invention is the finding that isolated microvesicles from human glioblastoma cells contain mRNAs, miRNAs and angiogenic proteins. Such glioblastoma microvesicles were taken up by primary human brain endothelial cells, likely via an endocytotic mechanism, and a reporter protein mRNA incorporated into the microvesicles was translated in those cells. This indicates that messages delivered by microvesicles can change the genetic and/or translational profile of a target cell (a cell which takes up a microvesicle). The microvesicles also contained miRNAs which are known to be abundant in glioblastomas (Krichevsky et al, manuscript in preparation). Thus microvesicles derived from glioblastoma tumors function as delivery vehicles for mRNA, miRNA and proteins which can change the translational state of other cells via delivery of specific mRNA species, promote angiogenesis of endothelial cells, and stimulate tumor growth.

In one embodiment, microvesicles are depleted from a bodily fluid from a donor subject before said bodily fluid is delivered to a recipient subject. The donor subject may be a subject with an undetectable tumor and the microvesicles in the bodily fluid are derived from the tumor. The tumor microvesicles in the donor bodily fluid, if unremoved, would be harmful since the genetic materials and proteins in the microvesicle may promote unrestricted growth of cells in the recipient subject.

Disclosed, but not part of the claimed invention is the use of the microvesicles identified herein to deliver a nucleic acid to a cell. In one embodiment, the cell is within the body of an individual. The method comprises administering a microvesicle(s) which contains the nucleic acid, or a cell that produces such microvesicles, to the individual such that the microvesicles contacts and/or enters the cell of the individual. The cell to which the nucleic acid gets delivered is referred to as the target cell.

The microvesicle can be engineered to contain a nucleic acid that it would not naturally contain (i.e. which is exogenous to the normal content of the microvesicle). This can be accomplished by physically inserting the nucleic acid into the microvesicles. Alternatively, a cell (e.g. grown in culture) can be engineered to target one or more specific nucleic acid into the exosome, and the exosome can be isolated from the cell. Alternatively, the engineered cell itself can be administered to the individual.

In one embodiment, the cell which produces the exosome for administration is of the same or similar origin or location in the body as the target cell. That is to say, for delivery of a microvesicle to a brain cell, the cell which produces the microvesicle would be a brain cell (e.g. a primary cell grown in culture). In another embodiment, the cell which produces the exosome is of a different cell type than the target cell. In one embodiment, the cell which produces the exosome is a type that is located proximally in the body to the target cell.

A nucleic acid sequence which can be delivered to a cell via an exosome can be RNA or DNA, and can be single or double stranded, and can be selected from a group comprising: nucleic acid encoding a protein of interest, oligonucleotides, nucleic acid analogues, for example peptide-nucleic acid (PNA), pseudo-complementary PNA (pc-PNA), locked nucleic acid (LNA) etc. Such nucleic acid sequences include, for example, but are not limited to, nucleic acid sequences encoding proteins, for example that act as transcriptional repressors, antisense molecules, ribozymes, small inhibitory nucleic acid sequences, for example but are not limited to RNAi, shRNA, siRNA, miRNA, antisense oligonucleotides, and combinations thereof.

Microvesicles isolated from a cell type are delivered to a recipient subject. Said microvesicles may benefit the recipient subject medically. For example, the angiogenesis and pro-proliferation effects of tumor exosomes may help the regeneration of injured tissues in the recipient subject. In one embodiment, the delivery means is by bodily fluid transfusion wherein microvesicles are added into a bodily fluid from a donor subject before said bodily fluid is delivered to a recipient subject.

In another embodiment, the microvesicle is an ingredient (e.g. the active ingredient in a pharmaceutically acceptable formulation suitable for administration to the subject (e.g. in the methods described herein). Generally this comprises a pharmaceutically acceptable carrier for the active ingredient. The specific carrier will depend upon a number of factors (e.g.. the route of administration).

The pharmaceutically acceptable carrier means any pharmaceutically acceptable means to mix and/or deliver the targeted delivery composition to a subject. This includes a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be acceptable in the sense of being compatible with the other ingredients of the formulation and is compatible with administration to a subject, for example a human.

Administration to the subject can be either systemic or localized. This includes, without limitation, dispensing, delivering or applying an active compound (e.g. in a pharmaceutical formulation) to the subject by any suitable route for delivery of the active compound to the desired location in the subject, including delivery by either the parenteral or oral route, intramuscular injection, subcutaneous/intradermal injection, intravenous injection, buccal administration, transdermal delivery and administration by the rectal, colonic, vaginal, intranasal or respiratory tract route.

It should be understood that this invention is not limited to the particular methodologies, protocols and reagents, described herein and as such may vary. The terminology used herein is for the purpose of describing particular embodiments only, and is not intended to limit the scope of the present invention, which is defined solely by the claims.

In one respect, the present disclosure relates to the herein described compositions, methods, and respective components thereof, as essential to the disclosure, yet open to the inclusion of unspecified elements, essential or not ( comprising ). In some embodiments, other elements to be included in the description of the composition, method or respective component thereof are limited to those that do not materially affect the basic and novel characteristic(s) of the disclosure ( consisting essentially of ). This applies equally to steps within a described method as well as compositions and components therein. In other embodiments, compositions, methods, and respective components thereof, described herein are intended to be exclusive of any element not deemed an essential element to the component, composition or method ( consisting of ).

### EXAMPLES

### Examples 1-7. Tumor cells shed microvesicles, which contain RNAs, including mRNAs and microRNAs, and that microvesicles contain more than 90% of the extracellular RNA in bodily fluids.

### Example 1: Microvesicles are shed from primary human glioblastoma cells.

Glioblastoma tissue was obtained from surgical resections and tumor cells were dissociated and cultured as monolayers. Specifically, brain tumor specimens from patients diagnosed by a neuropathologist as glioblastoma multiforme were taken directly from surgery and placed in cold sterile Neurobasal media (Invitrogen, Carlsbad, CA, USA). The specimens were dissociated into single cells within 1 hr from the time of surgery using a Neural Tissue Dissociation Kit (Miltenyi Biotech, Berisch Gladbach, Germany) and plated in DMEM 5% dFBS supplemented with penicillin-streptomycin (10 IU ml⁻¹ and 10 µg ml⁻¹, respectively, Sigma-Aldrich, St Louis, MO, USA). Because microvesicles can be found in the fetal bovine serum (FBS) traditionally used to cultivate cells, and these microvesicles contain substantial amounts of mRNA and miRNA, it was important to grow the tumor cells in media containing microvesicle-depleted FBS (dFBS). Cultured primary cells obtained from three glioblastoma tumors were found to produce microvesicles at both early and later passages (a passage is a cellular generation defined by the splitting of cells, which is a common cell culture technique and is necessary to keep the cells alive). The microvesicles were able to be detected by scanning electronmicroscopy (FIGS 1a and 1b) and transmission electronmicroscopy (FIG 1f). Briefly, human glioblastoma cells were placed on ornithine-coated cover-slips, fixed in 0.5x Karnovskys fixative and then washed 2x5min (2 times with 5 min each) with PBS. The cells were dehydrated in 35% EtOH 10 min, 50% EtOH 2x 10 min, 70% EtOH 2x 10 min, 95% EtOH 2x 10 min, and 100% EtOH 4x10 min. The cells were then transferred to critical point drying in a Tousimis SAMDR1-795 semi-automatic Critical Point Dryer followed by coating with chromium in a GATAN Model 681 High Resolution Ion Beam Coater. As shown in FIGS. 1a and 1b, tumor cells were covered with microvesicles varying in size from about 50 - 500 nm.

### Example 2: Glioblastoma microvesicles contain RNA.

To isolate microvesicles, glioblastoma cells at passage 1-15 were cultured in microvesicle-free media (DMEM containing 5% dFBS prepared by ultracentrifugation at 110,000 x g for 16 hours to remove bovine microvesicles). The conditioned medium from 40 million cells was harvested after 48 hours. The microvesicles were purified by differential centrifugation. Specifically, glioblastoma conditioned medium was centrifuged for 10 min at 300 x g to eliminate any cell contamination. Supernatants were further centrifuged for 20 min at 16,500 x g and filtered through a 0.22 m filter. Microvesicles were then pelleted by ultracentrifugation at 110,000 x g for 70 min. The microvesicle pellets were washed in 13 ml PBS, pelleted again and resuspended in PBS.

Isolated microvesicles were measured for their total protein content using DC Protein Assay (Bio-Rad, Hercules, CA, USA).

For the extraction of RNA from microvesicles, RNase A (Fermentas, Glen Burnie, MD, USA) at a final concentration of 100 µg/ml was added to suspensions of microvesicles and incubated for 15 min at 37°C to get rid of RNA outside of the microvesicles and thus ensure that the extracted RNA would come from inside the microvesicles. Total RNA was then extracted from the microvesicles using the MirVana RNA isolation kit (Ambion, Austin TX, USA) according to the manufacturer s protocol. After treatment with DNAse according to the manufacturer s protocol, the total RNA was quantified using a nanodrop ND-1000 instrument (Thermo Fischer Scientific, Wilmington, DE, USA).

Glioblastoma microvesicles were found to contain RNA and protein in a ratio of approximately 1:80 ( g RNA:µg protein). The average yield of proteins and RNAs isolated from microvesicles over a 48-hour period in culture was around 4 g protein and 50 ng RNA/million cells.

To confirm that the RNA was contained inside the microvesicles, microvesicles were either exposed to RNase A or mock treatment before RNA extraction (FIG. lc). There was never more than a 7% decrease in RNA content following RNase treatment. Thus, it appears that almost all of the extracellular RNA from the media is contained within the microvesicles and is thereby protected from external RNases by the surrounding vesicular membrane.

Total RNA from microvesicles and their donor cells were analyzed with a Bioanalyzer, showing that the microvesicles contain a broad range of RNA sizes consistent with a variety of mRNAs and miRNAs, but lack 18S and 28S the ribosomal RNA peaks characteristic of cellular RNA (FIGS. Id and 1e).

### Example 3: Microvesicles contain DNA.

To test if microvesicles also contain DNA, exosomes were isolated as mentioned in Example 2 and then treated with DNase before being lysed to release contents. The DNase treatment step was to remove DNA outside of the exosomes so that only DNA residing inside the exosomes was extracted. Specifically, the DNase treatment was performed using the DNA-free kit from Ambion according to manufacturer s recommendations (Catalog#AM1906). For the DNA purification step, an aliquot of isolated exosomes was lysed in 300µl lysis buffer that was part of the MirVana RNA isolation kit (Ambion) and the DNAs were purified from the lysed mixture using a DNA purification kit (Qiagen) according to the manufacturer s recommendation.

To examine whether the extracted DNA contains common genes, PCRs were performed using primer pairs specific to GAPDH, Human endogenous retrovirus K, Tenascin-c and Line-1. For the GAPDH gene, the following primers were used: Forw3GAPDHnew (SEQ ID NO: 1) and Rev3GAPDHnew (SEQ ID NO: 2). The primer pair amplifies a 112bp amplicon if the template is a spliced GAPDH cDNA and a 216bp amplicon if the template is an un-spliced genomic GAPDH DNA. In one experiment, isolated exosomes were treated with DNase before being lysed for DNA extraction (FIG. 3a). The 112bp fragments were amplified as expected from the exosomes from the tumor serum (See Lane 4 in FIG. 3a) and the primary tumor cells (See Lane 6 in FIG. 3a) but not from the exosomes from normal human fibroblasts (See Lane 5 in FIG. 3a). The 216bp fragment could not be amplified from exosomes of all three origins. However, fragments of both 112bp and 216bp were amplified when the genomic DNA isolated from the glioblastoma cell was used as templates (See Lane 3 in FIG. 3a). Thus, spliced GAPDH DNA exists within exosomes isolated from tumor cells but not within exosomes isolated from normal fibroblast cells.

In contrast, in another experiment, isolated exosomes were not treated with DNase before being lysed for DNA extraction (FIG. 3b). Not only the 112bp fragments but also the 216bp fragments were amplified from exosomes isolated from primary melanoma cells (See Lane 3 in FIG. 3b), suggesting that non-spliced GAPDH DNA or partially spliced cDNA that has been reverse transcribed exists outside of the exosomes.

For the Human Endogenous Retrovirus K (HERV-K) gene, the following primers were used: HERVK_6Forw (SEQ ID NO: 3) and HERVK_6Rev (SEQ ID NO: 4). The primer pair amplifies a 172bp amplicon. DNA was extracted from exosomes that were isolated and treated with DNase, and used as the template for PCR amplification. As shown in FIG. 3c, 172bp fragments were amplified in all tumor and normal human serum exosomes but not in exosomes from normal human fibroblasts. These data suggest that unlike exosomes from normal human fibroblasts, tumor and normal human serum exosomes contain endogenous retrovirus DNA sequences. To examine if tumor exosomes also contain transposable elements, the following LINE-1 specific primers were used for PCR amplifications: Linel_Forw (SEQ ID NO: 5) and Linel_Rev (SEQ ID NO: 6). These two primers are designed to detect LINE-1 in all species since each primer contains equal amounts of two different oligos. For the Linel_Forw primer, one oligo contains a C and the other oligo contains a G at the position designated with s . For the Linel_Rev primer, one oligo contains an A and the other oligo contains a G at the position designated with r . The primer pair amplifies a 290bp amplicon. The template was the DNA extracted from exosomes that were treated with DNase (as described above). As shown in FIG. 3e, 290bp LINE-1 fragments could be amplified from the exosomes from tumor cells and normal human serum but not from exosomes from the normal human fibroblasts.

To test if exosomes also contain Tenascin-C DNA, the following primer pair was used to perform PCR: Tenascin C Forw (SEQ ID NO: 7) and Tenascin C Rev (SEQ ID NO: 8). The primer pair amplifies a 197bp amplicon. The template was the DNA extracted from exosomes that were isolated and then treated with DNase before lysis. As shown in FIG. 3d, 197bp Tenascin C fragments were amplified in exosomes from tumor cells or normal human serum but not in exosomes from normal human fibroblasts. Thus, Tenascin-C DNA exists in tumor and normal human serum exosomes but not in exosomes from normal human fibroblasts.

To further confirm the presence of DNA in exosomes, exosomal DNA was extracted from D425 medulloblastoma cells using the method described above. Specifically, the exosomes were isolated and treated with DNase before lysis. Equal volumes of the final DNA extract were either treated with DNase or not treated with DNase before being visualized by Ethidium Bromide staining in 1% agarose gel. Ethidium Bromide is a dye that specifically stains nucleic acids and can be visualized under ultraviolet light. As shown in FIG. 3f, Ethidium Bromide staining disappeared after DNase treatment (See Lane 3 in FIG. 3f) while strong staining could be visualized in the un-treated aliquot (See Lane 2 in FIG. 3f). The DNase treated and non-treated extracts were also analyzed on a RNA pico chip (Agilent Technologies). As shown in FIG. 3g, single stranded DNA could be readily detected in the DNase-non-treated extract (See upper panel in FIG. 3g) but could barely be detected in the DNase-treated extract (See lower panel in FIG. 3g).

To test whether the extracted DNA was single-stranded, nucleic acids were extracted from the treated exosomes as described in the previous paragraph and further treated with RNAse to eliminate any RNA contamination. The treated nucleic acids were then analyzed on a RNA pico Bioanalyzer chip and in a DNA 1000 chip. The RNA pico chip only detects single stranded nucleic acids. The DNA 1000 chip detected double stranded nucleic acids. As shown in FIG. 3h, single stranded nucleic acids were detected (See upper panel) but double stranded nucleic acids were not detected (See lower panel). Thus, the DNA contained within tumor exosomes are mostly single stranded.

To demonstrate that single stranded DNA exists in tumor cells but not in normal human fibroblasts, nucleic acids were extracted from exosomes from either glioblastoma patient serum or normal human fibroblasts. The exosomes were treated with DNase before lysis and the purified nucleic acids were treated with RNase before analysis. As shown in FIG. 3i, exosomal nucleic acids extracted from glioblastoma patient serum could be detected by a RNA pico chip. In contrast, only a very small amount of single stranded DNA was extracted from normal human fibroblasts.

Accordingly, exosomes from tumor cells and normal human serum were found to contain contain single-stranded DNA. The single-stranded DNA is a reverse transcription product since the amplification products do not contain introns (FIG. 3a and FIG. 3b). It is known that tumor cells as well as normal progenitor cells/stem cells have active reverse transcriptase (RT) activity although the activity in normal progenitor cells/stem cells is relatively much lower. This RT activity makes it plausible that RNA transcripts in the cell can be reverse transcribed and packaged into exosomes as cDNA. Interestingly, exosomes from tumor cells contain more cDNAs corresponding to tumor-specific gene transcripts since tumor cells usually have up-regulated reverse transcriptase activity. Therefore, tumor specific cDNA in exosomes may be used as biomarkers for the diagnosis or prognosis of different tumor types. The use of cDNAs as biomarkers would skip the step of reverse transcription compared to the used of mRNA as biomarkers for tumors. In addition, the use of exosomal cDNA is advantageous over the use of whole serum/plasma DNA because serum/plasma contains genomic DNA released from dying cells. When testing amplified whole serum/plasma DNA, there will be more background.

### Example 4: Most extracellular RNA in human serum is contained within exosomes.

To determine the amount of RNA circulating in serum as free RNA /RNA-protein complex versus the amount of RNA contained within the exosomes, we isolated serum from a healthy human subject, and evenly split the serum into two samples with equal volume. For sample 1, the serum was ultracentrifuged to remove most microvesicles. Then the serum supernatant was collected and RNA left in the supernatant was extracted using Trizol LS. For sample 2, the serum was not ultracentrifuged and total RNA was extracted from the serum using Trizol LS. The amount of RNA in the sample 1 supernatant and sample 2 serum was measured. As a result, it was found that the amount of free RNA in sample 1 supernatant was less than 10% of the amount of total RNA isolated from the serum sample 2. Therefore, a majority of the RNA in serum is associated with the exosomes.

### Example 5: High efficiency of serum extracellular nucleic acid extraction is achieved by incorporating a serum exosome isolation step.

Whole serum and plasma contain large amounts of circulating DNA and possibly also RNA protected in protein complexes, while free RNA have a half-life of a few minutes in serum. Extracellular nucleic acid profiles in serum vary between normal and diseased mammals and thus may be biomarkers for certain diseases. To examine the profiles, nucleic acids need to be extracted. However, direct extraction of nucleic acids from serum and plasma is not practical, especially from large serum/plasma volumes. In this case, large volumes of Trizol LS (a RNA extraction reagent) are used to instantly inactivate all serum nucleases before extracting the exosomal nucleic acids. Subsequently, contaminants precipitate into the sample and affect subsequent analyses. As shown in Example 4, most extracellular RNAs in serum are contained in serum exosomes. Therefore, we tested whether it is more efficient to isolate extracellular nucleic acids by isolating the serum exosomes before nucleic acid extraction.

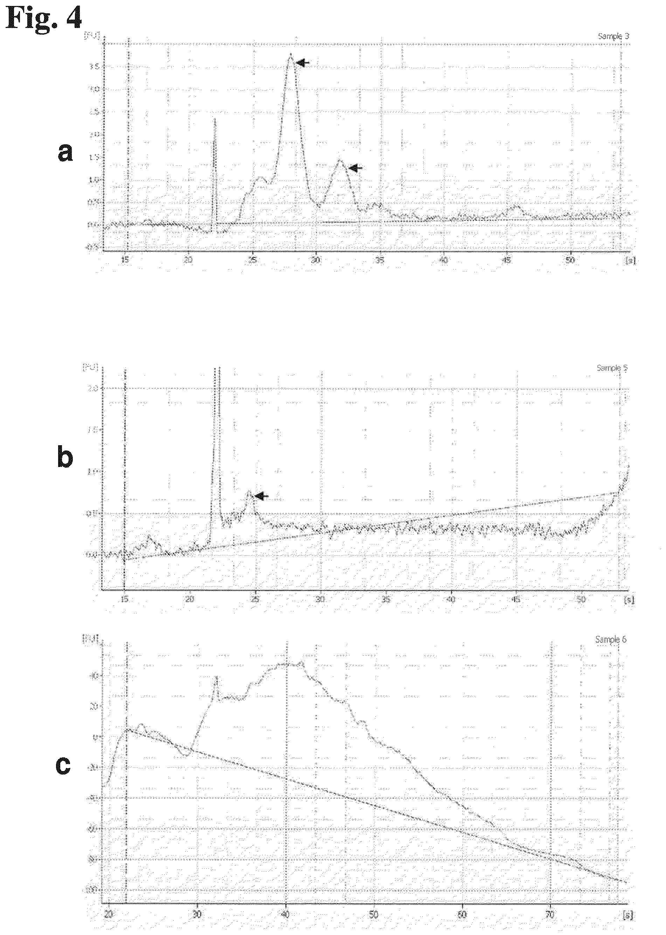

Four milliliter (ml) blood serum from a patient was split into 2 aliquots of 2 ml each. Serum exosomes from one aliquot were isolated prior to RNA extraction. The methods of exosome isolation and RNA extraction are the same as mentioned in Example 2. For the other aliquot, RNA was extracted directly using Trizol LS according to manufacturer s recommendation. The nucleic acids from these two extractions were analyzed on a Bioanalyzer RNA chip (Agilent Technologies). As shown in Figure 4, the amount of RNA extracted with the former method is significantly more than that obtained from the latter method. Further, the quality of RNA extracted with the latter method is relatively poor compared to that with the former method. Thus, the step of exosome isolation contributes to the efficiency of extracellular RNA extraction from serum.

### Example 6: Microarray analysis of mRNA.

Microarray analysis of the mRNA population in glioblastoma cells and microvesicles derived from them was performed by Miltenyi Biotech (Auburn, CA, USA) using the Agilent Whole Human Genome Microarray, 4x44K, two color array. The microarray analysis was performed on two different RNA preparations from primary glioblastoma cells and their corresponding microvesicles RNA preparations prepared as described in Examples 1 and 2. The data was analyzed using the GeneSifter software (Vizxlabs, Seattle, WA, USA). The Intersector software (Vizxlabs) was used to extract the genes readily detected on both arrays. The microarray data have been deposited in NCBI s Gene Expression Omnibus and are accessible through GEO series accession number GSE13470.

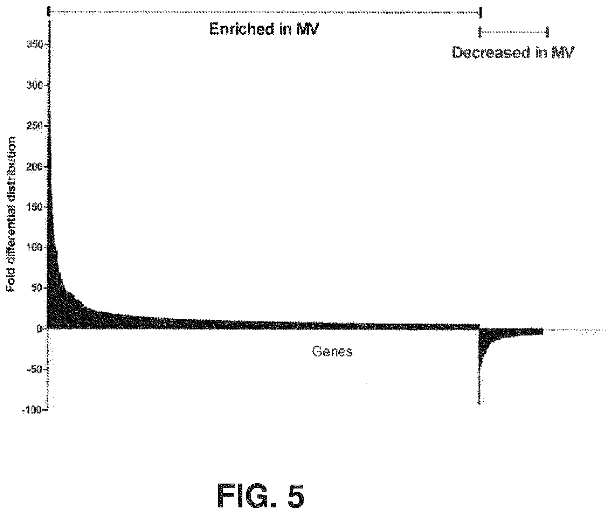

We found approximately 22,000 gene transcripts in the cells and 27,000 gene transcripts in the microvesicles that were detected well above background levels (99% confidence interval) on both arrays. Approximately 4,700 different mRNAs were detected exclusively in microvesicles on both arrays, indicating a selective enrichment process within the microvesicles. Consistent with this, there was a poor overall correlation in levels of mRNAs in the microvesicles as compared to their cells of origin from two tumor cell preparations (FIGS. 2a and 2b). In contrast, there was a good correlation in levels of mRNA from one cell culture (A) versus the second cell culture (B) (FIG. 2c) and a similar correlation in levels of mRNA from the corresponding microvesicles (A) and (B) (FIG. 2d). Accordingly, there is a consistency of mRNA distribution within the tumor cells and microvesicles. In comparing the ratio of transcripts in the microvesicles versus their cells of origin, we found 3,426 transcripts differentially distributed more than 5-fold (p-value <0.01). Of these, 2,238 transcripts were enriched (up to 380 fold) and 1,188 transcripts were less abundant (up to 90 fold) than in the cells (FIG. 5). The intensities and ratios of all gene transcripts were documented. The ontologies of mRNA transcripts enriched or reduced more than 10-fold were recorded and reviewed.

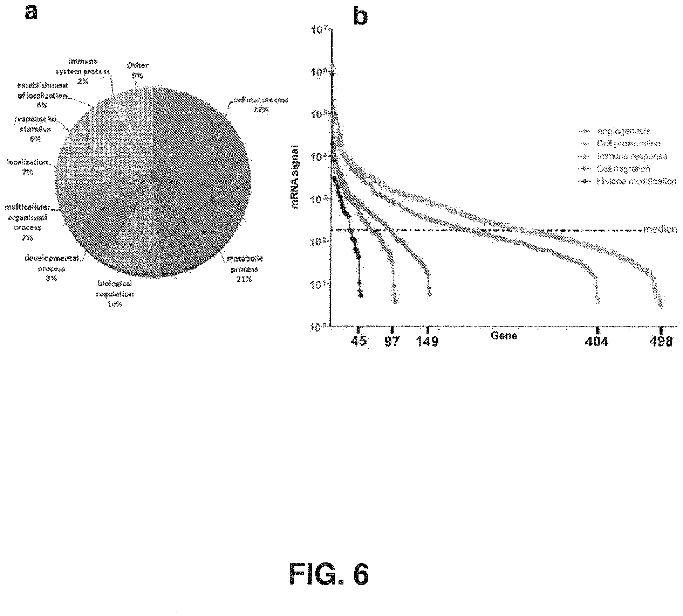

The mRNA transcripts that were highly enriched in the microvesicles were not always the ones that were most abundant in the microvesicles. The most abundant transcripts would be more likely to generate an effect in the recipient cell upon delivery, and therefore the 500 most abundant mRNA transcripts present in microvesicles were divided into different biological processes based on their ontology descriptions (FIG. 6a). Of the various ontologies, angiogenesis, cell proliferation, immune response, cell migration and histone modification were selected for further study as they represent specific functions that could be involved in remodeling the tumor stroma and enhancing tumor growth. Glioblastoma microvesicle mRNAs belonging to these five ontologies were plotted to compare their levels and contribution to the mRNA spectrum (FIG. 6b). All five ontologies contained mRNAs with very high expression levels compared to the median signal intensity level of the array.

A thorough analysis of mRNAs that are enriched in the microvesicles versus donor cells, suggests that there may be a cellular mechanism for localizing these messages into microvesicles, possibly via a zip code in the 3 UTR as described for mRNAs translated in specific cellular locations, such as that for beta actin (Kislauskis et al., 1994). The conformation of the mRNAs in the microvesicles is not known, but they may be present as ribonuclear particles (RNPs) (Mallardo et al., 2003) which would then prevent degradation and premature translation in the donor cell.

Microarray analysis of the mRNA populations in glioblastoma cells and microvesicles derived from glioblastoma cells, melanoma cells, and microvesicles derived from melanoma cells was performed by Illumina Inc. (San Diego, CA, USA) using the Whole-Genome cDNA-mediated Annealing, Selection, Extension, and Ligation (DASL) Assay. The Whole-Genome DASL Assay combines the PCR and labeling steps of Illumina s DASL Assay with the gene-based hybridization and whole-genome probe set of Illumina s HumanRef-8 BeadChip. This BeadChip covers more than 24,000 annotated genes derived from RefSeq (Build 36.2, Release 22). The microarray analysis was performed on two different RNA preparations from primary glioblastoma cells, microvesicles from glioblastomas cells (derived with the method as described in Examples 1 and 2), melanoma cells, and microvesicles from melanoma cells (derived with the method as described in Examples 1 and 2).

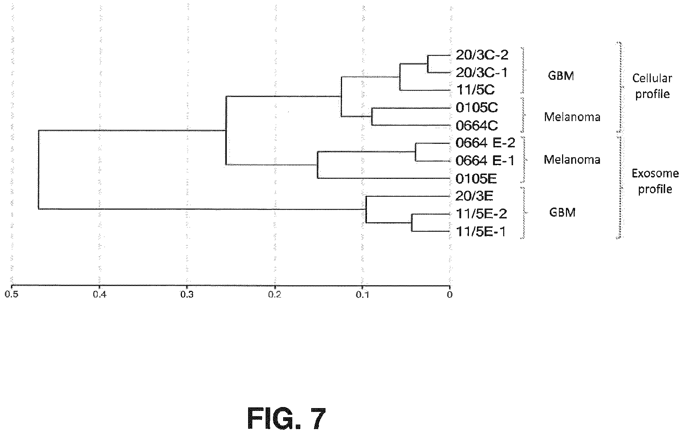

The expression data for each RNA preparation were pooled together and used to generate a cluster diagram. As shown in FIG. 7, mRNA expression profiles for glioblastoma cells, microvesicles from glioblastomas cells, melanoma cells, and microvesicles from melanoma cells are clustered together, respectively. Expression profiles of the two primary glioblastoma cell lines 20/3C and 11/5c are clustered with a distance of about 0.06. Expression profiles of the two primary melanoma cell lines 0105C and 0664C are clustered with a distance of about 0.09. Expression profiles of exosomes from the two primary melanoma cell lines 0105C and 0664C are clustered together with a distance of around 0.15. Expression profiles of exosomes from the two primary glioblastomas cell lines 20/3C and 11/5c are clustered together with a distance of around 0.098. Thus, exosomes from glioblastoma and melanoma have distinctive mRNA expression signatures and the gene expression signature of exosomes differs from that of their original cells. These data demonstrate that mRNA expression profiles from microvesicles may be used in the methods described herein for the diagnosis and prognosis of cancers.

### Example 7: Glioblastoma microvesicles contain miRNA

Mature miRNA from microvesicles and from donor cells was detected using a quantitative miRNA reverse transcription PCR. Specifically, total RNA was isolated from microvesicles and from donor cells using the mirVana RNA isolation kit (Applied Biosystems, Foster City, CA, USA). Using the TaqMan^{®} MicroRNA Assay kits (Applied Biosystems, Foster City, CA, USA), 30 ng total RNA was converted into cDNA using specific miR-primers and further amplified according to the manufacturer s protocol.

A subset of 11 miRNAs among those known to be up-regulated and abundant in gliomas was analyzed in microvesicles purified from two different primary glioblastomas (GBM 1 and GBM 2). These subset contained let-7a, miR-15b, miR-16, miR-19b, miR-21, miR-26a, miR-27a, miR-92, miR-93, miR-320 and miR-20. All of these miRNA were readily detected in donor cells and in microvesicles (FIG. 8). The levels were generally lower in microvesicles per g total RNA than in parental cells (10%, corresponding to approximately 3 Ct-values), but the levels were well correlated, indicating that these 11 miRNA species are not enriched in microvesicles.

Microarray analysis of the microRNA populations in glioblastoma cells and microvesicles derived from glioblastoma cells, melanoma cells, and microvesicles derived from melanoma cells was performed by Illumina Inc. (San Diego, CA, USA) using the MicroRNA Expression Profiling Panel, powered by the DASL Assay. The human MicroRNA Panels include 1146 microRNA species. The microarray analysis was performed on two different RNA preparations from primary glioblastoma cells, microvesicles from glioblastomas cells (derived using the method described in Examples 1 and 2), melanoma cells, and microvesicles from melanoma cells (derived using the method described in Examples 1 and 2).

The expression data for each RNA preparation were pooled together and used to generate a cluster diagram. As shown in FIG. 9, microRNA expression profiles for glioblastoma cells, microvesicles from glioblastomas cells, melanoma cells, and microvesicles from melanoma cells are clustered together, respectively. Expression profiles of the two primary melanoma cell lines 0105C and 0664C are clustered with a distance of about 0.13. Expression profiles of the two primary glioblastomas cell lines 20/3C and 11/5c are clustered with a distance of about 0.12. Expression profiles of exosomes from the two primary glioblastomas cell lines 20/3C and 11/5c are clustered together with a distance of around 0.12. Expression profiles of exosomes from the two primary melanoma cell lines 0105C and 0664C are clustered together with a distance of around 0.17. Thus, exosomes from glioblastoma and melanoma have distinctive microRNA expression signatures and that the gene expression signature of exosomes differs from that of their original cells. Furthermore, as demonstrated herein, microRNA expression profiles from microvesicles may be used in the methods described herein for the diagnosis and prognosis of cancers.

The finding of miRNAs in microvesicles suggests that tumor-derived microvesicles can modify the surrounding normal cells by changing their transcriptional/translational profiles. Furthermore, as demonstrated herein, miRNA expression profile from microvesicles may be used in the methods described herein for the diagnosis and prognosis of cancers, including but not limited to glioblastoma.

### Examples 8-15. These examples show that nucleic acids within exosomes from bodily fluids can be used as biomarkers for diseases or other medical conditions.

### Example 8: Expression profiles of miRNAs in microvesicles can be used as sensitive biomarkers for glioblastoma.

To determine if microRNAs within exosomes may be used as biomarkers for a disease and/or medical condition, we examined the existence of a correlation between the expression level of microRNA and disease status. Since microRNA-21 is expressed at high levels in glioblastoma cells and is readily detectable in exosomes isolated from serum of glioblastoma patients, we measured quantitatively microRNA-21 copy numbers within exosomes from the sera of glioblastoma patients by quantitative RT-PCR. Specifically, exosomes were isolated from 4 ml serum samples from 9 normal human subjects and 9 glioblastoma patients. The RNA extraction procedure was similar to the RNA extraction procedure as described in Example 2. The level of miR-21 was analyzed using singleplex qPCR (Applied Biosystems) and normalized to GAPDH expression level.

As shown in FIG. 10, the average Ct-value was 5.98 lower in the glioblastoma serum sample, suggesting that the exosomal miRNA-21 expression level in glioblastoma patients is approximately 63 fold higher than that in a normal human subject. The difference is statistically significant with a p value of 0.01. Therefore, there is a correlation between microRNA-21 expression level and glioblastoma disease status, which demonstrates that validity and applicability of the non-invasive diagnostic methods disclosed herein. For example, in one aspect, the method comprised the steps of isolating exosomes from the bodily fluid of a subject and analyzing microRNA-21 expression levels within the exosomes by measuring the copy number of microRNA-21 and comparing the number to that within exosomes from a normal subject or to a standard number generated by analyzing microRNA-21 contents within exosomes from a group of normal subjects. An increased copy number indicates the existence of glioblastoma in the subject; while the absence of an increased copy number indicates the absence of glioblastoma in the subject. This basic method may be extrapolated to diagnose/monitor other diseases and/or medical conditions associated with other species of microRNAs.

### Example 9: mRNAs in microvesicles can be used as sensitive biomarkers for diagnosis

Nucleic acids are of high value as biomarkers because of their ability to be detected with high sensitivity by PCR methods. Accordingly, the following tests were designed and carried out to determine whether the mRNA in microvesicles could be used as biomarkers for a medical disease or condition, in this case glioblastoma tumors. The epidermal growth factor receptor (EGFR) mRNA was selected because the expression of the EGFRvIII mutation is specific to some tumors and defines a clinically distinct subtype of glioma (Pelloski et al., 2007). In addition, EGFRvIII mutations traditionally cannot be detected using tissues other than the lesion tissues since these mutations are somatic mutations but not germ line mutations. Therefore, a biopsy from lesion tissues such as glioma tumor is conventionally required for detecting EGFRvIII mutations. As detailed below, nested RT-PCR was used to identify EGFRvIII mRNA in glioma tumor biopsy samples and the results compared with the mRNA species found in microvesicles purified from a serum sample from the same patient.

Microvesicles were purified from primary human glioblastoma cells followed by RNA extraction from both the microvesicles and donor cells (biopsy). The samples were coded and the PCRs were performed in a blind fashion. Gli-36EGFRvIII (human glioma cell stably expressing EGFRvIII) was included as a positive control. The microvesicles from 0.5-2 ml of frozen serum samples were pelleted as described in Example 2 and the RNA was extracted using the MirVana Microvesicles RNA isolation kit. Nested RT-PCR was then used to amplify both the wild type EGFR (1153 bp) and EGFRvIII (352 bp) transcripts from both the microvesicles and donor cells using the same set of primers. Specifically, the RNA was converted to cDNA using the Omniscript RT kit (Qiagen Inc, Valencia, CA, USA) according to the manufacturer s recommended protocol. GAPDH primers were GAPDH Forward (SEQ ID NO: 9) and GAPDH Reverse (SEQ ID NO: 10). The EGFR/EGFRvIII PCR1 primers were SEQ ID NO: 11 and SEQ ID NO: 12. The EGFR/EGFRvIII PCR2 primers were SEQ ID NO: 13 and SEQ ID NO: 14. The PCR cycling protocol was 94 °C for 3 minutes; 94 °C for 45 seconds, 60 °C for 45 seconds, 72 °C for 2 minutes for 35 cycles; and a final step 72 °C for 7 minutes.

We analyzed the biopsy sample to determine whether the EGFRvIII mRNA was present and compared the result with RNA extracted from exosomes purified from a frozen serum sample from the same patient. Fourteen of the 30 tumor samples (47%) contained the EGFRvIII transcript, which is consistent with the percentage of glioblastomas found to contain this mutation in other studies (Nishikawa et al., 2004). EGFRvIII could be amplified from exosomes in seven of the 25 patients (28%) from whom serum was drawn around the time of surgery (FIG. 11 and Table 1). When a new pair of primers EGFR/EGFRvIII PCR3: SEQ ID NO: 15 and SEQ ID NO: 16, were used as the second primer pair for the above nested PCR amplification, more individuals were found to harbor EGFRvIII mutations (Table 1). EGFRvIII could be amplified from exosomes in the six patients who was identified as negatives with the old pair of primers EGFRvIII PCR2: SEQ ID NO: 13 AND SEQ ID NO: 14. Notably, exosomes from individual 13, whose biopsy did not show EGFRvIII mutation, was shown to contain EGFRvIII mutation, suggesting an increased sensivity of EGFRvIII mutation detection using exosomes technology. From the exosomes isolated from 52 normal control serum samples, EGFRvIII could not be amplified (FIG. 12). Interestingly, two patients with an EGFRvIII negative tumor sample turned out to be EGFRvIII positive in the serum exosomes, supporting heterogeneous foci of EGFRvIII expression in the glioma tumor. Furthermore, our data also showed that intact RNAs in microvesicles were, unexpectedly, able to be isolated from frozen bodily serum of glioblastoma patients. These blind serum samples from confirmed glioblastoma patients were obtained from the Cancer Research Center (VU medical center, Amsterdam, the Netherlands) and were kept at -80°C until use. The identification of tumor specific RNAs in serum microvesicles allows the detection of somatic mutations which are present in the tumor cells. Such technology should result in improved diagnosis and therapeutic decisions.

The RNA found in the microvesicles contains a snapshot of a substantial array of the cellular gene expression profile at a given time. Among the mRNA found in glioblastoma-derived microvesicles, the EGFR mRNA is of special interest since the EGFRvIII splice variant is specifically associated with glioblastomas (Nishikawa et al., 2004). Here it is demonstrated that brain tumors release microvesicles into the bloodstream across the blood-brain-barrier (BBB), which has not been shown before. It is further demonstrated that mRNA variants, such as EGFRvIII in brain tumors, are able to be detected by a method comprising the steps of isolating exosomes from a small amount of patient serum and analyzing the RNA in said microvesicles.

Knowledge of the EGFRvIII mutation in tumors is important in choosing an optimal treatment regimen. EGFRvIII-positive gliomas are over 50 times more likely to respond to treatment with EGFR-inhibitors like erlotinib or gefitinib (Mellinghoff et al., 2005).

### Example 10: Diagnosis of iron metabolism disorders

The exosome diagnostics method can be adapted for other purposes as shown by the following example.

Hepcidin, an antimicrobial peptide, is the master hormonal regulator of iron metabolism. This peptide is produced mainly in mammalian liver and is controlled by the erythropoietic activity of the bone-marrow, the amount of circulating and stored body iron, and inflammation. Upon stimulation, hepcidin is secreted into the circulation or urine where it may act on target ferroportin-expressing cells. Ferroportin is the sole iron exporter identified to date and when bound to hepcidin, it is internalized and degraded. The resulting destruction of ferroportin leads to iron retention in ferroportin expressing cells such as macrophages and enterocytes. This pathophysiological mechanism underlies anemia of chronic diseases. More specifically, inappropriately high levels of hepcidin and elevated iron content within the reticuloendothelial system characterize anemia. Indeed, anemia may be associated with many diseases and/or medical conditions such as infections (acute and chronic), cancer, autoimmune, chronic rejection after solid-organ transplantation, and chronic kidney disease and inflammation (Weiss and Goodnough, 2005). On the other hand, in a genetic iron overload disease such as hereditary hemochromatosis, inappropriately low expression levels of hepcidin encourage a potentially fatal excessive efflux of iron from within the reticuloendothelial system. So, hepcidin is up-regulated in anemia associated with chronic disease, but down-regulated in hemochromatosis.

Currently, there is no suitable assay to quantitatively measure hepcidin levels in circulation or urine (Kemna et al., 2008) except time-of-flight mass spectrometry (TOF MS), which needs highly specialized equipment, and therefore is not readily accessible. Recently, the method of Enzyme Linked ImmunoSorbent Assay (ELISA) has been proposed to quantitatively measure hepcidin hormone levels but this method is not consistent because of the lack of clear correlations with hepcidin (Kemna et al., 2005; Kemna et al., 2007) and other iron related parameters (Brookes et al., 2005; Roe et al., 2007).

Hepcidin mRNA was detected in exosomes from human serum, as follows. Exosomes were first isolated from human serum and their mRNA contents extracted before conversion to cDNA and PCR amplification. PCR primers were designed to amplify a 129 nucleotide fragment of human Hepcidin. The sequences of the primers are SEQ ID NO: 57 and SEQ ID NO: 58. A hepcidin transcript of 129 nucleotides (the middle peak in FIG. 13D) was readily detected by Bioanalyzer. As a positive control (FIG. 13B), RNA from a human hepatoma cell line Huh-7 was extracted and converted to cDNA. The negative control (FIG. 13C) is without mRNA. These Bioanalyzer data are also shown in the pseudogel in FIG. 13A.

Hepcidin mRNA in microvesicles in circulation correlates with hepcidin mRNA in liver cells. Hence, measuring hepcidin mRNA within microvesicles in a bodily fluid sample would allow one to diagnose or monitor anemia or hemochromatosis in the subject.

Thus, it is possible to diagnose and/or monitor anemia and hemochromatosis in a subject by isolating microvesicles from a bodily fluid and comparing the hepcidin mRNA in said microvesicles with the mRNA from from a normal subject. With an anemic subject, the copy number of mRNA is increased over the normal, non-anemic level. In a subject suffering from hemochromatosis, the copy number is decreased relative to the mRNA in a normal subject.

### Example 11: Non-invasive transcriptional profiling of exosomes for diabetic nephropathy diagnosis

Diabetic nephropathy (DN) is a life threatening complication that currently lacks specific treatments. Thus, there is a need to develop sensitive diagnostics to identify patients developing or at risk of developing DN, enabling early intervention and monitoring.

Urine analysis provides a way to examine kidney function without having to take a biopsy. To date, this analysis has been limited to the study of protein in the urine. This Example sets forth a method to obtain from urine transcriptional profiles derived from cells that normally could only be obtained by kidney biopsy. Specifically, the method comprises the steps of isolating urine exosomes and analyzing the RNAs within said exosomes to obtain transcriptional profiles, which can be used to examine molecular changes being made by kidney cells in diabetic individuals and provide a 'snap shot' of any new proteins being made by the kidney. State-of-the-art technologies to obtain exosomal transcription profiles include, but are not limited to, contemporary hybridization arrays, PCR based technologies, and next generation sequencing methods. Since direct sequencing does not require pre-designed primers or spotted DNA oligos, it will provide a non-biased description of exosomal RNA profiles. An example of next generation sequencing technology is provided by the Illumina Genome Analyzer, which utilizes massively parallel sequencing technology which allows it to sequence the equivalent of 1/3 a human genome per run. The data obtainable from this analysis would enable one to rapidly and comprehensively examine the urinary exosomal transcriptional profile and allow comparison to the whole kidney. Using such a method, one could obtain much needed information regarding the transcription profile of urinary exosomes. A comparison of transcripts in control versus diabetes-derived urinary exosomes could further provide one with a comprehensive list of both predicted and new biomarkers for diabetic nephropathy.

In order to prove the feasibility of the diagnostic method described above, an experiment was designed and carried out to isolate urinary exosomes and to confirm the presence of renal specific biomarkers within these exosomes. In this experiment, a fresh morning urine sample of 220 ml was collected from a 28-year old healthy male subject and processed via differential centrifugation to isolate urinary exosomes. Specifically, urine was first spun at 300 x g spin for 10 minutes to remove any cells from the sample. The supernatant was collected and then underwent a 20-minute 16,500 x g spin to bring down any cell debris or protein aggregates. The supernatant was then passed through a 0.22 uM membrane filter to remove debris with diameters larger than 0.22uM. Finally, the sample underwent ultra-centrifugation at 100,000 x g for 1 hour to pellet the exosomes (Thery et al., 2006). The pellet was gently washed in phosphate buffered saline (PBS) and RNA was extracted using a Qiagen RNeasy kit pursuant to the manufacturer s instructions. The isolated RNA was converted to cDNA using the Omniscript RT kit (Qiagen) followed by PCR amplification of renal specific genes.

The renal specific genes examined and their corresponding renal area where the gene is expressed are as follows: AQP1 proximal tubules; AQP2 distal tubule (principal cells); CUBN proximal tubules; LRP2 proximal tubules; AVPR2 proximal and distal tubules; SLC9A3 (NHE-3) - Proximal tubule; ATP6V1B1 distal tubule (intercalated cells); NPHS1 glomerulus (podocyte cells); NPHS2 glomerulus (podocyte cells); and CLCN3 Type B intercalated cells of collecting ducts. The sequences of the primers designed to amplify each gene are AQP1-F (SEQ ID NO: 17) and AQP1-R (SEQ ID NO: 18); AQP2-F (SEQ ID NO: 19) and AQP2-R (SEQ ID NO: 20); CUBN-F (SEQ ID NO: 21) and CUBN-R (SEQ ID NO: 22); LRP2-F (SEQ ID NO: 23) and LRP2-R (SEQ ID NO: 24); AVPR2-F (SEQ ID NO: 25) and AVPR2-R (SEQ ID NO: 26); SLC9A3-F (SEQ ID NO: 27) and SLC9A3-R (SEQ ID NO: 28); ATP6V1B1-F (SEQ ID NO: 29) and ATP6V1B1-R (SEQ ID NO: 30); NPHS1-F (SEQ ID NO: 31) and NPHS1-R (SEQ ID NO: 32); NPHS2-F (SEQ ID NO: 33) and NPHS2-R (SEQ ID NO: 34); CLCN5-F (SEQ ID NO: 35) and CLCN5-R (SEQ ID NO: 36).

The expected sizes of the PCR products for each gene are AQP1-226bp, AQP2-208bp, CUBN-285bp, LRP2-220bp, AVPR2-290bp, SLC9A3-200bp, ATP6V1B1-226bp, NPHS1-201bp, NPHS2-266bp and CLCN5-204bp. The PCR cycling protocol was 95 °C for 8 minutes; 95 °C for 30 seconds, 60 °C for 30 seconds, 72 °C for 45 seconds for 30 cycles; and a final step 72 °C for 10 minutes.

As shown in FIG. 14a, kidney tubule cells contain multivesicular bodies, which is an intermediate step during exosome generation. Exosomes isolated from these cells can be identified by electron microscopy (FIG. 14b). Analysis of total RNA extracted from urinary exosomes indicates the presence of RNA species with a broad range of sizes (FIG. 14c). 18S and 28S ribosomal RNAs were not found. PCR analysis confirmed the presence of renal specific transcripts within urinary exosomes (FIG. 14d). These data show that kidney cells shed exosomes into urine and these urinary exosomes contain transcripts of renal origin, and that the exosome method can detect renal biomarkers associated with certain renal diseases and/or other medical conditions.

To further confirm the presence of renal specific mRNA transcripts in urinary exosomes, an independent set of experiments were performed using urine samples from six individuals. Exosomal nucleic acids were extracted from 200ml morning urine samples from each indivisual following a procedure as mentioned above. Specifically, urine samples underwent differential centrifugation starting with a 1000 g centrifugation to spin down whole cells and cell debris. The supernatant was carefully removed and centrifuged at 16,500 g for 20 minutes. The follow-on supernatant was then removed and filtered through a 0.8µm filter to remove residual debris from the exosome containing supernatant. The final supernatant then underwent ultracentrifugation at 100,000 g for 1hr lOmin. The pellet was washed in nuclease free PBS and re-centrifuged at 100,000 g for 1hr 10min to obtain the exosomes pellet which is ready for nucleic acid extraction. Nucleic acids were extracted from the pelleted exosomes using the Arcturus PicoPure RNA Isolation kit and the nucleic acid concentration and integrity was analyzed using a Bioanalyzer (Agilent) Pico chip. As shown in FIG. 14e, nucleic acids isolated from urinary exosomes vary from individual to individual. To test whether the presence of renal biomarkers also varies from individual to individual, PCR amplifications were carried out for Aquaporin1, Aquaporin2 and Cubilin gene using a new set of primer pairs: AQP1 new primer pair: SEQ ID NO: 37 and SEQ ID NO: 38; AQP2 new primer pair: SEQ ID NO: 39 and SEQ ID NO: 40; CUBN new primer pair: SEQ ID NO: 41 and SEQ ID NO: 42. These primer pairs were designed specifically to amplify the spliced and reverse transcribed cDNA fragments. Reverse transcription was performed using the Qiagen Sensiscript kit. As shown in FIG. 14f, no amplification was seen in individual 1, probably due to failed nucleic acid extraction. AQP1 was amplified only in individual 2. CUBN was amplified in indivisual 2 and 3. And AQP2 was amplified in individual 2, 3, 4 and 5. In comparison actin gene (indicated by House in FIG. 14f) was amplified in individual 2, 3, 4, 5 and 6. These data provide more evidence that urinary exosomes contain renal specific mRNA transcripts although the expression levels are different between different individuals.

To test the presence of cDNAs in urinary exosomes, a 200ml human urine sample was split into two 100ml urine samples. Urinary exosomes were isolated from each sample. Exosomes from one sample were treated with DNase and those from the other sample were mock treated. Exosomes from each sample were then lysed for nucleic acid extraction using PicoPure RNA isolation kit (Acturus). The nucleic acids were used as templates for nested-PCR amplification (PCR protocols described in Example 9) without prior reverse transcription. The primer pairs to amplify the actin gene were Actin-FOR (SEQ ID NO: 43) and Actin-REV (SEQ ID NO: 44); Actin-nest-FOR (SEQ ID NO: 45) and Actin-nest-REV (SEQ ID NO: 46) with an expected final amplicon of 100bp based on the actin gene cDNA sequence. As shown in FIG. 14g, the 100bp fragments were present in the positive control (human kidney cDNA as templates), DNase treated and non-treated exosomes, but absent in the negative control lane (without templates). Accordingly, actin cDNA is present in both the DNase treated and non-treated urinary exosomes.

To test whether most nucleic acids extracted using the method were present within exosomes, the nucleic acids extracted from the DNase treated and non-treated exosomes were dissolved in equal volumes and analyzed using a RNA Pico chip (Agilent Technologies). As shown in FIG. 14h, the concentration of the isolated nucleic acids from the DNase treated sample was 1,131 pg/ul and that from the non-treated sample was 1,378 pg/ul. Thus, more than 80% nucleic acids extracted from urinary exosomes using the above method were from inside exosomes.

To identify the content of urinary exosomes systematically, nucleic acids were extracted from urinary exosomes and submitted to the Broad Institute for sequencing. Approximately 14 million sequence reads were generated, each 76 nucleotides in length. These sequence reads correspond to fragments of DNA/RNA transcripts present within urinary exosomes. Using an extremely strict alignment parameter (100% identity over full length sequence), approximately 15% of the reads were aligned to the human genome. This percentage would likely increase if less stringent alignment criteria was used. A majority of these 15% reads did not align with protein coding genes but rather with non-coding genomic elements such are transposons and various LINE & SINE repeat elements. Notably, for those reads that are not aligned to the human genome, many are aligned to viral sequences. To the extent that the compositions and levels of nucleic acids contained in urinary exosomes change with respect to a disease status, profiles of the nucleic acids could be used according to the present methods as biomarkers for disease diagnosis.

This example demonstrates that the exosome method of analyzing urine exosomes can be used to determine cellular changes in the kidney in diabetes-related kidney disease without having to take a high-risk, invasive renal biopsy. The method provides a new and sensitive diagnostic tool using exosomes for early detection of kidney diseases such as diabetic nephropathy. This will allow immediate intervention and treatment. In sum, the exosome diagnostic method and technology described herein provides a means of much-needed diagnostics for diabetic nephropathy and other diseases which are associated with certain profiles of nucleic acids contained in urinary exosomes.

### Example 12: Prostate cancer diagnosis and urinary exosomes

Prostate cancer is the most common cancer in men today. The risk of prostate cancer is approximately 16%. More than 218,000 men in the United States were diagnosed in 2008. The earlier prostate cancer is detected, the greater are the chances of successful treatment. According to the American Cancer Society, if prostate cancers are found while they are still in the prostate itself or nearby areas, the five-year relative survival rate is over 98%.

One established diagnostic method is carried out by measuring the level of prostate specific antigen (PSA) in the blood, combined with a digital rectal examination. However, both the sensitivity and specificity of the PSA test requires significant improvement. This low specificity results in a high number of false positives, which generate numerous unnecessary and expensive biopsies. Other diagnostic methods are carried out by detecting the genetic profiles of newly identified biomarkers including, but not limited to, prostate cancer gene 3 (PCA3) (Groskopf et al., 2006; Nakanishi et al., 2008), a fusion gene between transmembrane protease serine 2 and ETS-related gene (TMPRSS2-ERG) (Tomlins et al., 2005), glutathione S-transferase pi (Goessl et al., 2000; Gonzalgo et al., 2004), and alpha-methylacyl CoA racemase (AMACR) (Zehentner et al., 2006; Zielie et al., 2004) in prostate cancer cells found in bodily fluids such as serum and urine (Groskopf et al., 2006; Wright and Lange, 2007). Although these biomarkers may give increased specificity due to overexpression in prostate cancer cells (e.g., PCA3 expression is increased 60- to 100-fold in prostate cancer cells), a digital rectal examination is required to milk prostate cells into the urine just before specimen collection (Nakanishi et al., 2008). Such rectal examinations have inherent disadvantages such as the bias on collecting those cancer cells that are easily milked into urine and the involvement of medical doctors which is costly and time consuming.

Here, a new method of detecting the genetic profiles of these biomarkers is proposed to overcome the limitation mentioned above. The method comprises the steps of isolating exosomes from a bodily fluid and analyzing the nucleic acid from said exosomes. The procedures of the method are similar to those detailed in Example 9. In this example, the urine samples were from four diagnosed prostate cancer patients. As shown in FIG. 15c, the cancer stages were characterized in terms of grade, Gleason stage and PSA levels. In addition, the nucleic acids analyzed by nested-RT-PCR as detailed in Example 7 were TMPRSS2-ERG and PCA3, two of the newly identified biomarkers of prostate cancer. For amplification of TMPRSS2-ERG, the primer pair for the first amplification step was TMPRSS2-ERG F1 (SEQ ID NO: 47) and TMPRSS2-ERG R1 (SEQ ID NO: 48); and the primer pair for the second amplification step was TMPRSS2-ERG F2 (SEQ ID NO: 49) and TMPRSS2-ERG R2 (SEQ ID NO: 50). The expected amplicon is 122 base pairs (bp) and gives two fragments (one is 68 bp, the other is 54 bp) after digestion with the restriction enzyme HaeII. For amplification of PCA3, the primer pair for the first amplification step was PCA3 F1 (SEQ ID NO: 51) and PCA3 R1 (SEQ ID NO: 52); and the primer pair for the second amplification step was PCA3 F2 (SEQ ID NO: 53) and PCA3 R2 (SEQ ID NO: 54). The expected amplicon is 152 bp in length and gives two fragments (one is 90 bp, the other is 62 bp) after digestion with the restriction enzyme Sca1.

As shown in FIG. 15a, in both patient 1 and 2, but not in patient 3 and 4, the expected amplicon of TMPRSS2-ERG could be detected and digested into two fragments of expected sizes. As shown in FIG. 15b, in all four patients, the expected amplicon of PCA3 could be detected and digested into two fragments of expected sizes. Therefore, PCA3 expression could be detected in urine samples from all four patients, while TMPRSS2-ERG expression could only be detected in urine samples from patient 1 and 2 (FIG. 15c). These data, although not conclusive due to the small sample size, demonstrate the applicability of the new method in detecting biomarkers of prostate cancer. Further, the exosome method is not limited to diagnosis but can be employed for prognosis and/or monitoring other medical conditions related to prostate cancer.

### Example 13: Microvesicles in non-invasive prenatal diagnosis

Prenatal diagnosis is now part of established obstetric practice all over the world. Conventional methods of obtaining fetal tissues for genetic analysis includes amniocentesis and chorionic villus sampling, both of which are invasive and confer risk to the unborn fetus. There is a long-felt need in clinical genetics to develop methods of non-invasive diagnosis. One approach that has been investigated extensively is based on the discovery of circulating fetal cells in maternal plasma. However, there are a number of barriers that hinder its application in clinical settings. Such barriers include the scarcity of fetal cells (only 1.2 cells/ml maternal blood), which requires relatively large volume blood samples, and the long half life of residual fetal cells from previous pregnancy, which may cause false positives. Another approach is based on the discovery of fetal DNA in maternal plasma. Sufficient fetal DNA amounts and short clearance time overcome the barriers associated with the fetal cell method. Nevertheless, DNA only confers inheritable genetic and some epigenetic information, both of which may not represent the dynamic gene expression profiles that are linked to fetal medical conditions. The discovery of circulating fetal RNA in maternal plasma (Ng et al., 2003b; Wong et al., 2005) may be the method of choice for non-invasive prenatal diagnosis.

Several studies suggest that fetal RNAs are of high diagnostic value. For example, elevated expression of fetal corticotropin-releasing hormone (CRH) transcript is associated with pre-eclampsia (a clinical condition manifested by hypertension, edema and proteinuria) during pregnancy (Ng et al., 2003a). In addition, the placenta-specific 4 (PLAC4) mRNA in maternal plasma was successfully used in a non-invasive test for aneuploid pregnancy (such as trisomy 21, Down syndrome) (Lo et al., 2007). Furthermore, fetal human chorionic gonadotropin (hCG) transcript in maternal plasma may be a marker of gestational trophoblastic diseases (GTDs), which is a tumorous growth of fetal tissues in a maternal host. Circulating fetal RNAs are mainly of placenta origin (Ng et al., 2003b). These fetal RNAs can be detected as early as the 4th week of gestation and such RNA is cleared rapidly postpartum.

Prenatal diagnosis using circulating fetal RNAs in maternal plasma, nevertheless, has several limitations. The first limitation is that circulating fetal RNA is mixed with circulating maternal RNA and is not effectively separable. Currently, fetal transcripts are identified, based on an assumption, as those that are detected in pregnant women antepartum as well as in their infant s cord blood, yet are significantly reduced or absent in maternal blood within 24 or 36 hours postpartum (Maron et al., 2007). The second limitation is that no method is established to enrich the circulating fetal RNA for enhanced diagnostic sensitivity since it is still unknown how fetal RNA is packaged and released. The way to overcome these limitations may lie in the isolation of microvesicles and the analysis of the fetal RNAs therein.

Several facts suggest that microvesicles, which are shed by eukaryotic cells, are the vehicles for circulating fetal RNAs in maternal plasma. First, circulating RNAs within microvesicles are protected from RNase degradation. Second, circulating fetal RNAs have been shown to remain in the non-cellular fraction of maternal plasma, which is consistent with the notion that microvesicles encompassing these fetal RNAs are able to be filtered through 0.22 um membrane. Third, similar to tumorous tissues which are know to shed microvesicles, placental cells, which are a pseudo-malignant fetal tissue, are most likely capable of shedding microvesicles. Thus, a novel method of non-invasive prenatal diagnosis is comprised of isolating fetal microvesicles from maternal blood plasma and then analyzing the nucleic acids within the microvesicles for any genetic variants associated with certain diseases and/or other medical conditions.

A hypothetical case of non-invasive prenatal diagnosis is as follows: peripheral blood samples are collected from pregnant women and undergo magnetic activated cell sorting (MACS) or other affinity purification to isolate and enrich fetus-specific microvesicles. The microvesicular pellet is resuspended in PBS and used immediately or stored at -20°C for further processing. RNA is extracted from the isolated microvesicles using the Qiagen RNA extraction kit as per the manufacturer s instructions. RNA content is analyzed for the expression level of fetal human chorionic gonadotropin (hCG) transcript. An increased expression level of hCG compared to the standard range points to the development of gestational trophoblastic diseases (GTDs) and entail further the need for clinical treatment for this abnormal growth in the fetus. The sensitivity of microvesicle technology makes it possible to detect the GTDs at a very early stage before any symptomatic manifestation or structural changes become detectable under ultrasonic examination. The standard range of hCG transcript levels may be determined by examining a statistically significant number of circulating fetal RNA samples from normal pregnancies.

This prenatal diagnostic method may be extrapolated to the prenatal diagnosis and/or monitoring of other diseases or medical conditions by examining those transcripts associated with these diseases or medical conditions. For example, extraction and analysis of anaplastic lymphoma kinase (ALK) nucleic acid from microvesicles of fetus origin from maternal blood is a non-invasive prenatal diagnosis of neuroblastoma, which is closely associated with mutations within the kinase domain or elevated expression of *ALK* (Mosse et al., 2008). Accordingly, the microvesicle methods and technology described herein may lead to a new era of much-needed, non-invasive prenatal genetic diagnosis.

### Example 14: Melanoma diagnosis

Melanoma is a malignant tumor of melanocytes (pigment cells) and is found predominantly in skin. It is a serious form of skin cancer and accounts for 75 percent of all deaths associated with skin cancer. Somatic activating mutations (e.g. V600E) of BRAF are the earliest and most common genetic abnormality detected in the genesis of human melanoma. Activated BRAF promotes melanoma cell cycle progression and/or survival.

Currently, the diagnosis of melanoma is made on the basis of physical examination and excisional biopsy. However, a biopsy can sample only a limited number of foci within the lesion and may give false positives or false negatives. The exosome method provides a more accurate means for diagnosing melanoma. As discussed above, the method is comprised of the steps of isolating exosomes from a bodily fluid of a subject and analyzing the nucleic acid from said exosomes.

To determine whether exosomes shed by melanoma cells contain BRAF mRNA, we cultured primary melanoma cells in DMEM media supplemented with exosome-depleted FBS and harvested the exosomes in the media using a similar procedure as detailed in Example 2. The primary cell lines were Yumel and M34. The Yumel cells do not have the V600E mutation in BRAF, while M34 cells have the V600E mutation in BRAF. RNAs were extracted from the exosomes and then analyzed for the presence of BRAF mRNA by RT-PCR. The primers used for PCR amplification were: BRAF forward (SEQ ID NO: 55) and BRAF reverse (SEQ ID NO: 56). The amplicon is 118 base pairs (bp) long and covers the part of BRAF cDNA sequence where the V600E mutation is located. As shown in FIG. 16a, a band of 118 bp was detected in exosomes from primary melanoma cells (Yumel and M34 cells), but not in exosomes from human fibroblast cells or negative controls. The negative detection of a band of 118 bp PCR product is not due to a mistaken RNA extraction since GAPDH transcripts could be detected in exosomes from both melanoma cell and human fibroblast cells (FIG. 16b). The 118 bp PCR products were further sequenced to detect the V600E mutation. As shown in FIGS. 16c and 16d, PCR products from YUMEL cells, as expected, contain wild type BRAF mRNA. In contrast, PCR products from M34 cells, as expected, contain mutant BRAF mRNA with a T-A point mutation, which causes the amino acid Valine (V) to be replaced by Glutamic acid (E) at the amino acid position 600 of the BRAF protein. Furthermore, BRAF mRNA cannot be detected in exosomes from normal human fibroblast cells, suggesting the BRAF mRNA is not contained in exosomes of all tissue origins.

These data suggest that melanoma cells shed exosomes into the blood circulation and thus melanoma can be diagnosed by isolating these exosomes from blood serum and analyzing the nucleic acid therefrom for the presence or absence of mutations (e.g., V600E) in BRAF. The method described above can also be employed to diagnose melanomas that are associated with other BRAF mutations and mutations in other genes. The method can also be employed to diagnose melanomas that are associated with the expression profiles of BRAF and other nucleic acids.

### Example 15: Detection of MMP levels from exosomes to monitor post transplantation conditions.

Organ transplants are usually effective treatments for organ failures. Kidney failure, heart disease, end-stage lung disease and cirrhosis of the liver are all conditions that can be effectively treated by a transplant. However, organ rejections caused by post-transplantation complications are major obstacles for long-term survival of the allograft recipients. For example, in lung transplantations, bronchiolitis obliterans syndrome is a severe complication affecting survival rates. In kidney transplants, chronic allograft nephropathy remains one of the major causes of renal allograft failure. Ischemia-reperfusion injury damages the donor heart after heart transplantation, as well as the donor liver after orthotopic liver transplantation. These post-transplantation complications may be ameliorated once detected at early stages. Therefore, it is essential to monitor post-transplantation conditions in order to alleviate adverse complications.

Alterations in the extracellular matrix contribute to the interstitial remodeling in post-transplantation complications. Matrix metalloproteinases (MMPs) are involved in both the turnover and degradation of extracellular matrix (ECM) proteins. MMPs are a family of proteolytic, zinc-dependent enzymes, with 27 members described to date, displaying multidomain structures and substrate specificities, and functioning in the processing, activation, or deactivation of a variety of soluble factors. Serum MMP levels may indicate the status of post-transplantation conditions. Indeed, circulating MMP-2 is associated with cystatin C, post-transplant duration, and diabetes mellitus in kidney transplant recipients (Chang et al., 2008). Disproportional expression of MMP-9 is linked to the development of bronchiolitis obliterans syndrome after lung transplantation (Hubner et al., 2005).

MMP mRNAs (MMP1, 8, 12, 15, 20, 21, 24, 26 and 27) can be detected in exosomes shed by glioblastoma cells as shown in Example 4 and Table 10. The present exosome method, isolating exosomes from a bodily fluid and analyzing nucleic acids from said exosomes, can be used to monitor transplantation conditions. The exosome isolation procedure is similar to that detailed in Example 2. The present procedures to analyze nucleic acid contained within exosomes are detailed in Example 9. A significant increase in the expression level of MMP-2 after kidney transplantation will indicate the onset and/or deterioration of post-transplantation complications. Similarly, a significantly elevated level of MMP-9 after lung transplantation, suggests the onset and/or deterioration of bronchiolitis obliterans syndrome.

Therefore, the exosome method can be used to monitor post-transplantation conditions by determining the expression levels of MMP proteins associated with post-transplantation complications. It is also expected that the method can be extrapolated to monitor post-transplantation conditions by determining the expression of other marker genes as well as monitor other medical conditions by determining the genetic profile of nucleic acids associated with these medical conditions.

### Examples 16-18. Microvesicles can be therapeutic agents or delivery vehicles of therapeutic agents.

### Example 16: Microvesicle proteins induce angiogenesis in vitro.

A study was designed and carried out to demonstrate glioblastoma microvesicles contribute to angiogenesis. HBMVECs (30,000 cells), a brain endothelial cell line, (Cell Systems, Catalogue #ACBRI-376, Kirkland, WA, USA) were cultured on Matrigel-coated wells in a 24-well plate in basal medium only (EBM) (Lonza Biologics Inc., Portsmouth, NH, USA), basal medium supplemented with glioblastoma microvesicles (EBM+ MV) (7 µg/well), or basal medium supplemented with a cocktail of angiogenic factors (EGM; hydrocortisone, EGF, FGF, VEGF, IGF, ascorbic acid, FBS, and heparin; Singlequots (EBM positive control). Tubule formation was measured after 16 hours and analyzed with the Image J software. HBMVECs cultured in the presence of glioblastoma microvesicles demonstrated a doubling of tubule length within 1 6 hours. The result was comparable to the result obtained with HBMCECs cultured in the presence of angiogenic factors (FIG. 18a). These results show that glioblastoma-derived microvesicles play a role in initiating angiogenesis in brain endothelial cells.

Levels of angiogenic proteins in microvesicles were also analyzed and compared with levels in glioblastoma donor cells. Using a human angiogenesis antibody array, we were able to detect 19 proteins involved in angiogenesis. Specifically, total protein from either primary glioblastoma cells or purified microvesicles isolated from said cells were lysed in lysis buffer (Promega, Madison, WI, USA) and added to the human angiogenesis antibody array (Panomics, Fremont CA, USA) according to manufacturer s recommendations. The arrays were scanned and analyzed with the Image J software. As shown in FIG. 18b, of the seven of the 19 angiogenic proteins were readily detected in the microvesicles, 6 (angiogenin, IL-6, lL-8, TIMP-I, VEGF and TIMP-2) were present at higher levels on a total protein basis as compared to the glioblastoma cells (FIG. 18c). The three angiogenic proteins most enriched in microvesicles compared to tumor cells were angiogenin, IL-6 and 1L-8, all of which have been implicated in glioma angiogenesis with higher levels associated with increased malignancy (25-27).

Microvesicles isolated from primary glioblastoma cells were also found to promote proliferation of a human U87 glioma cell line. In these studies, 100 000 U87 cells were seeded in wells of a 24-well plate and allowed to grow for three days (DMEM-5%FBS) or DMEM-5%FBS supplemented with 125 µg microvesicles isolated from primary glioblastoma cells. After three days, untreated U87 cells (FIG. 19a) were found to be fewer in number as determined using a Burker chamber, than those supplemented with microvesicles (FIG. 19b). Both non-supplemented and supplemented U87 cells had increased 5-and 8-fold in number over this period, respectively (FIG. 19c). Thus, glioblastoma microvesicles appear to stimulate proliferation of other glioma cells.

### Example 17: Glioblastoma microvesicles are taken up by HBMVECs.

To demonstrate that glioblastoma microvesicles are able to be taken up by human brain microvesicular endothelial cells (HBMVECs), purified glioblastoma microvesicles were labeled with PKH67 Green Fluorescent labeling kit (Sigma-Aldrich, St Louis, MO, USA). The labeled microvesicles were incubated with HBMVEC in culture (5 µg/50,000 cells) for 20 min at 4°C. The cells were washed and incubated at 37°C for 1 hour. Within 30 min the PKH67-labeled microvesicles were internalized into endosome-like structures within the HBMVECs (FIG. 17a). These results show that glioblastoma microvesicles can be internalized by brain endothelial cells.

Similar results were obtained when adding the fluorescently labeled microvesicles to primary glioblastoma cells.

### Example 18: mRNA delivered by glioblastoma microvesicles can be translated in recipient cells.

To determine whether glioblastoma-derived microvesicles mRNA could be delivered to and expressed in recipient cells, primary human glioblastoma cells were infected with a self-inactivating lentivirus vector expressing secreted Gaussia luciferase (Gluc) using a CMV promoter at an infection efficiency of >95%. The cells were stably transduced and generated microvesicles during the subsequent passages (2-10 passages were analyzed). Microvesicles were isolated from the cells and purified as described above. RT-PCR analysis showed that the mRNA for Gluc (555 bp) as well as GAPDH (226 bp) were present in the microvesicles (FIG. 17b). The level of *Gluc* mRNA was even higher than that for GAPDH as evaluated with quantitative RT-PCR.

Fifty micrograms of the purified microvesicles were added to 50,000 HBMVE cells and incubated for 24 hrs. The Gluc activity in the supernatant was measured directly after microvesicle addition (0 hrs), and after 15 hrs and 24 hrs. The Gluc activity in the supernatant was normalized to the Gluc protein activity associated with the microvesicles. The results are presented as the mean ± SEM (n=4). Specifically, the activity in the recipient HBMVE cells demonstrated a continual translation of the microvesicular *Gluc* mRNA. Thus, mRNA incorporated into the tumor microvesicles can be delivered into recipient cells and generate a functional protein.

The statistical analyses in all examples were performed using the Student's t-test.

### References

1. Abravaya, K., J.J. Carrino, S. Muldoon, and H.H. Lee. 1995. Detection of point mutations with a modified ligase chain reaction (Gap-LCR). Nucleic Acids Res. 23 :675-82.
2. Al-Nedawi, K., B. Meehan, J. Micallef, V. Lhotak, L. May, A. Guha, and J. Rak. 2008. Intercellular transfer of the oncogenic receptor EGFRvIII by microvesicles derived from tumour cells. Nat Cell Biol. 10:619-24.
3. Baj-Krzyworzeka, M., R. Szatanek, K. Weglarczyk, J. Baran, B. Urbanowicz, P. Branski, M.Z. Ratajczak, and M. Zembala. 2006. Tumour-derived microvesicles carry several surface determinants and mRNA of tumour cells and transfer some of these determinants to monocytes. Cancer Immunol Immunother. 55:808-18.
4. Balzar, M., M.J. Winter, C.J. de Boer, and S.V. Litvinov. 1999. The biology of the 17-1A antigen (Ep-CAM). J Mol Med. 77:699-712.
5. Booth, A.M., Y. Fang, J.K. Fallon, J.M. Yang, J.E. Hildreth, and S.J. Gould. 2006. Exosomes and HIV Gag bud from endosome-like domains of the T cell plasma membrane. J Cell Biol. 172:923-35.
6. Bossi, A., F. Bonini, A.P. Turner, and S.A. Piletsky. 2007. Molecularly imprinted polymers for the recognition of proteins: the state of the art. Biosens Bioelectron. 22:1131-7.
7. Brookes, M.J., N.K. Sharma, C. Tselepis, and T.H. Iqbal. 2005. Serum pro-hepcidin: measuring active hepcidin or a non-functional precursor? Gut. 54:169-70.
8. Carmeliet, P., and R.K. Jain. 2000. Angiogenesis in cancer and other diseases. Nature. 407:249-57.
9. Carpenter, G. 1987. Receptors for epidermal growth factor and other polypeptide mitogens. Annu Rev Biochem. 56:881-914.
10. Chang, H.R., W.H. Kuo, Y.S. Hsieh, S.F. Yang, C.C. Lin, M.L. Lee, J.D. Lian, and S.C. Chu. 2008. Circulating matrix metalloproteinase-2 is associated with cystatin C level, posttransplant duration, and diabetes mellitus in kidney transplant recipients. Transl Res. 151:217-23.
11. Chaput, N., J. Taieb, F. Andre, and L. Zitvogel. 2005. The potential of exosomes in immunotherapy. Expert Opin Biol Ther. 5:737-47.
12. Cheruvanky, A., H. Zhou, T. Pisitkun, J.B. Kopp, M.A. Knepper, P.S. Yuen, and R.A. Star. 2007. Rapid isolation of urinary exosomal biomarkers using a nanomembrane ultrafiltration concentrator. Am JPhysiol Renal Physiol. 292:F1657-61.
13. Clayton, A., J.P. Mitchell, J. Court, M.D. Mason, and Z. Tabi. 2007. Human tumor-derived exosomes selectively impair lymphocyte responses to interleukin-2. Cancer Res. 67:7458-66.
14. Cotton, R.G., N.R. Rodrigues, and R.D. Campbell. 1988. Reactivity of cytosine and thymine in single-base-pair mismatches with hydroxylamine and osmium tetroxide and its application to the study of mutations. Proc Natl Acad Sci U S A. 85:4397-401.
15. Delves, G.H., A.B. Stewart, A.J. Cooper, and B.A. Lwaleed. 2007. Prostasomes, angiogenesis, and tissue factor. Semin Thromb Hemost. 33:75-9.
16. Diehl, F., K. Schmidt, M.A. Choti, K. Romans, S. Goodman, M. Li, K. Thornton, N. Agrawal, L. Sokoll, S.A. Szabo, K.W. Kinzler, B. Vogelstein, and L.A. Diaz, Jr. 2008. Circulating mutant DNA to assess tumor dynamics. Nat Med. 14:985-90.
17. Fiorentino, F., M.C. Magli, D. Podini, A.P. Ferraretti, A. Nuccitelli, N. Vitale, M. Baldi, and L. Gianaroli. 2003. The minisequencing method: an alternative strategy for preimplantation genetic diagnosis of single gene disorders. Mol Hum Reprod. 9:399-410.
18. Fischer, S.G., and L.S. Lerman. 1979a. Length-independent separation of DNA restriction fragments in two-dimensional gel electrophoresis. Cell. 16:191-200.
19. Fischer, S.G., and L.S. Lerman. 1979b. Two-dimensional electrophoretic separation of restriction enzyme fragments of DNA. Methods Enzymol. 68:183-91.
20. Furnari, F.B., T. Fenton, R.M. Bachoo, A. Mukasa, J.M. Stommel, A. Stegh, W.C. Hahn, K.L. Ligon, D.N. Louis, C. Brennan, L. Chin, R.A. DePinho, and W.K. Cavenee. 2007. Malignant astrocytic glioma: genetics, biology, and paths to treatment. Genes Dev. 21:2683-710.
21. Gabrilovich, D.I. 2007. Molecular mechanisms and therapeutic reversal of immune suppression in cancer. Curr Cancer Drug Targets. 7:1.
22. Geiss, G.K., R.E. Bumgarner, B. Birditt, T. Dahl, N. Dowidar, D.L. Dunaway, H.P. Fell, S. Ferree, R.D. George, T. Grogan, J.J. James, M. Maysuria, J.D. Mitton, P. Oliveri, J.L. Osborn, T. Peng, A.L. Ratcliffe, P.J. Webster, E.H. Davidson, and L. Hood. 2008. Direct multiplexed measurement of gene expression with color-coded probe pairs. Nat Biotechnol. 26:317-25.
23. Goessl, C., H. Krause, M. Muller, R. Heicappell, M. Schrader, J. Sachsinger, and K. Miller. 2000. Fluorescent methylation-specific polymerase chain reaction for DNA-based detection of prostate cancer in bodily fluids. Cancer Res. 60:5941-5.
24. Gonzalgo, M.L., M. Nakayama, S.M. Lee, A.M. De Marzo, and W.G. Nelson. 2004. Detection of GSTP1 methylation in prostatic secretions using combinatorial MSP analysis. Urology. 63:414-8.
25. Gormally, E., E. Caboux, P. Vineis, and P. Hainaut. 2007. Circulating free DNA in plasma or serum as biomarker of carcinogenesis: practical aspects and biological significance. Mutat Res. 635:105-17.
26. Greco, V., M. Hannus, and S. Eaton. 2001. Argosomes: a potential vehicle for the spread of morphogens through epithelia. Cell. 106:633-45.
27. Groskopf, J., S.M. Aubin, I.L. Deras, A. Blase, S. Bodrug, C. Clark, S. Brentano, J. Mathis, J. Pham, T. Meyer, M. Cass, P. Hodge, M.L. Macairan, L.S. Marks, and H. Rittenhouse. 2006. APTIMA PCA3 molecular urine test: development of a method to aid in the diagnosis of prostate cancer. Clin Chem. 52:1089-95.
28. Guatelli, J.C., K.M. Whitfield, D.Y. Kwoh, K.J. Barringer, D.D. Richman, and T.R. Gingeras. 1990. Isothermal, in vitro amplification of nucleic acids by a multienzyme reaction modeled after retroviral replication. Proc Natl Acad Sci U S A. 87:1874-8.
29. Hahn, P.J. 1993. Molecular biology of double-minute chromosomes. Bioessays. 15:477-84.
30. Hubner, R.H., S. Meffert, U. Mundt, H. Bottcher, S. Freitag, N.E. El Mokhtari, T. Pufe, S. Hirt, U.R. Folsch, and B. Bewig. 2005. Matrix metalloproteinase-9 in bronchiolitis obliterans syndrome after lung transplantation. Eur Respir J. 25:494-501.
31. Janowska-Wieczorek, A., M. Wysoczynski, J. Kijowski, L. Marquez-Curtis, B. Machalinski, J. Ratajczak, and M.Z. Ratajczak. 2005. Microvesicles derived from activated platelets induce metastasis and angiogenesis in lung cancer. Int J Cancer. 113:752-60.
32. Johnson, S., D. Evans, S. Laurenson, D. Paul, A.G. Davies, P.K. Ferrigno, and C. Walti. 2008. Surface-immobilized peptide aptamers as probe molecules for protein detection. Anal Chem. 80:978-83.
33. Jones, S., X. Zhang, D.W. Parsons, J.C. Lin, R.J. Leary, P. Angenendt, P. Mankoo, H. Carter, H. Kamiyama, A. Jimeno, S.M. Hong, B. Fu, M.T. Lin, E.S. Calhoun, M. Kamiyama, K. Walter, T. Nikolskaya, Y. Nikolsky, J. Hartigan, D.R. Smith, M. Hidalgo, S.D. Leach, A.P. Klein, E.M. Jaffee, M. Goggins, A. Maitra, C. Iacobuzio-Donahue, J.R. Eshleman, S.E. Kern, R.H. Hruban, R. Karchin, N. Papadopoulos, G. Parmigiani, B. Vogelstein, V.E. Velculescu, and K.W. Kinzler. 2008. Core Signaling Pathways in Human Pancreatic Cancers Revealed by Global Genomic Analyses. Science*.*
34. Kan, Y.W., and A.M. Dozy. 1978a. Antenatal diagnosis of sickle-cell anaemia by D.N.A. analysis of amniotic-fluid cells. Lancet. 2:910-2.
35. Kan, Y.W., and A.M. Dozy. 1978b. Polymorphism of DNA sequence adjacent to human beta-globin structural gene: relationship to sickle mutation. Proc Natl Acad Sci USA. 75:5631-5.
36. Keller, S., C. Rupp, A. Stoeck, S. Runz, M. Fogel, S. Lugert, H.D. Hager, M.S. Abdel-Bakky, P. Gutwein, and P. Altevogt. 2007. CD24 is a marker of exosomes secreted into urine and amniotic fluid. Kidney Int. 72:1095-102.
37. Kemna, E., P. Pickkers, E. Nemeth, H. van der Hoeven, and D. Swinkels. 2005. Time-course analysis of hepcidin, serum iron, and plasma cytokine levels in humans injected with LPS. Blood. 106:1864-6.
38. Kemna, E.H., H. Tjalsma, V.N. Podust, and D.W. Swinkels. 2007. Mass spectrometry-based hepcidin measurements in serum and urine: analytical aspects and clinical implications. Clin Chem. 53:620-8.
39. Kemna, E.H., H. Tjalsma, H.L. Willems, and D.W. Swinkels. 2008. Hepcidin: from discovery to differential diagnosis. Haematologica. 93:90-7.
40. Kislauskis, E.H., X. Zhu, and R.H. Singer. 1994. Sequences responsible for intracellular localization of beta-actin messenger RNA also affect cell phenotype. J Cell Biol. 127:441-51.
41. Kwoh, D.Y., G.R. Davis, K.M. Whitfield, H.L. Chappelle, L.J. DiMichele, and T.R. Gingeras. 1989. Transcription-based amplification system and detection of amplified human immunodeficiency virus type 1 with a bead-based sandwich hybridization format. Proc Natl Acad Sci USA. 86:1173-7.
42. Landegren, U., R. Kaiser, J. Sanders, and L. Hood. 1988. A ligase-mediated gene detection technique. Science. 241:1077-80.
43. Li, J., L. Wang, H. Mamon, M.H. Kulke, R. Berbeco, and G.M. Makrigiorgos. 2008. Replacing PCR with COLD-PCR enriches variant DNA sequences and redefines the sensitivity of genetic testing. Nat Med. 14:579-84.
44. Liu, C., S. Yu, K. Zinn, J. Wang, L. Zhang, Y. Jia, J.C. Kappes, S. Barnes, R.P. Kimberly, W.E. Grizzle, and H.G. Zhang. 2006a. Murine mammary carcinoma exosomes promote tumor growth by suppression of NK cell function. J Immunol. 176:1375-85.
45. Liu, Q., J.C. Greimann, and C.D. Lima. 2006b. Reconstitution, activities, and structure of the eukaryotic RNA exosome. Cell. 127:1223-37.
46. Lo, Y.M., N.B. Tsui, R.W. Chiu, T.K. Lau, T.N. Leung, M.M. Heung, A. Gerovassili, Y. Jin, K.H. Nicolaides, C.R. Cantor, and C. Ding. 2007. Plasma placental RNA allelic ratio permits noninvasive prenatal chromosomal aneuploidy detection. Nat Med. 13:218-23.
47. Louis, D.N., H. Ohgaki, O.D. Wiestler, W.K. Cavenee, P.C. Burger, A. Jouvet, B.W. Scheithauer, and P. Kleihues. 2007. The 2007 WHO classification of tumours of the central nervous system. Acta Neuropathol. 114:97-109.
48. Mack, M., A. Kleinschmidt, H. Bruhl, C. Klier, P.J. Nelson, J. Cihak, J. Plachy, M. Stangassinger, V. Erfle, and D. Schlondorff. 2000. Transfer of the chemokine receptor CCR5 between cells by membrane-derived microparticles: a mechanism for cellular human immunodeficiency virus 1 infection. Nat Med. 6:769-75.
49. Mallardo, M., A. Deitinghoff, J. Muller, B. Goetze, P. Macchi, C. Peters, and M.A. Kiebler. 2003. Isolation and characterization of Staufen-containing ribonucleoprotein particles from rat brain. Proc Natl Acad Sci USA. 100:2100-5.
50. Maron, J.L., K.L. Johnson, D. Slonim, C.Q. Lai, M. Ramoni, G. Alterovitz, Z. Jarrah, Z. Yang, and D.W. Bianchi. 2007. Gene expression analysis in pregnant women and their infants identifies unique fetal biomarkers that circulate in maternal blood. J Clin Invest. 117:3007-19.
51. Mazzocca, A., R. Coppari, R. De Franco, J.Y. Cho, T.A. Libermann, M. Pinzani, and A. Toker. 2005. A secreted form of ADAM9 promotes carcinoma invasion through tumor-stromal interactions. Cancer Res. 65:4728-38.
52. McLendon, R., A. Friedman, D. Bigner, E.G. Van Meir, D.J. Brat, G. Marie Mastrogianakis, J.J. Olson, T. Mikkelsen, N. Lehman, K. Aldape, W.K. Alfred Yung, O. Bogler, S. Vandenberg, M. Berger, M. Prados, D. Muzny, M. Morgan, S. Scherer, A. Sabo, L. Nazareth, L. Lewis, O. Hall, Y. Zhu, Y. Ren, O. Alvi, J. Yao, A. Hawes, S. Jhangiani, G. Fowler, A. San Lucas, C. Kovar, A. Cree, H. Dinh, J. Santibanez, V. Joshi, M.L. Gonzalez-Garay, C.A. Miller, A. Milosavljevic, L. Donehower, D.A. Wheeler, R.A. Gibbs, K. Cibulskis, C. Sougnez, T. Fennell, S. Mahan, J. Wilkinson, L. Ziaugra, R. Onofrio, T. Bloom, R. Nicol, K. Ardlie, J. Baldwin, S. Gabriel, E.S. Lander, L. Ding, R.S. Fulton, M.D. McLellan, J. Wallis, D.E. Larson, X. Shi, R. Abbott, L. Fulton, K. Chen, D.C. Koboldt, M.C. Wendl, R. Meyer, Y. Tang, L. Lin, J.R. Osborne, B.H. Dunford-Shore, T.L. Miner, K. Delehaunty, C. Markovic, G. Swift, W. Courtney, C. Pohl, S. Abbott, A. Hawkins, S. Leong, C. Haipek, H. Schmidt, M. Wiechert, T. Vickery, S. Scott, D.J. Dooling, A. Chinwalla, G.M. Weinstock, E.R. Mardis, R.K. Wilson, G. Getz, W. Winckler, R.G. Verhaak, M.S. Lawrence, M. O'Kelly, J. Robinson, G. Alexe, R. Beroukhim, S. Carter, D. Chiang, J. Gould, et al. 2008. Comprehensive genomic characterization defines human glioblastoma genes and core pathways. Nature*.*
53. Mellinghoff, I.K., M.Y. Wang, I. Vivanco, D.A. Haas-Kogan, S. Zhu, E.Q. Dia, K.V. Lu, K. Yoshimoto, J.H. Huang, D.J. Chute, B.L. Riggs, S. Horvath, L.M. Liau, W.K. Cavenee, P.N. Rao, R. Beroukhim, T.C. Peck, J.C. Lee, W.R. Sellers, D. Stokoe, M. Prados, T.F. Cloughesy, C.L. Sawyers, andP.S. Mischel. 2005. Molecular determinants of the response of glioblastomas to EGFR kinase inhibitors. N Engl J Med. 353:2012-24.
54. Miele, E.A., D.R. Mills, and F.R. Kramer. 1983. Autocatalytic replication of a recombinant RNA. J Mol Biol. 171:281-95.
55. Millimaggi, D., M. Mari, S. D'Ascenzo, E. Carosa, E.A. Jannini, S. Zucker, G. Carta, A. Pavan, and V. Dolo. 2007. Tumor vesicle-associated CD147 modulates the angiogenic capability of endothelial cells. Neoplasia. 9:349-57.
56. Mosse, Y.P., M. Laudenslager, L. Longo, K.A. Cole, A. Wood, E.F. Attiyeh, M.J. Laquaglia, R. Sennett, J.E. Lynch, P. Perri, G. Laureys, F. Speleman, C. Kim, C. Hou, H. Hakonarson, A. Torkamani, N.J. Schork, G.M. Brodeur, G.P. Tonini, E. Rappaport, M. Devoto, and J.M. Maris. 2008. Identification of ALK as a major familial neuroblastoma predisposition gene. Nature*.*
57. Muerkoster, S., K. Wegehenkel, A. Arlt, M. Witt, B. Sipos, M.L. Kruse, T. Sebens, G. Kloppel, H. Kalthoff, U.R. Folsch, and H. Schafer. 2004. Tumor stroma interactions induce chemoresistance in pancreatic ductal carcinoma cells involving increased secretion and paracrine effects of nitric oxide and interleukin-lbeta. Cancer Res. 64:1331-7.
58. Myers, R.M., Z. Larin, and T. Maniatis. 1985. Detection of single base substitutions by ribonuclease cleavage at mismatches in RNA:DNA duplexes. Science. 230:1242-6.
59. Nagrath, S., L.V. Sequist, S. Maheswaran, D.W. Bell, D. Irimia, L. Ulkus, M.R. Smith, E.L. Kwak, S. Digumarthy, A. Muzikansky, P. Ryan, U.J. Balis, R.G. Tompkins, D.A. Haber, and M. Toner. 2007. Isolation of rare circulating tumour cells in cancer patients by microchip technology. Nature. 450:1235-9.
60. Nakanishi, H., J. Groskopf, H.A. Fritsche, V. Bhadkamkar, A. Blase, S.V. Kumar, J.W. Davis, P. Troncoso, H. Rittenhouse, and R.J. Babaian. 2008. PCA3 molecular urine assay correlates with prostate cancer tumor volume: implication in selecting candidates for active surveillance. J Urol. 179:1804-9; discussion 1809-10.
61. Nakazawa, H., D. English, P.L. Randell, K. Nakazawa, N. Martel, B.K. Armstrong, and H. Yamasaki. 1994. UV and skin cancer: specific p53 gene mutation in normal skin as a biologically relevant exposure measurement. Proc Natl Acad Sci U S A. 91:360-4.
62. Ng, E.K., T.N. Leung, N.B. Tsui, T.K. Lau, N.S. Panesar, R.W. Chiu, and Y.M. Lo. 2003a. The concentration of circulating corticotropin-releasing hormone mRNA in maternal plasma is increased in preeclampsia. Clin Chem. 49:727-31.
63. Ng, E.K., N.B. Tsui, T.K. Lau, T.N. Leung, R.W. Chiu, N.S. Panesar, L.C. Lit, K.W. Chan, and Y.M. Lo. 2003b. mRNA of placental origin is readily detectable in maternal plasma. Proc Natl Acad Sci USA. 100:4748-53.
64. Nishikawa, R., T. Sugiyama, Y. Narita, F. Furnari, W.K. Cavenee, and M. Matsutani. 2004. Immunohistochemical analysis of the mutant epidermal growth factor, deltaEGFR, in glioblastoma. Brain Tumor Pathol. 21:53-6.
65. Orita, M., H. Iwahana, H. Kanazawa, K. Hayashi, and T. Sekiya. 1989. Detection of polymorphisms of human DNA by gel electrophoresis as single-strand conformation polymorphisms. Proc Natl Acad Sci USA. 86:2766-70.
66. Pan, B.T., and R.M. Johnstone. 1983. Fate of the transferrin receptor during maturation of sheep reticulocytes in vitro: selective externalization of the receptor. Cell. 33:967-78.
67. Parsons, D.W., S. Jones, X. Zhang, J.C. Lin, R.J. Leary, P. Angenendt, P. Mankoo, H. Carter, I.M. Siu, G.L. Gallia, A. Olivi, R. McLendon, B.A. Rasheed, S. Keir, T. Nikolskaya, Y. Nikolsky, D.A. Busam, H. Tekleab, L.A. Diaz, Jr., J. Hartigan, D.R. Smith, R.L. Strausberg, S.K. Marie, S.M. Shinjo, H. Yan, G.J. Riggins, D.D. Bigner, R. Karchin, N. Papadopoulos, G. Parmigiani, B. Vogelstein, V.E. Velculescu, and K.W. Kinzler. 2008. An Integrated Genomic Analysis of Human Glioblastoma Multiforme. Science*.*
68. Pelloski, C.E., K.V. Ballman, A.F. Furth, L. Zhang, E. Lin, E.P. Sulman, K. Bhat, J.M. McDonald, W.K. Yung, H. Colman, S.Y. Woo, A.B. Heimberger, D. Suki, M.D. Prados, S.M. Chang, F.G. Barker, 2nd, J.C. Buckner, C.D. James, and K. Aldape. 2007. Epidermal growth factor receptor variant III status defines clinically distinct subtypes of glioblastoma. J Clin Oncol. 25:2288-94.
69. Raposo, G., H.W. Nijman, W. Stoorvogel, R. Liejendekker, C.V. Harding, C.J. Melief, and H.J. Geuze. 1996. B lymphocytes secrete antigen-presenting vesicles. J Exp Med. 183:1161-72.
70. Roe, M.A., C. Spinks, A.L. Heath, L.J. Harvey, R. Foxall, J. Wimperis, C. Wolf, and S.J. Fairweather-Tait. 2007. Serum prohepcidin concentration: no association with iron absorption in healthy men; and no relationship with iron status in men carrying HFE mutations, hereditary haemochromatosis patients undergoing phlebotomy treatment, or pregnant women. Br JNutr. 97:544-9.
71. Schetter, A.J., S.Y. Leung, J.J. Sohn, K.A. Zanetti, E.D. Bowman, N. Yanaihara, S.T. Yuen, T.L. Chan, D.L. Kwong, G.K. Au, C.G. Liu, G.A. Calin, C.M. Croce, and C.C. Harris. 2008. MicroRNA expression profiles associated with prognosis and therapeutic outcome in colon adenocarcinoma. JAMA. 299:425-36.
72. Singer, C.F., D. Gschwantler-Kaulich, A. Fink-Retter, C. Haas, G. Hudelist, K. Czerwenka, and E. Kubista. 2007. Differential gene expression profile in breast cancer-derived stromal fibroblasts. Breast Cancer Res Treat.
73. Steemers, F.J., W. Chang, G. Lee, D.L. Barker, R. Shen, and K.L. Gunderson. 2006. Whole-genome genotyping with the single-base extension assay. Nat Methods. 3:31-3.
74. Stupp, R., W.P. Mason, M.J. van den Bent, M. Weller, B. Fisher, M.J. Taphoorn, K. Belanger, A.A. Brandes, C. Marosi, U. Bogdahn, J. Curschmann, R.C. Janzer, S.K. Ludwin, T. Gorlia, A. Allgeier, D. Lacombe, J.G. Cairncross, E. Eisenhauer, and R.O. Mirimanoff. 2005. Radiotherapy plus concomitant and adjuvant temozolomide for glioblastoma. N Engl J Med. 352:987-96.
75. Taylor, D.D., and C. Gercel-Taylor. 2008. MicroRNA signatures of tumor-derived exosomes as diagnostic biomarkers of ovarian cancer. Gynecol Oncol. 110:13-21.
76. Thery, C., S. Amigorena, G. Raposo, and A. Clayton. 2006. Isolation and characterization of exosomes from cell culture supernatants and biological fluids. Curr Protoc Cell Biol. Chapter 3 :Unit 3 22.
77. Thery, C., L. Zitvogel, and S. Amigorena. 2002. Exosomes: composition, biogenesis and function. Nat Rev Immunol. 2:569-79.
78. Tomlins, S.A., D.R. Rhodes, S. Perner, S.M. Dhanasekaran, R. Mehra, X.W. Sun, S. Varambally, X. Cao, J. Tchinda, R. Kuefer, C. Lee, J.E. Montie, R.B. Shah, K.J. Pienta, M.A. Rubin, and A.M. Chinnaiyan. 2005. Recurrent fusion of TMPRSS2 and ETS transcription factor genes in prostate cancer. Science. 310:644-8.
79. Valadi, H., K. Ekstrom, A. Bossios, M. Sjostrand, J.J. Lee, and J.O. Lotvall. 2007. Exosome-mediated transfer of mRNAs and microRNAs is a novel mechanism of genetic exchange between cells. Nat Cell Biol. 9:654-9.
80. van Dijk, E.L., G. Schilders, and G.J. Pruijn. 2007. Human cell growth requires a functional cytoplasmic exosome, which is involved in various mRNA decay pathways. RNA. 13:1027-35.
81. Velculescu, V.E., L. Zhang, B. Vogelstein, and K.W. Kinzler. 1995. Serial analysis of gene expression. Science. 270:484-7.
82. Weiss, G., and L.T. Goodnough. 2005. Anemia of chronic disease. N Engl J Med. 352:1011-23.
83. Went, P.T., A. Lugli, S. Meier, M. Bundi, M. Mirlacher, G. Sauter, and S. Dirnhofer. 2004. Frequent EpCam protein expression in human carcinomas. Hum Pathol. 35:122-8.
84. Wieckowski, E., and T.L. Whiteside. 2006. Human tumor-derived vs dendritic cell-derived exosomes have distinct biologic roles and molecular profiles. Immunol Res. 36:247-54.
85. Wong, B.C., R.W. Chiu, N.B. Tsui, K.C. Chan, L.W. Chan, T.K. Lau, T.N. Leung, and Y.M. Lo. 2005. Circulating placental RNA in maternal plasma is associated with a preponderance of 5' mRNA fragments: implications for noninvasive prenatal diagnosis and monitoring. Clin Chem. 51:1786-95.
86. Wood, L.D., D.W. Parsons, S. Jones, J. Lin, T. Sjoblom, R.J. Leary, D. Shen, S.M. Boca, T. Barber, J. Ptak, N. Silliman, S. Szabo, Z. Dezso, V. Ustyanksky, T. Nikolskaya, Y. Nikolsky, R. Karchin, P.A. Wilson, J.S. Kaminker, Z. Zhang, R. Croshaw, J. Willis, D. Dawson, M. Shipitsin, J.K. Willson, S. Sukumar, K. Polyak, B.H. Park, C.L. Pethiyagoda, P.V. Pant, D.G. Ballinger, A.B. Sparks, J. Hartigan, D.R. Smith, E. Suh, N. Papadopoulos, P. Buckhaults, S.D. Markowitz, G. Parmigiani, K.W. Kinzler, V.E. Velculescu, and B. Vogelstein. 2007. The genomic landscapes of human breast and colorectal cancers. Science. 318:1108-13.
87. Wright, J.L., and P.H. Lange. 2007. Newer potential biomarkers in prostate cancer. Rev Urol. 9:207-13.
88. Zehentner, B.K., H. Secrist, X. Zhang, D.C. Hayes, R. Ostenson, G. Goodman, J. Xu, M. Kiviat, N. Kiviat, D.H. Persing, and R.L. Houghton. 2006. Detection of alpha-methylacyl-coenzyme-A racemase transcripts in blood and urine samples of prostate cancer patients. Mol Diagn Ther. 10:397-403.
89. Zielie, P.J., J.A. Mobley, R.G. Ebb, Z. Jiang, R.D. Blute, and S.M. Ho. 2004. A novel diagnostic test for prostate cancer emerges from the determination of alpha-methylacyl-coenzyme a racemase in prostatic secretions. J Urol. 172:1130-3.

**Table 1. RNA in glioblastoma microvesicles can be used as sensitive biomarkers.**

| Nested RT-PCR was used to monitor EGFRvIII mRNA in glioma biopsy tissue as well as exosomes purified from a frozen serum sample from the same patient. Samples from 30 patients were analysed in a blinded fashion and PCR reactions were repeated at least three times for each sample. No EGFRvIII mRNA was found in serum microvesicles from 30 normal controls. PP1 refers to primer pair composed of SEQ ID NOs: 13 and 14. PP2 refers to primer pair composed of SEQ ID NOS: 15 and 16. - refers to not available . | | | | | |
|---|---|---|---|---|---|
| **Patient#** | **Time of serum collection^{∗}** | **Serum volume** | **Biopsy EGFRvIII** | **Serum exosome EGFRvIII(PP1)** | **Serum exosome EGFRvIII(PP2)** |
| **1** | 0 | 3 ml | **Yes** | **Yes** | **-** |
| **2** | 0 | 2 ml | **No** | **No** | **-** |
| **3** | 0 | 2.5 ml | **No** | **No** | **-** |
| **4** | 0 | 1 ml | **Yes** | **No** | **Yes** |
| **5** | 0 | 1 ml | **Yes** | **No** | **Yes** |
| **6** | 0 | 1 ml | **No** | **No** | - |
| **7** | 0 | 0.6 ml | **Yes** | **Yes** | - |
| **8** | 0 | 1 ml | **No** | **No** | - |
| **9** | 0 | 1 ml | **Yes** | **Yes** | - |
| **10** | 0 | 1 ml | **No** | **Yes** | - |
| **11** | 0 | 2 ml | **Yes** | **No** | **Yes** |
| **12** | 0 | 2 ml | **Yes** | **Yes** | - |
| **13** | 0 | 2 ml | **No** | **Yes** | - |
| **14** | 0 | 2 ml | **Yes** | **Yes** | - |
| **15** | 0 | 2 ml | **No** | **No** | - |
| **16** | 0 | 2 ml | **No** | **No** | - |
| **17** | 0 | 1 ml | **Yes** | **No** | - |
| **18** | 0 | 0.8 ml | **Yes** | **No** | - |
| **19** | 0 | 1 ml | **No** | **No** | - |
| **20** | 0 | 1 ml | **No** | **No** | - |
| **21** | 0 | 1 ml | **No** | **No** | - |
| **22** | 0 | 1 ml | **No** | **No** | - |
| **23** | 0 | 1 ml | **No** | **No** | - |
| **24** | 0 | 1 ml | **No** | **No** | - |
| **25** | 0 | 1 ml | **No** | **No** | - |
| **26** | **14** | 0.6 ml | **Yes** | **No** | **Yes** |
| **27** | **14** | 1.2 ml | **No** | **No** | **No** |
| **28** | **14** | 0.8 ml | **Yes** | **No** | **Yes** |
| **29** | **14** | 0.9 ml | **Yes** | **No** | **No** |
| **30** | **14** | 0.6 ml | **Yes** | **No** | **Yes** |

| | | | | | |
|---|---|---|---|---|---|
| ^{∗}Days post-surgery of tumor removal | | | | | |

**Table 2 Abbreviations used in Table 3.**

| Abbreviation | Term |
|---|---|
| A | amplification |
| AEL | acute eosinophilic leukemia |
| AL | acute leukemia |
| ALCL | anaplastic large-cell lymphoma |
| ALL | acute lymphocytic leukemia |
| AML | acute myelogenous leukemia |
| AML^{∗} | acute myelogenous leukemia (primarily treatment associated) |
| APL | acute promyelocytic leukemia |
| B-ALL | B-cell acute lymphocyte leukemia |
| B-CLL | B-cell Lymphocytic leukemia |
| B-NHL | B-cell Non-Hodgkin Lymphoma |
| CLL | chronic lymphatic leukemia |
| CML | chronic myeloid leukemia |
| CMML | chronic myelomonocytic leukemia |
| CNS | central nervous system |
| D | large deletion |
| DFSP | dermatfibrosarcoma protuberans |
| DLBL | diffuse large B-cell lymphoma |
| DLCL | diffuse large-cell lymphoma |
| Dom | dominant |
| E | epithelial |
| F | frames |
| GIST | gastrointestinal stromal tumour |
| JMML | juvenile myelomonocytic leukemia |
| L | leukaemia/lymphoma |
| M | mesenchymal |
| MALT | mucosa-associated lymphoid tissue lymphoma |
| MDS | myelodysplastic syndrome |
| Mis | Missense |
| MLCLS | mediastinal large cell lymphoma with sclerosis |
| MM | multiple myeloma |
| MPD | Myeloproliferative disorder |
| N | nonsense |
| NHL | non-Hodgkin lymphoma |
| NK/T | natural killer T cell |
| NSCLC | non small cell lung cancer |
| O | other |
| PMBL | primary mediastinal B-cell lymphoma |
| pre-B All | pre-B-cell acute lymphablastic leukaemia |
| Rec | reccesive |
| S | splice site |
| T | translocation |
| T-ALL | T-cell acute lymphoblastic leukemia |
| T-CLL | T-cell chronic lymphocytic leukaemia |
| TGCT | testicular germ cell tumour |
| T-PLL | T cell prolymphocytic leukaemia |

| **Table 3: Genes Commonly Mutated in Cancers** | | | | | | | | | | |
|---|---|---|---|---|---|---|---|---|---|---|
| **Symbol** | **Locuslink ID** | **Protein ID*** | **Chromosome band** | **Tumour types (somatic)** | **Tumour types (germline)** | **Cancer syndrome** | **Tissue type** | **Cancer molecular genetics** | **Mutation type** | **Translocation partner** |
| *ABL1* | *25* | P00519 | 9q34.1 | CML, ALL | | | L | Dom | T | *BCR, ETV6* |
| *ABL2* | *27* | P42684 | 1q24-q25 | AML | | | L | Dom | T | *ETV6* |
| *AF15Q14* | 57082 | NP_06511 3 | 15q14 | AML | | | L | Dom | T | *MLL* |
| *AF1Q* | 10962 | Q13015 | 1q21 | ALL | | | L | Dom | T | *MLL* |
| *AF3p21* | 51517 | Q9NZQ3 | 3p21 | ALL | | | L | Dom | T | *MLL* |
| *AF5q31* | 27125 | NP_05523 8 | 5q31 | ALL | | | L | Dom | T | *MLL* |
| *AKT2* | 208 | P31751 | 19q13.1-q13.2 | Ovarian, pancreatic | | | E | Dom | A | |
| *ALK* | 238 | Q9UM73 | 2p23 | ALCL | | | L | Dom | T | *NPM1, TPM3, TFG, TPM4, ATIC, CLTC, MSN, ALO17* |
| *ALO17* | 57714 | XP 29076 9 | 17q25.3 | ALCL | | | L | Dom | T | *ALK* |
| *APC* | 324 | P25054 | 5q21 | Colorectal, pancreatic, desmoid, hepatoblastoma, glioma, other CNS | Colorectal, pancreatic, desmoid, hepatoblastoma, glioma, other CNS | Adenomatous polyposis coli; Turcot syndrome | E, M, O | Rec | D , Mis, N, F, S | |
| *ARHGEF1* 2 | 23365 | NP_05612 8 | 11q23.3 | AML | | | L | Dom | T | *MLL* |
| *ARHH* | 399 | Q15669 | 4p13 | NHL | | | L | Dom | T | *BCL6* |
| *ARNT* | 405 | P27540 | 1q21 | AML | | | L | Dom | T | *ETV6* |
| *ASPSCR1* | 79058 | NP_07698 8 | 17q25 | Alveolar soft part sarcoma | | | M | Dom | T | *TFE3* |
| *ATF1* | 466 | P18846 | 12q13 | Malignant melanoma of soft parts, angiomatoid fibrous histiocytoma | | | E, M | Dom | T | *EWSR1* |
| *ATIC* | 471 | P31939 | 2q35 | ALCL | | | L | Dom | T | *ALK* |
| *ATM* | 472 | Q13315 | 11q22.3 | T-PLL | Leukaemia, lymphoma, medulloblastoma, glioma | Ataxia telangiectasia | L, O | Rec | D, Mis, N, F, S | |
| *BCL10* | 8915 | 095999 | 1p22 | MALT | | | L | Dom | T | *IGHa* |
| *BCL11A* | 53335 | NP 06048 4 | 2p13 | B-CLL | | | L | Dom | T | *IGHa* |
| *BCL11B* | 64919 | NP_61280 8 | 14q32.1 | T-ALL | | | L | Dom | T | *TLX3* |
| *BCL2* | 596 | P10415 | 18q21.3 | NHL, CLL | | | L | Dom | T | *IGHa* |
| *BCL3* | 602 | P20749 | 19q13 | CLL | | | L | Dom | T | *IGHa* |
| *BCL5* | 603 | I52586 | 17q22 | CLL | | | L | Dom | T | *MYC* |
| *BCL6* | 604 | P41182 | 3q27 | NHL, CLL | | | L | Dom | T, Mis | *IG* loci, *ZNFN1A1, LCP1, PIM1, TFRC, MHC2TA, NACA, HSPCB, HSPCA, HIST1H4I, IL21R, POU2AF1, ARHH, EIF4A2* |
| *BCL7A* | 605 | NP 06627 3 | 12q24.1 | B-NHL | | | L | Dom | T | *MYC* |
| *BCL9* | 607 | O00512 | 1q21 | B-ALL | | | L | Dom | T | *IGHα, IGLα* |
| *BCR* | 613 | P11274 | 22q11.21 | CML, ALL | | | L | Dom | T | *ABL1, FGFRI* |
| *BHD* | 201163 | NP_65943 4 | 17p11.2 | | Renal, fibrofolliculomas, trichodiscomas | Birt Hogg Dube syndrome | E, M | Rec? | Mis, N, F | |
| *BIRC3* | 330 | Q13489 | 11q22-q23 | MALT | | | L | Dom | T | *MALT1* |
| *BLM* | 641 | P54132 | 15q26.1 | | Leukaemia, lymphoma, skin squamous cell, other cancers | Bloom Syndrome | L, E | Rec | Mis, N, F | |
| *BMPR1A* | 657 | P36894 | 10q22.3 | | Gastrointestinal polyps | Juvenile polyposis | E | Rec | Mis, N, F | |
| *BRAF* | 673 | P15056 | 7q34 | Melanoma, colorectal, papillary thyroid, borderline ovarian, NSCLC, cholangiocarcinoma | | | E | Dom ; | M | |
| *BRCA1* | *672* | P38398 | 17q21 | Ovarian | Breast, ovarian | Hereditary breast/ovarian | E | Rec | D, Mis, N, F, S | |
| *BRCA2* | 675 | P51587 | 13q12 | Breast, ovarian, pancreatic | Breast, ovarian, pancreatic, leukaemia (FANCB, FANCD1) | Hereditary breast/ | L, E ovarian | Rec | D, Mis, N, | *F, S* |
| *BRD4* | 23476 | 060885 | 19p13.1 | Lethal midline carcinoma of young people | | | E | Dom | T | *NUT* |
| *BTG1* | 694 | P31607 | 12q22 | BCLL | | | L | Dom | T | *MYC* |
| *CBFA2T1* | 862 | Q06455 | 8q22 | AML | | | L | Dom | T | *MLL, RUNX1* |
| *CBFA2T3* | 863 | NP_00517 8 | 16q24 | AML | | | L | Dom | T | *RUNX1* |
| *CBFB* | 865 | Q13951 | 16q22 | AML | | | L | Dom | T | *MYH11* |
| *CBL* | 867 | P22681 | 11q23.3 | AML | | | L | Dom | T | *MLL* |
| *CCND1* | 595 | P24385 | 11q13 | CLL, B-ALL, breast | | | L, E | Dom | T | *IGHα, FSTL3* |
| *CDH1* | 999 | P12830 | 16q22.1 | Lobular breast, gastric | Gastric | Familial gastric carcinoma | E | Rec | Mis, N, F, S | |
| *CDK4* | 1019 | P11802 | 12q14 | | Melanoma | Familial malignant melanoma | E | Dom | Mis | |
| *CDKN2A- p14^{ARF}* | 1029 | NP 47810 2 | 9p21 | Melanoma, multiple other | Melanoma, pancreatic | Familial malignant melanoma | L, E, M, O | Rec | D, S | |
| *CDKN2A- p16^{INK4A}* | 1029 | P42771 | 9p21 | Melanoma, multiple other | Melanoma, pancreatic | Familial malignant melanoma | L, E, M, O | Rec | D, Mis, N, F, S | |
| *CDX2* | 1045 | Q99626 | 13q12.3 | AML | | | L | Dom | T | *ETV6* |
| *CEBPA* | 1050 | NP 00435 5 | 11p15.5 | AML, MDS | | | L | Dom | Mis, N, F | |
| *CEP1* | 11064 | NP 00894 9 | 9q33 | MPD/NHL | | | L | Dom | T | *FGFR1* |
| *CHIC2* | 26511 | NP 03624 2 | 4q11-q12 | AML | | | L | Dom | T | *ETV6* |
| *CHN1* | 1123 | P15882 | 2q31-q32.1 | Extraskeletal myxoid chondrosarcoma | | | M | Dom | T | *TAF15* |
| *CLTC* | 1213 | Q00610 | 17q11-qter | ALCL | | | L | Dom | T | *ALK* |
| *COL1A1* | 1277 | P02452 | 17q21.31-q22 | Dermatofibrosarc oma protuberans | | | M | Dom | T | *PDGFB* |
| *COPEB* | 1316 | Q99612 | 10p15 | Prostatic, glioma | | | E, O | Rec | Mis, N | |
| *COX6C* | 1345 | P09669 | 8q22-q23 | Uterine leiomyoma | | | M | Dom | T | *HMGA2* |
| *CREBBP* | 1387 | Q92793 | 16p13.3 | AL, AML | | | L | Dom | T | *MLL, MORF, RUNXBP2* |
| *CTNNB1* | 1499 | P35222 | 3p22-p21.3 | Colorectal, ovarian, hepatoblastoma, others | | | E, M, O | Dom | H, Mis | |
| *CYLD* | 1540 | NP 05606 2 | 16q12-q13 | Cylindroma | Cylindroma | Familial cylindromatosis | E | Rec | Mis, N, F, | |
| *D10S170* | 8030 | NP 00542 7 | 10q21 | Papillary thyroid, CML | | | E | Dom | T | *RET, PDGFRB* |
| *DDB2* | 1643 | Q92466 | 11p12 | | Skin basal cell, skin squamous cell, melanoma | Xeroderma pigmentosum E | E | Rec | M, N | |
| *DDIT3* | 1649 | P35638 | 12q13.1- q13.2 | Liposarcoma | | | M | Dom | T | *FUS* |
| *DDX10* | 1662 | Q13206 | 11q22-q23 | AML§ | | | L | Dom | T | *NUP98* |
| *DEK* | 7913 | P35659 | 6p23 | AML | | | L | Dom | T | *NUP214* |
| *EGFR* | 1956 | P00533 | 7p12.3-p12.1 | Glioma | | | O | Dom | A, O | |
| *EIF4A2* | 1974 | Q14240 | 3q27.3 | NHL | | | L | Dom | T | *BCL6* |
| | *ELKS* 23085 | NP_05587 | 12p13.3 | Papillary thyroid | | | E | Dom | T | RET |
| *ELL* | 8178 | P55199 | 19p13.1 | AL | | | L | Dom | T | *MLL* |
| EP300 2033 | | Q09472 | 22q13 | Colorectal, breast, pancreatic, AML | | | L, E | Rec | T | *MLL, RUNXBP2* |
| *EPS15* | 2060 | P42566 | 1p32 | ALL | | | L | Dom | T | *MLL* |
| *ERBB2* | 2064 | P04626 | 17q21.1 | Breast, ovarian, other tumour | types | | E | Dom | A | |
| *ERCC2* | 2068 | P18074 | 19q13.2-q13.3 | | Skin basal cell, skin squamous cell, melanoma | Xeroderma pigmentosum D | E | Rec | M, N, F, S | |
| *ERCC3* | 2071 | P19447 | 2q21 | squamous | Skin basal cell, skin cell, melanoma | Xeroderma pigmentosum B | E | Rec | M, S | |
| *ERCC4* | 2072 | Q92889 | 16p13.3- | | Skin basal cell, skin squamous cell, melanoma | Xeroderma pigmentosum F | E | Rec | M, N, F | |
| *ERCC5* | 2073 | P28715 | 13q33 | | Skin basal cell, skin squamous cell, melanoma | Xeroderma pigmentosum G | E | Rec | M, N, F | |
| *ERG* | 2078 | P11308 | 21q22.3 | Ewing s sarcoma | | | M | Dom | T | *EWSR1* |
| *ETV1* | 2115 | P50549 | 7p22 | Ewing s sarcoma | | | M | Dom | T | *EWSR1* |
| *ETV4* | 2118 | P43268 | 17q21 | Ewing s sarcoma | | | M | Dom | T | *EWSR1* |
| *ETV6* | 2120 | P41212 | 12p13 | Congenital fibrosarcoma, multiple leukaemia and lymphoma, secretory breast | | | L, E, M | Dom | T | *NTRK3, RUNX1, PDGFRB ABL1*, *MN1*, *ABL2, FACL6, CHIC2, ARNT, JAK2, EVI1, CDX2, STL* |
| *EVI1* | 2122 | Q03112 | 3q26 | AML CML | | | L | Dom | T | *RUNX1, ETV6* |
| *EWSR1* | 2130 | NP-_00523 4 | 22q12 | Ewing s sarcoma, desmoplastic small round cell, ALL | | | L, M | Dom | T | *FLI1, ERG, ZNF278, NR4A3, TEC, FEV, ATF1*, *ETV1, ETV4, WT1, ZNF384* |
| EXT1 | 2131 NP-_ | 00011 8 | 8q24.11-q24.13 | | Exostoses, osteosarcoma | Multiple exostoses type 1 | M | Rec | Mis, N, F, S | |
| *EXT2* | 2132 | Q93063 | 11p12-p11 | | Exostoses, osteosarcoma | Multiple exostoses 2 | M | Rec | Mis, S N, F, S | |
| *FACL6* | 23305 | NP_05607 1 | 5q31 | AML, AEL | | type | L | Dom | T | *ETV6* |
| *FANCA* | 2175 | NP-_00012 6 | 16q24.3 | | AML, leukaemia | Fanconi A anaemia A | L | Rec | D, Mis, N, F, S | |
| *FANCC* | 2176 | Q00597 | 9q22.3 | | AML, leukaemia | Fanconi C anaemia | L | Rec | D, F, Mis, N, | |
| *FANCD2* | 2177 | NP-_14907 5 | 3p26 | | AML, leukaemia | Fanconi anaemia D2 | L | Rec | D, Mis, N, F | |
| *FANCE* | 2178 | NP-_06874 | 6p21-p22 | | AML, leukaemia | Fanconi E anaemia | L | Rec | N, F, S | |
| *FANCF* | 2188 | Q9NPI8 | 11p15 | | AML, leukaemia | Fanconi F anaemia | L | Rec | N, F | |
| *FANCG* | 2189 | O15287 | 9p13 | | AML, leukaemia | Fanconi G anaemia | L | Rec | Mis, N, F, | |
| *FEV* | 54738 | NP-_05999 | 2q36 | Ewing s sarcoma | | | M | Dom | T | *EWSR1* |
| *FGFR1* | 2260 | P11362 | 8p11.2-p11.1 | MPD/NHL | | | L | Dom | T | BCR, *FOP, ZNF198, CEP1* |
| *FGFRIOP* | 11116 | NP_0089766 | 6q27 | MPD/NHL | | | L | Dom | T | *FGFRI* |
| *FGFR2* | 2263 | P21802 | 10q26 | Gastric | | | E | Dom | Mis | |
| *FGFR3* | 2261 | P22607 | 4p16.3 | Bladder, MM | | | L, E | Dom | Mis, T | *IGH* |
| *FH* | 2271 | P07954 | 1q42.1 | | Leiomyomatosis, renal | Hereditary leiomyomatosis and renal-cell cancer | E, M | Rec | Mis, N, F | |
| *FIPIL1* | 81608 | NP-_11217 9 | 4q12 | Idiopathic hypereosinophilic syndrome | | | L | Dom | T | *PDGFRA* |
| *FLI1* | 2313 | Q01543 | 11q24 | Ewing s sarcoma | | | M | Dom | T | *EWSR1* |
| *FLT3* | 2322 | P36888 | 13q12 | AML, ALL | | | L | Dom | Mis, O | |
| *FLT4* | 2324 | P35916 | 5q35.3 | Angiosarcoma | | | M | Dom | Mis | |
| *FNBP1* | 23048 | XP 05266 6 | 9q23 | AML | | | L | Dom | T | *MLL* |
| *FOXO1A* | 2308 | Q12778 | 13q14.1 | Alveolar rhabdomyosarcoma s | | | M | Dom | T | *PAX3* |
| *FOXO3A* | 2309 | 043524 | 6q21 | AL | | | L | Dom | T | *MLL* |
| *FSTL3* | 10272 | O95633 | 19p13 | B-CLL | | | L | Dom | T | *CCND1* |
| *FUS* | 2521 | P35637 | 16ρ11.2 | Liposarcoma | | | M | Dom | T | *DDIT3* |
| *GAS7* | 8522 | 060861 | 17p | AML^{§} | | | L | Dom | T | *MLL* |
| *GATA1* | 2623 | P15976 | Xp11.23 | Megakaryoblastic leukaemia of Down syndrome | | | L | Dom | Mis, F | |
| | | | | | | | | | | |
| *GMPS* | 8833 | P49915 | 3q24 | AML | | | L | Dom | T | *MLL* |
| *GNAS* | 2778 | P04895 | 20q13.2 | Pituitary adenoma | | | E | Dom | Mis | |
| *GOLGA5* | 9950 | NP_00510 4 | 14q | Papillary thyroid | | | E | Dom | T | *RET* |
| *GPC3* | 2719 | P51654 | Xq26.1 | | Wilms tumour | Simpson Golabi Behmel O syndrome | O | Rec | T, D, Mis, N, F, S | |
| *GPHN* | 10243 | Q9NQX3 | 14q24 | AL | | | L | Dom | T | *MLL* |
| *GRAF* | 23092 | NP_05588 6 | 5q3l | AML, MDS | | | L | Dom | T, F, S | *MLL* |
| *HEI10* | 57820 | NP_06700 1 | 14q11.1 | Uterine leiomyoma | | | M | Dom | T | *HMGA2* |
| *HIP1* | 3092 | 000291 | 7q11.23 | CMML | | | L | Dom | T | *PDGFRB* |
| *HIST1H4I* | 8294 | NP_003486 | 6p21.3 | NHL | | | L | Dom | T | *BCL6* |
| *HLF* | 3131 | Q16534 | 17q22 | ALL | | | L | Dom | T | *TCF3* |
| *HMGA2* | 8091 | P52926 | 12q15 | Lipoma | | | M | Dom | T | *LHFP, RAD51L1, LPP, HEI10, COX6C* |
| *HOXA11* | 3207 | P31270 | 7p15-p14.2 | CML | | | L | Dom | T | *NUP98* |
| *HOXA13* | 3209 | P31271 | 7p15-p14.2 | AML | | | L | Dom | T | *NUP98* |
| *HOXA9 HOXC13* | 3205 3229 | P31269 P31276 | 7p15-p14.2 12q13.3 | AML^{§} AML | | | L L | Dom Dom | T T | *NUP98 NUP98* |
| *HOXD11* | 3237 | P31277 | 2q31-q32 | AML | | | L | Dom | T | *NUP98* |
| *HOXD13* | 3239 | P35453 | 2q31-q32 | AML^{§} | | | L | Dom | T | *NUP98* |
| *HRAS* | 3265 | P01112 | 11p15.5 | Infrequent sarcomas, rare other types | | | L, M | Dom | Mis | |
| *HRPT2* | 3279 | NP 01352 2 | 1q21-q31 | Parathyroid adenoma | Parathyroid adenoma, multiple ossifying jaw fibroma | Hyperparathyroidism jaw tumour syndrome | E, M | Rec | Mis, N, F | |
| *HSPCA* | 3320 | P07900 | 1q212-q22 | NHL | | | L | Dom | T | *BCL6* |
| *HSPCB* | 3326 | P08238 | 6p12 | NHL | | | L | Dom | T | *BCL6* |
| *IGH* | 3492 | | 14q32.33 | MM, Burkitt s lymphoma, NHL, CLL, B-ALL, MALT | | | L | Dom | T | *MYC, FGFR3, PAX5, IRTA1, IRF4, CCND1, BCL9, BCL6, BCL8, BCL2, BCL3, BCL10, BCL11A. LHX4* |
| *IGK* | 50802 | | 2p12 | Burkitt s lymphoma | | | L | Dom | T | *MYC* |
| *IGL* | 3535 | | 22q11.1-q11.2 | Burkitt s lymphoma | | | L | Dom | T | *BCL9, MYC* |
| *IL21R* | 50615 | Q9HBE5 | 16p11 | NHL | | | L | Dom | T | *BCL6* |
| *IRF4* | 3662 | Q15306 | 6p25-p23 | MM | | | L | Dom | T | *IGH* |
| *IRTA1* | 83417 | NP_11257 2 | 1q21 | B-NHL | | | L | Dom | T | *IGH* |
| *JAK2* | 3717 | 060674 | 9p24 | ALL, AML | | | L | Dom | T | *ETV6* |
| *KIT* | 3815 | P10721 | 4q12 | GIST, AML, TGCT | GIST, epithelioma | Familial gastrointestinal stromal | L, M, O | Dom | Mis, O | |
| *KRAS2* | 3845 | NP_00497 6 | 12p12.1 | Pancreatic, colorectal, lung, thyroid, AML, others | | | L, E, M, O | Dom | Mis | |
| *LAF4* | 3899 | P51826 | 2q11.2-q12 | ALL | | | L | Dom | T | *MLL* |
| *LASP1* | 3927 | Q14847 | 17q11-q21.3 | AML | | | L | Dom | T | *MLL* |
| *LCK* | 3932 | NP 00534 7 | 1p35-p34.3 | T-ALL | | | L | Dom | T | *TRB* |
| *LCP1* | 3936 | P13796 | 13q14.1-q14.3 | NHL | | | L | Dom | T | *BCL6* |
| *LCX* | 80312 | XP_16761 2 | 10q21 | AML | | | L | Dom | T | *MLL* |
| *LHFP* | 10186 | NP 00577 1 | 13q12 | Lipoma | | | M | Dom | T | *HMGA2* |
| *LMO1* | 4004 | P25800 | 11p15 | T-ALL | | | L | Dom | T | *TRD* |
| *LMO2* | 4005 | P25791 | 11p13 | T-ALL | | | L | Dom | T | *TRD* |
| *LPP* | 4026 | NP 00556 9 | 3q28 | Lipoma, leukaemia | | | L, M | Dom | T | *HMGA2, MLL* |
| *LYL1* | 4066 | P12980 | 19p13.2-p13.1 | T-ALL | | | L | Dom | T | *TRB* |
| *MADH4* | 4089 | Q13485 | 18q21.1 | Colorectal, pancreatic, small intestine | Gastrointestinal polyps | Juvenile polyposis | E | Rec | D, Mis, N, F | |
| *MALT1* | 10892 | Q9UDY8 | 18q21 | MALT | | | L | Dom | T | *BIRC3* |
| *MAML2* | 84441 | XP_04571 6 | 11q22-q23 | Salivary-gland mucoepidermoid | | | E | Dom | T | *MECT1* |
| *MAP2K4* | 6416 | P45985 | 17p11.2 | Pancreatic, breast, colorectal | | | E | Rec | D, Mis, N | |
| *MDS1* | 4197 | Q13465 | 3q26 | MDS, AML | | | L | Dom | T | *RUNX1* |
| *MECT1* | 94159 | AAK9383 2.1 | 19p13 | Salivary-gland mucoepidermoid | | | E | Dom | T | *MAML2* |
| *MEN1* | 4221 | 000255 | 11q13 | Parathyroid | Parathyroid adenoma, pituitary adenoma, pancreatic islet cell, carcinoid | Multiple endocrine neoplasia type 1 | E | Rec | D, Mis, N, F, S | |
| *MET* | 4233 | P08581 | 7q31 | Papillary renal, head-neck squamous cell | Papillary renal | Familial papillary renal | E | Dom | Mis | |
| *MHC2TA* | 4261 | P33076 | 16p13 | NHL | | | L | Dom | T | *BCL6* |
| *MLF1* | 4291 | P58340 | 3q25.1 | AML | | | L | Dom | T | *NPM1* |
| *MLH1* | 4292 | P40692 | 3p21.3 | Colorectal, endometrial, ovarian, CNS | Colorectal, endometrial, ovarian, CNS | Hereditary non- polyposis colorectal, Turcot syndrome | E, O | Rec | D, Mis, N, F, S | |
| *MLL* | 4297 | Q03164 | 11q23 | AML, ALL | | | L | Dom | T, O | *MLL, MLLT1, MLLT2, MLLT3, MLLT4, MLLT7, MLLT10, MLLT6, ELL, EPS15, AF1Q, CREBBP, SH3GL1, FNBP1, PNUTL1, MSF, GPHN, GMPS, SSH3BP1, ARHGEF12, GAS7, FOXO3A, LAF4, LCX, SEPT6, LPP, CBFA2T1, GRAF, EP300, PICALM* |
| *MLLT1* | 4298 | Q03111 | 19p13.3 | AL | | | L | Dom | T | *MLL* |
| *MLLT10* | 8028 | P55197 | 10p12 | AL | | | L | Dom | T | *MLL, PICALM* |
| *MLLT2* | 4299 | P51825 | 4q21 | AL | | | L | Dom | T | *MLL* |
| *MLLT3* | 4300 | P42568 | 9p22 | ALL | | | L | Dom | T | *MLL* |
| *MLLT4* | 4301 | P55196 | 6q27 | AL | | | L | Dom | T | *MLL* |
| *MLLT6* | 4302 | P55198 | 17q21 | AL | | | L | Dom | T | *MLL* |
| *MLLT7* | 4303 | NP_00592 9 | Xq13.1 | AL | | | L | Dom | T | *MLL* |
| *MN1* 4330 | | Q10571 | 22q13 | AML, meningioma | | | L, O | Dom | T | *ETV6* |
| *MSF* | 10801 | NP 00663 1 | 17q25 | AML^{§} | | | L | Dom | T | *MLL* |
| *MSH2* | 4436 | P43246 | 2p22-p21 | Colorectal, endometrial, ovarian | Colorectal, endometrial, ovarian | Hereditary non- polyposis colorectal | E | Rec | D, Mis, N, F, S | |
| *MSH6* | 2956 | P52701 | 2p16 | Colorectal | Colorectal, endometrial, ovarian | Hereditary non- polyposis colorectal | E | Rec | Mis, N, F, S | |
| *MSN* | 4478 | P26038 | Xq11.2-q12 | ALCL | | | L | Dom | T | *ALK* |
| *MUTYH* | 4595 | NP_03635 4 | = 1p34.3-1p32.1 | | Colorectal | Adenomatous polyposis coli | E | Rec | Mis, N, F, S | |
| *MYC* | 4609 | P01106 | 8q24.12-q24.13 | Burkitt s lymphoma, amplified in other cancers, B-CLL | | | L, E | Dom | A, T | *IGK , BCL5, BCL7A, BTG1, TRA , IGH* |
| *MYCL1* | 4610 | P12524 | 1p34.3 | Small cell lung | | | E | Dom | A | |
| *MYCN* | 4613 | P04198 | 2p24.1 | Neuroblastoma | | | O | Dom | A | |
| *MYH11* | 4629 | P35749 | 16p13.13-p13.12 | AML | | | L | Dom | T | *CBFB* |
| *MYH9* | 4627 | P35579 | 22q13.1 | ALCL | | | L | Dom | T | *ALK* |
| *MYST4* | 23522 | NP 03646 2 | 10q22 | AML | | | L | Dom | T | *CREBBP* |
| *NACA* | 4666 | NP 00558 5 | 12q23-q24.1 | NHL | | | L | Dom | T | *BCL6* |
| *NBS1* | 4683 | NP_00247 6 | 8q21 | | NHL, glioma, medulloblastoma, rhabdomyosarcoma | Nijmegen breakage syndrome | L, E, M, O | Rec | Mis, N, F | |
| *NCOA2* | 10499 | Q15596 | 8q13.1 | AML | | | L | Dom | T | *RUNXBP2* |
| *NCOA4* | 8031 | Q13772 | 10q11.2 | Papillary thyroid | | | E | Dom | T | *RET* |
| *NF1* | 4763 | P21359 | 17q12 | Neurofibroma, glioma | Neurofibroma, glioma | Neurofibromatos is type 1 | O | Rec | D, Mis, N, F, S, O | |
| *NF2* | 4771 | P35240 | 22q12.2 | Meningioma, acoustic neuroma | Meningioma, acoustic neuroma | Neurofibromatos is type 2 | O | Rec | D, Mis, N, F, S, O | |
| *NOTCH1* | 4851 | P46531 | 9q34.3 | T-ALL | | | L | Dom | T | *TRB* |
| *NPM1* | 4869 | P06748 | 5q35 | NHL, APL, AML | | | L | Dom | T | *ALK, RARA, MLF1* |
| *NR4A3* | 8013 | Q92570 | 9q22 | Extraskeletal myxoid chondrosarcoma | | | M | Dom | T | *EWSR1* |
| *NRAS* | 4893 | P01111 | 1p13.2 | Melanoma, MM, AML, thyroid | | | L, E | Dom | Mis | |
| *NSD1* | 64324 | NP 07190 0 | 5q35 | AML | | | L | Dom | T | *NUP98* |
| *NTRK1* | 4914 | P04629 | 1q21-q22 | Papillary thyroid | | | E | Dom | T | *TPM3, TPR, TFG* |
| *NTRK3* | 4916 | Q16288 | 15q25 | Congenital fibrosarcoma, secretory breast | | | E, M | Dom | T | *ETV6* |
| *NUMA1* | 4926 | NP 00617 6 | 11q13 | APL | | | L | Dom | T | *RARA* |
| *NUP214* | 8021 | P35658 | 9q34.1 | AML | | | L | Dom | T | *DEK, SET* |
| *NUP98* | 4928 | P52948 | 11p15 | AML | | | L | Dom | T | *HOXA9, NSD1 WHSC1L1, DDX10, TOP1, HOXD13, PMX1, HOXA13, HOXD11, HOXA11, RAPGDS1* |
| *NUT* | 256646 | XP_17172 4 | 15q13 | Lethal midline carcinoma of young people | | | E | Dom | T | *BRD4* |
| *OLIG2* | 10215 | Q13516 | 21q22.11 | T-ALL | | | L | Dom | T | *TRA* |
| *PAX3* | 5077 | P23760 | 2q35 | Alveolar rhabdomyosarcoma | | | M | Dom | T | *FOXO1A* |
| *PAX5* | 5079 | Q02548 | 9p13 | NHL | | | L | Dom | T | *IGH* |
| *PAX7* | 5081 | P23759 | 1p36.2-p36.12 | Alveolar rhabdomyosarcoma | | | M | Dom | T | *FOXO1A* |
| *PAX8* | 7849 | Q06710 | 2q12-q14 | Follicular thyroid | | | E | Dom | T | *PPARG* |
| *PBX1* | 5087 | NP 00257 6 | 1q23 | Pre-B-ALL | | | L | Dom | T | *TCF3* |
| *PCM1* | 5108 | NP_00618 8 | Rp22-p21.3 | Papillary thyroid | | | E | Dom | T | *RFT* |
| *PDGFB* | 5155 | P01127 | 22q12.3-q13.1 | DFSP | | | M | Dom | T | *COL1A1* |
| *PDGFRA* | 5156 | P16234 | 4q11-q13 | GIST | | | M, O | Dom | Mis, O | |
| *PDGFRB* | 5159 | NP_00260 0 | 5q31-q32 | MPD, AML, CMML, CML | | | L | Dom | T | *ETV6, TRIP11, HIP1, RAB5EP, H4* |
| *PICALM* | 8301 | Q13492 | 11q14 | T-ALL, AML | | | L | Dom | T | *MLLT10, MLL* |
| *PIM1* | 5292 | P11309 | 6p21.2 | NHL | | | L | Dom | T | *BCL6* |
| *PML* | 5371 | P29590 | 15q22 | APL | | | L | Dom | T | *RARA* |
| *PMS1* | 5378 | P54277 | 2q31-q33 | | Colorectal, endometrial, ovarian | Hereditary non- polyposis colorectal cancer | E | Rec | Mis, N | |
| *PMS2* | 5395 | P54278 | 7p22 | | Colorectal, endometrial, ovarian, medulloblastoma, glioma | Hereditary non- polyposis colorectal cancer, Turcot syndrome | E | Rec | Mis, N, F | |
| *PMX1* | 5396 | P54821 | 1q24 | AML | | | L | Dom | T | *NUP98* |
| *PNUTL1* | 5413 | NP 00267 9 | 22q11.2 | AML | | | L | Dom | T | *MLL* |
| *POU2AF1* | 5450 | Q16633 | 11q23.1 | NHL | | | L | Dom | T | *BCL6* |
| *PPARG* | 5468 | P37231 | 3p25 | Follicular thyroid | | | E | Dom | T | *PAX8* |
| *PRCC* | 5546 | Q92733 | 1q21.1 | Papillary renal | | | E | Dom | T | *TFE3* |
| *PRKAR1A* | 5573 | P10644 | 17q23-q24 | Papillary thyroid | Myxoma, endocrine, papillary thyroid | Carney complex | E , M | Dom, Rec | T, Mis, N, F, S | *RET* |
| *PRO1073* | 29005 | Q9UHZ2 | 11q31.1 | Renal-cell carcinoma (childhood epithelioid) | | | E | Dom | T | *TFEB* |
| *PSIP2* | 11168 | NP_15009 1 | 9p22.2 | AML | | | L | Dom | T | *NUP98* |
| *PTCH* | 5727 | Q13635 | 9q22.3 | Skin basal cell, medulloblastoma | Skin basal cell, medulloblastoma | Nevoid basal-cell carcinoma syndrome | E, M | Rec | Mis, N, F, S | |
| *PTEN* | 5728 | 000633 | 10q23.3 | Glioma, prostatic, endometrial | Harmartoma, glioma, prostatic, endometrial | Cowden syndrome, Bannayan Riley Ruvalcaba syndrome | L, E, M, O | Rec | D, Mis, N, F, S | |
| *PTPN11* | 5781 | Q06124 | 12q24.1 | JMML, AML, MDS | | | L | Dom | Mis | |
| *RAB5EP* 9135 | | NP_00469 4 | 17p13 | CMML | | | L | Dom | T | *PDGFRB* |
| *RAD51L1* | 5890 | NP_00286 8 | 14q23-q24.2 | Lipoma, uterine leiomyoma | | . | M | Dom | T | *HMGA2* |
| *RAP1GDS1* | 5910 | P52306 | 4q21-q25 | T-ALL | | | L | Dom | T | *NUP98* |
| *RARA* | 5914 | P10276 | 17q12 | APL | | | L | Dom | T | *PML, ZNF145, TIF1, NUMA1, NPM1* |
| *RBI* | 5925 | P06400 | 13q14 | Retinoblastoma, sarcoma, breast, small-cell lung | Retinoblastoma, sarcoma, breast, small-cell lung | Familial retinoblastoma | L, E, M, O | Rec | D, Mis, N, F, S | |
| *RECQL4* | 9401 | 094761 | 8q24.3 | | Osteosarcoma, skin basal and squamous cell | Rothmund Thompson syndrome | M | Rec | N, F, S | |
| *REL* | 5966 | Q04864 | 2p13-p12 | Hodgkin | | | L | Dom | A | |
| *RET* | 5979 | P07949 | 10q11.2 | Lymphoma Medullary thyroid, | Medullary thyroid, | Multiple | E, O | Dom | T, Mis, N, | *H4, PRKAR1A,* |
| | | | | papillary thyroid, pheochromocytoma | papillary thyroid, pheochromocytomaneoplas ia | endocrine 2A/2B | | | F | *NCOA4, PCM1, GOLGA5, TR1M33* |
| *RPL22* | 6146 | P35268 | 3q26 | AML, CML | | | L | Dom | T | |
| *RUNX1* | 861 | Q01196 | 21q22.3 | AML, pre-B-ALL | | | L | Dom | T | *RPL22, MDS1, EVI1, CBFA2T3, CBFA2T1, ETV6* |
| *RUNXBP2* | 799 | NP _00675 7 | 8p11 | AML | | | L | Dom | T | *CREBBP, NCOA2, EP300* |
| *SBDS* | 51119 | Q9Y3A5 | 7q11 | | AML, MDS | Schwachman Diamond syndrome | L | Rec | Gene conversion | |
| *SDHB* | 6390 | P21912 | 1p36.1-p35 | | Paraganglioma, pheochromocytoma | Familial paraganglioma | O | Rec | Mis, N, F | |
| *SDHC* | 6391 | 075609 | 1q21 | | Paraganglioma, pheochromocytoma | Familial paraganglioma | O | Rec | Mis, N, F | |
| *SDHD* | 6392 | 014521 | 11q23 | | Paraganglioma, pheochromocytoma | Familial paraganglioma | O | Rec | Mis, N, F, S | |
| *SEPT6* | 23157 | NP_05594 4 | Xq24 | AML | | | L | Dom | T | *MLL* |
| *SET* | 6418 | Q01105 | 9q34 | AML | | | L | Dom | T | *NUP214* |
| *SFPQ* | 6421 | P23246 | 1p34.3 | Papillary renal cell | | | E | Dom | T | *TFE3* |
| *SH3GL1* | 6455 | Q99961 | 19p13.3 | AL | | | L | Dom | T | *MLL* |
| *SMARCB1* | 6598 | Q12824 | 22q11 | Malignant rhabdoid | Malignant rhabdoid | Rhabdoid predisposition syndrome | M | Rec | D, N, F, S | |
| *SMO* | 6608 | Q99835 | 7q31-q32 | Skin basal cell | | | E | Dom | Mis | |
| *SS18* | 6760 | Q15532 | 18q11.2 | Synovial sarcoma | | | M | Dom | T | *SSX1, SSX2* |
| *SS18L1* | 26039 | O75177 | 20q13.3 | Synovial sarcoma | | | M | Dom | T | *SSX1* |
| *SSH3BP1* | 10006 | NP 00546 1 | 10p11.2 | AML | | | L | Dom | T | *MLL* |
| *SSX1* | 6756 | Q16384 | Xp11.23-p11.22 | Synovial sarcoma | | | M | Dom | T | *SS18* |
| *SSX2* | 6757 | Q16385 | Xpll.23-pll.22 | Synovial sarcoma | | | M | Dom | T | *SS18* |
| *SSX4* | 6759 | 060224 | Xp11.23 | Synovial sarcoma | | | M | Dom | T | *SS18* |
| *STK11* | 6794 | Q15831 | 19p13.3 | NSCLC | Jejunal harmartoma, ovarian, testicular, pancreatic | Peutz Jeghers syndrome | E, M, O | Rec | ' D, Mis, N, | |
| *STL* | 7955 | NOPROTEIN | 6q23 | B-ALL | | | L | Dom | T | *ETV6* |
| *SUFU* | 51684 | NP 05725 3 | 10q24.32 | Medulloblastoma | Medulloblastoma | Medulloblastom a predisposition | O | Rec | D, F, S | |
| *TAFI5* | 8148 | Q92804 | 17q11.1-q11.2 | Extraskeletal myxoid chondrosarcomas, ALL | | | L, M | Dom | T | *TEC, CHN1, ZNF384* |
| *TAL1* | 6886 | P17542 | 1p32 | Lymphoblastic leukaemia/ biphasic | | | L | Dom | T | *TRD* |
| *TAL2* | 6887 | Q16559 | 9q31 | T-ALL | | | L | Dom | T | *TRB* |
| *TCF1* | 6927 | P20823 | 12q24.2 | Hepatic adenoma, hepatocellular carcinoma | Hepatic adenoma, hepatocellular carcinoma | Familial hepatic adenoma | E | Rec | Mis, F | |
| *TCF12* | 6938 | Q99081 | 15q21 | Extraskeletal myxoid chondrosarcoma | | | M | Dom | T | *TEC* |
| *TCF3* | 6929 | P15923 | 19p13.3 | pre-B-ALL | | | L | Dom | T | *PBX1, HLF, TFPT* |
| *TCL1A* | 8115 | NP 06880 1 | 14q32.1 | T-CLL | | | L | Dom | T | *TRA* |
| *TEC* | 7006 | P42680 | 4p12 | Extraskeletal myxoid chondrosarcoma | | | M | Dom | T | *EWSR1, TAF15, TCF12* |
| *TFE3* | 7030 | P19532 | Xp11.22 | Papillary renal, alveolar soft part sarcoma | | | E | Dom | T | *SFPQ, ASPSCR1, PRCC* |
| *TFEB* | 7942 | P19484 | 6p21 | Renal (childhood epithelioid) | | | E, M | Dom | T | *ALPHA* |
| *TFG* | 10342 | NP_00606 1 | 3q11-q12 | Papillary thyroid, ALCL | | | E, L | Dom | T | *NTRK1, ALK* |
| *TFPT* | 29844 | NP 03747 4 | 19q13 | Pre-B-ALL | | | L | Dom | T | *TCF3* |
| *TFRC* | 7037 | P02786 | 3q29 | NHL | | | L | Dom | T | *BCL6* |
| *TIF1* | 8805 | 015164 | 7q32-q34 | APL | | | L | Dom | T | *RARA* |
| *TLX1* | 3195 | P31314 | 10q24 | T-ALL | | | L | Dom | T | *TRB , TRD* |
| *TLX3* | 30012 | 043711 | 5q35.1 | T-ALL | | | L | Dom | T | *BCL11B* |
| *TNFRSF6* | 355 | P25445 | 10q24.1 | TGCT, nasal NK/T lymphoma, skin squamous-cell carcinoma (burn-scar related) | | | L, E, O | Rec | Mis | |
| *TOP1* | 7150 | P11387 | 20q12-q13.1 | AML^{§} | | | L | Dom | T | *NUP98* |
| *TP53* | 7157 | P04637 | 17p13 | Breast, colorectal, lung, sarcoma, adrenocortical, glioma, multiple other types | Breast, sarcoma, adrenocortical carcinoma, glioma, multiple other types | Li Fraumeni syndrome | L, E, M, O | Rec | Mis, N, F | |
| *TPM3* | 7170 | P06753 | 1q22-q23 | Papillary thyroid, ALCL | | | E, L | Dom | T | *NTRK1, ALK* |
| *TPM4* | 7171 | P07226 | 19p13.1 | ALCL | | | L | Dom | T | *ALK* |
| *TPR* | 7175 | P12270 | 1q25 | Papillary thyroid | | | E | Dom | T | *NTRK1* |
| *TRA* | 6955 | | 14q11.2 | T-ALL | | | L | Dom | T | *ATL, OLIG2, MYC, TCL1A* |
| *TRB* | 6957 | | 7q35 | T-ALL | | | L | Dom | T | *HOX11, LCK, NOTCH1, TAL2, LYL1* |
| *TRD* | 6964 | | 14q11 | T-cell leukaemia | | | L | Dom | T | *TAL1, HOX11, TLX1, LMO1, LMO2* |
| *TRIM33* | 51592 | Q9UPN9 | 1p13 | Papillary thyroid | | | E | Dom | T | *RET* |
| *TRIP11* | 9321 | NP 00423 0 | 14q31-q32 | AML | | | L | Dom | T | *PDGFRB* |
| *TSC1* | 7248 | Q92574 | 9q34 | | Hamartoma, renal cell | Tuberous sclerosis 1 | E, O | Rec | D, Mis, N, F, S | |
| *TSC2* | 7249 | P49815 | 16p13.3 | | Hamartoma, renal cell | Tuberous sclerosis 2 | E, O | Rec | D, Mis, N, F, S | |
| *TSHR* | 7253 | P16473 | 14q31 | Toxic thyroid adenoma | Thyroid adenoma | | E | Dom | Mis | |
| *VHL* | 7428 | P40337 | 3p25 | Renal, hemangioma, pheochromocytoma | Renal, hemangioma, pheochromocytoma | von Hippcl Lindau syndrome | E, M, O | Rcc | D, Mis, N, F, S | |
| *WAS* | 7454 | P42768 | Xp11.23-p11.22 | | Lymphoma | Wiskott Aldrich syndrome | L | Rec | Mis, N, F, S | |
| *WHSC1L1* | 54904 | NP 06024 8 | 8p12 | AML | | | L | Dom | T | *NUP98* |
| *WRN* | 7486 | Q14191 | 8p12-p11.2 | | Osteosarcoma, meningioma, others | Werner syndrome | L, E, M, O | Rec | Mis, N, F, S | |
| *WT1* | 7490 | NP 00036 9 | 11p13 | Wilms, desmoplastic small round cell | Wilms | Deny s Drash syndrome, Frasier syndrome, Familial Wilms tumour | O | Rec | D, Mis, N, F, S | *EWSR1* |
| *XPA* | 7507 | P23025 | 9q22.3 | | Skin basal cell, skin squamous cell, melanoma | Xeroderma pigmentosum A | E | Rec | Mis, N, F, S | |
| *XPC* | 7508 | Q01831 | 3p25 | | Skin basal cell, skin squamous cell, melanoma | Xeroderma pigmentosum C | E | Rec | Mis, N, F, S | |
| *ZNF145* | 7704 | Q05516 | 11q23.1 | APL | | | L | Dom | T | *RARA* |
| *ZNF198* | 7750 | Q9UBW7 | 13q11-q12 | MPD/NHL | | | L | Dom | T | *FGFR1* |
| *ZNF278* | 23598 | NP_05513 8 | 22q12-q14 | Ewing s sarcoma | | | M | Dom | T | *EWSR1* |
| *ZNF384* | 171017 | NP_59773 3 | 12p13 | ALL | | | L | Dom | T | *EWSR1, TAF15* |
| *ZNFN1A1* | 10320 | NP_00605 1 | 7p12 | ALL, DLBCL | | | L | Dom | T | *BCL6* |
| ^{∗}From Swiss-Prot/Refseq. D (large deletion) covers the abnormalities that result in allele loss/loss of heterozygosity at many recessive cancer genes. §Refers to cases of acute myeloid leukaemia that are associated with treatment. ∥O (other) in the mutation type column refers primarily to small in-frame deletions/insertions as found in KIT/PDGFRA, and larger duplications/insertions as found in FLT3 and EGFR. Note that where an inversion/large deletion has been shown to result in a fusions protein, these have been listed under translocations. The Wellcome Trust Sanger Institute web version of the cancer-gene set can be found at http://www.sanger.ac.uk/genetics/CPG/Census/. A, amplification; AEL, acute eosinophilic leukaemia; AL, acute leukaemia; ALCL, anaplastic large-cell lymphoma; ALL, acute lymphocytic leukaemia; AML, acute myelogenous leukaemia; APL, acute promyelocytic leukaemia; B-ALL, B-cell acute lymphocytic leukaemia; B-CLL, B-cell lymphocytic leukaemia; B-NHL, B-cell non-Hodgkin's lymphoma; CLL, chronic lymphatic leukaemia; CML, chronic myeloid leukaemia; CMML, chronic myelomonocytic leukaemia; CNS, central nervous system; D, large deletion; DFSP, dermatofibrosarcoma protuberans; DLBCL, diffuse large B-cell lymphoma; Dom, dominant; E, epithelial; F, frameshift; GIST, gastrointestinal stromal tumour; JMML, juvenile myelomonocytic leukaemia; L, leukaemia/lymphoma; M, mesenchymal; MALT, mucosa-associated lymphoid tissue; MDS, myelodysplastic syndrome; MM, multiple myeloma; Mis, missense; N, nonsense; NHL, non-Hodgkin's lymphoma; NK/T, natural killer T cell; NSCLC, non-small-cell lung cancer; O, other; pre-B-ALL, pre-B-cell acute lymphoblastic leukaemia; Rec, recessive; S, splice site; T, translocation; T-ALL, T-cell acute lymphoblastic leukaemia; T-CLL, T-cell chronic lymphocytic leukaemia; TGCT, testicular germ-cell tumour; T-PLL, T-cell prolymphocytic leukaemia. | | | | | | | | | | |

| **Table 4: Commonly Upregulated Genes in Cancers** | | | | | | | | | |
|---|---|---|---|---|---|---|---|---|---|
| **UnlGene** | **Gene symbol** | **N** | **Up #** | **Down #** | **UniGene** | **Gene symbol** | **N** | **Up #** | **Down #** |
| Hs. 159430 | FNDC3B | 11 | 10 | 0 | Hs.239388 | PAQR8 | 8 | 5 | 1 |
| Hs. 518201 | DTX3L | 8 | 7 | 0 | Hs.592827 | RBAK | 8 | 5 | 1 |
| Hs.530899 | LOC162073 | 8 | 7 | 0 | Hs.525157 | TNFSF13B | 8 | 5 | 1 |
| Hs.15159 | CKLF | 11 | 9 | 1 | Hs.126774 | DTL | 13 | 8 | 0 |
| Hs.474150 | BID | 16 | 13 | 0 | Hs.385913 | ANP32E | 13 | 8 | 1 |
| Hs.7753 | CALU | 15 | 12 | 0 | Hs.532968 | DKFP762E1312 | 13 | 8 | 1 |
| Hs. 418795 | GLT2SDI | 10 | 8 | 0 | Hs.372429 | PDIA6 | 13 | 8 | 1 |
| Hs 435556 | BFAR | 12 | 9 | 0 | Hs.233952 | PSMA7 | 13 | 8 | 1 |
| Hs. 459362 | PACI | 12 | 9 | 1 | Hs.533770 | SLC38A1 | 13 | 8 | 1 |
| Hs. 521800 | Cborf76 | 8 | 6 | 0 | Hs.489284 | ARPC18 | 18 | 11 | 0 |
| Hs. 209561 | KIAA1715 | 8 | 6 | 0 | Hs.497788 | EPRS | 18 | 11 | 0 |
| Hs. 585011 | Clorf96 | 8 | 6 | 1 | Hs. 79110 | NCL | 18 | 11 | 0 |
| Hs..403933 | FBX032 | 8 | 6 | 1 | Hs.251531 | PSMA4 | 18 | 11 | 0 |
| Hs. 368853 | AYTL2 | 15 | 11 | 1 | Hs.429180 | Elf2S2 | 18 | 11 | 1 |
| Hs. 511093 | NUSAP1 | 11 | 8 | 0 | Hs.46S885 | ILF3 | 18 | 11 | 1 |
| Hs. 370895 | RPN2 | 14 | 10 | 0 | Hs.169840 | TTK | 18 | 11 | 1 |
| Hs. 180062 | PSMBB | 17 | 12 | 0 | Hs.489365 | APIST | 15 | 9 | 1 |
| Hs. 444600 | BOLAZ | 10 | 7 | 0 | Hs.256639 | PPIH | 15 | 9 | 1 |
| Hs. 445890 | CHIH4 | 13 | 9 | 0 | Hs. 14559 | CEP55 | 10 | 6 | 1 |
| Hs. 534392 | KDELR3 | 13 | 9 | 0 | Hs. 308613 | MTERFD1 | 10 | 6 | 1 |
| Hs. 632 191 | XTP3TPA | 13 | 9 | 0 | Hs. 21331 | ZW1LCH | 10 | 6 | 1 |
| Hs.387567 | ACLV | 19 | 13 | 1 | Hs. 524S99 | NAPIL! | 17 | 10 | 1 |
| Hs.533282 | NONO | 18 | 12 | 0 | Hs. 78171 | PGKI | 17 | 10 | 2 |
| Hs.83753 | SNRPB | 18 | 12 | 0 | Hs. 512380 | PLEKHB2 | 12 | 7 | 1 |
| Hs. 471441 | PSMBZ | 18 | 12 | 1 | Hs. 352018 | TAP1 | 19 | 11 | 1 |
| Hs. 482497 | TNPOI | 18 | 12 | 1 | Hs. 194698 | CCNB2 | 14 | 8 | 1 |
| Hs. 370937 | TAPBP | 15 | 10 | 0 | Hs. 153357 | PLOD3 | 14 | 8 | 1 |
| Hs .126941 | FAM49B | 12 | 8 | 0 | Hs. 471200 | NRP2 | 14 | 8 | 2 |
| Hs. 408629 | KDELCI | 12 | 8 | 0 | Hs.250822 | AURKA | 16 | 9 | 1 |
| Hs. 49?384 | IPO9 | 12 | 8 | 1 | Hs.75528 | GN12 | 16 | 9 | 1 |
| Hs. 8752 | TMEM4 | 12 | 8 | 1 | Hs. 1197 | HSPEI | 16 | 9 | 1 |
| Hs. 195642 | C17orf27 | 9 | 6 | 0 | Hs.202672 | DNMTI | 18 | 10 | 1 |
| Hs.358997 | TTL | 9 | 6 | 0 | Hs. 433670 | FTL | 18 | 10 | 1 |
| Hs. 1600 | CCT5 | 20 | 13 | 0 | Hs. 519972 | HLA-F | 18 | 10 | 1 |
| Hs. 269408 | E2F3 | 17 | 11 | 0 | HS. 520210 | KDELR2 | 18 | 10 | 1 |
| Hs.234027 | ZBTB12 | 17 | 11 | 1 | Hs. 40515.1 | CARD-4 | 11 | 6 | 1 |
| Hs. 520205 | EIF2AK1 | 14 | 9 | 0 | Hs. 477700 | DBRI | 11 | 6 | 1 |
| Hs.89545 | PSMB4 | 14 | 9 | 0 | Hs. I4468 | FLJ11286 | 11 | 6 | 1 |
| Hs.449415 | EIF2C2 | 14 | 9 | 1 | Hs. 516077 | FLJ14668 | 11 | 6 | 1 |
| Hs. 409065 | FEN1 | 14 | 9 | 1 | HS. 494337 | GOLPH2 | 11 | 6 | 1 |
| Hs. 313 | SPP1 | 14 | 9 | 2 | Hs.. 371036 | NOX4 | 11 | 6 | 1 |
| Hs..525135 | FARP1 | 14 | 9 | 2 | Hs..438683 | SLAMF8 | 11 | 6 | 1 |
| Hs. 524390 | K-ALPHA-1 | 11 | 7 | 0 | Hs.520714 | SNXIO | 11 | 6 | 1 |
| Hs..432360 | SCNM1 | 11 7 | | 0 | Hs. 159428 | BAX | 13 | 7 | 1 |
| Hs. 172028 | ADAM10 | 19 | 12 | 0 | Hs..311609 | DDX39 | 13 | 7 | 1 |
| Hs. 381189 | CBX3 | 19 | 12 | 0 | Hs. 463035 | EKBP10 | 13 | 7 | 1 |
| Hs. 522257 | HNRPK | 19 | 12 | 0 | Hs.438695 | FKBP11 | 13 | 7 | 1 |
| Hs. 470943 | STATI | 19 | 12 | 0 | Hs.515255 | LSM4 | 13 | 7 | 1 |
| Hs. 118638 | NME1 | 19 | 12 | 1 | Hs. 55285 | MORC2 | 13 | 7 | 1 |
| Hs. 519452 | NPM1 | 19 | 12 | 1 | Hs. 43666 | PTP4A3 | 13 | 7 | 1 |
| Hs. 506748 | HDGF | 16 | 10 | 0 | Hs. 369440 | SFXN1 | 13 | 7 | 1 |
| Hs.386283 | ADAM12 | 16 | 10 | 2 | Hs. 5I7155 | TMEPAI | 13 | 7 | 1 |
| Hs. 474740 | APOL2 | 8 | 5 | 0 | Hs. 631580 | UBA2 | 13 | 7 | 1 |
| Hs. 552608 | Clorf58 | 8 | 5 | 0 | Hs.46346S | UTP16 | 13 | 7 | 1 |
| Hs.470654 | CDCA7 | 8 | 5 | 0 | Hs. 492974 | WISP1 | 13 | 7 | 1 |
| Hs. 179'B8 | EMNL3 | 8 | 5 | 0 | Hs. 113876 | WHSC1 | 13 | 7 | 1 |
| Hs. 143618 | GEMIN6 | 8 | 5 | 0 | Hs. 494614 | BAT2D1 | 15 | 8 | 2 |
| Hs.6459 | GPRI72A | 8 | 5 | 0 | Hs. 166463 | HNRPU | 19 | 10 | 2 |
| Hs. 133294 | IQGAP3 | 8 | 5 | 0 | | | | | |

| | | | | | | | | | |
|---|---|---|---|---|---|---|---|---|---|
| No number of studies (types of cancer) which have available expression data on a test gene. Up # or down # number of cancer types whose expression of the tested gene is up or down regulated. All these genes are significantly consistently up-regulated (P<10) in a large majority of cancer types. doi: 10.137/journal pone. 0001149.001 | | | | | | | | | |

| **Table 5: Commonly Downregulated Genes in Cancers** | | | | | | | | | |
|---|---|---|---|---|---|---|---|---|---|
| **UnlGene** | **Gene symbol** | **N** | **Up #** | **Down #** | **UniGene** | **Gene symbol** | **N** | **Up #** | **Down #** |
| Hs. 401835 | TCEA12 | 10 | 0 | 8 | Hs. 306083 | LOC91689 | 8 | 0 | 5 |
| Hs. 58351 | ABCA8 | 13 | 0 | 10 | Hs. 160953 | PS3AIP1 | 8 | 0 | 5 |
| Hs. 525205 | NDRG2 | 12 | 0 | 9 | Hs.2112252 | SLC24A3 | 8 | 0 | 5 |
| Hs. 524085 | USP2 12 0 9 | | | | Hs. 163079 | TUBAL3 | 8 | 0 | 5 |
| Hs. 172755 | BRP44L | 11 | 0 | 8 | Hs. 389171 | PINK1 | 13 | 0 | 8 |
| Hs. 22242 | ECHDC3 | 11 | 0 | 8 | Hs. 470887 | GULP1 | 13 | 1 | 8 |
| Hs. 196952 | HLF | 19 | 1 | 13 | Hs. 490981 | MSRA | 13 | 1 | 8 |
| Hs.496587 | CHRDL1 | 12 | 0 | 8 | Hs. 476092 | CLEC3B | 18 | 0 | 11 |
| Hs. 476319 | ECHDC2 | 12 | 0 | 8 | Hs.386502 | FMO4 | 18 | 0 | 11 |
| Hs. 409352 | FLJ20701 | 12 | 0 | 8 | Hs. 137367 | ANK2 | 18 | 1 | 11 |
| Hs. 103253 | PLIN | 12 | 0 | 8 | Hs. 212088 | EPHX2 | 18 | 1 | 11 |
| Hs. 293970 | ALDH6A1 | 18 | 1 | 12 | Hs. 157818 | KCNAB1 | 18 | 1 | 11 |
| Hs. 390729 | ERBB4 | 17 | 0 | 11 | Hs. 163924 | NR3C2 | 18 | 1 | 11 |
| Hs. 553502 | RORA | 17 | 0 | 11 | Hs. 269128 | PPP2R1B | 18 | 1 | 11 |
| Hs. 388918 | RECK | 14 | 0 | 9 | Hs.40582 | CDC148 | 15 | 1 | 9 |
| Hs. 216226 | SYNGR1 | 14 | 0 | 9 | Hs. 438867 | FL20489 | 10 | 1 | 6 |
| Hs.506357 | fam107a | 14 | 1 | 9 | Hs. 224008 | FEZ1 | 17 | 1 | 10 |
| Hs.476454 | ABHD6 | 11 | 0 | 7 | Hs.443789 | C6orf60 | 12 | 1 | 7 |
| Hs. 519694 | Csorf4 | 11 | 0 | 7 | Hs. 475319 | LRRFIP2 | 12 | 1 | 7 |
| Hs. 528385 | DHR54 | 11 | 0 | 7 | Hs. 514713 | MPPE1 | 12 | 1 | 7 |
| Hs. 477288 | TRPM3 | 1 | 0 | 7 | Hs. 183153 | ARL4D | 19 | 1 | 11 |
| Hs. 420830 | HIF3A | 11 | 1 | 7 | Hs. 642660 | C10orfl116 | 19 | 1 | 11 |
| Hs. 511265 | SEMA6D | 11 | 1 | 7 | Hs. 495912 | DMD | 19 | 1 | 11 |
| Hs.436657 | CLU | 19 | 1 | 12 | Hs. 503126 | SHANK2 | 14 | 1 | 8 |
| Hs. 78482 | PALM | 16 | 0 | 10 | Hs. 481342 | SORBS2 | 14 | 1 | 8 |
| Hs. 82318 | WASF3 | 16 | 0 | 10 | Hs. 169441 | MAGI1 | 16 | 1 | 9 |
| Hs. 268869 | ADHFE1 | 8 | 0 | 5 | Hs.75652 | GSTM5 | 18 | 1 | 10 |
| Hs. 34494 | AGXT2 | 8 | 0 | 5 | Hs. 405156 | PPAP28 | 18 | 1 | 10 |
| Hs. 249129 | CIDEA | 8 | 0 | 5 | Hs. 271771 | SNCA | 18 | 1 | 10 |
| Hs. 302754 | EFCBP1 | 8 | 0 | 5 | Hs. 181855 | CASC5 | 9 | 1 | 5 |
| Hs.521953 | EFHC2 | 8 | 0 | 5 | Hs.506458 | ANKS1B | 11 | 1 | 6 |
| Hs. 200100 | E11s1 | 8 | 0 | 5 | Hs. 445885 | KIAA1217 | 11 | 1 | 6 |
| Hs. 479703 | FL21511 | 8 | 0 | 5 | Hs.643583 | DKFZp667G2110 | 13 | 1 | 7 |
| Hs.. 500750 | HPSE2 | 8 | 0 | 5 | Hs. 406787 | FBX03 | 13 | 1 | 7 |
| Hs. 380929 | LDHD | 8 | 0 | 5 | Hs. 431498 | FOXP1 | 13 | 1 | 7 |

| | | | | | | | | | |
|---|---|---|---|---|---|---|---|---|---|
| All these genes are significantly consistently down-regulated (P < 10⁻⁵) in a large majority of cancer types. doi: 10.1371/journal.pone.0001149.t002 | | | | | | | | | |

| **Table 6: Commonly Upregulated Genes in Pancreatic Cancer** | | | |
|---|---|---|---|
| **Accession** | **Gene Symbol** | **Gene Name** | **FC** |
| NM 006475 | POSTN | periostin, osteoblast specific factor | 13.28 |
| NM 005980 | S100P | S100 calcium binding protein P | 12.36 |
| NM 004385 | CSPG2 | chondroitin sulfate proteoglycan 2 (versican) | 10.57 |
| NM 003118 | SPARC | secreted protein, acidic cysteine-rich (osteonectin) | 10.46 |
| NM 003225 | TFF1 | trefoil factor 1 (breast cancer, estrogen-inducible sequence expressed in) | 8.13 |
| NM 002026 | FN1 | fibronectin 1 | 7.93 |
| NM 006142 | SFN | stratifin | 7.81 |
| NM 000393 | COL5A2 | collagen, type V, alpha 2 | 7.22 |
| NM 005940 | MMP11 | matrix metalloproteinase 11 (stromelysin 3) | 7.17 |
| NM 000088 | COL1A1 | collagen, type I, alpha 1 | 6.50 |
| NM 000930 | PLAT | plasminogen activator, tissue | 6.46 |
| NM 003064 | SLPI | secretory leukocyte protease inhibitor (antileukoproteinase) | 6.01 |
| NM 006516 | SLC2A1 | solute carrier family 2 (facilitated glucose transporter), member 1 | 5.39 |
| NM 003226 | TFF3 | trefoil factor 3 (intestinal) | 5.28 |
| NM 004460 | FAP | fibroblast activation protein alpha | 5.20 |
| NM 003467 | CXCR4 | chemokine (C-X-C motif) receptor 4 | 5.18 |
| NM 003247 | THBS2 | thrombospondin 2 | 5.04 |
| NM 012101 | TRIM29 | tripartite motif-containing | 4.91 |
| NM 033664 | CDH11 | cadherin 11, type 2, OB-cadherin (osteoblast) | 4.52 |
| NM 006169 | NNMT | nicotinamide N-methvltransferase | 4.51 |
| NM 004425 | ECM1 | extracellular matrix protein 1 | 4.39 |
| NM 003358 | UGCG | UDP-glucose ceramide glucosyltransferase | 4.36 |
| NM 000700 | ANXA1 | annexin A1 | 4.31 |
| NM 004772 | C5orf13 | chromosome 5 open reading frame 13 | 4.29 |
| NM 182470 | PKM2 | pyruvate kinase, muscle | 4.28 |
| NM 004994 | MMP9 | matrix metalloproteinase 9 (gelatinase B, 92kDa gelatinase, 92kDa type IV collagenase) | 4.19 |
| NM 006868 | RAB31 | RAB31, member RAS oncogene family | 4.18 |
| NM 001932 | MPP3 | membrane protein, palmitoylated 3 (MAGUK p55 subfamily member 3) | 4.16 |
| AF200348 | D2S448 | Melanoma associated gene | 4.14 |
| NM 000574 | DAF | decay accelerating factor for complement (CD55, Cromer blood group system) | 4.11 |
| NM 000213 | ITGB4 | integrin beta | 4.11 |
| NM 001645 | APOC1 | apolipoprotein C-I | 3.86 |
| NM 198129 | LAMA3 | laminin alpha 3 | 3.86 |
| NM 002997 | SDC1 | syndecan 1 | 3.80 |
| NM 001769 | CD9 | CD9 antigen (p24) | 3.78 |
| BC004376 | ANXA8 | annexim A8 | 3.74 |
| NM 005620 | S100A11 | S100 calcium binding protein A11 (calgizzarin) | 3.72 |
| NM 002659 | PLAUR | plasminogen activator urokinase receptor | 3.70 |
| NM 002966 | S100A10 | S100 calcium binding protein A10 (annexin II ligand, calpactin I, light polypeptide (p11)) | 3.67 |
| NM 004898 | CLOCK | clock homolog (mouse) | 3.65 |
| NM 002345 | LUM | lumican | 3.59 |
| NM 006097 | MYL9 | myosin light polypeptide 9, regulatory | 3.44 |
| NM 004120 | GBP2 | guanylate binding protein 2, interferon-inducible | 3.44 |
| AK056875 | LOC91316 | similar to bK246H3.1 (immunoglobulin lambda-like polypeptide 1, pre-B-cell specific) | 3.40 |
| NM 001827 | CKS2 | CDC28 protein kinase requlatorv subunit 2 | 3.36 |
| NM 002203 | ITGA2 | integrin alpha 2 (CD49B, alpha 2 subunit of VLA-2 receptor) | 3.35 |
| NM 000599 | IGFBP5 | insulin-like growth factor binding protein 5 | 3.33 |
| NM 004530 | MMP2 | matrix metalloproteinase 2 (gelatinase A, 72kDa gelatinase, 72kDa type IV collagenase) | 3.33 |
| NM 004335 | BST2 | bone marrow stromal cell antigen | 3.30 |
| NM 000593 | TAP1 | transporter 1, ATP-binding cassette, sub-family B (MDR/TAP) | 3.29 |
| NM 004915 | ABCG1 | ATP-bindina cassette sub-familv G (WHITE), member | 3.27 |
| NM 001235 | SERPINH 1 | serine (or cysteine) proteinase inhibitor, clade H (heat shock protein 47), member 1 (collagen binding protein 1) | 3.25 |
| NM 001165 | BIRC3 | baculoviral IAP repeat-containing 3 | 3.23 |
| NM 002658 | PLAU | plasminogen activator, urokinase | 3.20 |
| NM 021103 | TMSB 10 | thymosin, beta 10 | 3.18 |
| NM 000304 | PMP22 | peripheral myelin protein 22 | 3.15 |
| XM 371541 | K1AA1641 | KIAA1641 protein | 3.11 |
| NM 012329 | MMD | monocyte to macrophage differentiation-associated | 3.07 |
| NM 182744 | NBL1 | neuroblastoma suppression of tumorigenicity 1 | 3.06 |
| NM 002245 | KCNK1 | potassium channel , subfamily K, member 1 | 3.03 |
| NM 000627 | LTBP1 | latent transforming growth factor beta binding protein 1 | 3.02 |
| NM 000063 | C2 | complement component 2 | 3.01 |
| NM 000100 | CSTB | cystatin B (stefin B) | 2.99 |
| NM 000396 | CTSK | cathepsin K (pycnodysostosis) | 2.98 |
| NM 016816 | OAS1 | 2' 5'-oliaoadenylate synthetase 1, 40/46kDa | 2.98 |
| NM 004240 | TRIP 10 | thyroid hormone receptor interactor 10 | 2.95 |
| NM 000138 | FBN1 | fibrillin 1 (Marfan syndrome) | 2.94 |
| NM 002318 | LOXL2 | lysyl oxidase-like 2 | 2.92 |
| NM 002053 | GBP1 | guanylate binding orotein 1 interferon-inducible, lysyl 67kDa | 2.90 |
| NM 005564 | LCN2 | lipocalin 2 (oncogene 24p3) | 2.88 |
| NM 153490 | KRT13 | keratin 13 | 2.85 |
| NM 004723 | ARHGEF 2 | rho/rac guanine nucleotide exchange factor (GEF) 2 | 2.80 |
| NM 004146 | NDUFB7 | NADH dehydrozenase (ubiquinone) 1 beta subcomplex, 7, 18kDa | 2.79 |
| NM 003937 | KYNU | kynureninase (L-kynurenine hydrolase) | 2.77 |
| NM 002574 | PRDX1 | Peroxiredoxin 1 | 2.77 |
| NM 002444 | MSN | moesin | 2.73 |
| NM 002901 | RCN1 | reticulocalbin 1, EF-hand calcium binding domain | 2.73 |
| NM 005165 | ALDOC | aldolase C, fructose-bisphosphate | 2.72 |
| NM 002204 | ITGA3 | integrin, alpha 3 (antigen CD49C, alpha 3 subunit of VLA-3 receptor) | 2.72 |
| NM 033138 | CALD1 | caldesmon 1 | 2.71 |
| NM 003816 | ADAM9 | a disintegrin and metalloproteinase domain 9 (meltrin gamma) | 2.69 |
| NM 173843 | IL1RN | interleukin 1 receptor antagonist | 2.66 |
| NM 000602 | SERPINE 1 | serine (or cysteine) proteinase inhibitor, clade E (nexin, plasminggen activator inhibitor type 1), member 1 | 2.65 |
| NM 002213 | ITGB5 | integrin, beta 5 | 2.64 |
| NM 004447 | EPS8 | epidermal growth factor receptor pathway substrate 8 | 2.64 |
| NM 002928 | RGS16 | regulator of G-protein singalling 16 | 2.62 |
| NM 001288 | CLIC1 | chloride intracellular channel 1 | 2.61 |
| NM 015996 | TAGLN | transgelin | 2.57 |
| NM 002087 | GRN | granulin | 2.55 |
| NM 001183 | ATP6AP1 | ATPase, H+ transporting, lysosomal accessory protein 1 | 2.54 |
| NM 001730 | KLF5 | Kruppel-like factor 5 (intestinal) | 2.51 |
| NM 003516 | HIST2H2 AA | histone 2, H2aa | 2.50 |
| NM 014736 | KIAA0101 | KIAA0101 gene product | 2.49 |
| NM 002290 | LAMA4 | laminin alpha 4 | 2.49 |
| NM 001826 | CKS1B | CDC28 protein kinase reaulatory subunit 1B | 2.48 |
| NM 001814 | CTSC | cathepsin C | 2.45 |
| NM 176825 | SULT1C1 | sulfotransferase family cytosolic, 1C, member 1 | 2.43 |
| NM 002862 | PYGB | phosphorylase, glycogen; brain | 2.41 |
| NM 000917 | P4HA1 | procollagen-proline, 2-oxoglutarate 4-dioxygenase (proline 4- hydroxylase), alpha polypeptideI | 2.41 |
| NM 001428 | EN01 | enolase 1 (alpha) | 2.40 |
| NM 001425 | EMP3 | epithelial membrane protein 3 | 2.40 |
| NM 019111 | HLA-DRA | maior histocompatibility complex, class II, DR alpha | 2.38 |
| NM 001387 | DPYSL3 | dihydropyrimidinase-like 3 | 2.36 |
| NM 006471 | MRCL3 | myosin regulatory light chain MRCL3 | 2.34 |
| NM 006332 | IFI30 | interferon gamma-inducible protein 30 | 2.34 |
| NM 001312 | CRIP2 | cysteine-rich protein 2 | 2.33 |
| NM 002224 | ITPR3 | inositol 1 4 5-triphosphate receptor tvpe 3 | 2.31 |
| NM 053025 | MYLK | myosin light peptide kinase | 2.29 |
| NM 002785 | PSG11 | pregnancy specific beta-1-glycoprotein 11 | 2.27 |
| NM 000900 | MGP | matrix Gla protein | 2.26 |
| NM 000962 | PTGS 1 | prostaglandin-endoperoxide synthase 1 (prostaglandin G/H synthase and cyclooxyenase) | 2.25 |
| NM 005915 | MCM6 | minichromosome maintenance deficient 6 (MIS5 homolog, S. pombe) (S. cerevisiae) | 2.24 |
| NM 001067 | TOP2A | topoisomerase (DNA) II alpha 170kDa | 2.23 |
| NM 001878 | CRABP2 | cellular retinoic acid binding protein 2 | 2.23 |
| NM 006745 | SC4MOL | sterol-C4-methyl oxidase-like | 2.22 |
| NM 003528 | HIST2H2 | histone 2, H2be | 2.22 |
| BF347579 | | Transcribed sequence with strong similarity to protein pir:I38500 (H.sapiens) 138500 interferon gamma receptor accessory factor-1 precursor - human | 2.21 |
| NM 005261 | GEM | GTP binding protein overexpressed in skeletal muscle | 2.19 |
| NM 021874 | CDC25B | cell division cycle 25B | 2.18 |
| NM 022550 | XRCC4 | X-ray repair complementing defective repair in Chinese hamster cells 4 | 2.17 |
| NM 020250 | GSN | gelsolin (amyloidosis, Finnish type) | 2.17 |
| NM 002916 | RFC4 | replication factor C (activator 1) 4, 37kDa | 2.16 |
| NM 005606 | LGMN | legumain | 2.14 |
| NM 006762 | LAPTM5 | Lysosomal-associated multispanning membrane protein-5 | 2.14 |
| NM 002727 | PRG1 | proteoglycan 1, secretory granule | 2.14 |
| NM 002609 | PDGFRB | platelet-derived growth factor receptor, beta polypeptide | 2.14 |
| NM 001424 | EMP2 | epithelial membrane protein 2 | 2.12 |
| NM 005022 | PFN1 | profilin 1 | 2.12 |
| NM 001657 | AREG | amphiregulin amphireaulin (schwannoma-derived growth factor) | 2.11 |
| NM 005100 | AKAP12 | A kinase (PRKA) anchor protein (gravin) 12 | 2.11 |
| NM 000860 | HPGD | hydroxyprostaglandin dehydrogenase 15 (NAD) | 2.10 |
| NM 007115 | TNFAIP6 | tumor necrosis factor alpha-induced protein 6 | 2.09 |
| NM 021638 | AFAP | actin filament associated protein | 2.08 |
| NM 001946 | DUSP6 | dual specificity phosphatase 6 | 2.05 |
| NM 181802 | UBE2C | ubiquitin-coniugating enzyme E2C | 2.04 |
| NM 002593 | PCOLCE | procollagen C-endopeptidase enhancer | 2.02 |
| NM 033292 | CASP1 | caspase 1, apoptosis-related cysteine protease (interleukin 1, beta, convertase) | 2.02 |
| NM 003870 | IQGAP1 | IQ motif containing GTPase activating protein 1 | 2.02 |
| NM 005563 | STMN1 | stathmin 1/oncoprotein 18 | 2.01 |
| NM 005558 | LAD1 | ladinin 1 | 2.01 |
| NM 001776 | ENTPD1 | ectonucleoside triphosphate diphosphohydrolase 1 | 2.00 |
| NM_001299 | CNN1 | calponin 1, basic, smooth muscle | 2.00 |
| AK055128 | PSMD14 | proteasome (prosome, macropain) 26S subunit, non-ATPase, 14 | 2.00 |
| NM_006304 | SHFM1 | split hand/foot malformation (ectrodactyly) type 1 | 1.98 |
| NM_004024 | ATF3 | activating transcription factor 3 | 1.98 |
| NM_000291 | PGK1 | phosphoglycerate kinase 1 | 1.98 |
| NM_006520 | TCTE1L | t-complex-associated-testis-expressed 1-like | 1.97 |
| NM_201380 | PLEC1 | plectin 1 intermediate filament binding protein 500kDa | 1.97 |
| NM_002838 | PTPRC | protein tyrosine phosphatase, receptor type, C | 1.97 |
| NM_000211 | ITGB2 | integrin, beta 2 (antigen CD18 (p95), lymphocyte function-associated antigen 1; macrophage antigen 1 (mac-1) beta subunit) | 1.97 |
| NM_002577 | PAK2 | p21 (CDKN1A)-activated kinase 2 | 1.96 |
| NM_000295 | SERPINA 1 | serine (or cysteine) proteinase inhibitor, clade A (alpha-1 antiproteinase, antitrypsin), member 1 | 1.96 |
| NM_183001 | SHC1 | SHC (Src homology 2 domain containing transforming protein 1 | 1.96 |
| NM_005019 | PDE1A | phosphodiesterase 1A, calmodulin-dependent | 1.95 |
| NM_002298 | LCP1 | lymphocyte cytosolic protein 1 (L-plastin) | 1.95 |
| NM_006769 | LMO4 | LIM domain only 4 | 1.94 |
| NM_001465 | FYB | FYN binding protein (FYB-120/130) | 1.93 |
| NM_183422 | TSC22 | transforming growth factor beta-stimulated protein TSC-22 | 1.92 |
| NM_001777 | CD47 | CD47 antigen (Rh-related antigen, integrin-associated signal transducer) | 1.92 |
| NM 001755 | CBFB | core-binding factor, beta subunit | 1.90 |
| NM_005544 | IRS1 | insulin receptor substrate 1 | 1.88 |
| NM_000698 | ALOX5 | arachidonate 5-lipoxvgenase | 1.88 |
| NM_006096 | NDRG1 | N-myc downstream regulated gene 1 | 1.88 |
| NM_001105 | ACVR1 | activin A receptor, type 1 | 1.87 |
| NM 003105 | SORL1 | sortilin-related receptor, L(DLR class) A repeats-containing | 1.85 |
| NM_001998 | FBLN2 | fibulin 2 | 1.85 |
| NM_014791 | MELK | maternal embryonic leucine zipper kinase | 1.85 |
| NM_003092 | SNRPB2 | small nuclear ribonucleoprotein polvpeptide B | 1.84 |
| NM_001120 | TETRAN | tetracycline transporter-like protein | 1.84 |
| NM 182943 | PLOD2 | procollagen-lysine, 2-oxoglutarate 5-dioxygenase (lysine hydroxylase) 2 | 1.83 |
| NM_181862 | BACH | brain acyl-CoA hydrolase | 1.82 |
| NM_021102 | SPINT2 | serine protease inhibitor, Kunitz type, 2 | 1.82 |
| NM_004419 | DUSP5 | dual specificity phosphatase 5 | 1.81 |
| NM_006482 | DYRK2 | dual specificity tyrosine-(Y)-phosphorylation regulated kinase 2 | 1.81 |
| NM 145690 | YWHAZ | tyrosine 3-monooxypenase/tryptophan 5-monooxygenase activation protein, zeta polypeptide | 1.81 |
| NM_000714 | BZRP | benzodiazapine receptor (peripheral) | 1.81 |
| NM_013995 | LAMP2 | lysosomal-associated membrane protein 2 | 1.80 |
| CA450153 | ACYP1 | acylphosphatase 1, erythrocyte (common) type | 1.80 |
| NM 000405 | GM2A | GM2 ganglioside activator protein | 1.79 |
| NM_139275 | AKAP1 | A kinase (PRKA) anchor protein 1 | 1.79 |
| NM_001679 | ATP1B3 | ATPase, Na+/K+ transporting, beta 3 polypeptide | 1.79 |
| NM_016343 | CENPF | centromere protein F, 350/400ka (mitosin) | 1.79 |
| NM_002201 | ISG20 | interferon stimulated gene 20kDa | 1.79 |
| NM_002463 | MX2 | myxovirus (influenza virus) resistance 2 (mouse) | 1.79 |
| NM_006820 | C1orf29 | chromosome 1 open reading frame 29 | 1.79 |
| NM_201397 | GPX1 | glutathione peroxidase 1 | 1.79 |
| NM_005738 | ARL4 | ADP-ribosylation factor-like 4 | 1.78 |
| NM_001038 | SCNN1A | sodium channel nonvoltage-gated 1 alpha | 1.78 |
| NM 002863 | PYGL | phosphorylase, glycogen; liver (Hers disease, glycogen storage disease type VI) | 1.78 |
| NM_001281 | CKAP1 | cytoskeleton associated protein 1 | 1.77 |
| NM_003879 | CFLAR | CASP8 and FADD-like apoptosis regulator | 1.76 |
| NM_182948 | PRKACB | protein kinase, cAMP-dependent catalytic, beta | 1.75 |
| NM_006009 | TUBA3 | tubulin, alpha 3 | 1.75 |
| NM 201444 | DGKA | diacylglycerol kinase, alpha 80kDa | 1.74 |
| NM_005471 | GNPDA1 | glucosamine-6-phosphate deaminase 1 | 1.74 |
| NM_001451 | FOXF1 | forkheadboxFl | 1.74 |
| NM_001988 | EVPL | envoplakin | 1.73 |
| NM 021724 | NR1D1 | nuclear receptor subfamily 1, group D member 1 | 1.73 |
| NM_006364 | SEC23A | Sec23 homolog A (S. cerevisiae) | 1.72 |
| NM_002129 | HMGB2 | high-mobility group box 2 | 1.72 |
| NM_004172 | SLC1A3 | solute carrier family 1 (glial high affinity glutamate transporter), member 3 | 1.71 |
| NM_001421 | ELF4 | E74-like factor 4 (ets domain transcription factor) | 1.71 |
| NM 005566 | LDHA | lactate dehydrogenase A | 1.70 |
| NM_000270 | NP | nucleoside phosphorylase | 1.69 |
| NM_153425 | TRADD | TNFRSF1A-associated via death domain | 1.67 |
| NM_004762 | PSCD1 | pleckstrin homology, Sec7 and coiled-coil domains (cytohesin 1) | 1.67 |
| NM_001985 | ETFB | electron-transfer-flavoprotein, beta polypeptide | 1.67 |
| NM 016587 | CBX3 | chromobox homolog 3 (HP1 gamma homolog, Drosophila) | 1.66 |
| NM_002085 | GPX4 | glutathione peroxidase 4 (phospholipid hydroperoxidase) | 1.66 |
| NM_002795 | PSMB3 | proteasome (prosome, macropain) subunit, beta type, 3 | 1.65 |
| NM_000963 | PTGS2 | prostaglandin-endoperoxide synthase 2 (prostaglandin G/H synthase and cyclopxyoenase) | 1.65 |
| NM_001642 | APLP2 | amyloid beta (A4) precursor-like protein 2 | 1.65 |
| NM 000569 | FCGR3A | Fc fragment of 1gG low affinity iiia receptor for (CD16) | 1.64 |
| NM_000362 | TIMP3 | tissue inhibitor of metalloproteinase 3 (Sorsby fundus dystrophy, pse-udoinflammatory) | 1.63 |
| NM_002417 | MKI67 | antigen identified by monoclonal antibody Ki-67 | 1.63 |
| NM_000175 | GPI | glucose phosophate isomerase | 1.63 |
| AF179995 | SEPT8 | septin 8 | 1.62 |
| NM 004121 | GGTLA1 | gamma-glutamyltransferase-like activity 1 | 1.62 |
| NM_002690 | POLB | polymerase (DNA directed), beta | 1.62 |
| NM_004334 | BST1 | bone marrow stromal cell antigen 1 | 1.61 |
| NM_001892 | CSNK1A1 | casein kinase 1, alpha 1 | 1.61 |
| NM_014670 | BZW1 | basic leucine zipper and W2 domains 1 | 1.60 |
| NM_001110 | ADAM10 | a disintegrin and metalloproteinase domain 10 | 1.60 |
| NM_005792 | MPHOSP H6 | M-phase phosphoprotein 6 | 1.60 |
| NM_001126 | ADSS | adenylosuccinate synthase | 1.59 |
| XM 376059 | SERTAD2 | SERTA domain containing 2 | 1.59 |
| NM 001664 | ARHA | ras homolog gene family, member A | 1.59 |
| NM 002475 | MLC1SA | myosin light chain 1 slow a | 1.59 |
| NM_014498 | GOLPH4 | golgi phosphoprotein 4 | 1.59 |
| NM_005964 | MYH10 | myosin heavy polypeptide 10 non-muscle | 1.59 |
| NM_003330 | TXNRD 1 | thioredoxin reductase 1 | 1.59 |
| NM_001757 | CBR1 | carbonyl reductase 1 | 1.58 |
| NM_003130 | SRI | sorcin | 1.57 |
| NM_006765 | TUSC3 | tumor suppressor candidate 3 | 1.57 |
| NM_183047 | PRKCBP 1 | protein kinase C binding protein 1 | 1.57 |
| NM_005333 | HCCS | holocytochrome c synthase (cytochrome c heme-lyase) | 1.57 |
| NM 001444 | FABP5 | fatty acid binding protein 5 (psoriasis-associated) | 1.57 |
| NM_001799 | CDK7 | cyclin-dependent kinase 7 (M015 homolog, Xenopus laevis, cdk-activating kinase) | 1.57 |
| NM_001539 | DNAJA1 | DnaJ (Hsp40) homolog subfamily A member 1 | 1.57 |
| NM_004475 | FLOT2 | flotillin 2 | 1.57 |
| NM_004308 | ARHGAP 1 | Rho GTPase activating protein 1 | 1.56 |
| NM 002388 | MCM3 | MCM3 minichromosome maintenance deficient 3 (S. cerevisiae) | 1.56 |
| NM_006435 | IFITM2 | interferon induced transmembrane protein 2 (1-8D) | 1.56 |
| NM_000454 | SOD1 | superoxide dismutase 1, soluble (amyotrophic lateral sclerosis 1 (adult)) | 1.56 |
| NM_015161 | ARL6IP | ADP-ribosylation factor-like 6 interacting protein | 1.56 |
| NM_078480 | SIAHBP1 | fuse-binding protein-interacting repressor | 1.56 |
| NM 025207 | PP591 | FAD-svnthetase | 1.56 |
| NM_002833 | PTPN9 | protein tyrosine phosphatase non-receptor type 9 | 1.55 |
| NM_001753 | CAV1 | caveolin 1 caveolae protein 22kDa | 1.55 |
| NM_003286 | TOP1 | topoisomerase (DNA) I | 1.55 |
| BU739663 | | Transcribed sequence with moderate similarity to protein sp:P13196 (H.sapiens) HEM1_HUMAN 5-aminolevulinic acid synthase, nonspecific mitochondrial precursor | 1.55 |
| NM_006788 | RALBP1 | ralA binding protein 1 | 1.54 |
| NM_000944 | PPP3CA | protein phosphatase 3 (formerly 2B), catalytic subunit, alpha isoform (calcineurin A alpha) | 1.54 |
| NM_003374 | VDAC1 | voltaqe-dependent anion channel 1 | 1.54 |
| NM_000560 | CD53 | CD53 antigen | 1.54 |
| NM 002037 | FYN | FYN oncogene related to SRC FGR, YES | 1.54 |
| NM_002885 | RAP1GA1 | RAP1 GTPase activating protein 1 | 1.53 |
| NM_018979 | PRKWNK 1 | lprotein kinase, lysine deficient 1 | 1.53 |
| NM_002835 | PTPN12 | protein tyrosine phosphatase, non-receptor type 12 | 1.53 |
| NM_007315 | STAT1 | signal transducer and activator of transcription 1, 91kDa | 1.52 |
| NM 014846 | KIAA0196 | KIAA0196 gene product | 1.52 |
| NM_001237 | CCNA2 | cyclin A2 | 1.52 |
| NM_004596 | SNRPA | small nuclear ribonucleoprotein polypeptide A | 1.52 |
| NM_002790 | PSMA5 | proteasome (prosome, macropoain) subunit, alpha type, 5 | 1.52 |
| NM 015361 | R3HDM | R3H domain (binds single-stranded nucleic acids) containing | 1.52 |
| NM_001665 | ARHG | ras homolog gene family, member G (rho G) | 1.51 |
| NM_002788 | PSMA3 | proteasome (prosome macropain) subunit, alpha type, 3 | 1.50 |
| NM_006904 | PRKDC | protein kinase, DNA-activated, catalytic polypeptide | 1.50 |
| NM_003400 | XPO1 | exportin 1 (CRM1 homolog, yeast) | 1.50 |
| NM 178014 | OK/SW-cl.56 | beta 5-tubulin | 1.50 |
| NM_002634 | PHB | prohibitin | 1.49 |
| NM_004792 | PPIG | peptidyl-prolyl isomerase G (cyclophilin G) | 1.49 |
| NM_002508 | NID | nidogen (enactin) | 1.49 |
| NM_001765 | CD1C | CD1C antigen, c polypeptide | 1.48 |
| NM_000311 | PRNP | prion protein (p27-30) (Creutzfeld-Jakob disease, Gerstmann-Strausler-Scheinker syndrome, fatal familial insomnia) | 1.48 |
| NM_006437 | ADPRTL1 | ADP-ribosyltransferase (NAD+; poly (ADP-ribose) polymerase)-like 1 | 1.48 |
| NM_002759 | PRKR | protein kinase, interferon-inducible double stranded RNA dependent | 1.48 |
| NM_014669 | KIAA0095 | KIAA0095 gene product | 1.47 |
| NM 003391 | WNT2 | wingless-type MMTV integration site family member 2 | 1.47 |
| NM_004309 | ARHGDIA | Rho GDP dissociation inhibitor (GDI) alpha | 1.47 |
| NM_000418 | IL4R | interleukin 4 receptor | 1.46 |
| NM_003352 | UBL1 | ubiquitin-like 1 (sentrin) | 1.46 |
| NM_006290 | TNFAIP3 | tumor necrosis factor alpha-induced protein 3 | 1.45 |
| NM 004763 | ITGB1BP1 | integrin beta 1 binding protein 1 | 1.45 |
| NM_005754 | G3BP | Ras-GTPase-activating protein SH3-domain-binding protein | 1.45 |
| NM_021990 | GABRE | gamma-aminobutyric acid (GABA) A receptor, epsilon | 1.44 |
| NM_001379 | DNMT1 | DNA (cytosine-5-)-methyltransferase 1 | 1.44 |
| NM_001154 | ANXA5 | annexin A5 | 1.44 |
| NM 004354 | CCNG2 | cyclin G2 | 1.44 |
| NM_005002 | NDUFA9 | NADH dehydroaenase (ubiquinone) 1 alpha subcomplex, 9, 39kDa | 1.43 |
| NM_001931 | DLAT | dihydrolipoamide S-acetyltransferase (E2 component of pyruvate dehydroaenase complex) | 1.43 |
| NM_005902 | MADH3 | MAD mothers against decapentaplegic homolog 3 (Drosophila) | 1.43 |
| NM_000110 | DPYD | dihydropyrimidine dehydrogenase | 1.43 |
| NM 001316 | CSE1L | CSE1 chromosome segregation 1-like (veast) | 1.43 |
| NM_000167 | GK | glycerol kinase | 1.43 |
| NM_001924 | GADD45 A | growth arrest and DNA-damage-inducible, alpha | 1.42 |
| NM_014225 | PPP2R1A | protein phosphatase 2 (formerly 2A), regulatory subunit A (PR 65), alpha isoform | 1.42 |
| NM_001233 | CAV2 | caveolin 2 | 1.42 |
| NM_176863 | PSME3 | proteasome (prosome, macropain) activator subunit 3 (PA28 gamma; Ki) | 1.42 |
| NM_001905 | CTPS | CTP synthase | 1.41 |
| NM_005653 | TFCP2 | transcription factor CP2 | 1.41 |
| NM_003405 | YWHAH | tyrosine 3-monooxygenase/tryptophan 5-monooxygenase activation protein, eta polypeptide | 1.41 |
| NM_003392 | WNT5A | wingless-type MMTV integration site family, member 5A | 1.40 |
| NM 002375 | MAP4 | microtubule-associated protein 4 | 1.40 |
| NM_006353 | HMGN4 | high mobility qroup nucleosomal binding domain 4 | 1.39 |
| NM_006527 | SLBP | stem-loop (histone) bindino protein | 1.39 |
| NM_000517 | HBA2 | hemoglobin alpha 2 | 1.38 |
| NM_002661 | PLCG2 | phospholipase C, gamma 2 (phosphatidylinositol-specific) | 1.38 |
| NM 001493 | GDI1 | GDP dissociation inhibitor 1 | 1.38 |
| NM_181430 | FOXK2 | forkhead box K2 | 1.38 |
| NM_002086 | GRB2 | growth factor receptor-bound protein 2 | 1.38 |
| NM_002868 | RAB5B | RAB5B, member RAS oncogene family | 1.37 |
| NM_002768 | PCOLN3 | procollagen (type III) N-endopeptidase | 1.37 |
| NM_014742 | TM9SF4 | transmembrane 9 superfamily protein member 4 | 1.37 |
| NM_004344 | CETN2 | centrin, EF-hand protein, 2 | 1.37 |
| NM_002881 | RALB | v-ral simian leukemia viral oncogene homolog B (ras related; GTP binding protein) | 1.36 |
| NM_004099 | STOM | stomatin | 1.36 |
| NM_031844 | HNRPU | heterogeneous nuclear ribonucleoprotein U (scaffold attachment factor A) | 1.36 |
| NM_000480 | AMPD3 | adenosine monophosphate deaminase (isoform E) | 1.35 |
| NM_006561 | CUGBP2 | CUG triplet repeat RNA binding protein 2 | 1.35 |
| NM_152879 | DGKD | diacylglycerol kinase delta 130kDa | 1.35 |
| NM_138558 | PPP1R8 | protein phosphatase 1 reQulatory (inhibitor) subunit 8 | 1.35 |
| NM 004941 | DHX8 | DEAH (Asp-Glu-Ala-His) box polypeptide 8 | 1.34 |
| NM_021079 | NMT1 | N-myristoyltransferase 1 | 1.33 |
| NM_004622 | TSN | translin | 1.33 |
| NM_002473 | MYH9 | myosin, heavy polypeptide 9, non-muscle | 1.33 |
| NM_006889 | CD86 | CD86 antigen (CD28 antigen ligand 2, B7-2 antigen) | 1.33 |
| NM 004383 | CSK | c-src tyrosine kinase | 1.33 |
| NM_004317 | ASNA1 | arsA arsenite transoorter ATP-binding homolog 1 (bacterial) | 1.33 |
| NM_024298 | LENG4 | leukocyte receptor cluster (LRC) member 4 | 1.32 |
| NM_001912 | CTSL | cathepsin L | 1.32 |
| NM 001357 | DHX9 | DEAH (Asp-Glu-Ala-His) box polypeptide 9 | 1.32 |
| NM_006849 | PDIP | protein disulfide isomerase, pancreatic | 1.32 |
| NM_018457 | DKFZP56 4J157 | DKFZ, 0564J157 protein | 1.31 |
| NM 024880 | TCF7L2 | transcription factor 7-like 2 (T-cell specific, HMG-box) | 1.31 |
| NM_002081 | GPC1 | glypican 1 | 1.31 |
| NM 004235 | KLF4 | Kruppel-like factor 4 (gut) | 1.31 |
| NM_005565 | LCP2 | lymphocyte cytosolic protein 2 (SH2 domain containing leukocyte protein of 76kDa) | 1.30 |
| NM_002667 | PLN | phospholamban | 1.30 |
| NM_004946 | DOCK2 | dedicator of cytokinesis 2 | 1.30 |
| NM_002035 | FVT1 | follicular lymphoma variant translocation 1 | 1.29 |
| NM_002865 | RAB2 | RAB2 member RAS oncogene family | 1.29 |
| NM_002806 | PSMC6 | proteasome (prosome macropain) 26S subunit ATPase 6 | 1.29 |
| NM_004240 | TRIP 10 | thyroid hormone receptor interactor 10 | 1.28 |
| NM_003760 | EIF4G3 | eukaryotic translation initiation factor 4 gamma, 3 | 1.28 |
| NM 005151 | USP14 | ubiquitin specific protease 14 (tRNA quanine transglycosylase) | 1.28 |
| NM_015922 | H105E3 | NAD(P) deoendent steroid dehydropenase-like | 1.27 |
| NM_033306 | CASP4 | caspase 4 apoptosis-related cysteine protease | 1.27 |
| NM_198189 | COPS8 | COP9 constitutive photomorohogenic homolog subunit 8 (Arabidopsis) | 1.27 |
| NM_001933 | DLST | dihydrolipoamide S-succinyltransferase (E2 component of 2-oxo-qlutarate complex) | 1.27 |
| NM 015004 | K1AA0116 | K1AA0 116 protein | 1.27 |
| NM_033362 | MRPS12 | mitochondrial ribosomal protein S12 | 1.27 |
| NM_004180 | TANK | TRAF family member-associated NFKB activator | 1.26 |
| NM_014734 | K1AA0247 | K1AA0247 | 1.26 |
| NM_005271 | GLUD1 | glutamate dehydropenase 1 | 1.25 |
| NM_003009 | SEPW1 | selenoprotein W, 1 | 1.25 |
| NM_182641 | FALZ | fetal Alzheimer antigen | 1.24 |
| NM_007362 | NCBP2 | nuclear cap binding protein subunit 2 20kDa | 1.24 |
| NM_004292 | RIN1 | Ras and Rab interactor 1 | 1.24 |
| NM_014608 | CYFIP1 | cytoplasmic FMR1 interacting protein 1 | 1.23 |
| NM 022333 | TIAL1 | TIA1 cytotoxic oranule-associated RNA binding protein-like 1 | 1.23 |
| NM_003126 | SPTA1 | spectrin alpha erythrocytic 1 (elliptocytosis 2) | 1.22 |
| NM_014602 | PIK3R4 | phosphoinositide-3-kinase regulatory subunit 4, p150 | 1.18 |
| NM_002194 | INPP1 | inositol polyphosphate-1-phosphatase | 1.16 |

| | | | |
|---|---|---|---|
| Note: Accession IDs NM_XXXX are uniquely assigned to each gene by National Center for Biotechnology Information (NCBI) (http://www.ncbi.nlm.nih.gov/sites/entrez?db=nuccore). | | | |

| **Table 7: Commonly Downregulated Genes in Pancreatic Cancer** | | | |
|---|---|---|---|
| **Accession** | **Gene Symbol** | **Gene Name** | **FC** |
| NM 006499 | LGALS8 | galoctosite-binding, soluble, 8 (galectin 8) | 0.87 |
| NM_000466 | PEX1 | peroxisome biogenesis factor 1 | 0.81 |
| NM_002766 | PRPSAP1 | phosphoribosyl pyrophosphate synthetase-associated protein 1 | 0.81 |
| NM_147131 | GALT | galactose-1-phosphate uridylyltransferase | 0.80 |
| NM_002101 | GYPC | glycophorin C (Gerbich blood group) | 0.80 |
| NM_002880 | RAF1 | v-raf-1 murine leukemia viral oncogene homolog 1 | 0.80 |
| NM_004649 | C218rf33 | chromosome 21 open reading frame 33 | 0.80 |
| NM_003262 | TLOC1 | translocation protein 1 | 0.79 |
| NM_147223 | NCOA1 | nuclear receptor coactivator 1 | 0.79 |
| NM_007062 | PWP1 | nuclear phosphoprotein similar to S. cerevisiae PWP 1 | 0.79 |
| NM 005561 | LAMP1 | lysosomal-associated membrane protein 1 | 0.79 |
| NM_006810 | PDIR | for protein disulfide isomerase-related | 0.78 |
| NM_033360 | KRAS2 | v-Ki-ras2 Kirsten rat sarcoma 2 viral oncogene homolog | 0.77 |
| NM_001513 | GSTZ1 | glutathione transferase zeta 1 (maleylacetoacetate isomerase) | 0.77 |
| NM_006184 | NUCB1 | nucleobindin 1 | 0.77 |
| NM 001634 | AMD1 | adenosvlmethionine decarboxylase 1 | 0.76 |
| NM_006749 | SLC20A2 | solute carrier family 20 (phosphate transporter), member 2 | 0.76 |
| NM_003144 | SSR1 | signal sequence receptor alpha (translocon-associated protein alpha) | 0.76 |
| NM_004606 | TAF1 | TAF1 RNA polymerase II, TATA box binding protein (TBP)-associated factor 250kDa | 0.75 |
| BX648788 | | MRNA; cDNA DKFZP686M12165 (from clone DKFZP686M12165) | 0.75 |
| NM 004035 | ACOX1 | acyl-Coenzyme A oxidase 1 palmitoyl | 0.74 |
| NM_000287 | PEX6 | peroxisomal biogenesis factor 6 | 0.73 |
| NM_003884 | PCAF | p300/CBP-associated factor | 0.73 |
| NM_006870 | DSTN | destrin (actin depolymerizing factor) | 0.73 |
| NM_001604 | PAX6 | paired box gene 6 (aniridia keratitis) | 0.72 |
| NM_000722 | CACNA2 D1 | calcium channel voltage-dependent alpha 2/delta subunit 1 | 0.72 |
| NM_033022 | RPS24 | ribosomal protein S24 | 0.72 |
| NM_004563 | PCK2 | phosphoenolpyruvate carboxykinase 2 (mitochondrial) | 0.72 |
| NM_002602 | PDE6G | phosphodiesterase 6G cGMP-specific, rod, gamma | 0.72 |
| NM_001889 | CRYZ | crystalline, zeta (quinone reductase) | 0.72 |
| NM 002339 | LSP1 | lymphocyte-specific protein 1 | 0.72 |
| NM_016848 | SHC3 | src homology 2 domain containing transforming protein C3 | 0.71 |
| NM_002906 | RDX | radixin | 0.71 |
| NM_007014 | WWP2 | Nedd-4-like ubiquitin-protein ligase | 0.71 |
| NM_000414 | HSD17B4 | hydroxysteroid (17-beta) dehydrogenase 4 | 0.71 |
| NM_001127 | AP1B1 | adaptor-related protein complex 1, beta 1 subunit | 0.71 |
| NM_002402 | MEST | mesoderm specific transcript homolog (mouse) | 0.70 |
| NM_033251 | RPL13 | ribosomal protein L13 | 0.70 |
| NM_139069 | MAPK9 | mitogen-activated protein kinase 9 | 0.70 |
| NM_002913 | RFC1 | replication factor C (activator 1) 1, 145kDa | 0.70 |
| NM_000487 | ARSA | arvlsulfatase A | 0.70 |
| NM_006973 | ZNF32 | zinc finger protein 32 (KOX 30) | 0.70 |
| NM_005310 | GRB7 | growth factor receptor-bound protein 7 | 0.70 |
| NM_005962 | MX11 | MAX interacting protein 1 | 0.69 |
| NM 005359 | MADH4 | MAD, mothers against decapentaplegic homolog 4 (Drosophila) | 0.69 |
| NM_002340 | LSS | lanosterol synthase (2 3-oxidosqualene-lanosterol cyclase) | 0.69 |
| NM_003684 | MKNK1 | MAP kinase-interacting serine/threonine kinase 1 | 0.68 |
| NM_005671 | D8S2298 E | reproduction 8 | 0.68 |
| NM_000309 | PPOX | protoporphyrinogen oxidase | 0.68 |
| NM 000994 | RPL32 | ribosomal protein L32 | 0.68 |
| NM_000972 | RPL7A | ribosomal protein L7a | 0.68 |
| NM_005101 | G1P2 | interferon, alpha-inducible protein (clone IFI-15K) | 0.67 |
| NM_001129 | AEBP1 | AE binding protein 1 | 0.67 |
| NM_001011 | RPS7 | ribosomal protein S7 | 0.67 |
| NM_001153 | ANXA4 | annexin A4 | 0.67 |
| NM_012335 | MY01F | myosin IF | 0.66 |
| NM_005007 | NFKBIL1 | nuclear factor of kappa light polypeptide gene enhancer in B-cells inhibitor-like 1 | 0.66 |
| NM_001870 | CPA3 | carboxypeptidase A3 (mast cell) | 0.66 |
| NM 181826 | NF2 | neurofibromin 2 (bilateral acoustic neuroma) | 0.66 |
| NM_000285 | PEPD | peptidase D | 0.66 |
| NM_006180 | NTRK2 | neurotrophic tyrosine kinase, receptor type 2 | 0.66 |
| NM_000543 | SMPD1 | sphingomyelin phosphodiesterase 1, acid lysosomal (acid sphinagmyelinase) | 0.66 |
| NM_001459 | FLT3LG | fms-related tyrosine kinase 3 ligand | 0.65 |
| NM_003750 | EIF3S10 | eukaryotic translation initiation factor 3, subunit 10 theta, 150/170kDa | 0.65 |
| NM_005570 | LMAN1 | lectin mannose-binding, 1 | 0.65 |
| NM_004409 | DMPK | dystrophia myotonica-protein kinase | 0.65 |
| NM_172159 | KCNAB1 | potassium voltage-gated channel, shaker-related subfamily, beta member 1 | 0.65 |
| XM 352750 | COL14A1 | collagen, type XIV, alpha 1 (undulin) | 0.65 |
| NM_001731 | BTG1 | B-cell translocation gene 1, anti-proliferative | 0.65 |
| NM_000884 | IMPDH2 | IMP (inosine monophosphate) dehydrogenase 2 | 0.64 |
| NM 001885 | CRYAB | crystallin, alpha B | 0.64 |
| NM_000240 | MAOA | monoamine oxidase A | 0.64 |
| NM_003136 | SRP54 | siqnal recognition particle 54kDa | 0.63 |
| NM_000281 | PCBD | 6-pyruvoyl-tetrahydropterin synthase/dimerization cofactor of hepatocyte nuclear factor 1 alpha (TCF1) | 0.63 |
| NM 005729 | PPIF | peptidylprolpyl isomerase F (cyclophilin F) | 0.63 |
| NM_006481 | TCF2 | transcription factor 2, hepatic; LF-B3' variant hepatic nuclear factor | 0.63 |
| NM_002089 | CXCL2 | chemokine (C-X-C motif) liqand 2 | 0.63 |
| NM_001961 | EEF2 | eukaryotic translation elonqation factor 2 | 0.63 |
| NM_001801 | CDOl | cysteine dioxygenase type I | 0.63 |
| NM 006389 | HYOU1 | hypoxia up-regulated 1 | 0.63 |
| XM 167711 | ITGA8 | integrin, alpha 8 | 0.62 |
| NM_014765 | TOMM20 | translocase of outer mitochondrial membrane 20 homolog (yeast) | 0.62 |
| NM_006714 | SMPDL3 A | sphingomyelin phosphodiesterase, acid-like 3A | 0.62 |
| NM_000016 | ACAOM | acyl-Coenzyme A dehydrogenase C-4 to C-12 straiqht chain | 0.62 |
| NM 003924 | PHOX2B | paired-like homeobox 2b | 0.62 |
| NM_002078 | GOLGA4 | golgi autoantigen, golgin subfamily a 4 | 0.62 |
| NM_002736 | PRKAR2 B | protein kinase cAMP-dependent, requlatory, type II beta | 0.62 |
| BQ217469 | K1AA0114 | K1AA0114 gene product | 0.61 |
| NM 006307 | SRPX | sushi-repeat-containing protein X-linked | 0.61 |
| NM 002184 | IL6ST | interleukin 6 siqnal transducer (gp130 oncostatin M receptor) | 0.61 |
| NM_153186 | ANKR015 | ankyrin repeat domain 15 | 0.61 |
| NM_003038 | S1C1A4 | solute carrier family 1 (glutamate/neutral amino acid transporter), member 4 | 0.60 |
| NM_006195 | PBX3 | pre-B-cell leukemia transcription factor 3 | 0.60 |
| NM_000327 | ROM1 | retinal outer segment membrane protein 1 | 0.60 |
| NM 003463 | PTP4A1 | protein tyrosine phosphatase type IVA, member 1 | 0.60 |
| NM_001520 | GTF3C1 | general transcription factor iiiC polypeptide 1 alpha 220kDa | 0.60 |
| NM_006277 | ITSN2 | intersectin 2 | 0.59 |
| NM_000985 | RPL17 | ribosomal protein L17 | 0.59 |
| NM_000909 | NPY1R | neuropeptide Y receptor Y1 | 0.59 |
| NM 001014 | RPS10 | ribosomal protein S10 | 0.59 |
| NM_022307 | ICA1 | islet cell autoantigen 1 69kDa | 0.58 |
| NM_002567 | PBP | prostatic binding protein | 0.58 |
| NM_012324 | MAPK81P 2 | mitogen-activated protein kinase 8 interacting protein 2 | 0.58 |
| NM_004490 | GRB14 | growth factor receptor-bound protein 14 | 0.58 |
| NM 004733 | SLC33A1 | solute carrier family 33 (acetyl-CoA transporter), member 1 | 0.57 |
| NM_002197 | AC01 | aconitase 1, soluble | 0.57 |
| NM_000505 | F12 | coagulation factor Xii (Hageman factor) | 0.57 |
| NM_005010 | NRCAM | neuronal cell adhesion molecule | 0.56 |
| NM_006963 | ZNF22 | zinc finger protein 22 (KOX 15) | 0.56 |
| NM_006827 | TMP21 | transmembrane trafficking protein | 0.55 |
| NM_004394 | DAP | death-associated protein | 0.54 |
| NM_001089 | ABCA3 | ATP-binding cassette, sub-family A (ABC), member 3 | 0.54 |
| NM_004470 | FKBP2 | FK506 binding protein 2, 13kDa | 0.53 |
| NM_005749 | TOB1 | transducer of ERBB2, 1 | 0.53 |
| NM_001355 | DDT | D-dopachrome tautomerase | 0.53 |
| NM_002111 | HD | huntington (Huntington disease) | 0.53 |
| NM_002635 | S1C25A3 | solute carrier family 25 (mitochondrial carrier; phosphate carrier), member 3 | 0.53 |
| NM_005596 | NFIB | nuclear factor I/B | 0.53 |
| NM_006273 | CCL7 | chemokine (C-C motif) liqand 7 | 0.53 |
| NM_001013 | RPS9 | ribosomal protein S9 | 0.52 |
| NM_001551 | IGBP1 | immunoglobulin (CD79A) binding protein 1 | 0.52 |
| NM_004498 | ONECUT 1 | one cut domain, family member 1 | 0.52 |
| NM_004484 | GPC3 | glypican 3 | 0.52 |
| NM 130797 | DPP6 | dipeptidylpeptidase 6 | 0.52 |
| NM_000746 | CHRNA7 | cholineragic receptor, nicotinic, alpha polypeptide 7 | 0.51 |
| NM_001756 | SERPINA 6 | serine (or cysteine) proteinase inhibitor, clade A (alpha-1 antiproteinase antitrypsin), member 6 | 0.51 |
| NM_001327 | CTAG1 | cancer/testis antigen 1 | 0.51 |
| NM_003651 | CSDA | cold shock domain protein A | 0.50 |
| NM 005848 | IRLB | c-mvc promoter-binding protein | 0.50 |
| BC040073 | H19 | H19, imprinted maternally expressed untranslated mRNA | 0.50 |
| NM_002228 | JUN | v-jun sarcoma virus 17 oncogene homolog (avian) | 0.49 |
| NM_000795 | DRD2 | dopamine receptor D2 | 0.48 |
| NM 002084 | GPX3 | glutathione peroxidase 3 (plasma) | 0.48 |
| NM_002716 | PPP2R1B | protein phosphatase 2 (formerly 2A), regulatory subunit A (PR 65), beta isoform | 0.48 |
| NM_005166 | APLP1 | amyloid beta (A4) precursor-like protein 1 | 0.48 |
| NM_005911 | MAT2A | methionine adenosyltransferase II, alpha | 0.47 |
| NM_000208 | INSR | insulin receptor | 0.47 |
| NM 170736 | KCNJ15 | potassium inwardly-rectifying channel, subfamily J, member 15 | 0.47 |
| NM_001190 | BCAT2 | branched chain aminotransferase 2, mitochondrial | 0.47 |
| NM_005336 | HDLBP | hiqh density lipoprotein binding protein (viqilin) | 0.46 |
| NM_001076 | UGT2B15 | UDP glycosyltransferase 2 family, polypeptide B15 | 0.46 |
| NM_001152 | SLC25A5 | solute carrier family 25 (mitochondrial carrier; adenine nucleotide translocator, member 5 | 0.46 |
| NM_002729 | HHEX | hematopoietically expressed homeobox | 0.46 |
| NM_002847 | PTPRN2 | protein tyrosine phosphatase, receptor type, N polypeptide 2 | 0.44 |
| NM_000447 | PSEN2 | presenilin 2 (Alzheimer disease 4) | 0.44 |
| NM_152868 | KCNJ4 | potassium inwardly-rectifying channel, subfamily J, member 4 | 0.44 |
| NM_001759 | CCND2 | cyclin D2 | 0.44 |
| NM 000316 | PTHR1 | parathyroid hormone receptor 1 | 0.44 |
| NM_001612 | ACRV1 | acrosomal vesicle protein 1 | 0.43 |
| NM_002467 | MYC | v-mc myelocytomatosis viral oncogene homolog (avian) | 0.43 |
| NM_004454 | ETV5 | ets variant gene 5 (ets-related molecule) | 0.43 |
| NM_002846 | PTPRN | protein tyrosine phosphatase, receptor type N | 0.43 |
| NM 005622 | SAH | SA hypertension-associated homolog (rat) | 0.42 |
| NM_001989 | EVX1 | eve, even-skipped homeo box homolog 1 (Drosophila) | 0.42 |
| NM_000166 | GJB1 | gap junction protein, beta 1, 32kDa (connexin 32, Charcot-Marie-Tooth neuropathy, X-linked) | 0.42 |
| NM_014685 | HERPUD 1 | homocysteine-inducible, endoplasmic reticulum stress-inducible, ubiquitin-like domain member 1 | 0.42 |
| NM_001735 | C5 | complement component 5 | 0.41 |
| NM 005504 | BCAT1 | branched chain aminotransferase 1, ctyosolic | 0.41 |
| NM_006808 | SEC61B | Sec61 beta subunit | 0.40 |
| NM 006751 | SSFA02 | sperm specific antigen 2 | 0.39 |
| NM 005947 | MT1B | metallothionein 1B (functional) | 0.38 |
| NM 005576 | LOXL1 | lysyl oxidase-like 1 | 0.37 |
| NM 005627 | SGK | serum/glucocorticoid regulated kinase | 0.36 |
| NM 004683 | RGN | regucalcin (senescence marker protein-30) | 0.36 |
| NM_00918 | P4HB | procollagen-proline, 2-oxoglutarate 4-dioxygenase (proline 4-hydroxylase), beta polypeptide (protein disulfide isomerase; thyroid hormone binding protein p55) | 0.36 |
| BC044862 | | Macrophage stimulating 1 (hepatocyte growth factor-like), mRNA (cDNA clone IMAGE:4821945), with apparent retained intron | 0.35 |
| NM 005952 | MT1X | metallothionein IX | 0.35 |
| NM 000429 | MAT1A | methionine adenosyltransferase 1, alpha | 0.35 |
| NM 004010 | DMD | dystrophin (muscular dystrophy, Duchenne and Becker types) | 0.34 |
| NM_000689 | ALDH1A1 | aldehyde dehydrogenase 1 family, member A1 | 0.34 |
| NM_002889 | RARRES2 | retinoic acid receptor responder (tazarotene induced) 2 | 0.33 |
| NM_006280 | SSRA | signal sequence receptor, delta (translocon-associated protein delta) | 0.33 |
| NM 003819 | PABPC4 | poly(A) binding protein, cytoplasmic 4 (inducible form) | 0.32 |
| NM 000755 | CRAT | carnitine aceltyltransferase | 0.32 |
| NM 015684 | ATP5S | ATP synthase, H+ transporting, mitochondrial F0 complex, subunit s (factor B) | .030 |
| NM 033200 | BC002942 | hypothetical protein BC002942 | 0.30 |
| BCG986717 | | Transcribed sequences | 0.29 |
| NM 148923 | CYB5 | cytochrome b-5 | 0.29 |
| NM_000609 | CXCL12 | chemokine (C-X-C motif) ligand 12 (stromal cell-derived factor 1) | 0.29 |
| NM_001979 | EPHX2 | epoxide hydrolase 2, cytoplasmic | 0.28 |
| NM_001332 | CTNND2 | catenin (caherin-associated protein), delta 2 (neural plakophilin-related arm-repeat protein) | 0.27 |
| NM 001831 | CLU | clusterin (complement lysis inhibitor, SP-40, 40, sulfated glycoprotein 2, testosterone-repressed prostate message 2, apolipoprotein J) | 0.27 |
| NM 005080 | XBP1 | X-box binding protein 1 | 0.27 |
| NM 000156 | GAMT | guanidinoacetate N-methyltransferase | 0.27 |
| NM 182848 | CLDN10 | claudin 10 | 0.26 |
| NM 000065 | C6 | complement component 6 | 0.26 |
| NM_000128 | F11 | coagulation factor XI (plasma thromboplasin antecedent) | 0.24 |
| NM 003822 | MR5A2 | nuclear receptor subfamily 5, group A, member 2 | 0.24 |
| NM 006406 | PRDX4 | peroxiredoxin 4 | 0.21 |
| BM799844 | BNIP3 | BCL2/adenovirus E1B 19kDa interacting protein 3 | 0.21 |
| NM 018646 | TRPV6 | transient receptor potential cation channel, subfamily V, member 6 | 0.21 |
| NM_005013 | NUCB2 | nucleobindin 2 | 0.21 |
| NM 000624 | SERPINA 3 | serine (or cysteine) proteinase inhibitor, clade A (alpha-1 antiproteinase, antitrypsin), member 3 | 0.19 |
| NM 005065 | SEL 1L | sel-1 suppressor of lin-12-like (C. elegans) | 0.18 |
| NM 198235 | RNASE 1 | ribonuclease, RNase A family, 1 (pancreatic) | 0.17 |
| NM 006498 | LGALS2 | lectin, galactoside-binding, soluble, 2 (galectin 2) | 0.16 |
| NM_002899 | RBP1 | retinol binding protein 1, cellular | 0.12 |
| NM 004413 | DPEP1 | dipeptidase 1 (renal) | 0.12 |
| NM 021603 | FXYD2 | FXYD domain contaning ion transport regulator 2 | 0.09 |
| NM 138938 | PAP | pancreatitis-associated protein | 0.08 |
| NM 201553 | FGL | fibrinogen-like 1 | 0.07 |
| NM 001482 | GATM | glycerine amidinotransferase (L-arrginine: glycine amidinotransferase) | 0.04 |
| NM 033240 | ELA2A | elastase 2^{a} | 0.02 |
| NM 000101 | CYBA | cytochrome b-245, alpha polypeptide | 0.02 |

| | | | |
|---|---|---|---|
| Note: Accession IDs NM XXXX are uniquely assigned to each gene by National Center for Biotechnology Information (NCBI) (http://www.ncbi.nlm.nih.gov/sites/entrez?db=nuccore). | | | |

**Table 8. microRNAs that are up-regulated in glioblastoma cells.**

| **Fold change** | **microRNA** |
|---|---|
| Up 10X | miR-10b, miR-10a, miR-96 |
| Up 2-10X | miR-182, miR-199b, miR-21, miR124, miR-199a, miR-199-s, miR-199a, miR-106b, miR-15b, miR-188, miR-148a, miR-104, miR-224, miR-368, miR-23a, miR-210N, miR-183, miR-25, miR-200cN, miR-373, miR-17-5p, let-7a, miR-16, miR-19b, miR-26a, miR-27a, miR-92, miR-93, miR-320 and miR-20 |
| Up 1-2 X | miR-143, miR-186. miR-337, miR-30a-3p, miR-355, miR-324-3p etc. |

**Table 9. microRNAs that are down-regulated in glioblastoma cells.**

| **Fold change** | **microRNA** |
|---|---|
| Down 10X | miR-218, miR-124a, miR-124b, miR-137, miR-184, miR-129, miR-33, miR-139, miR-128b, miR-128a, miR-330, miR-133a, miR-203, miR-153, miR-326, miR-105, miR-338, miR-133b, miR-132, miR-154, miR-29bN |
| Down 2-10X | miR-7N, miR-323, miR-219, miR-328, miR-149, miR-122a, miR-321, miR-107, miR-190, miR-29cN, miR-95, miR-154, miR-221, miR-299, miR-31, miR-370, miR-331, miR-342, miR-340 |

**Table 10. MMP genes contained within microvesicles isolated from glioblastoma cell line.**

| **Gene Symbol** | **Accession ID** | **Gene Description** |
|---|---|---|
| MMP1 | AK097805 | Homo sapiens cDNA FLJ40486 fis, clone TESTI2043866. [AK097805] |
| MMP8 | NM_002424 | Homo sapiens matrix metallopeptidase 8 (neutrophil collagenase) (MMP8), mRNA [NM_002424] |
| MMP12 | NM_002426 | Homo sapiens matrix metallopeptidase 12 (macrophage elastase) (MMP12), mRNA [NM_002426] |
| MMP15 | NM_002428 | Homo sapiens matrix metallopeptidase 15 (membrane-inserted) (MMP15), mRNA [NM_002428] |
| MMP20 | NM_004771 | Homo sapiens matrix metallopeptidase 20 (enamelysin) (MMP20), mRNA [NM_004771] |
| MMP21 | NM_147191 | Homo sapiens matrix metallopeptidase 21 (MMP21), mRNA [NM_147191] |
| MMP24 | NM_006690 | Homo sapiens matrix metallopeptidase 24 (membrane-inserted) (MMP24), mRNA [NM_006690] |
| MMP26 | NM_021801 | Homo sapiens matrix metallopeptidase 26 (MMP26), mRNA [NM_021801] |
| MMP27 | NM_022122 | Homo sapiens matrix metallopeptidase 27 (MMP27), mRNA [NM_022122] |

| | | |
|---|---|---|
| Note: Gene symbols are standard symbols assigned by Entrz Gene (http://www.ncbi.nlm.nih.gov/sites/entrez?db=gene). Accession IDs are uniquely assigned to each gene by National Center for Biotechnology Information (NCBI) (http://www.ncbi.nlm.nih.gov/sites/entrez?db=nuccore). | | |

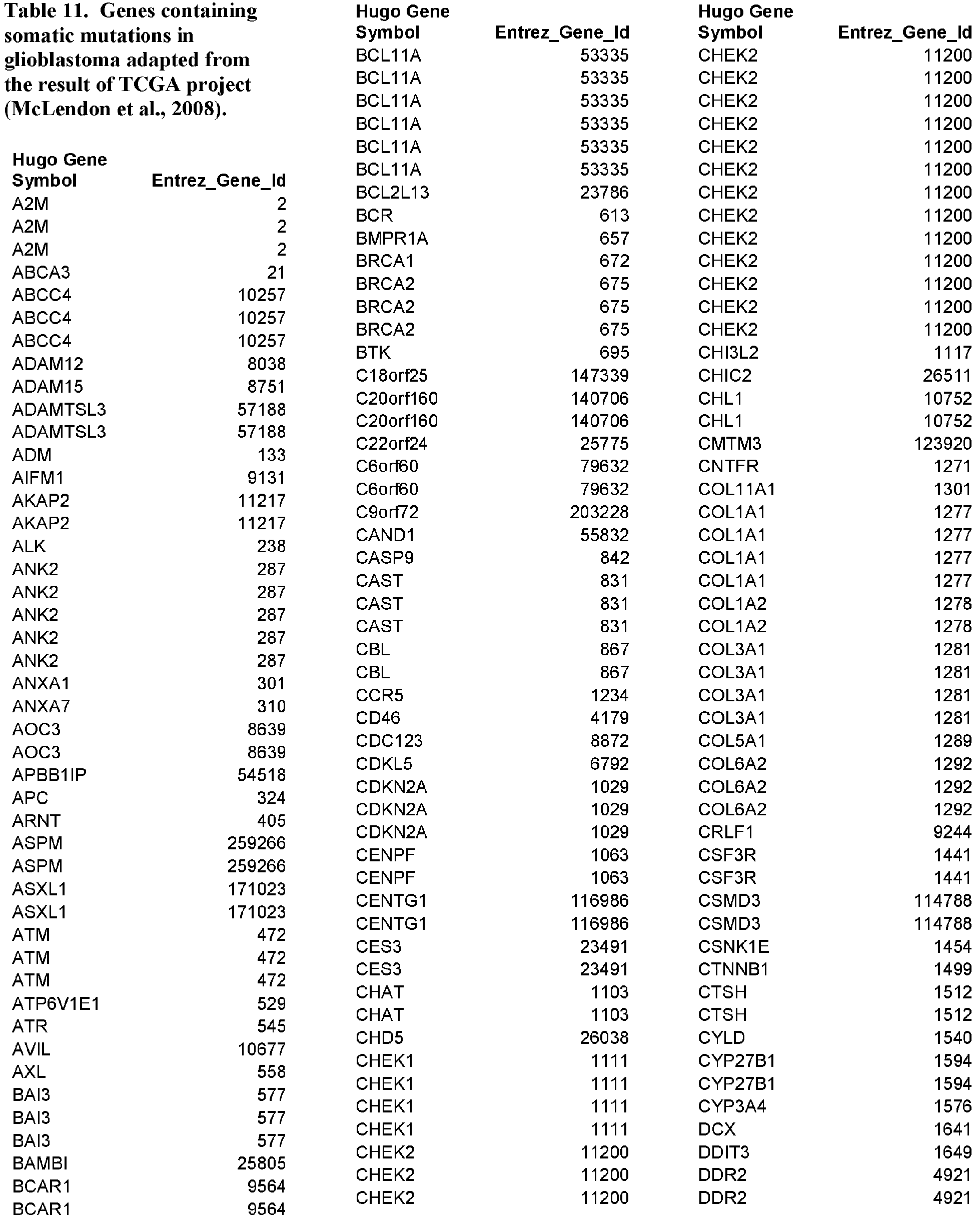

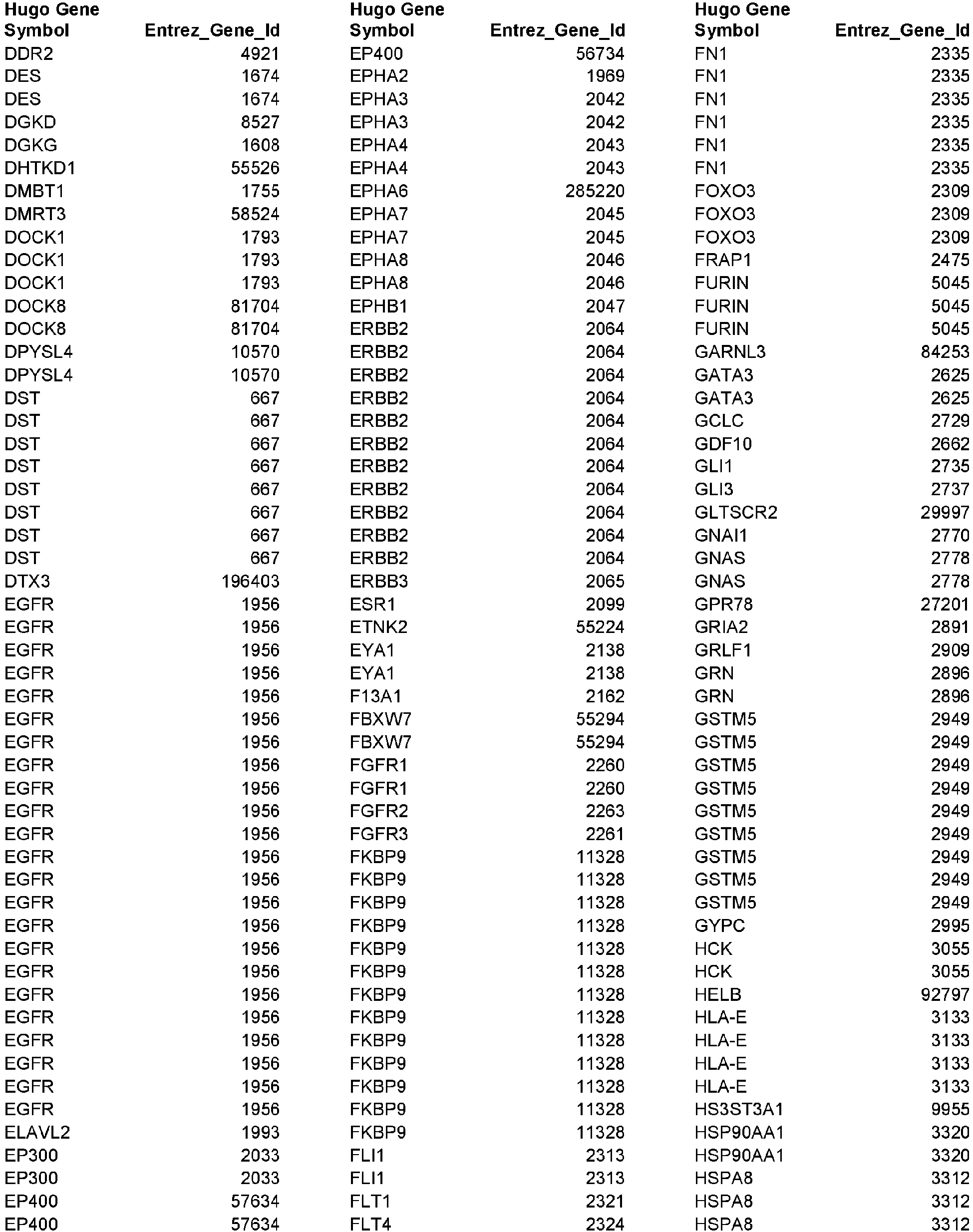

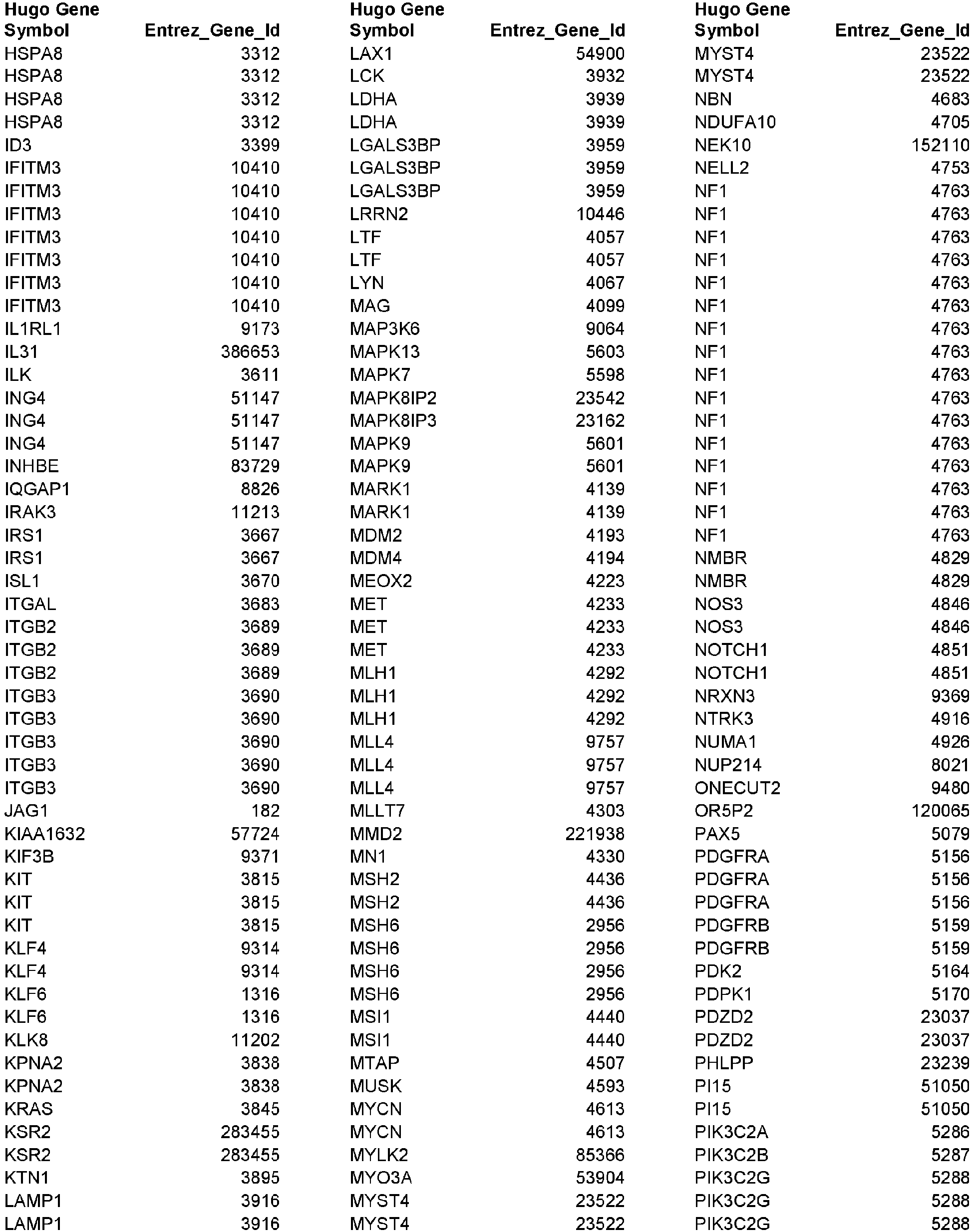

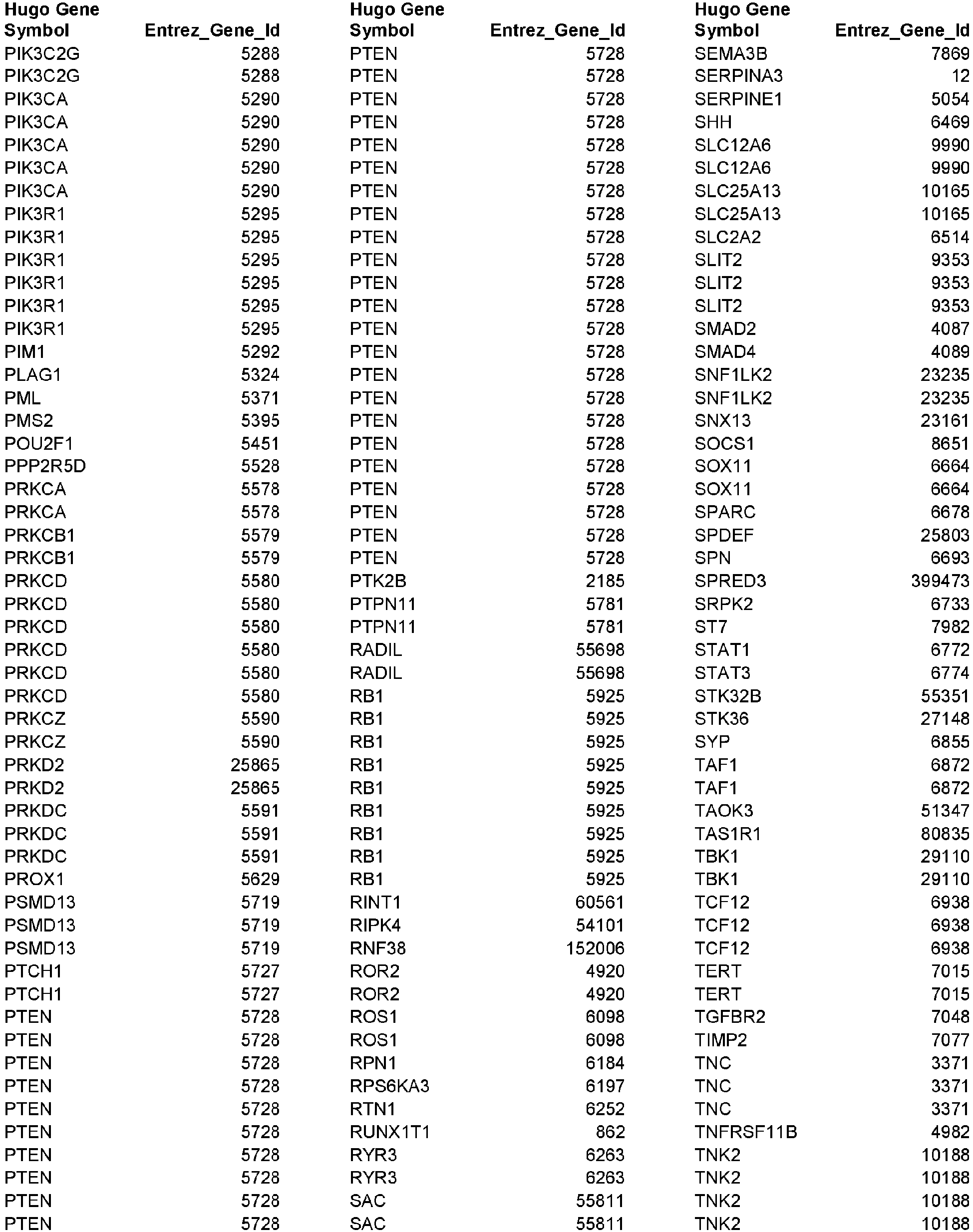

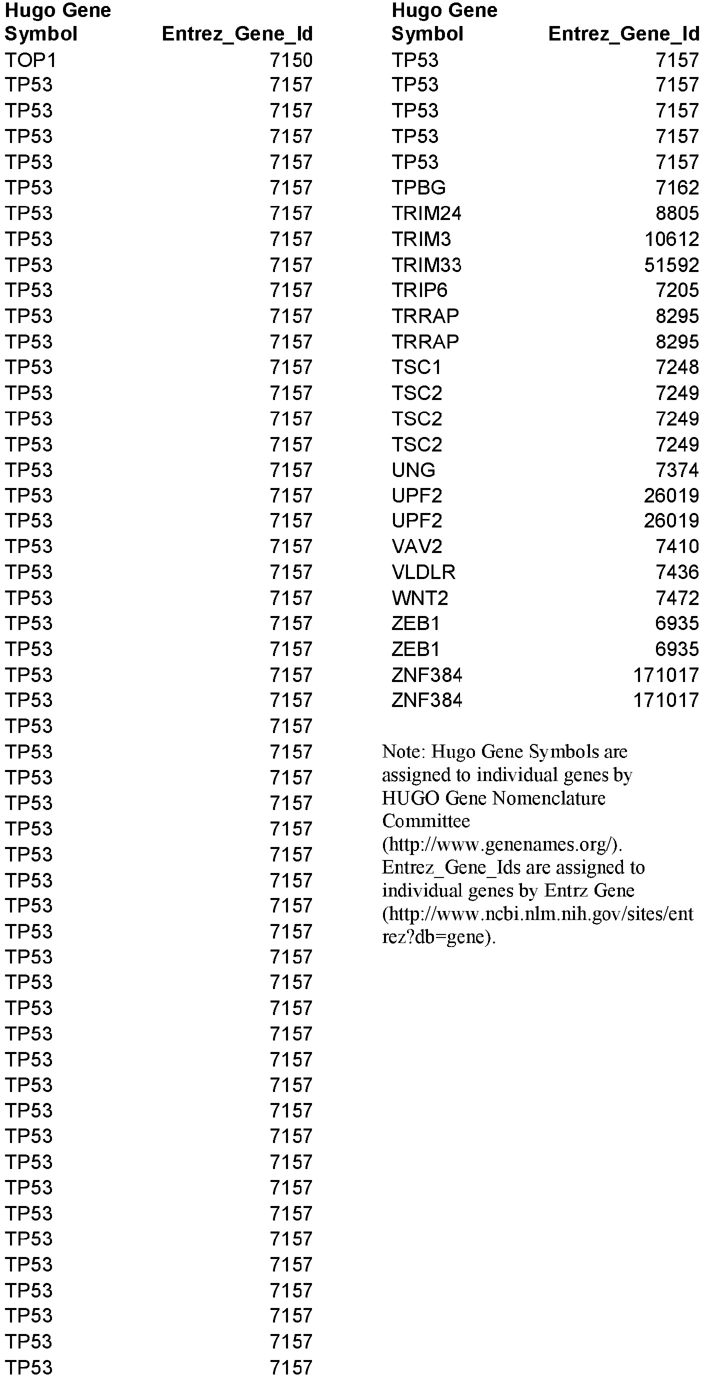

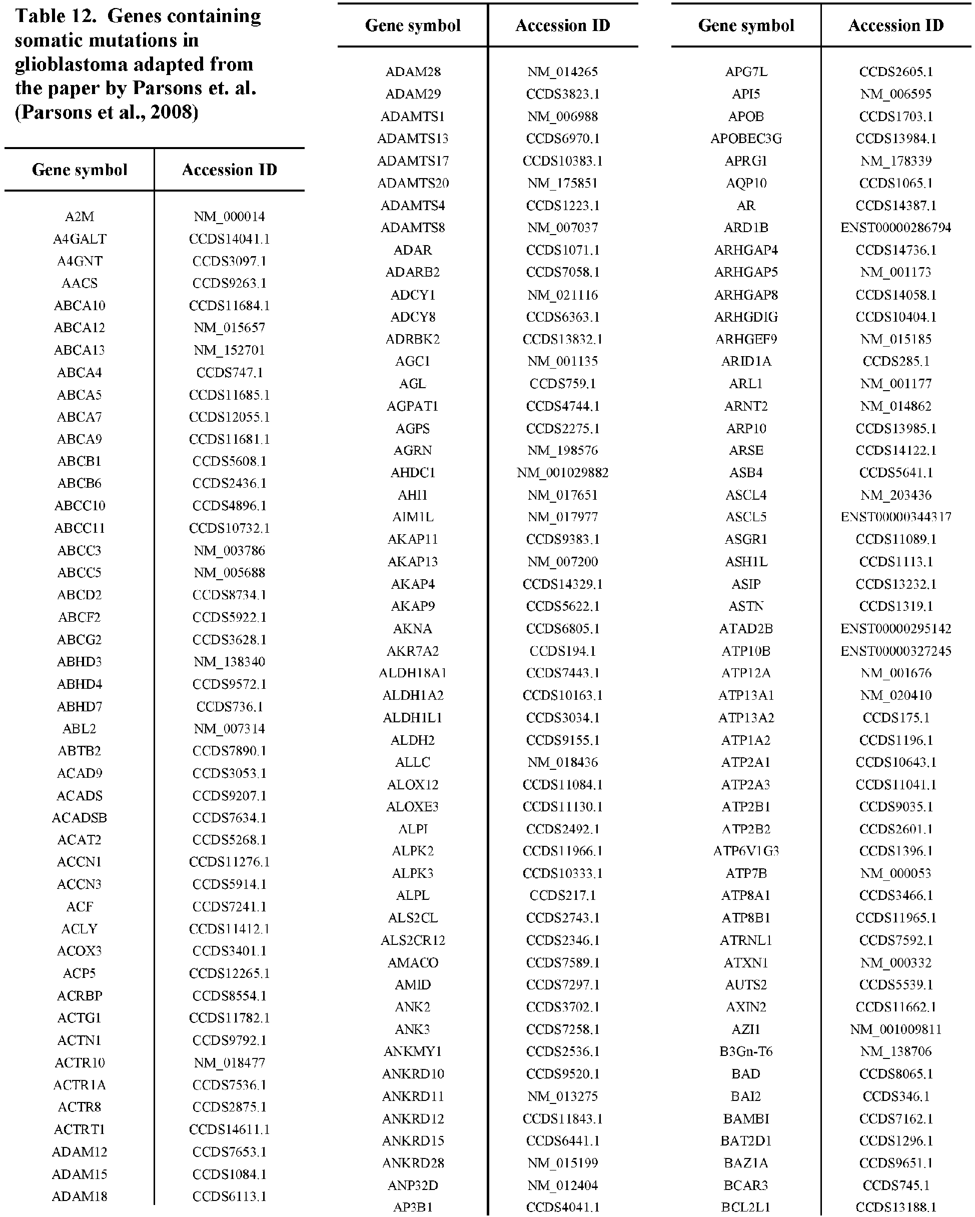

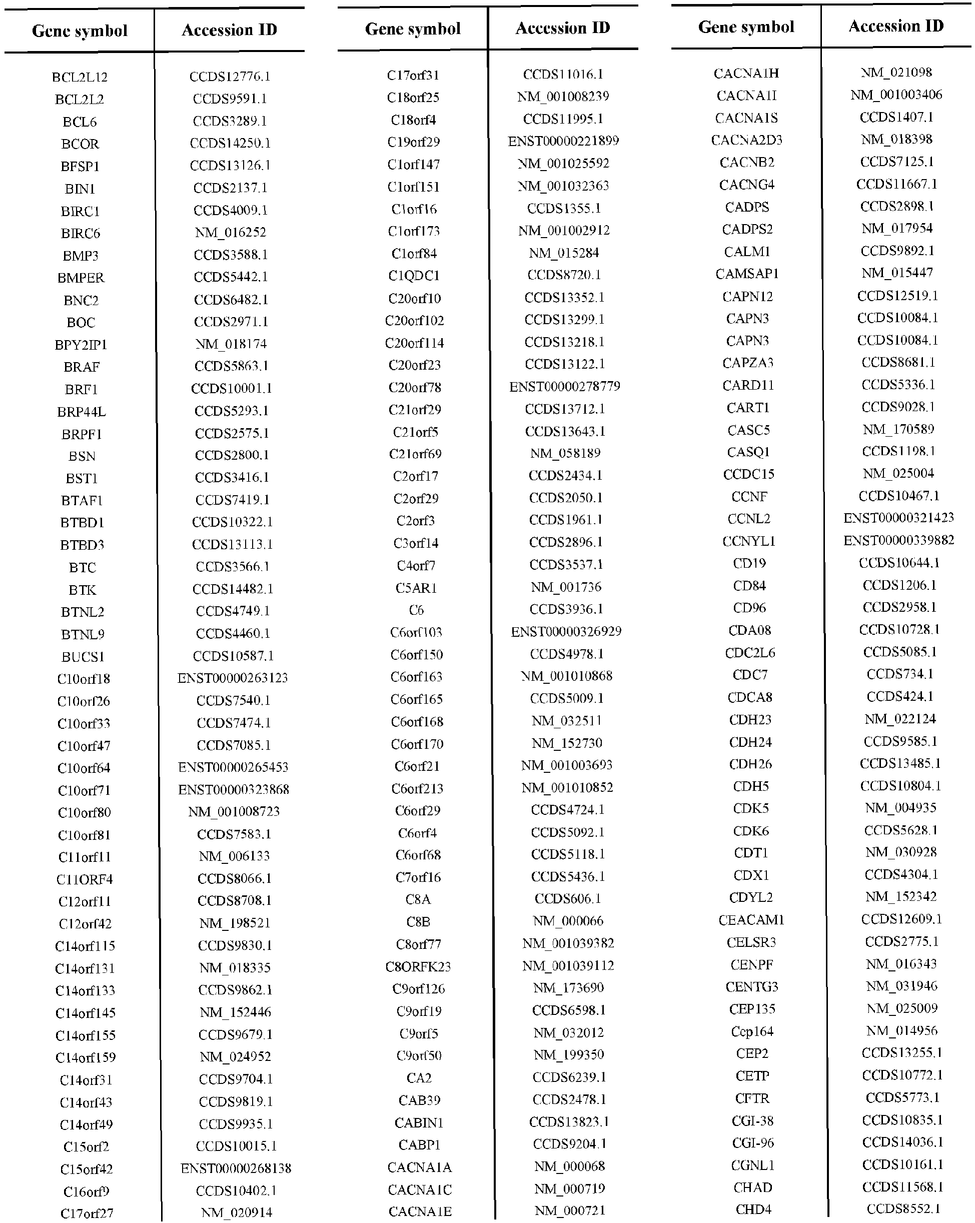

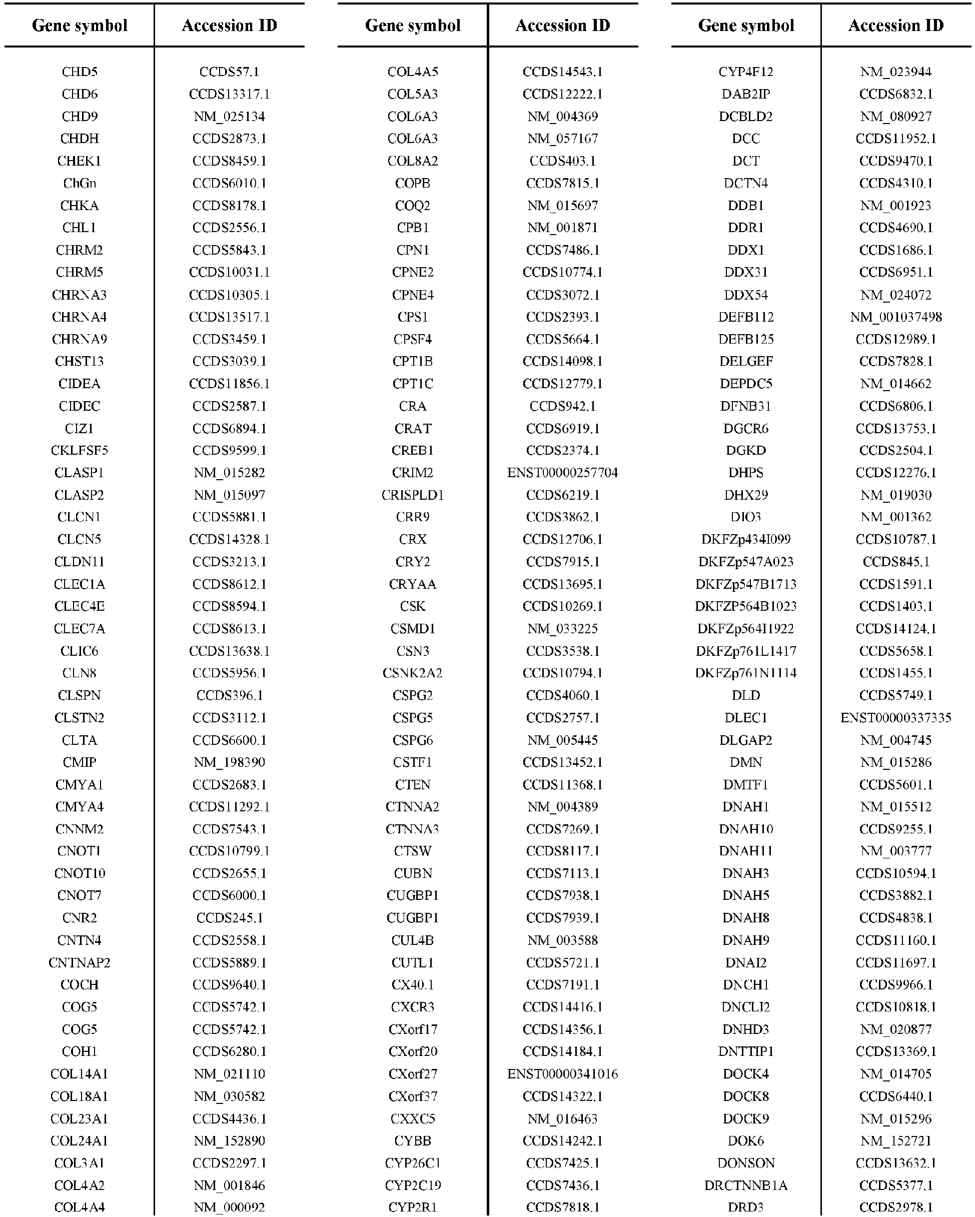

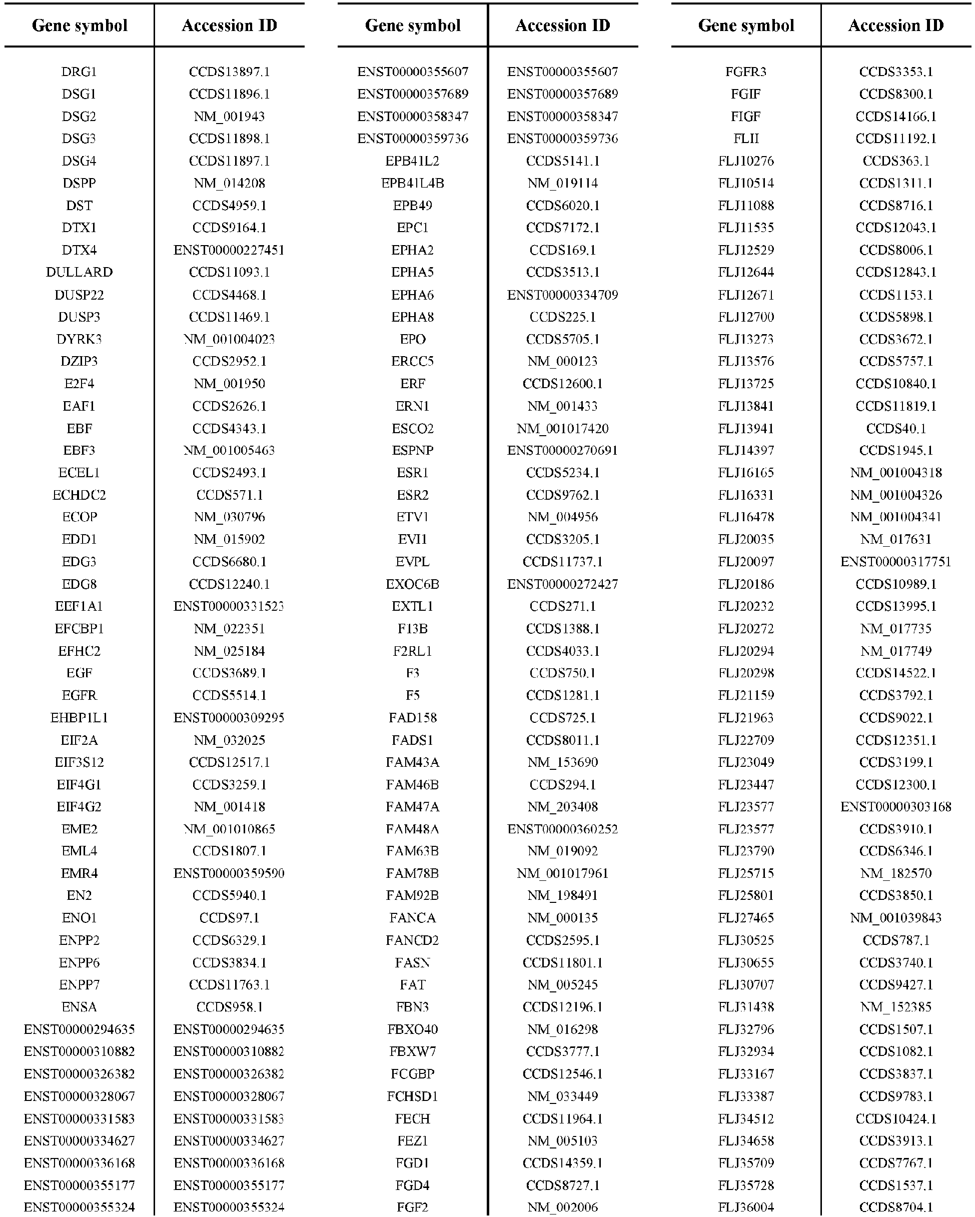

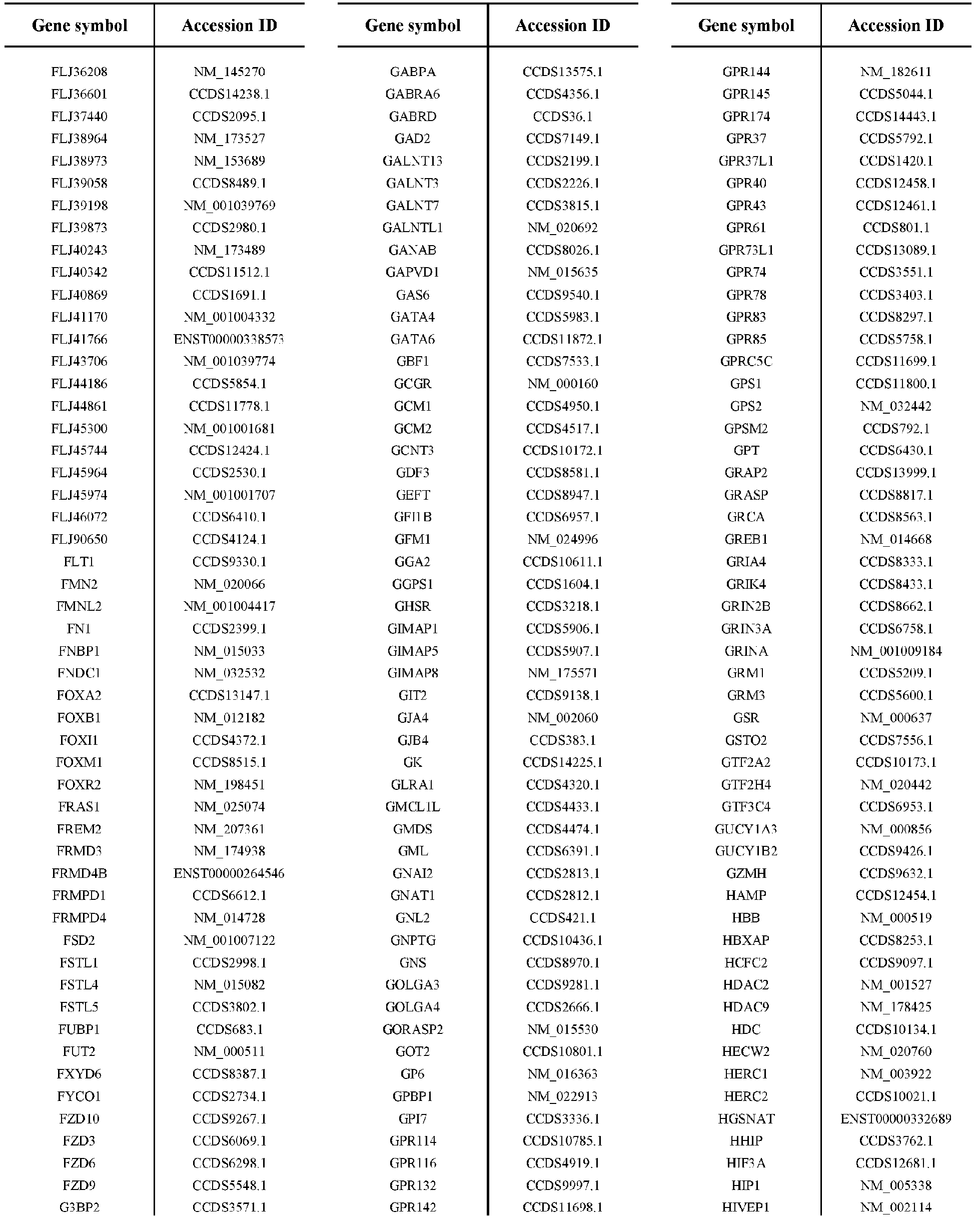

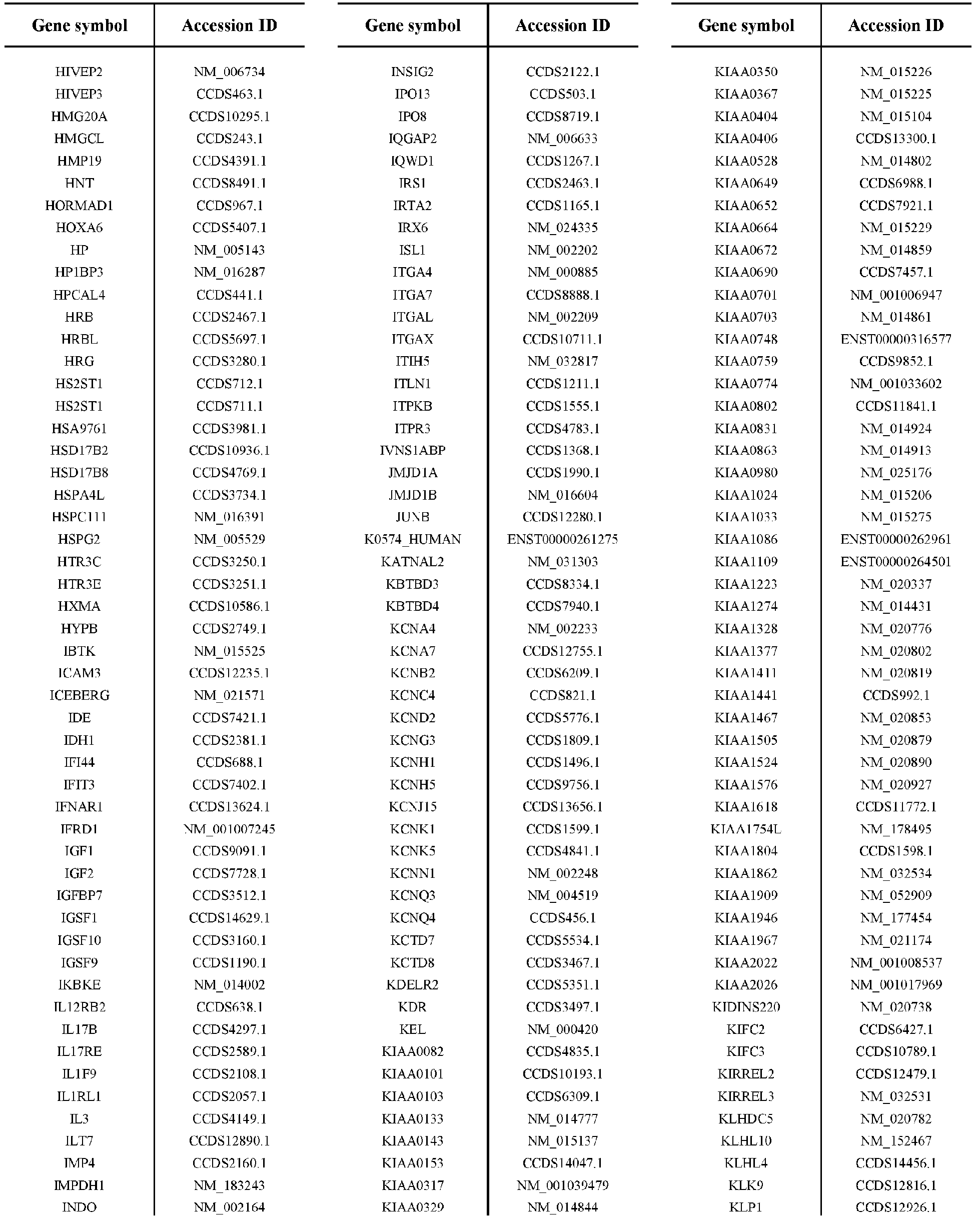

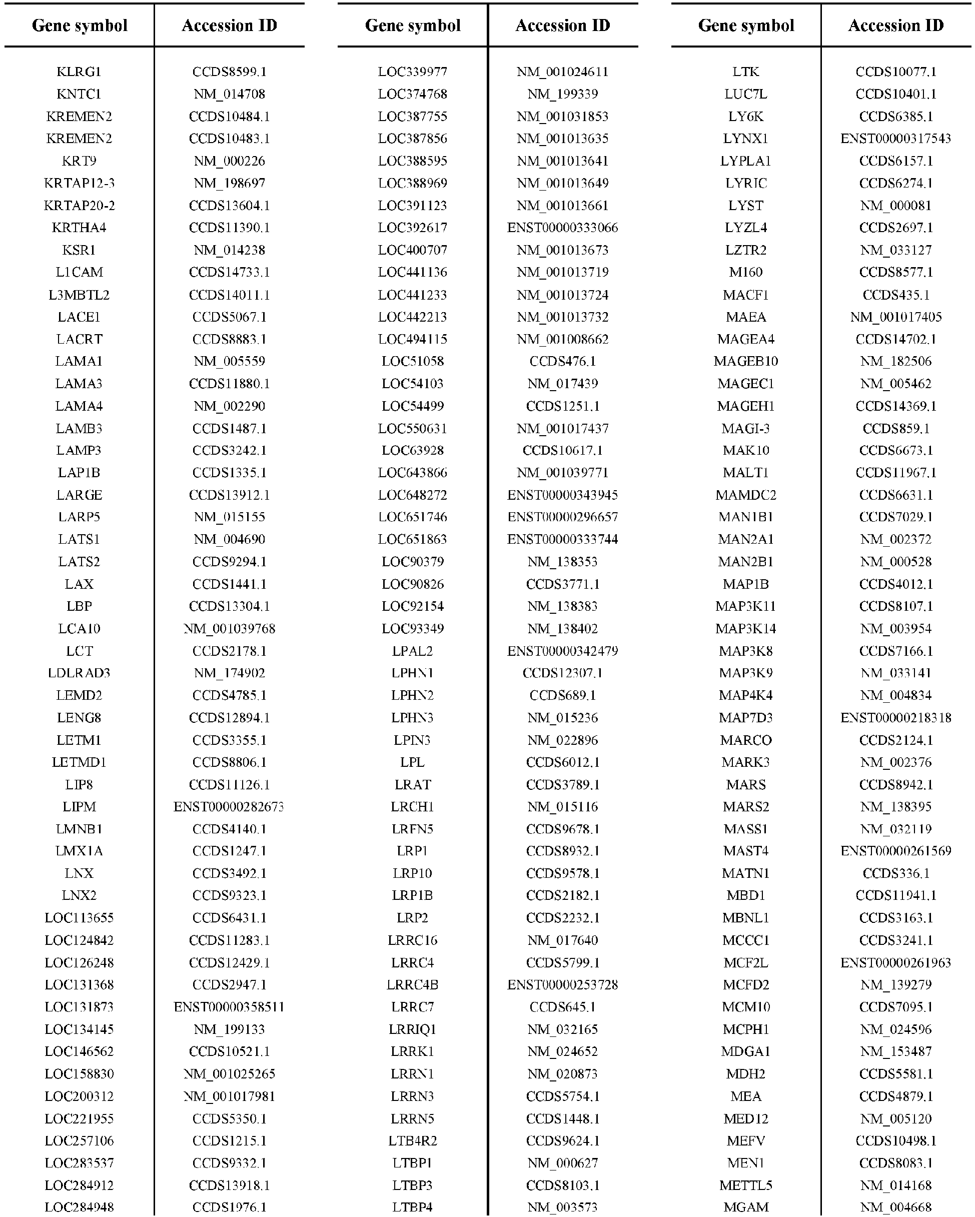

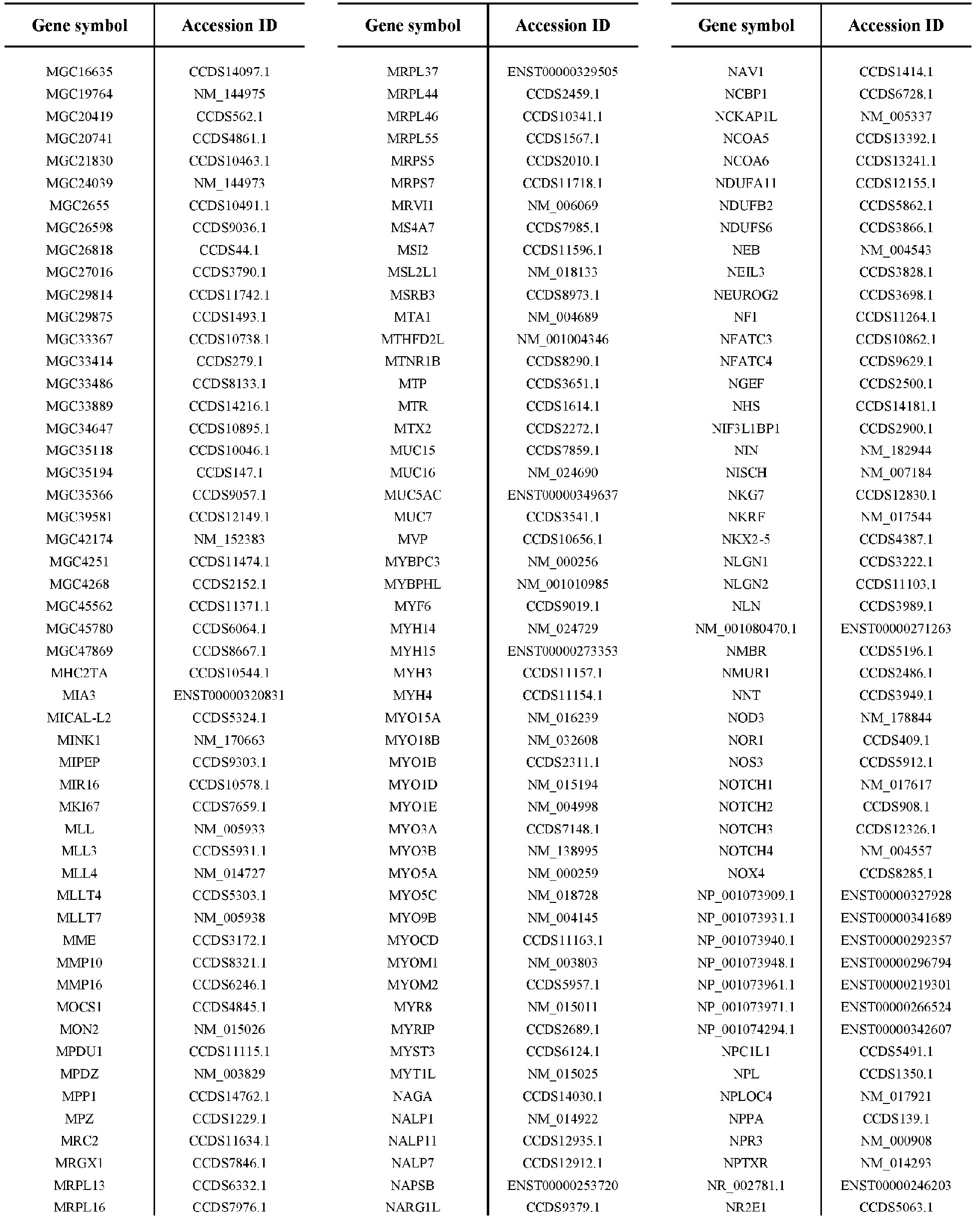

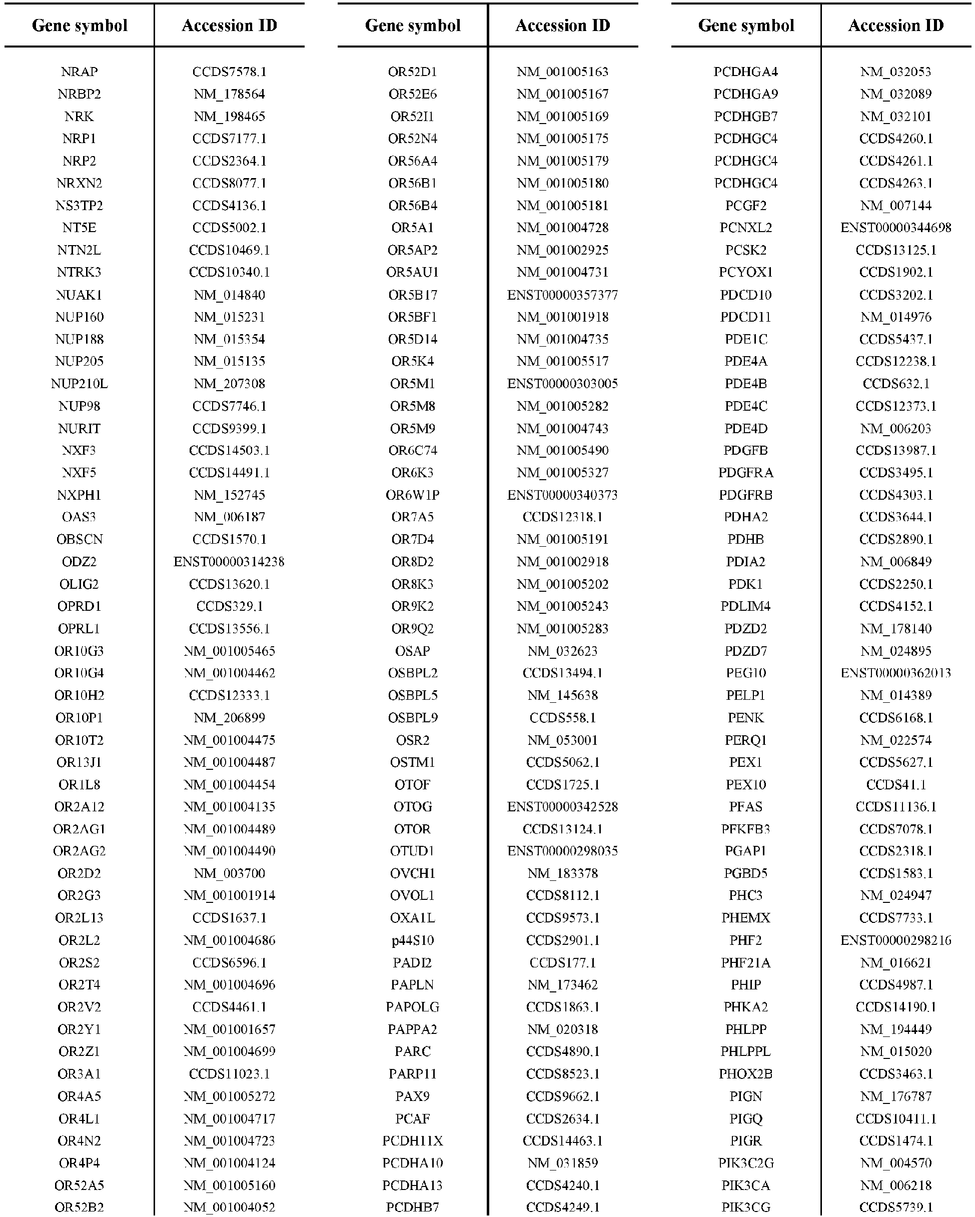

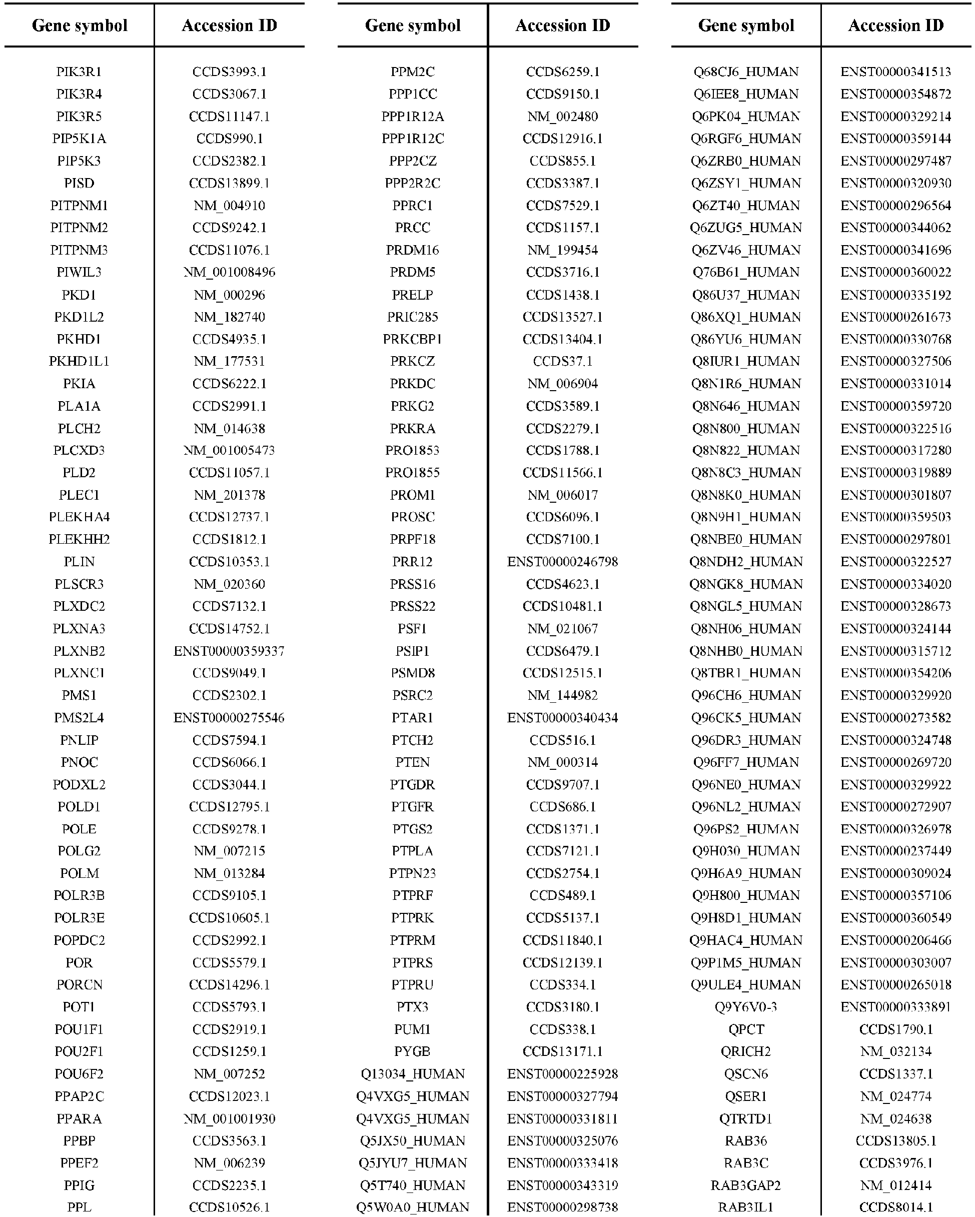

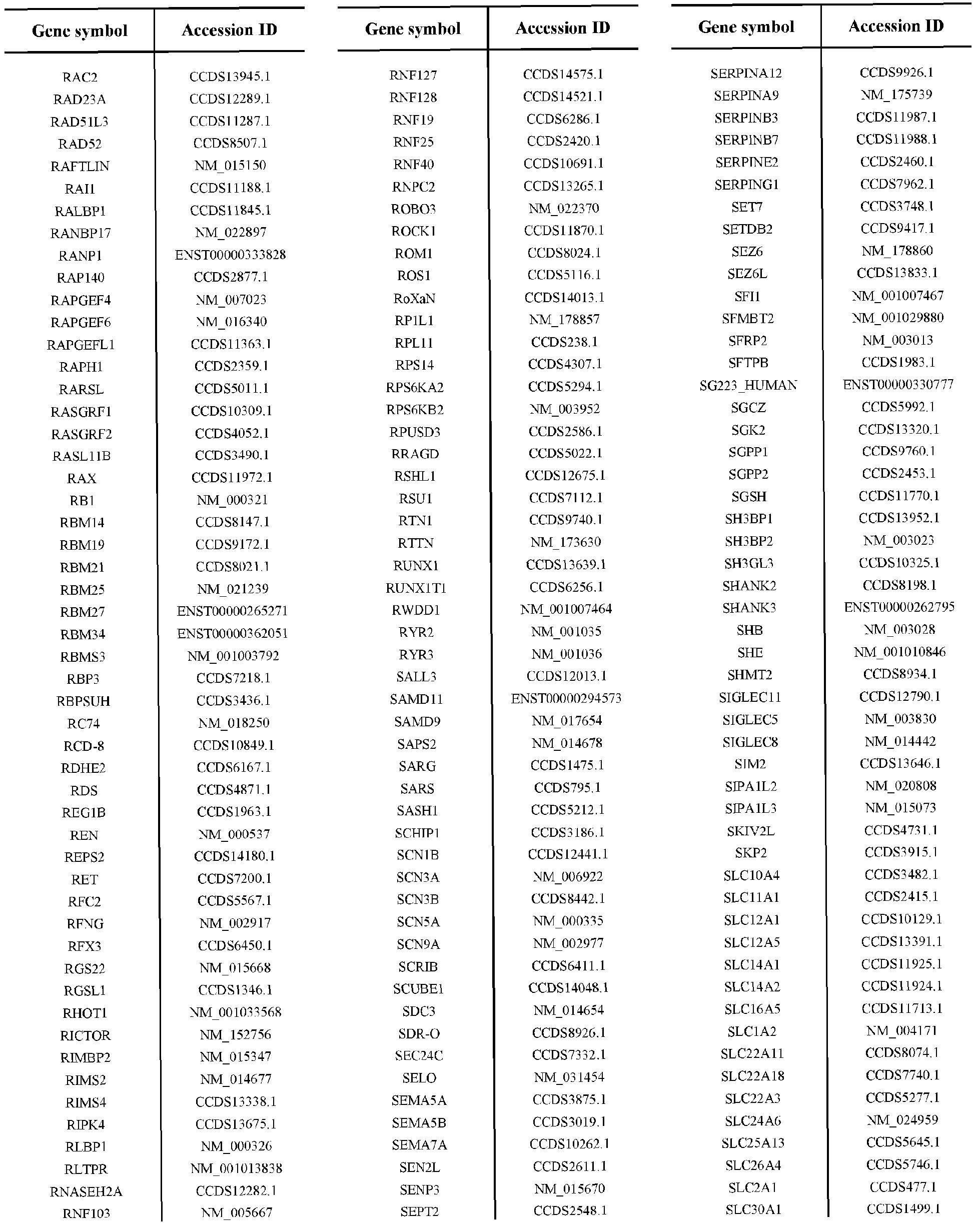

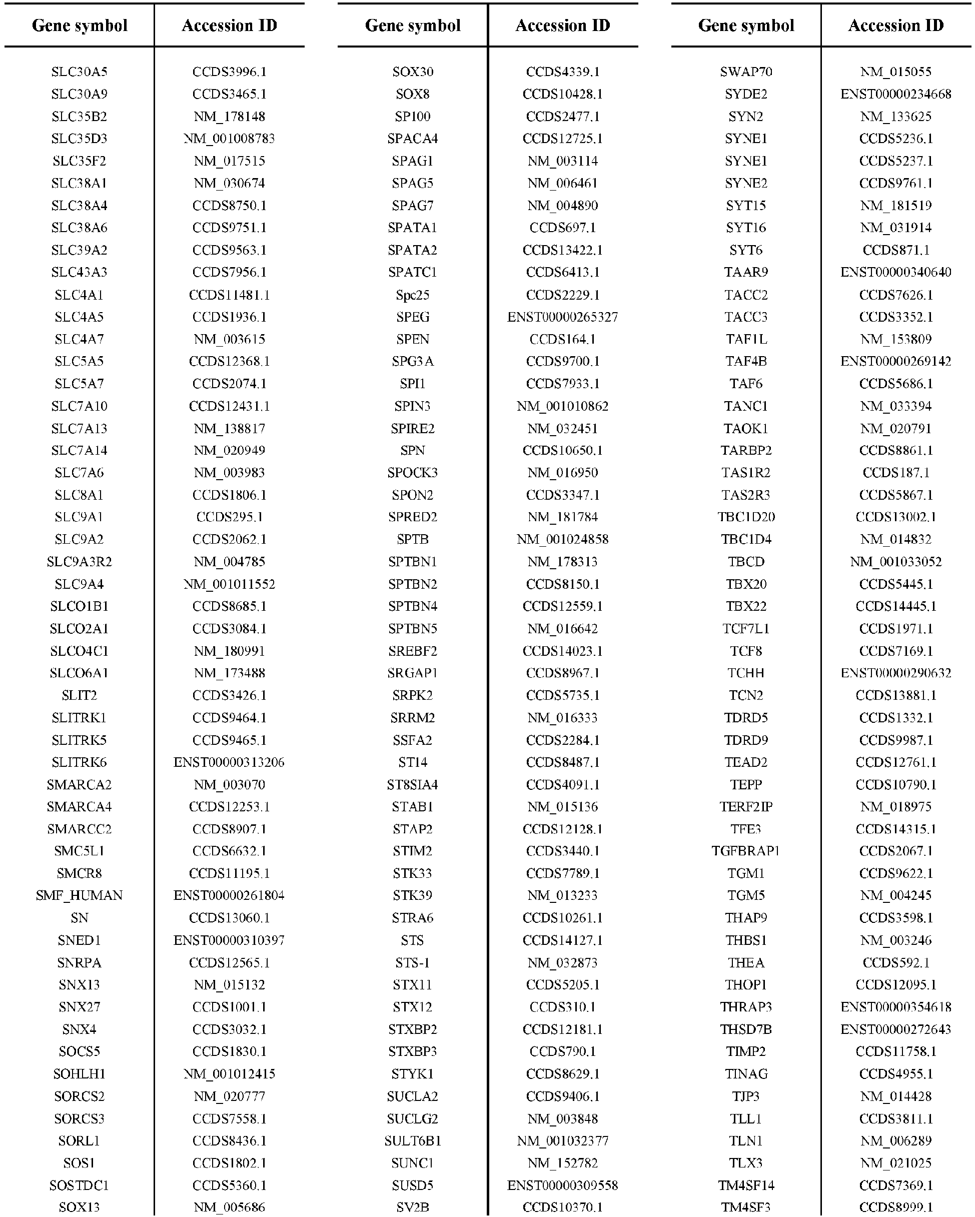

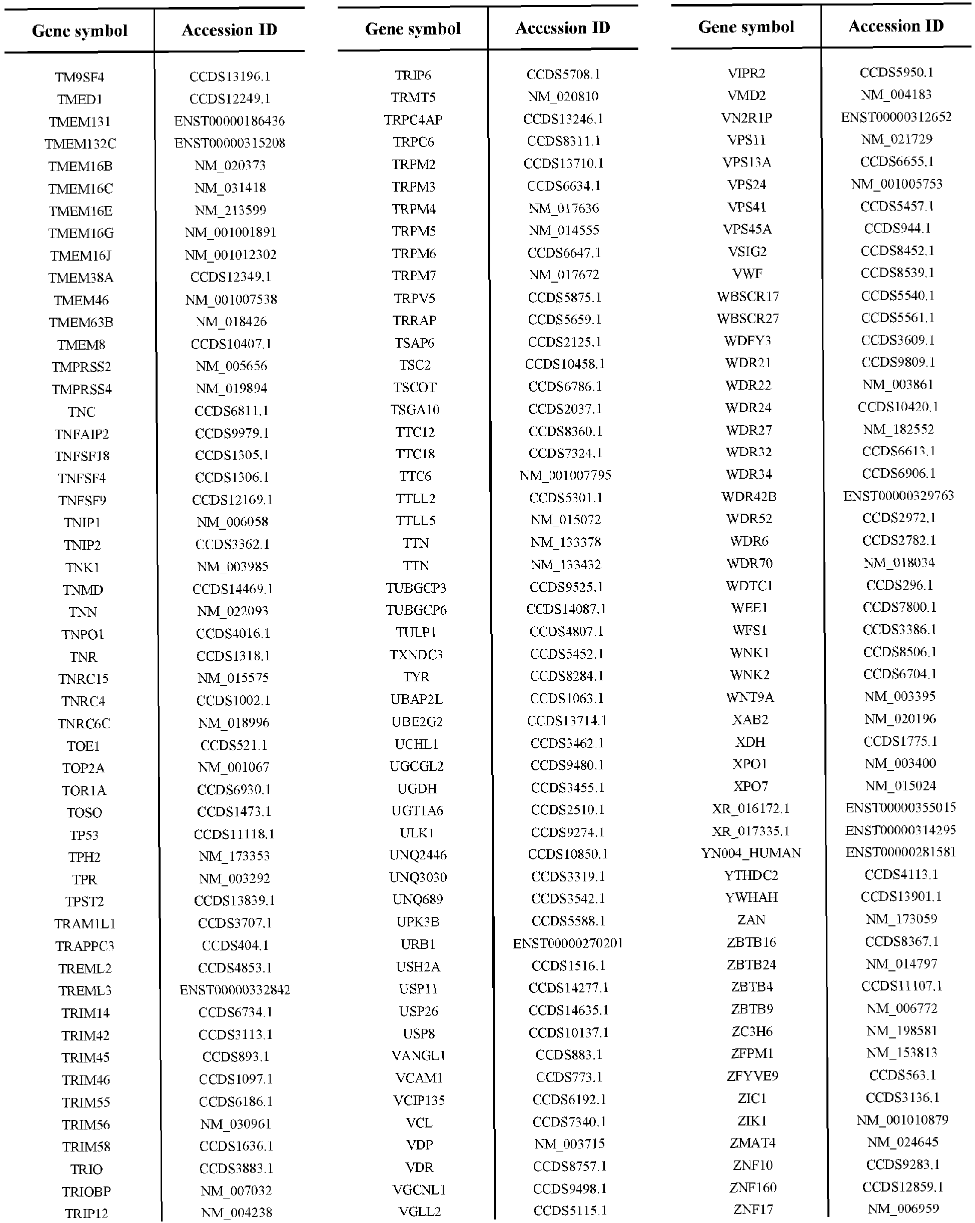

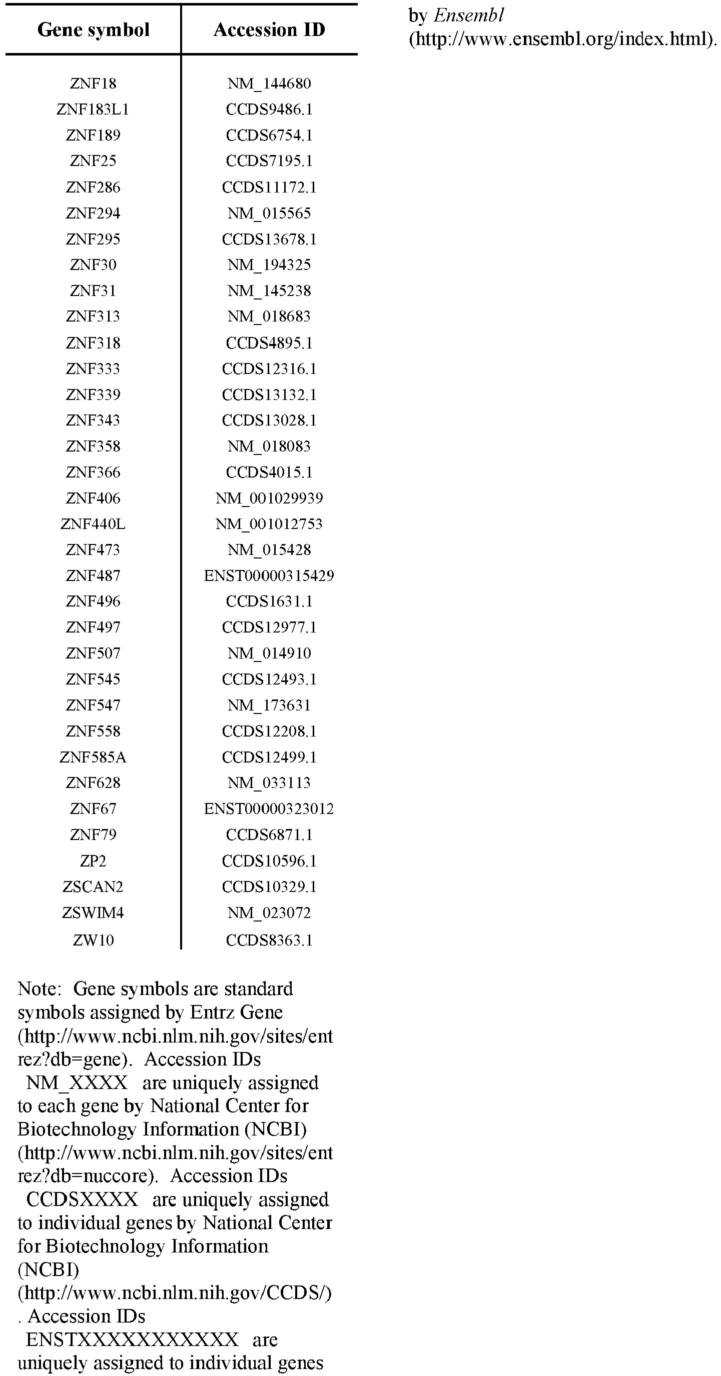

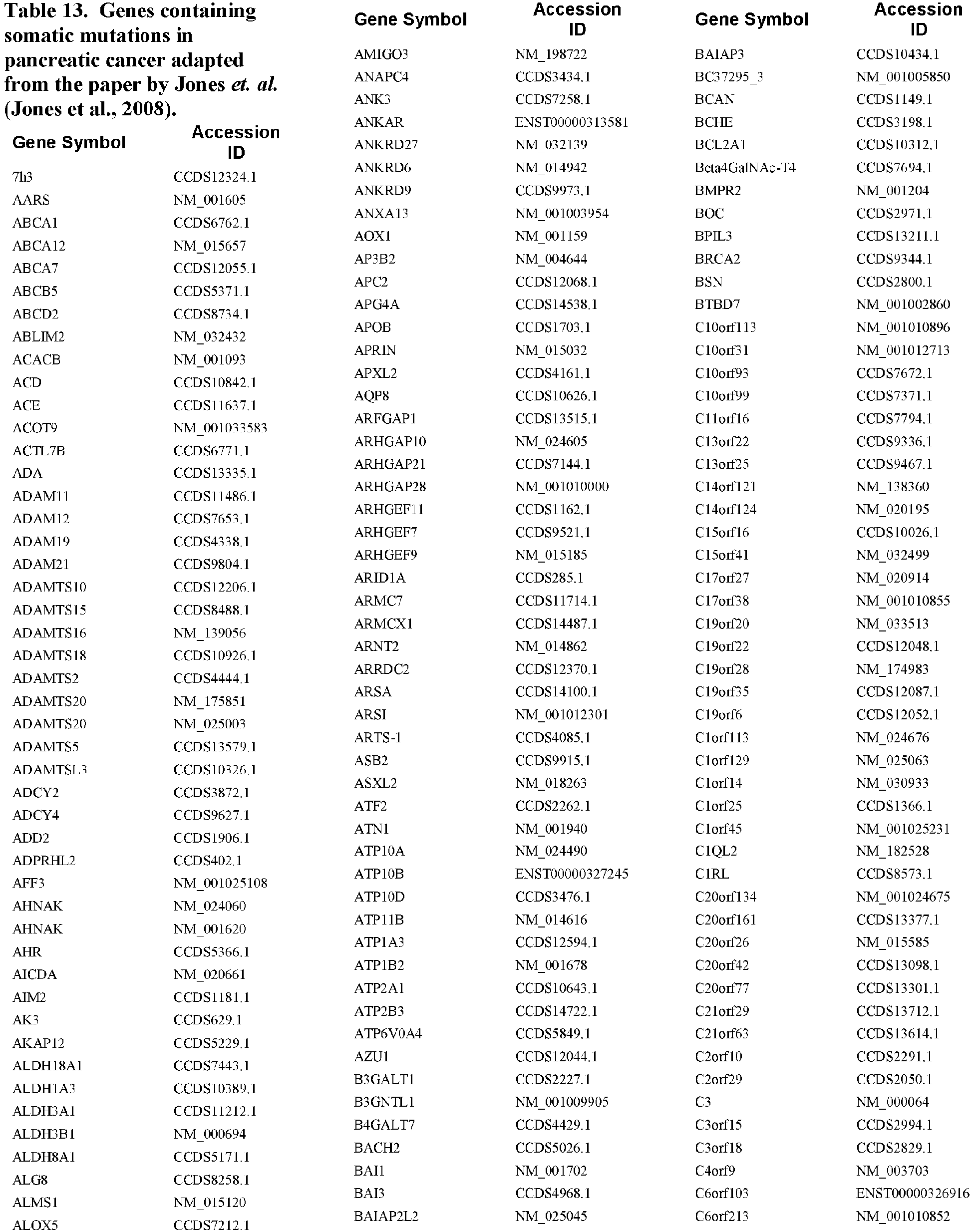

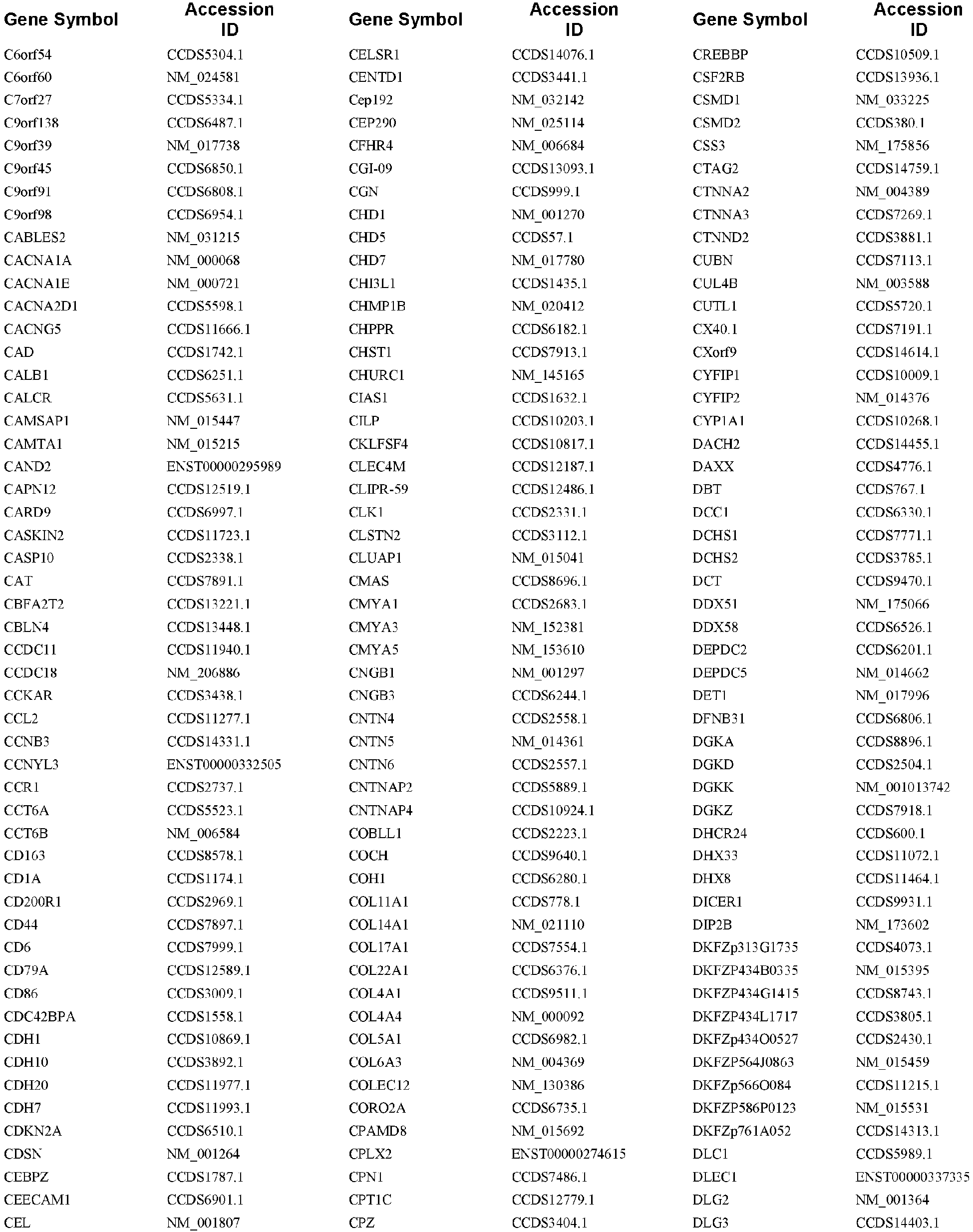

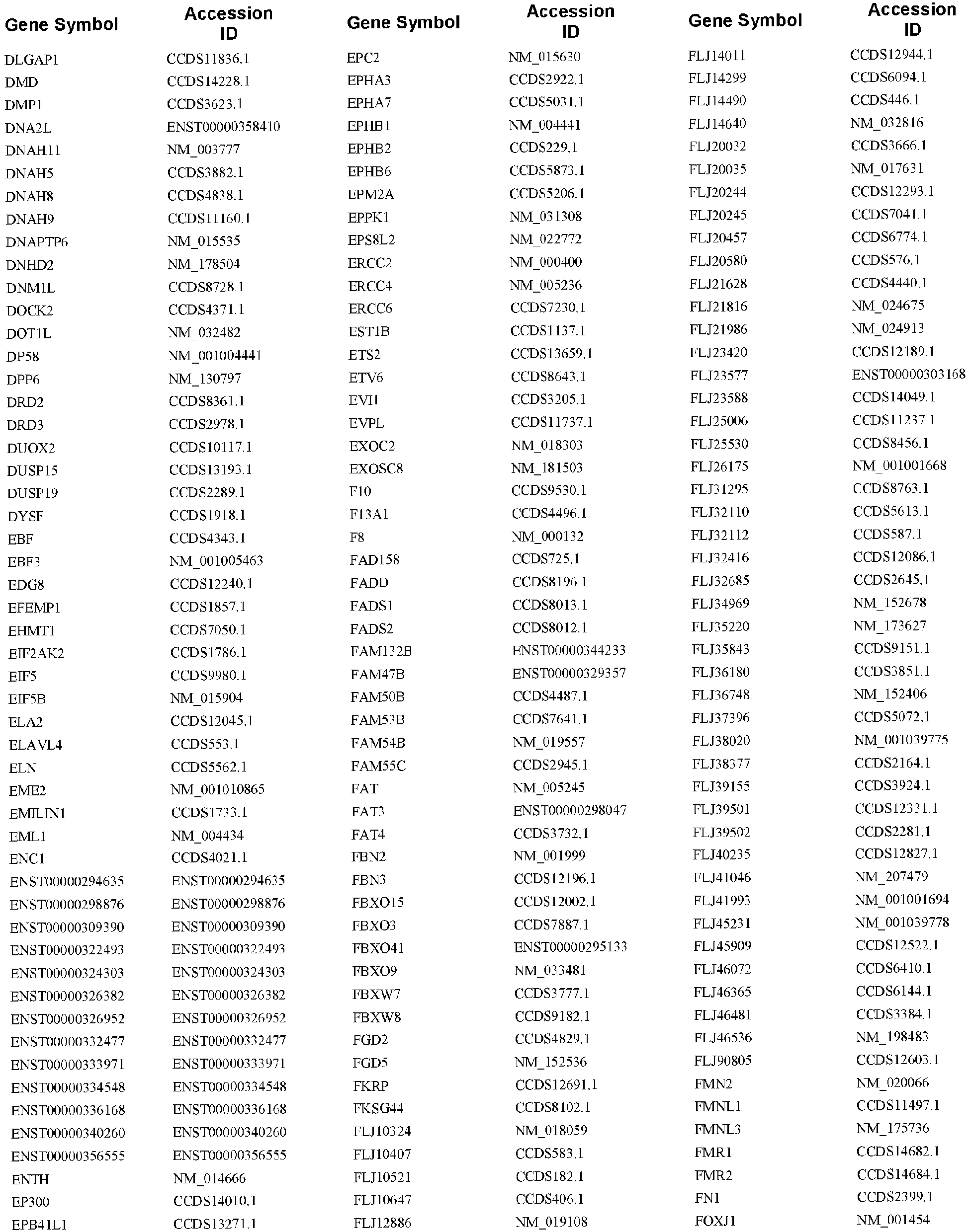

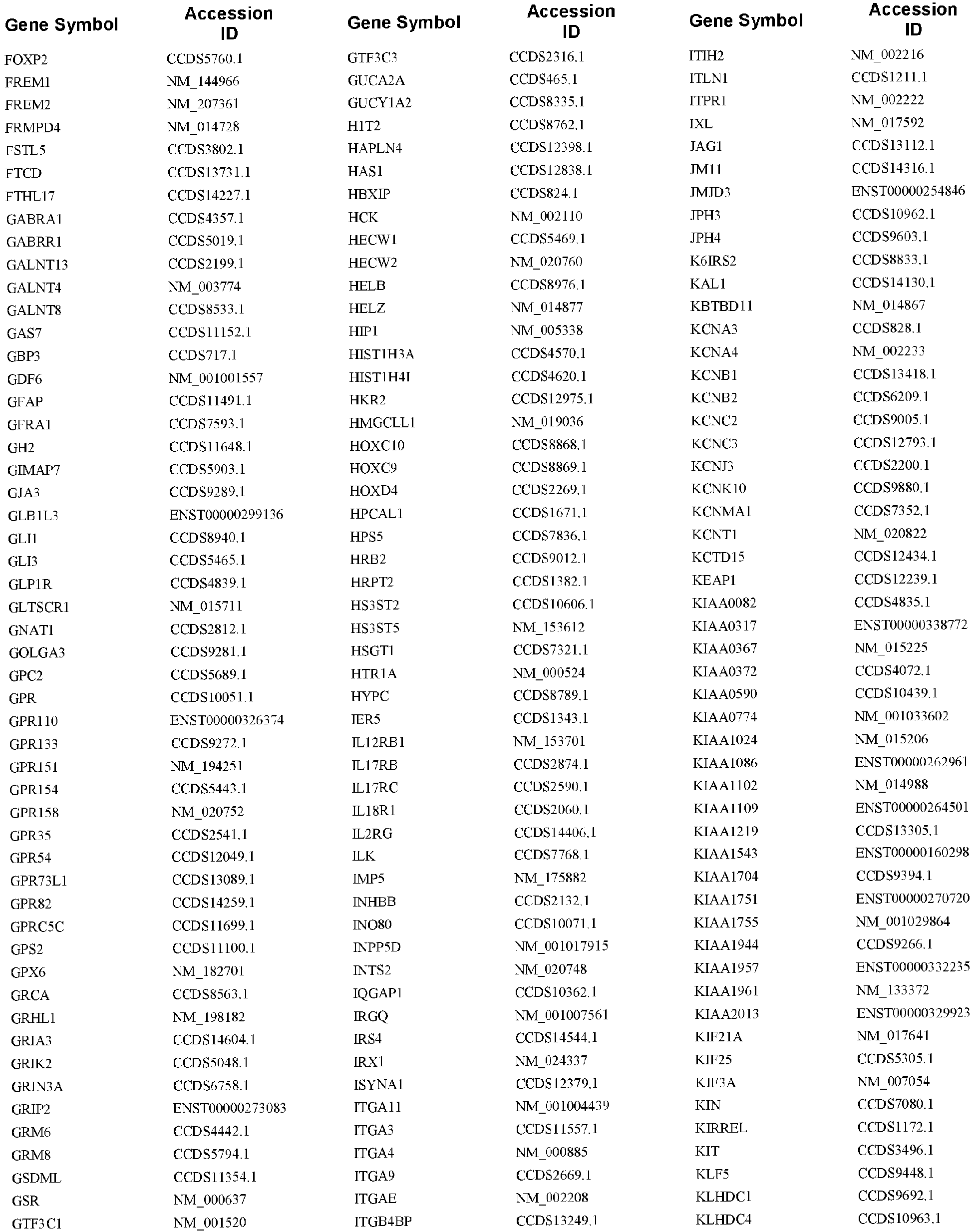

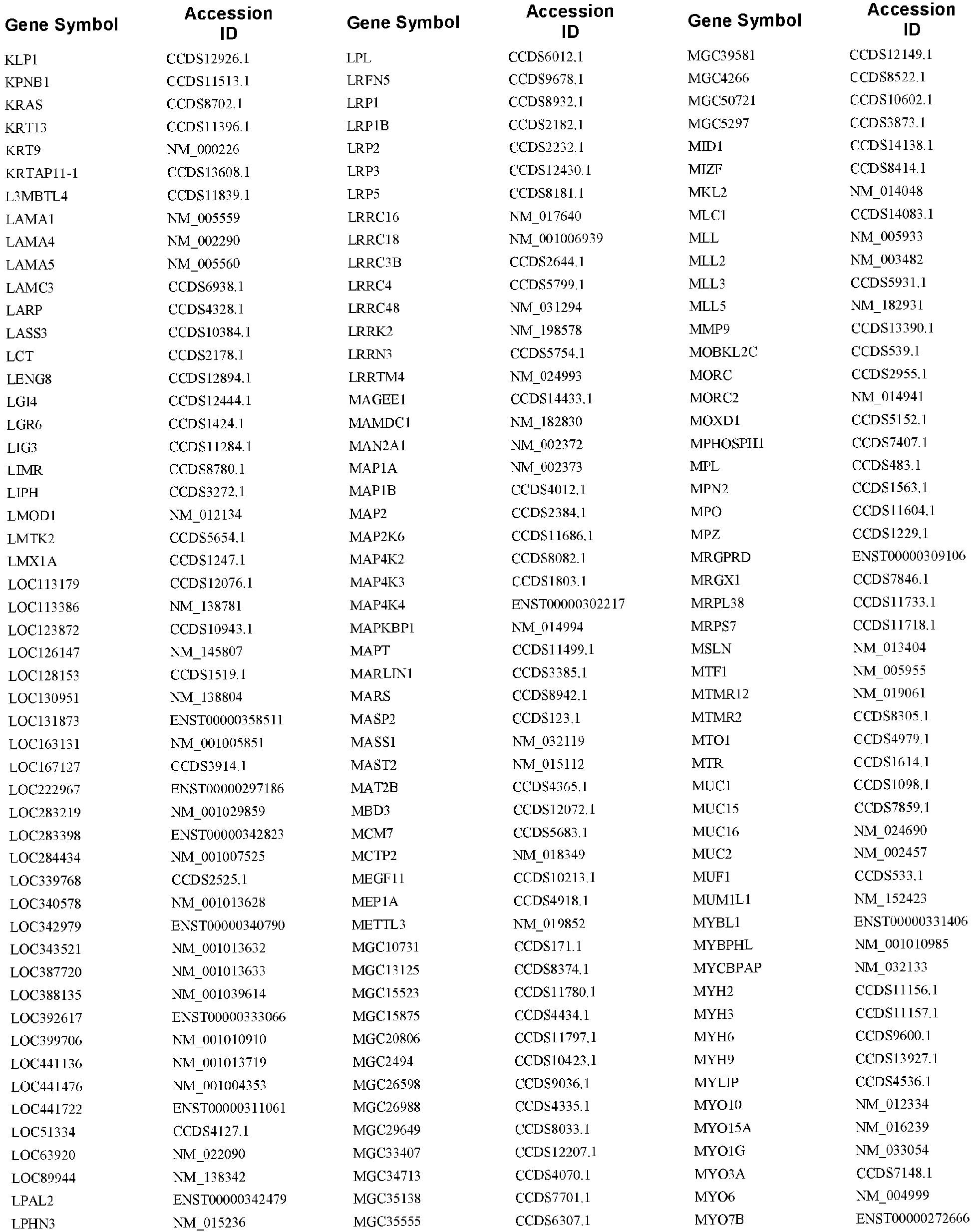

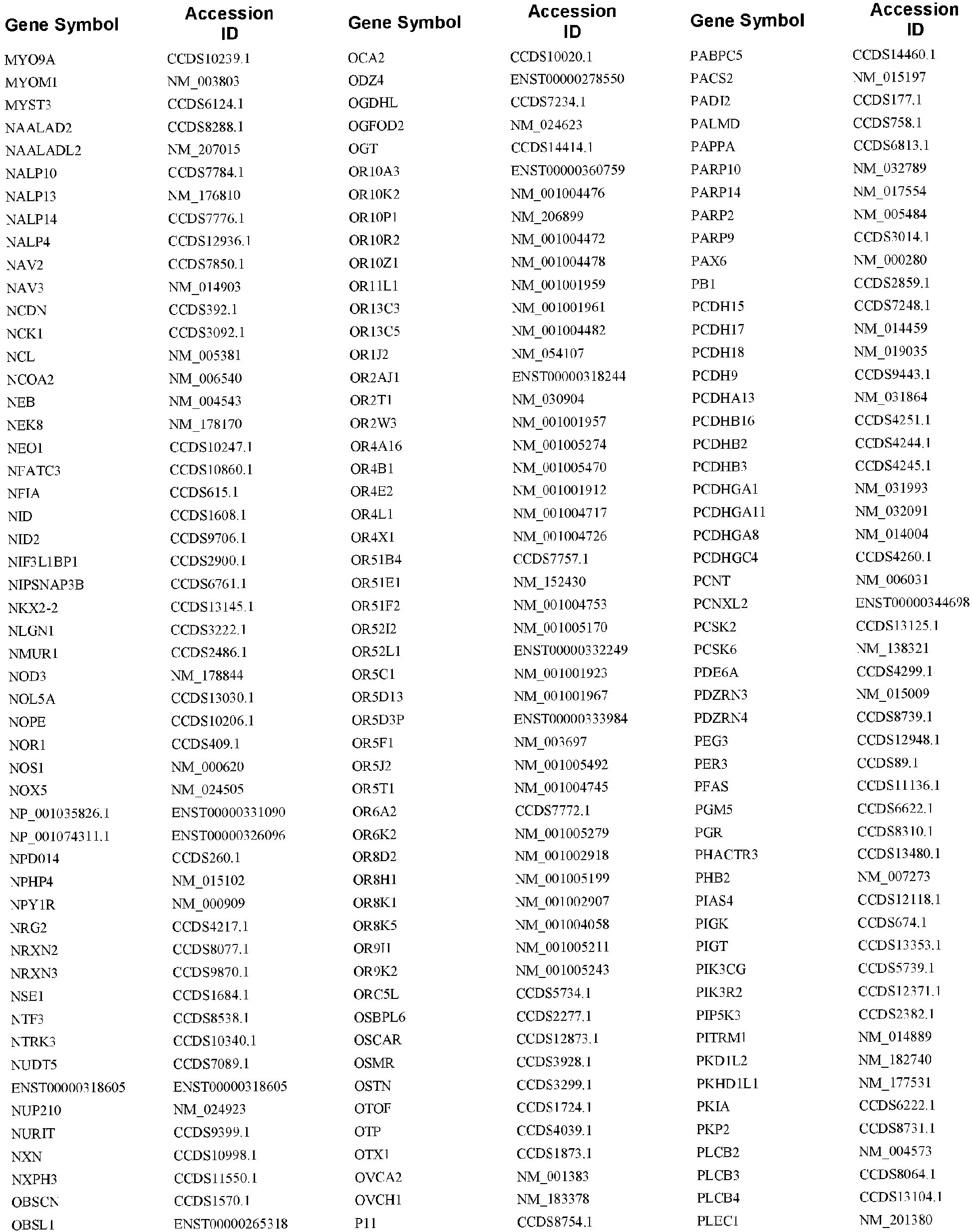

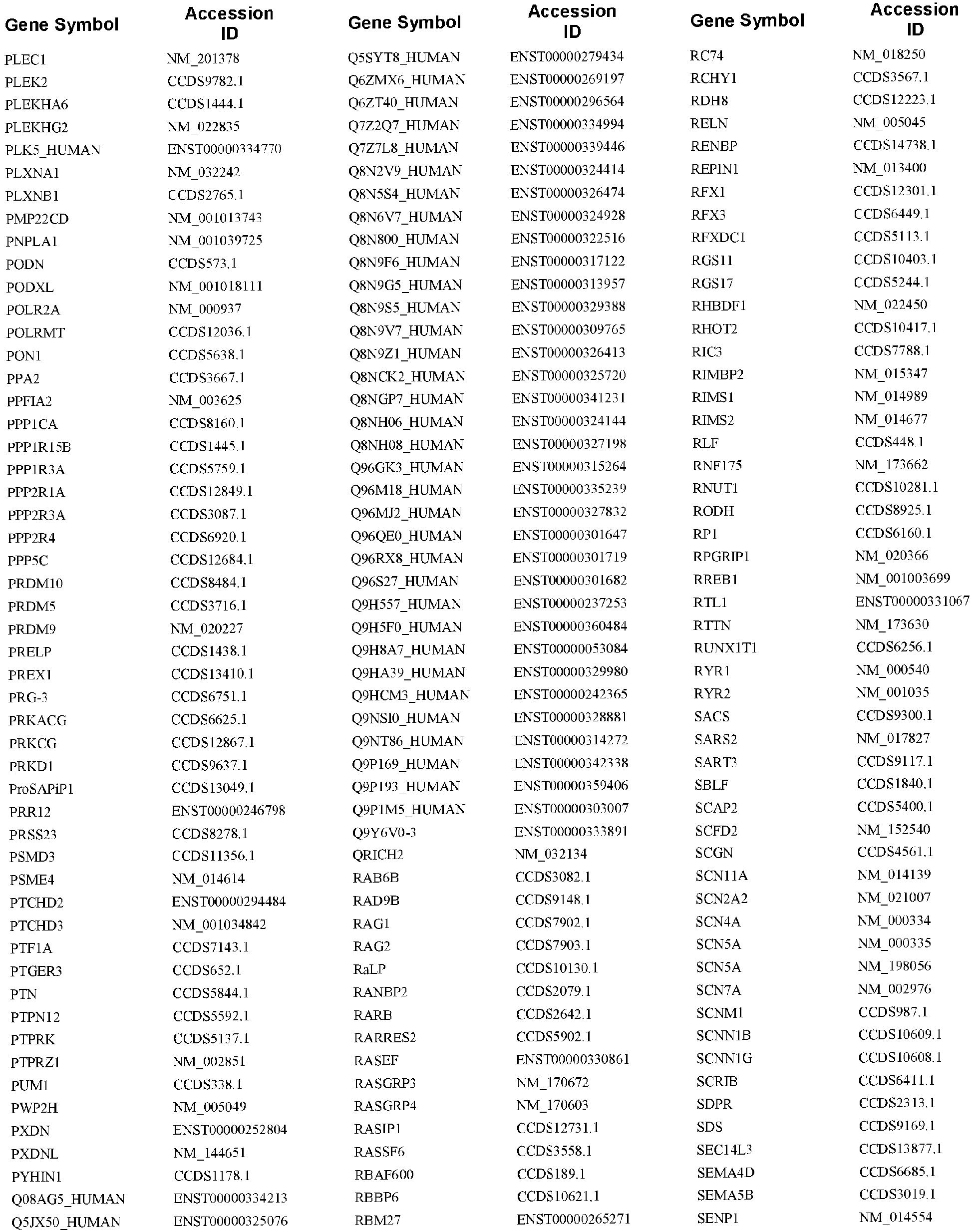

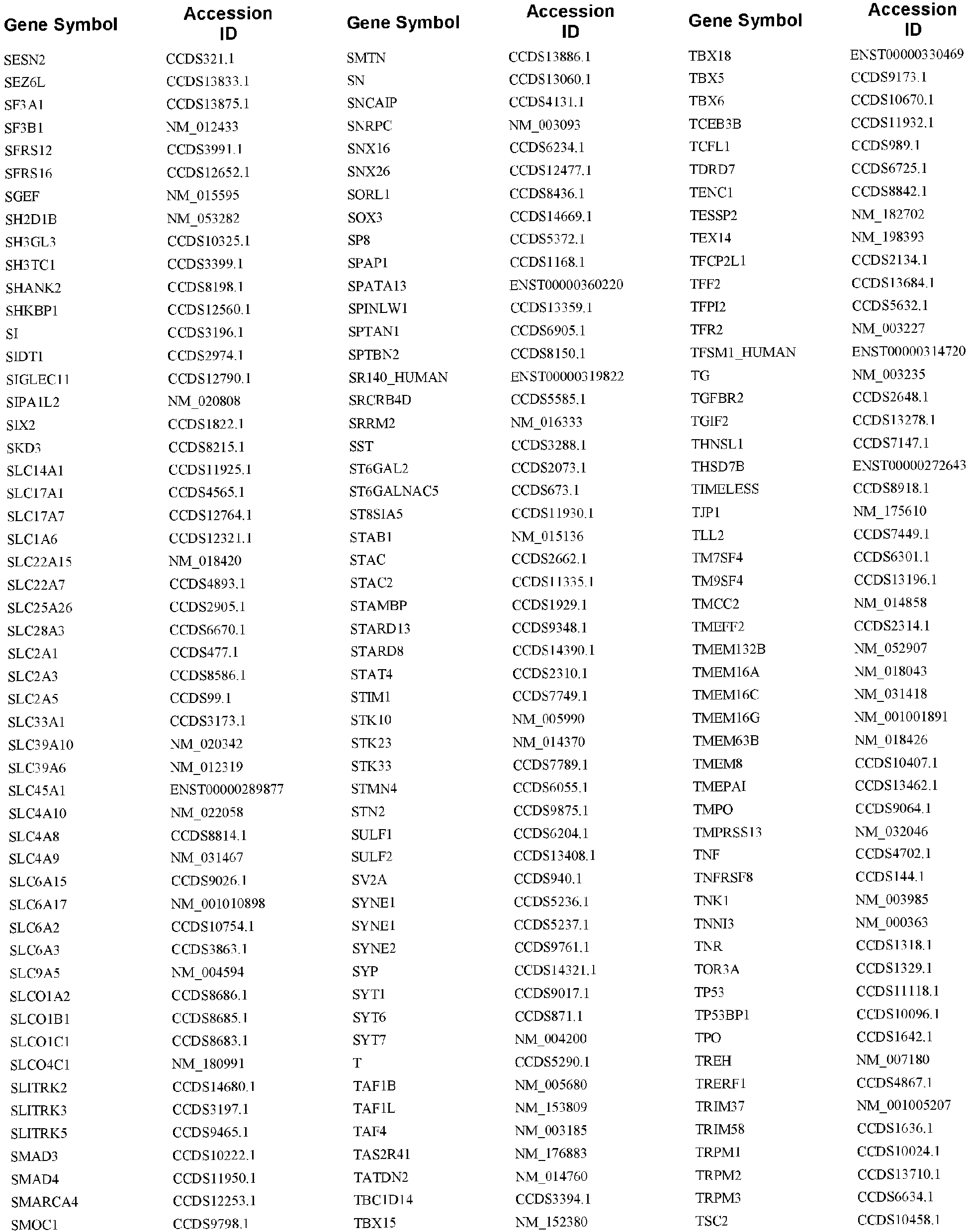

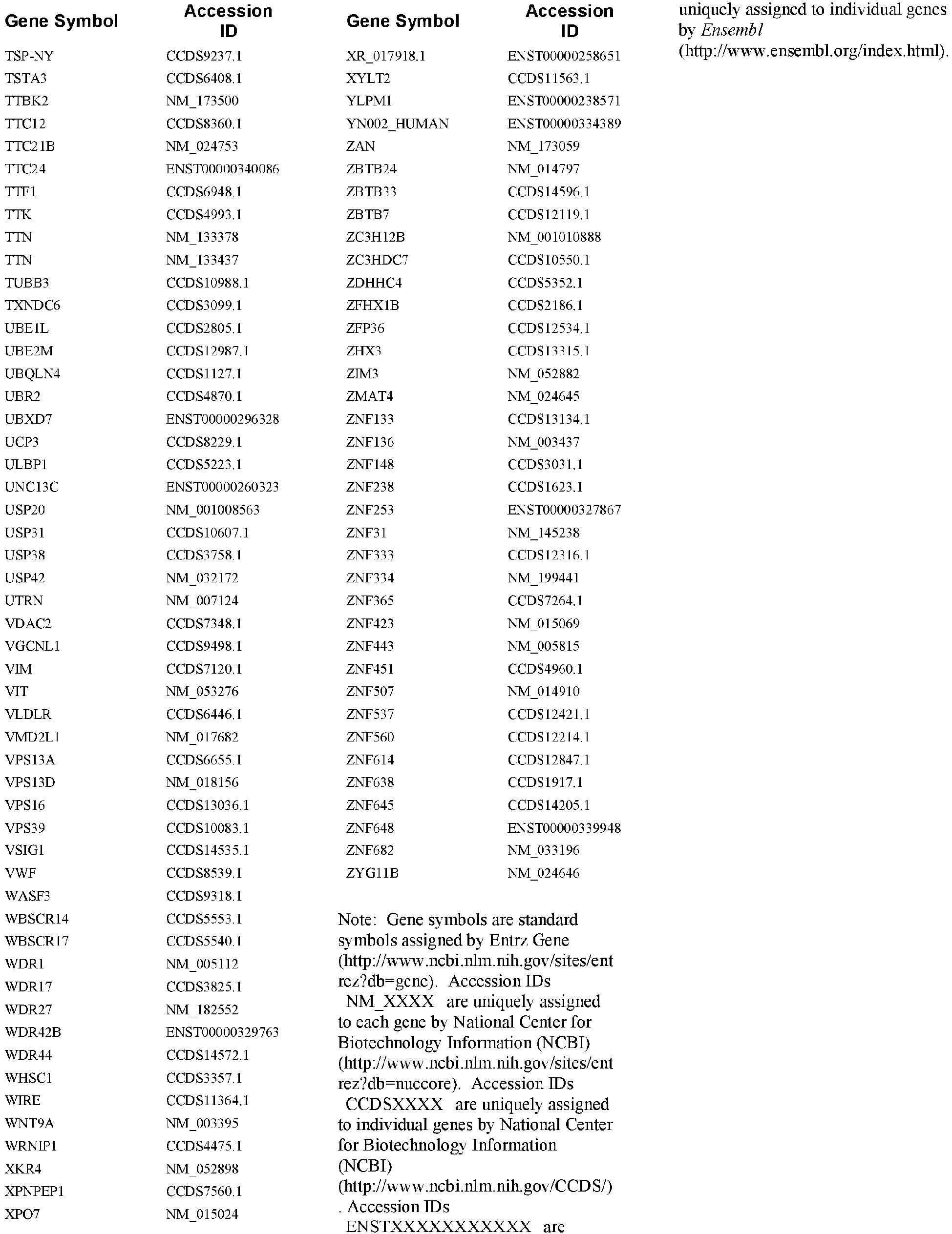

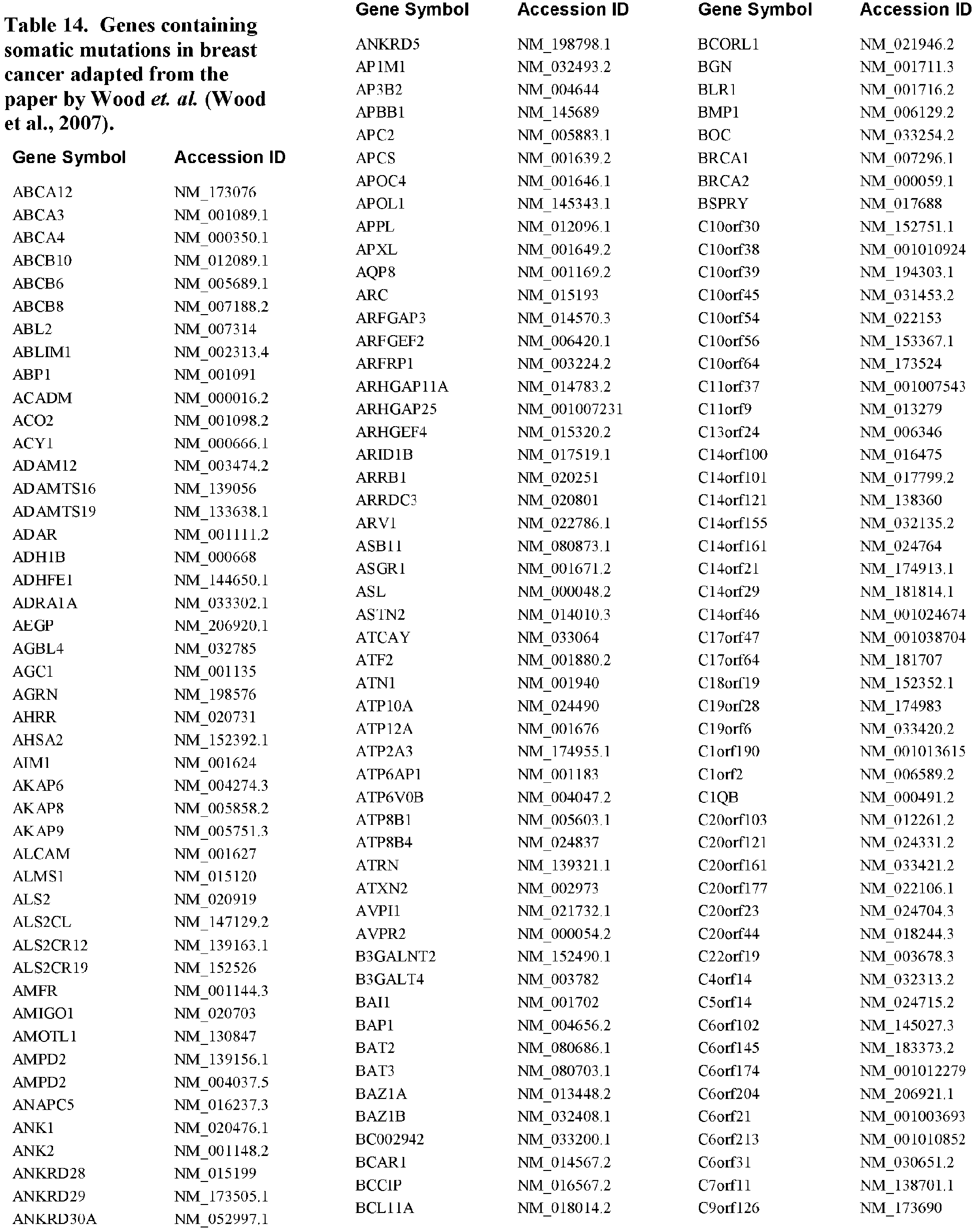

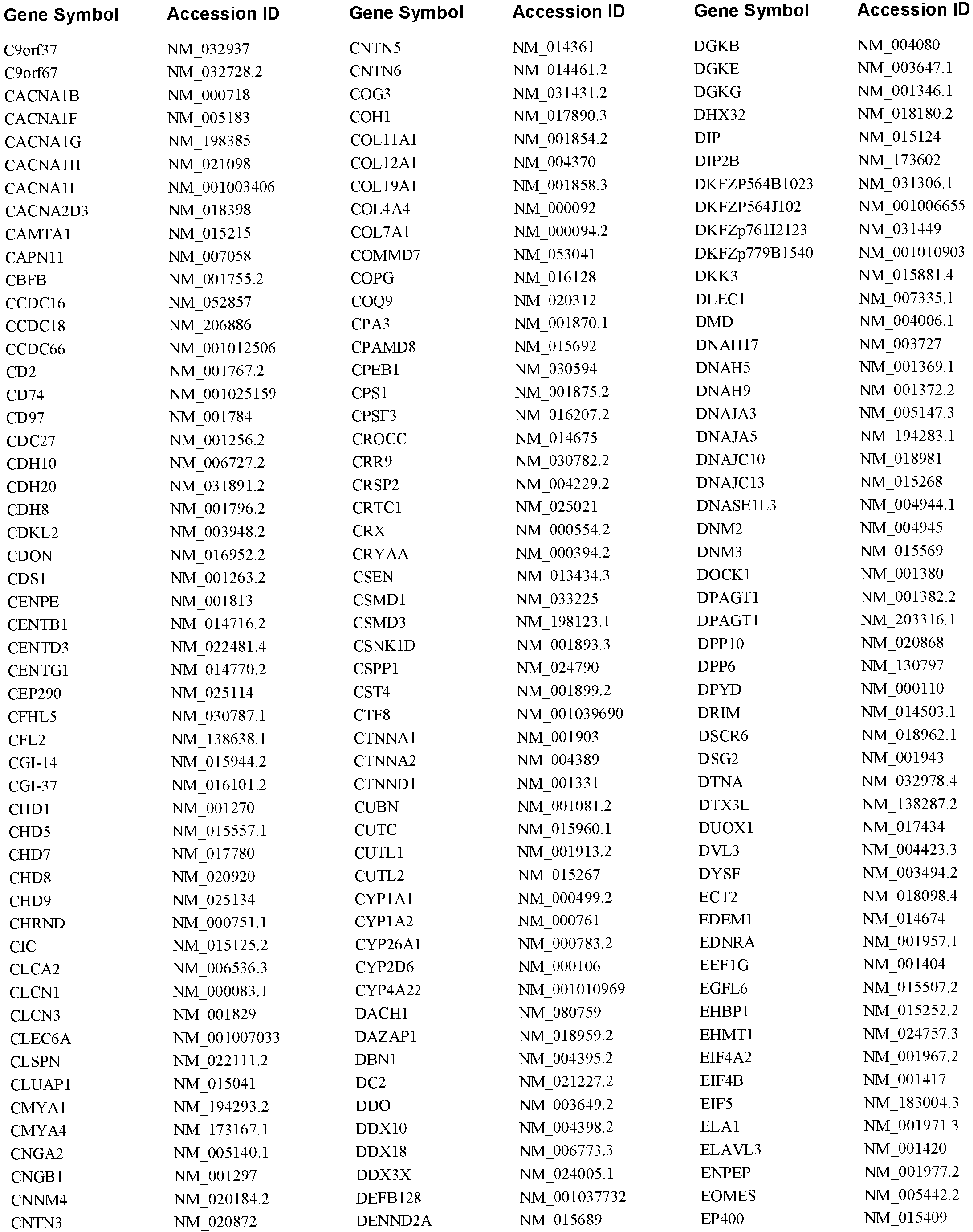

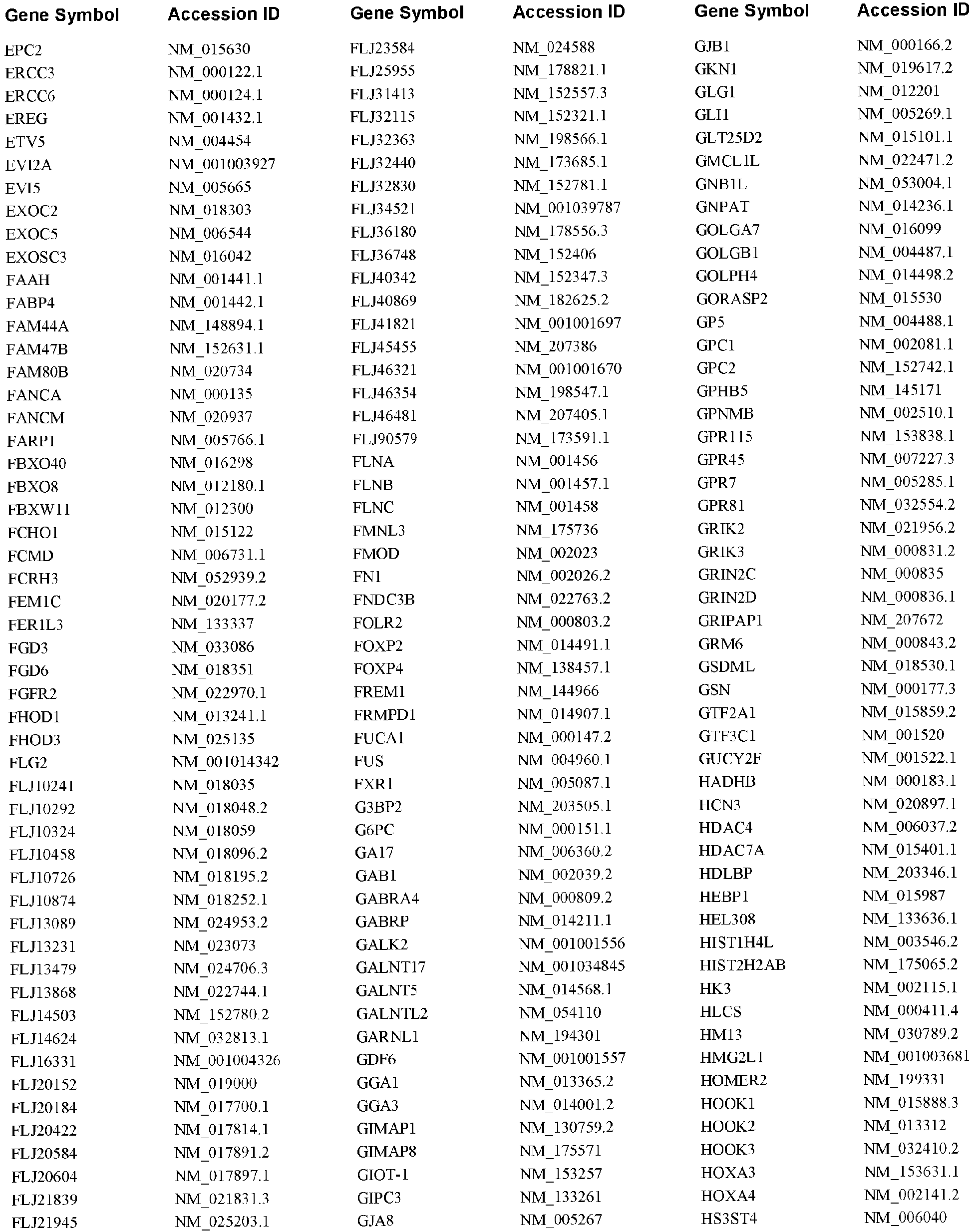

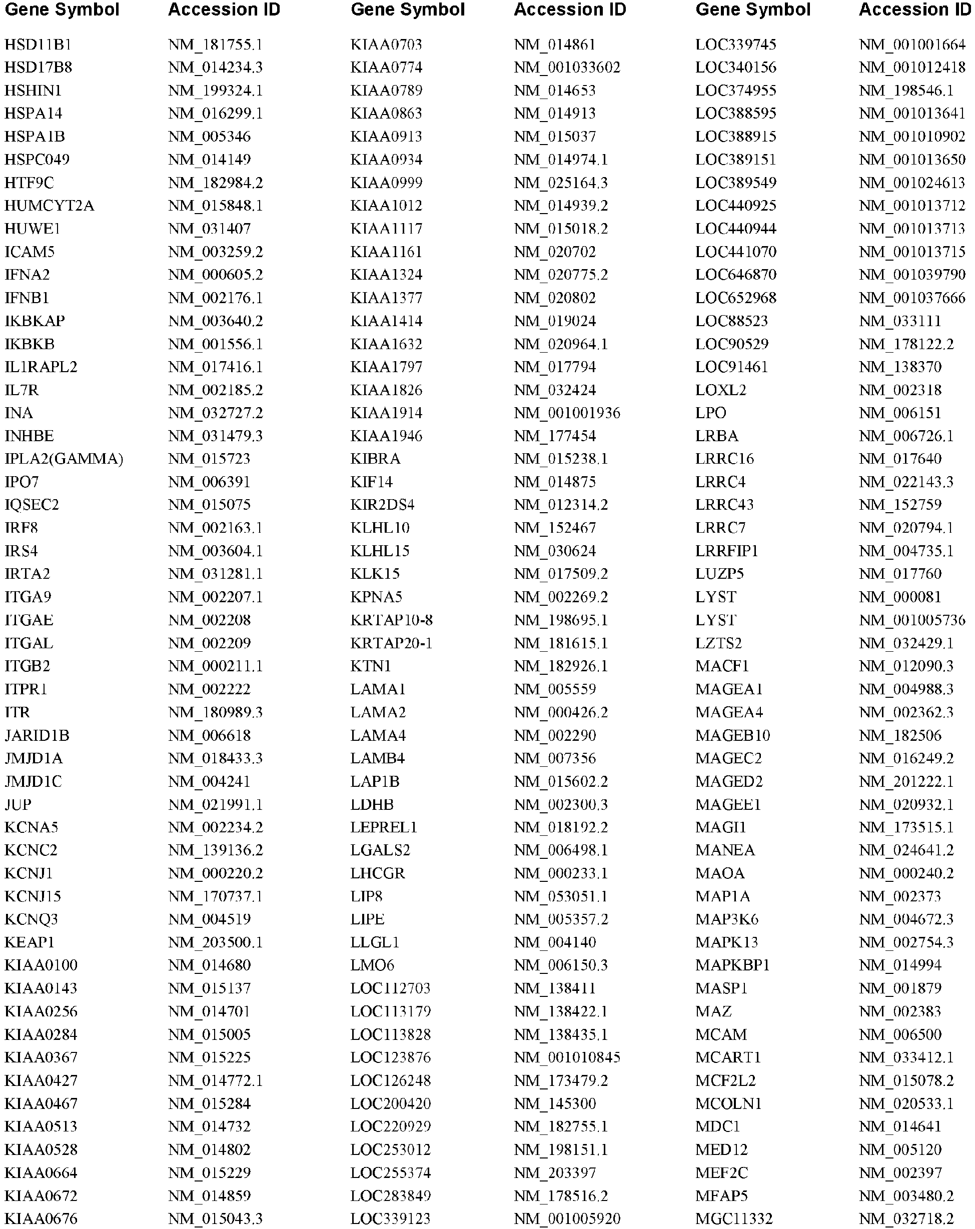

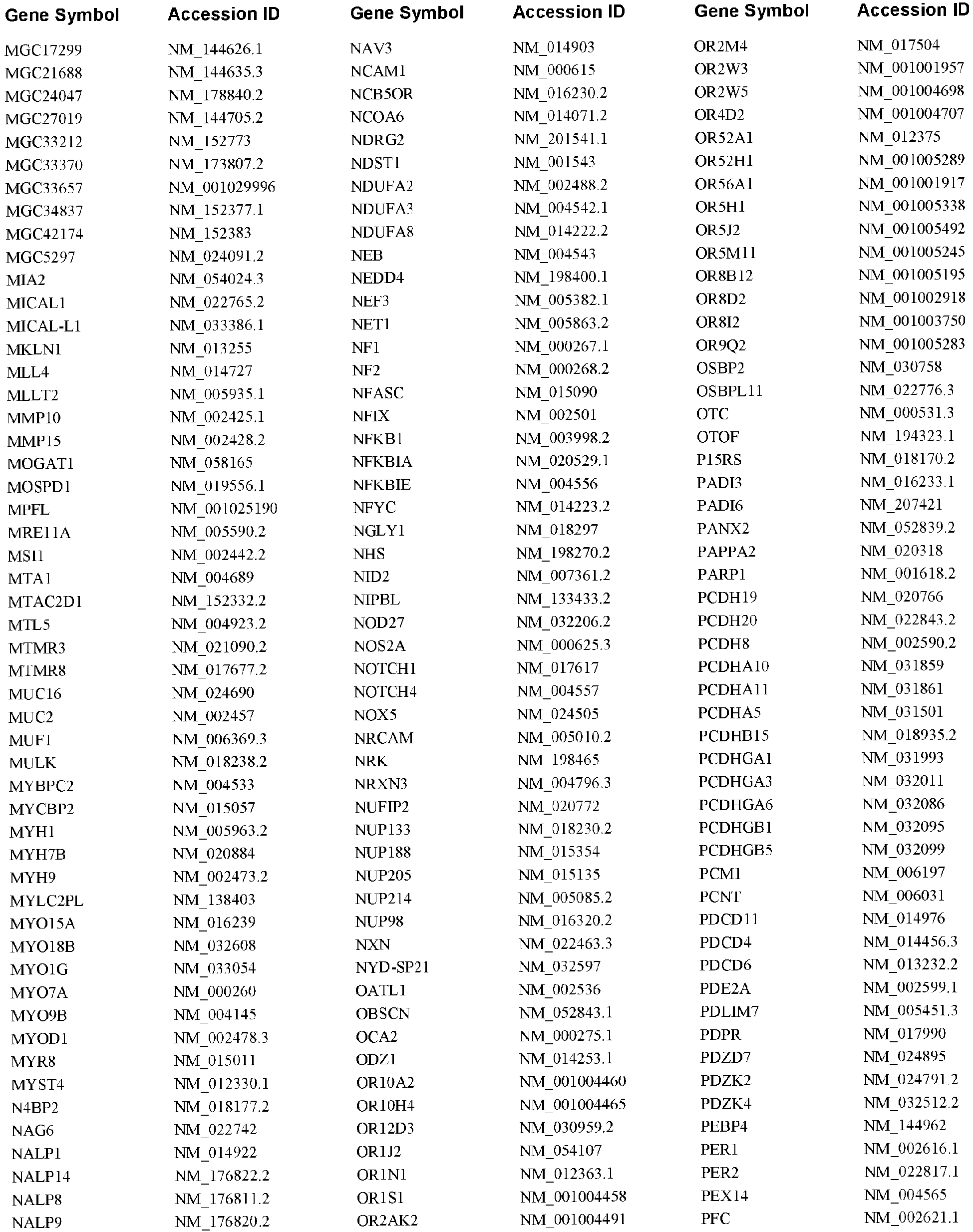

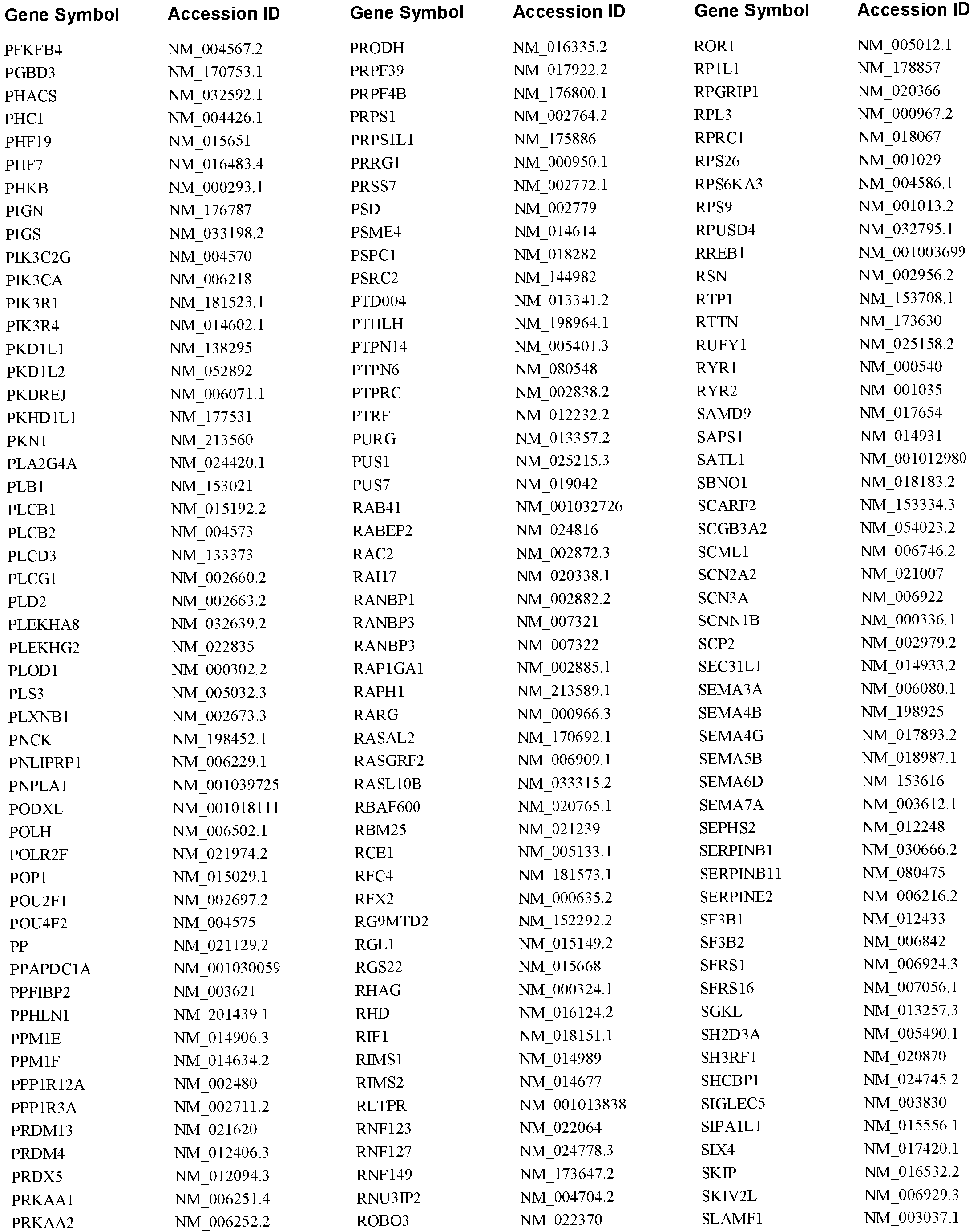

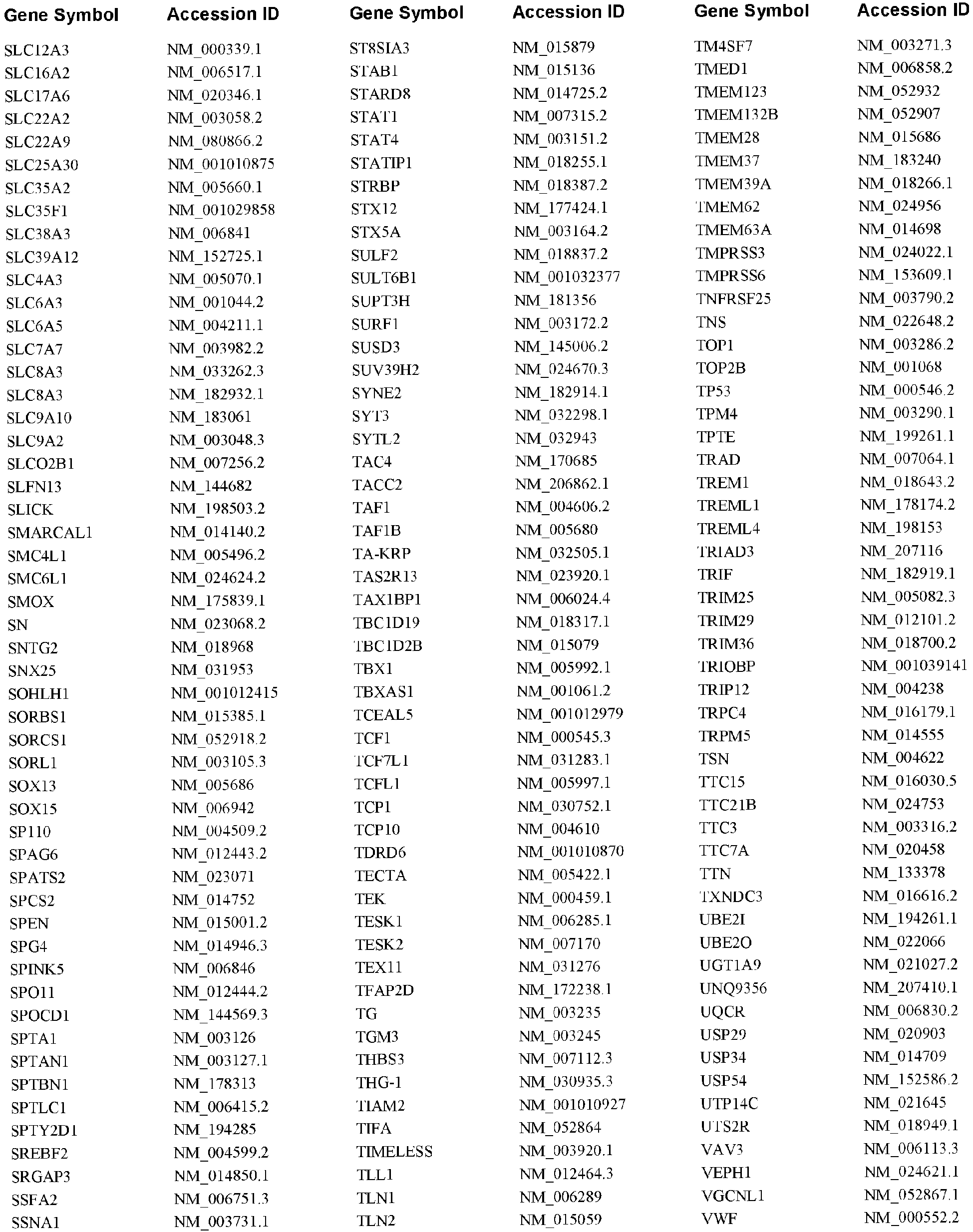

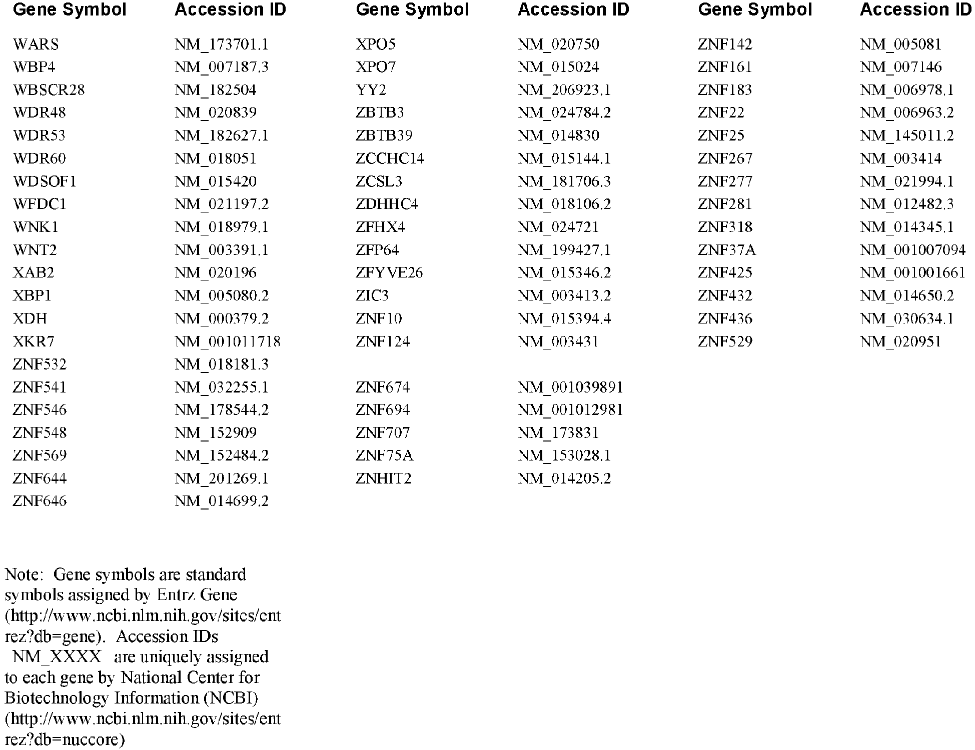

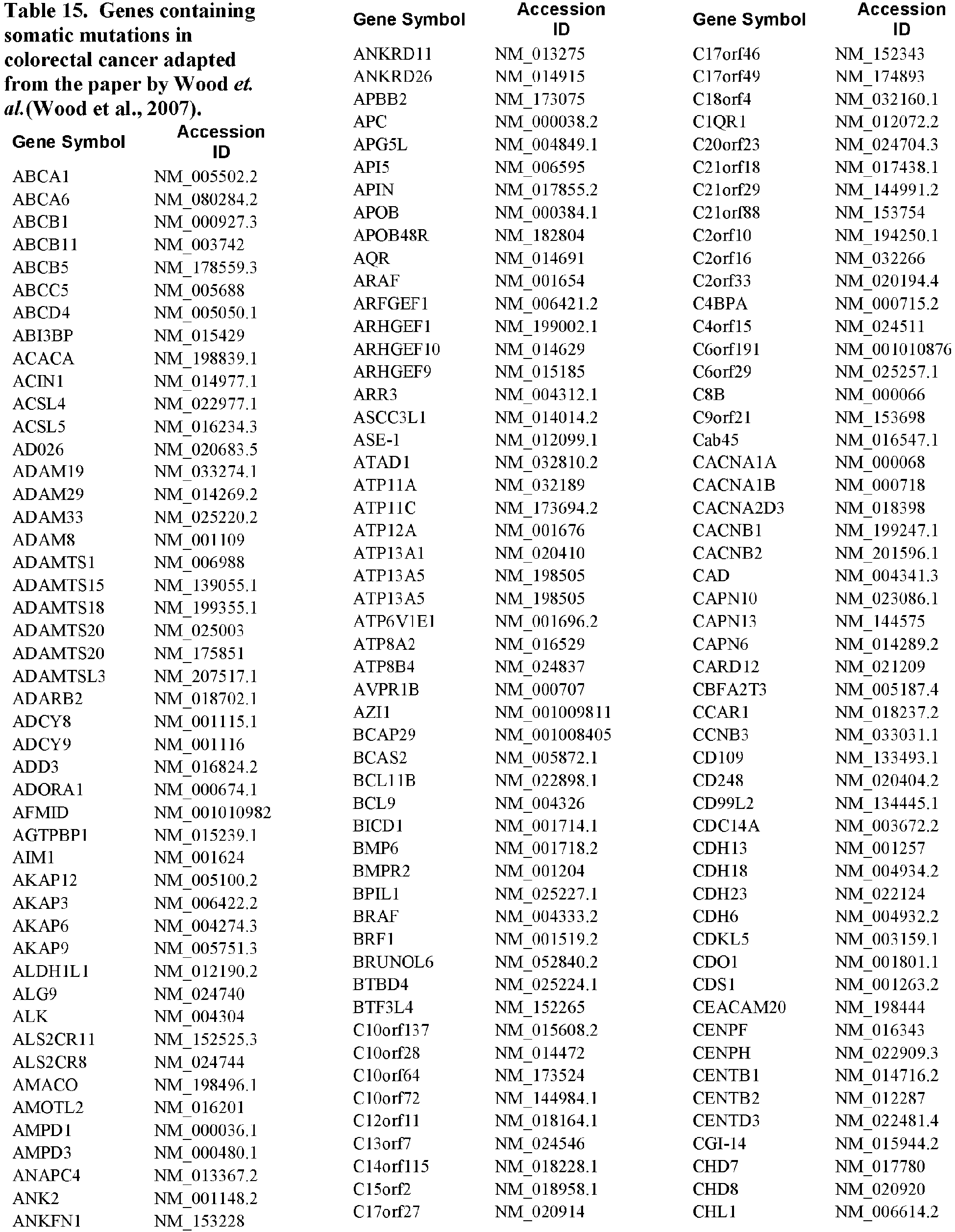

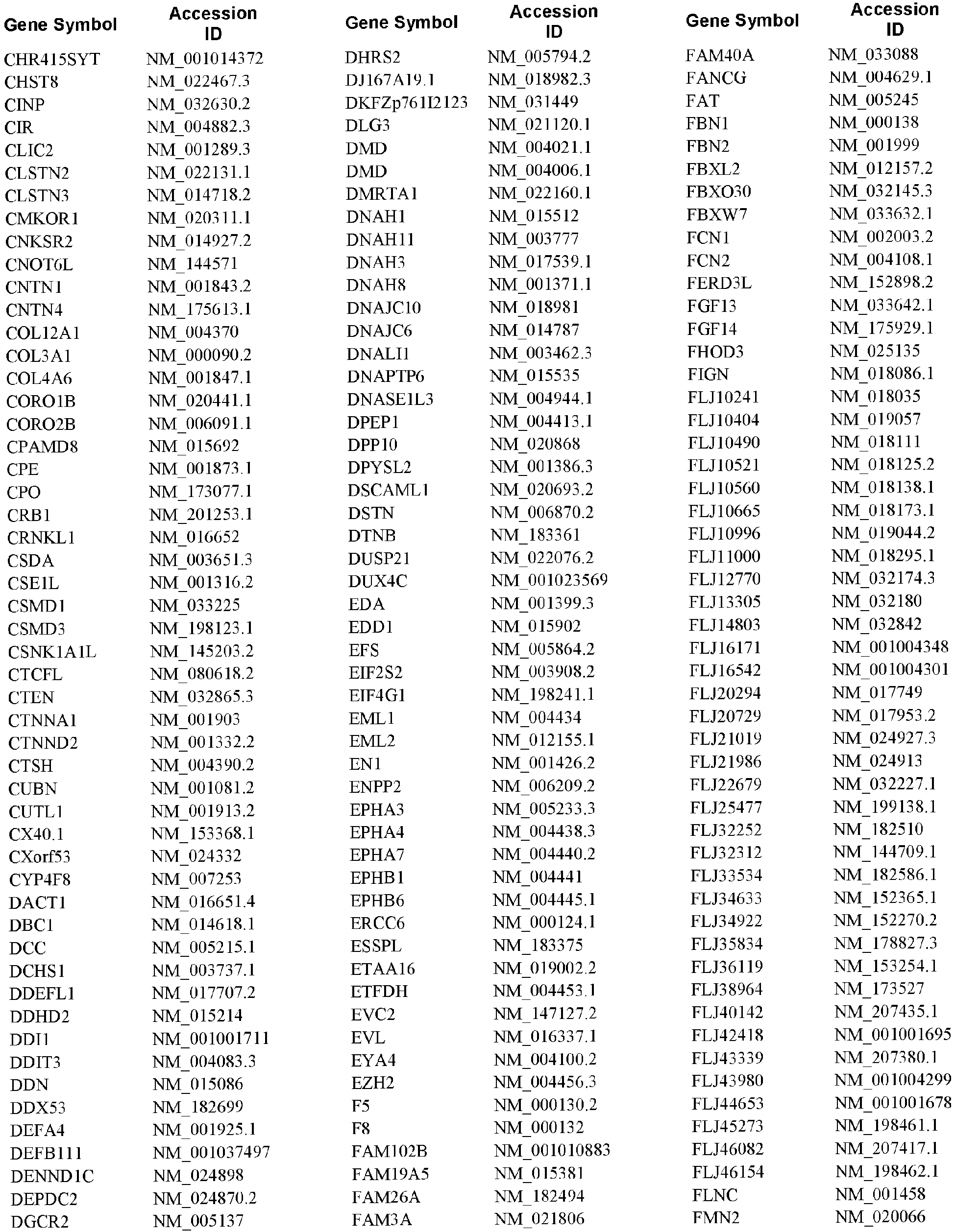

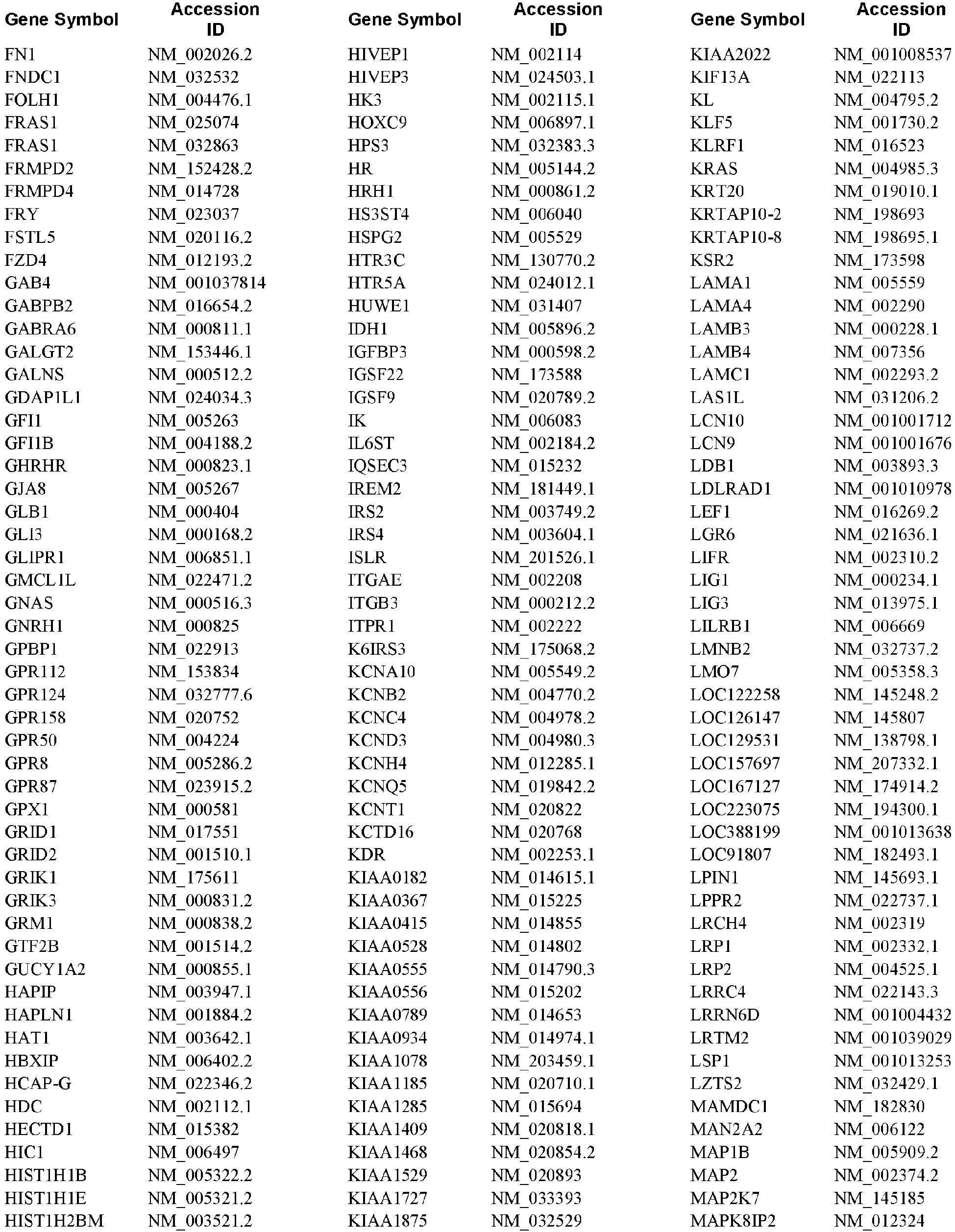

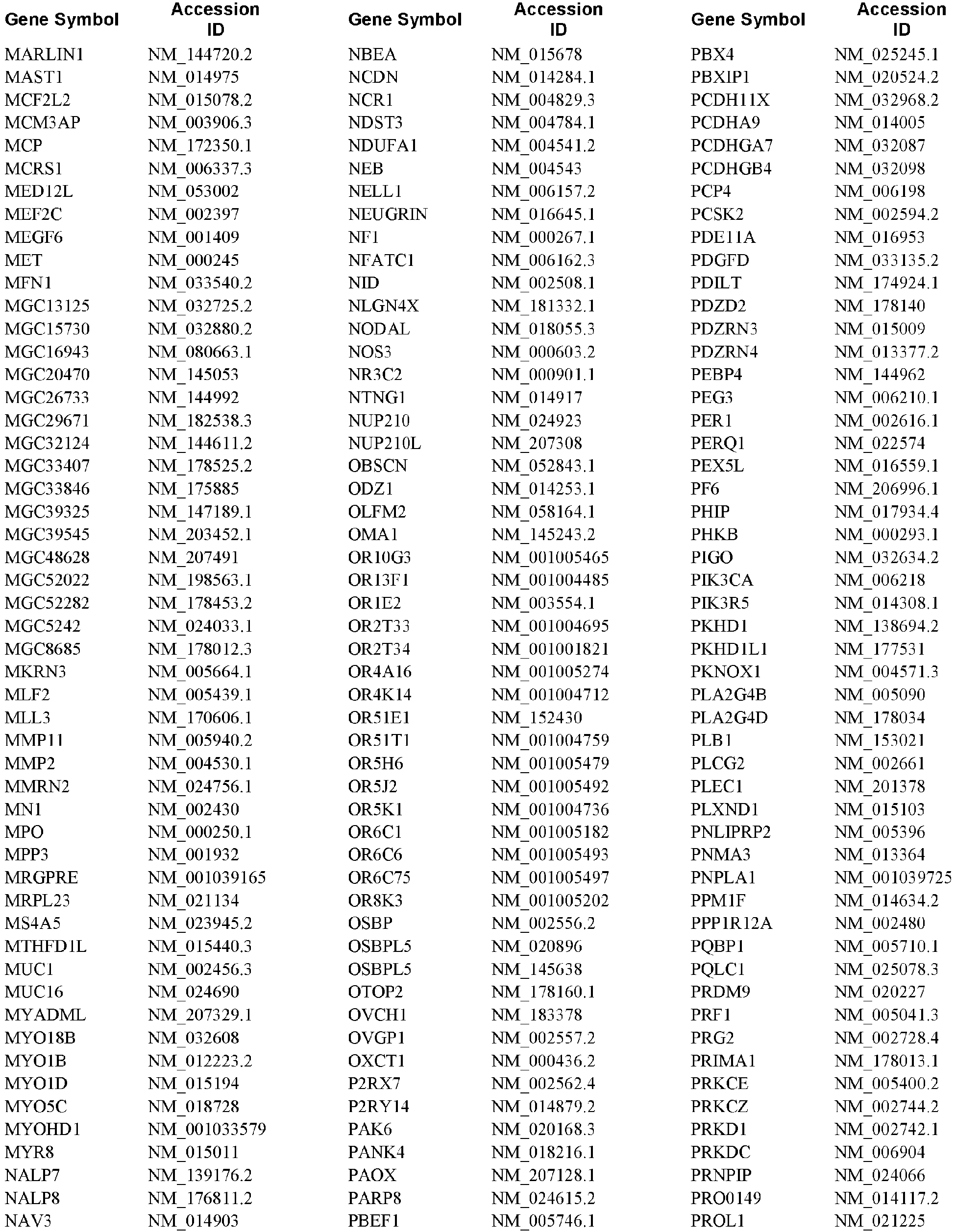

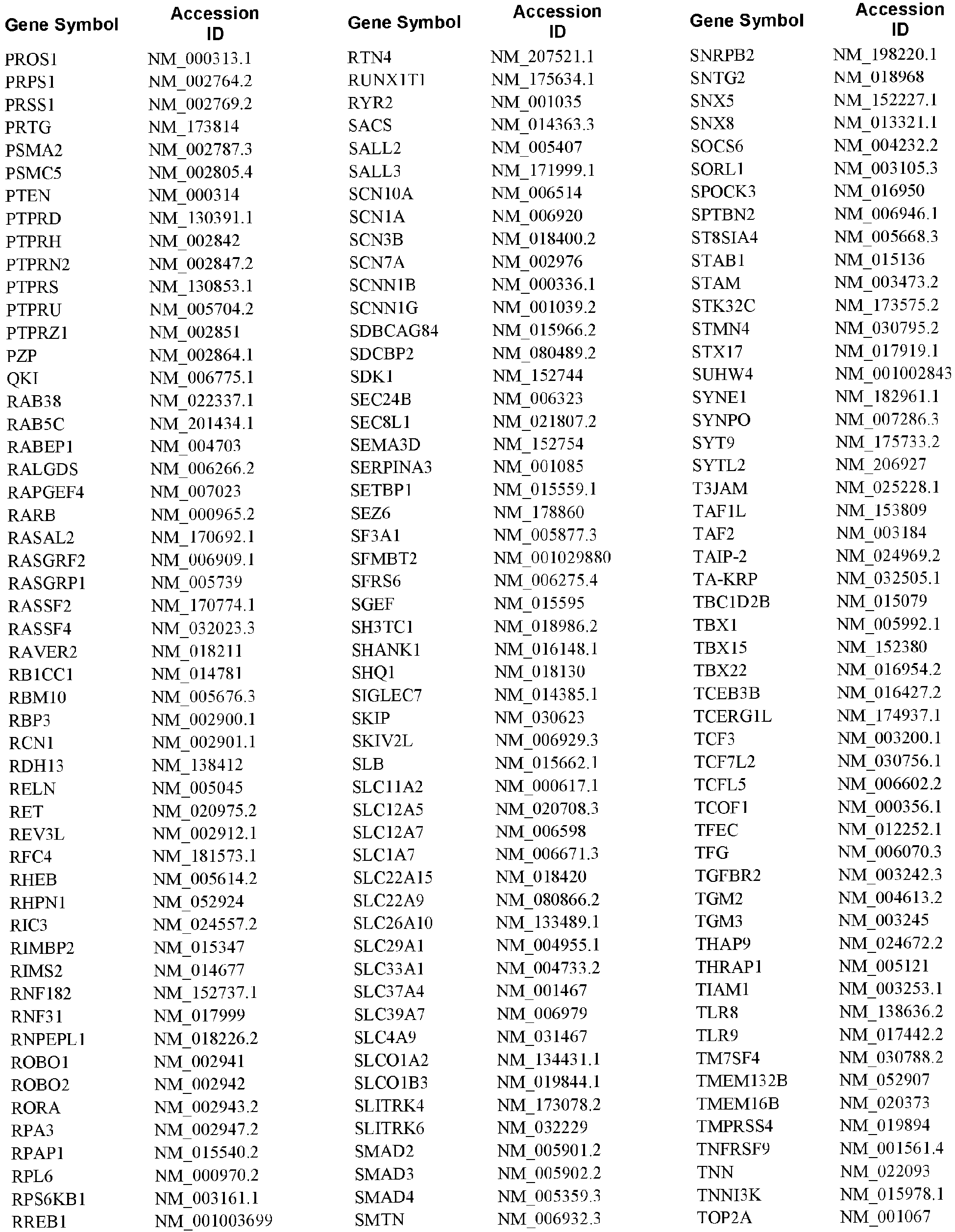

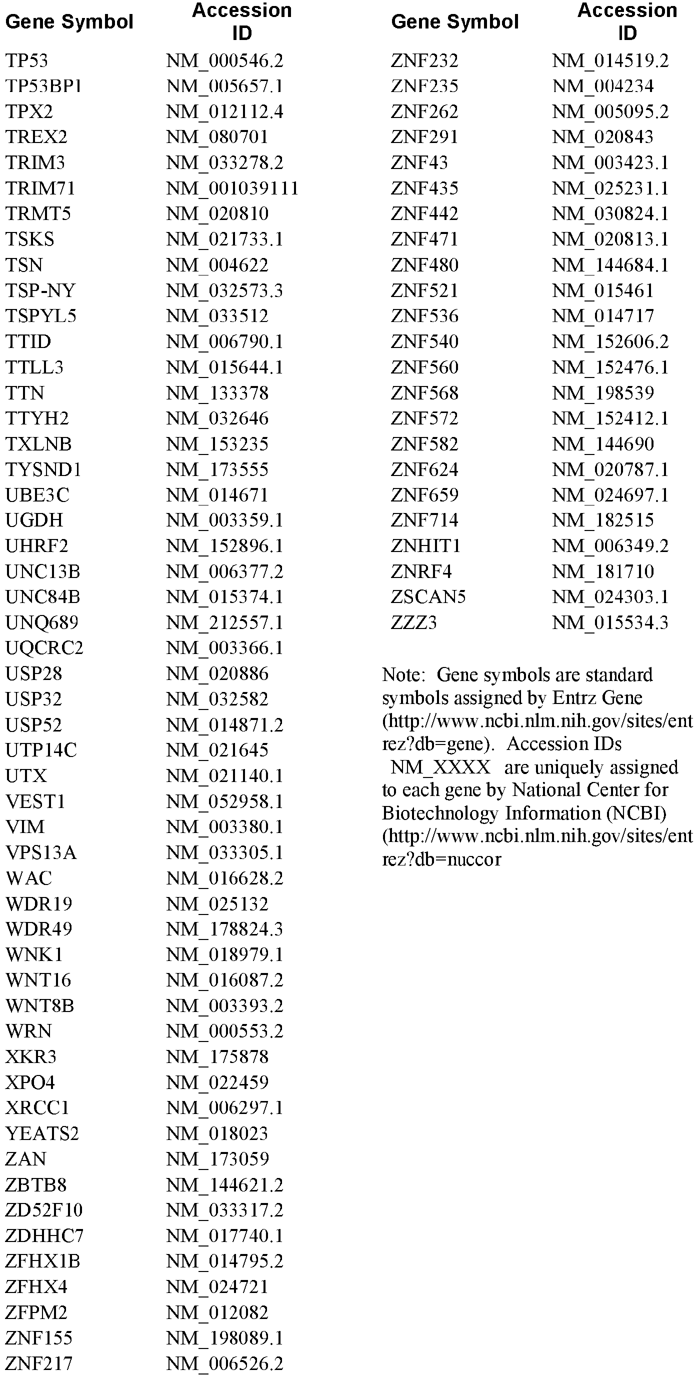

## Claims

1. A method for detecting a genome-wide genetic profile in microvesicles from a subject, thereby aiding in the diagnosis, monitoring and/or evaluation of a disease or other medical condition in a subject, comprising the steps of:
(a) isolating a microvesicle fraction from a biological sample from the subject, wherein the biological sample from the subject is a bodily fluid sample, wherein the bodily fluid sample is serum or urine;
(b) extracting nucleic acids from the microvesicle fraction, wherein the extracted nucleic acids comprise mRNAs; and
(c) detecting the expression level of the mRNAs, thereby detecting the genome-wide genetic profile in the microvesicles;
(d) comparing the genome-wide genetic profile detected in (c) to a genome-wide genetic profile derived from a healthy individual, thereby aiding in the diagnosis, monitoring and/or evaluation of a disease or other medical condition in a subject.

2. The method of claim 1, wherein step (a) comprises processing a microvesicle fraction to exclude proteins, lipids, debris from dead cells and other contaminants.

3. The method of claim 2, wherein processing the microvesicle fraction comprises filtering, preferably wherein the filtering comprises filtering through a 0.8 µm or 0.22 µm filter.

4. The method of any of the preceding claims, wherein step (a) comprises purifying microvesicles from the microvesicle fraction using size exclusion chromatography, density gradient centrifugation, centrifugation, differential centrifugation, ultracentrifugation, nanomembrane ultrafiltration, immunosorbent capture, affinity purification, microfluidic separation, anion exchange chromatography, gel permeation chromatography or any combination thereof.

5. The method of any of the preceding claims, wherein the detecting step c) is performed by microarray analysis, PCR, hybridization with allele-specific probes, enzymatic mutation detection, ligation chain reaction (LCR), oligonucleotide ligation assay (OLA), flow-cytometric heteroduplex analysis, chemical cleavage of mismatches, mass spectrometry, nucleic acid sequencing, single strand conformation polymorphism (SSCP), denaturing gradient gel electrophoresis (DGGE), temperature gradient gel electrophoresis (TGGE), restriction fragment polymorphisms, serial analysis of gene expression (SAGE), next generation sequencing, massively parallel sequencing or any combination thereof.

6. The method of claim 1, wherein the disease or other medical condition is cancer.

7. The method of claim 6, wherein the cancer is melanoma, glioblastoma or prostate cancer.

8. The method of any one of the preceding claims, wherein the disease or other medical condition is glioblastoma.

## Patentansprüche

1. Verfahren zum Nachweisen eines genomweiten genetischen Profils in Mikrovesikeln eines Subjekts, wobei dadurch die Diagnose, Überwachung und/oder Bewertung einer Erkrankung oder eines anderen medizinischen Zustands in einem Subjekt unterstützt wird, umfassend die Schritte:
(a) Isolieren einer Mikrovesikelfraktion aus einer biologischen Probe von dem Subjekt, wobei die biologische Probe von dem Subjekt eine Körperfluidprobe ist, wobei die Körperfluidprobe Serum oder Urin ist;
(b) Extrahieren von Nukleinsäuren aus der Mikrovesikelfraktion, wobei die extrahierten Nukleinsäuren mRNAs umfassen; und
(c) Nachweisen des Expressionsniveaus der mRNAs, wobei dadurch das genomweite genetische Profil in den Mikrovesikeln nachgewiesen wird;
(d) Vergleichen des in (c) nachgewiesenen genomweiten genetischen Profils mit einem genomweiten genetischen Profil, das von einem gesunden Individuum stammt, wobei dadurch die Diagnose, Überwachung und/oder Bewertung einer Erkrankung oder eines anderen medizinischen Zustands in einem Subjekt unterstützt wird.

2. Verfahren nach Anspruch 1, wobei Schritt (a) das Verarbeiten einer Mikrovesikelfraktion umfasst, um Proteine, Lipide, Überbleibsel von toten Zellen und andere Verunreinigungen auszuschließen.

3. Verfahren nach Anspruch 2, wobei das Verarbeiten der Mikrovesikelfraktion ein Filtern umfasst, bevorzugt wobei das Filtern das Filtern durch einen 0,8 µm oder 0,22 µm-Filter umfasst.

4. Verfahren nach einem der vorangehenden Ansprüche, wobei Schritt (a) ein Reinigen von Mikrovesikeln aus der Mikrovesikelfraktion unter Verwendung von Größenausschlusschromatographie, Dichtegradientenzentrifugation, Zentrifugation, differentieller Zentrifugation, Ultrazentrifugation, Nanomembranultrafiltration, Immunadsorptionsmitteleinfang, Affinitätsreinigung, mikrofluidischer Trennung, Anionaustauschchromatographie, Gelpermeationschromatographie oder eine beliebige Kombination davon umfasst.

5. Verfahren nach einem der vorhergehenden Ansprüche, wobei der Nachweisschritt c) mittels Microarray-Analyse, PCR, Hybridisierung mit Allel-spezifischen Sonden, enzymatischem Mutationsnachweis, Ligationskettenreaktion (ligation chain reaction - LCR), Oligonukleotidligationsassay (OLA), flusszytometrischer Heteroduplexanalyse, chemischer Spaltung von Fehlpaarungen, Massenspektrometrie, Nukleinsäuresequenzierung, Einzelstrangkonformationspolymorphismus (single strand conformation polymorphism - SSCP), denaturierende Gradientengelelektrophorese (DGGE), Temperaturgradientengelelektrophorese (TGGE), Restriktionsfragmentpolymorphismen, serieller Analyse der Genexpression (SAGE), Sequenzierung der nächsten Generation, massiver Parallelsequenzierung oder einer beliebigen Kombination davon durchgeführt wird.

6. Verfahren nach Anspruch 1, wobei die Erkrankung oder der andere medizinische Zustand Krebs ist.

7. Verfahren nach Anspruch 6, wobei der Krebs ein Melanom, ein Glioblastom oder Prostatakrebs ist.

8. Verfahren nach einem der vorhergehenden Ansprüche, wobei die Erkrankung oder der andere medizinische Zustand Glioblastom ist.

## Revendications

1. Procédé de détection d'un profil génétique à l'échelle du génome dans des microvésicules provenant d'un sujet, aidant ainsi au diagnostic, à la surveillance et/ou à l'évaluation d'une maladie ou d'un autre état de santé chez un sujet, comprenant les étapes suivantes :
(a) l'isolement d'une fraction de microvésicules d'un échantillon biologique du sujet, l'échantillon biologique du sujet étant un échantillon de fluide corporel, l'échantillon de fluide corporel étant du sérum ou de l'urine ;
(b) l'extraction des acides nucléiques de la fraction de microvésicules, les acides nucléiques extraits comprenant des ARNm ; et
(c) la détection du niveau d'expression des ARNm, détectant ainsi le profil génétique à l'échelle du génome dans les microvésicules ;
(d) la comparaison du profil génétique à l'échelle du génome détecté en (c) à un profil génétique à l'échelle du génome dérivé d'un individu sain, aidant ainsi au diagnostic, à la surveillance et/ou à l'évaluation d'une maladie ou d'un autre état de santé chez un sujet.

2. Procédé selon la revendication 1, dans lequel l'étape (a) comprend le traitement d'une fraction de microvésicules pour exclure les protéines, les lipides, les débris des cellules mortes et d'autres contaminants.

3. Procédé selon la revendication 2, dans lequel le traitement de la fraction de microvésicules comprend le filtrage, de préférence, le filtrage comprenant le filtrage à travers un filtre de 0,8 µm ou 0,22 µm.

4. Procédé selon l'une quelconque des revendications précédentes, dans lequel l'étape (a) comprend la purification de microvésicules à partir de la fraction de microvésicules à l'aide de la chromatographie d'exclusion de taille, la centrifugation en gradient de densité, la centrifugation, la centrifugation différentielle, l'ultracentrifugation, l'ultrafiltration de nanomembrane, la capture d'immunosorbant, la purification par affinité, la séparation microfluidique, la séparation anionique la chromatographie d'échange, la chromatographie par perméation de gel ou toute combinaison de celles-ci.

5. Procédé selon l'une quelconque des revendications précédentes, dans lequel l'étape de détection c) est effectuée par hybridation d'analyse de microréseau, PCR, avec des sondes spécifiques d'allèles, détection de mutation enzymatique, réaction de ligature en chaîne (LCR), dosage de ligature oligonucléotidique (OLA), analyse hétéroduplex cytométrique en flux, clivage chimique des mésappariements, spectrométrie de masse, séquençage des acides nucléiques, polymorphisme de conformation des simples brins (SSCP), électrophorèse sur gel en gradient dénaturant (DGGE), électrophorèse sur gel en gradient de température (TGGE), polymorphismes des fragments de restriction, analyse en série de l'expression des gènes (SAGE), séquençage de nouvelle génération, séquençage massivement parallèle ou toute combinaison de ceux-ci.

6. Procédé selon la revendication 1, dans lequel la maladie ou autre état de santé est le cancer.

7. Procédé selon la revendication 6, dans lequel le cancer est un mélanome, un glioblastome ou un cancer de la prostate.

8. Procédé selon l'une quelconque des revendications précédentes, dans lequel la maladie ou autre état de santé est le glioblastome.
